(19) Europäisches Patentamt
European Patent Office
Office européen des brevets

(11) EP 2 586 790 A2

(12) # EUROPEAN PATENT APPLICATION

(43) Date of publication:
**01.05.2013 Bulletin 2013/18**

(21) Application number: **12194903.6**

(22) Date of filing: **15.08.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **16.08.2006 US 838975 P**

(62) Document number(s) of the earlier application(s) in accordance with Art. 76 EPC:
**07825559.3 / 2 064 230**

(71) Applicant: **Novartis AG**
**4056 Basel (CH)**

(72) Inventors:
- **Hacker, Jörg**
  **97070 Würzburg (DE)**
- **Scorza, Francesco Berlanda**
  **53100 Siena (IT)**
- **Moriel, Danilo Gomes**
  **53100 Siena (IT)**
- **Pizza, Mariagrazia**
  **53100 Siena (IT)**
- **Serino, Laura**
  **53100 Siena (IT)**
- **Fontana, Maria Rita**
  **53100 Siena (IT)**

(74) Representative: **Marshall, Cameron John et al**
**Carpmaels & Ransford**
**One Southampton Row**
**London**
**WC1B 5HA (GB)**

(51) Int Cl.:
***C07K 14/245*** *(2006.01)* ***A61K 39/00*** *(2006.01)*

Remarks:
This application was filed on 29-11-2012 as a divisional application to the application mentioned under INID code 62.

(54) **Immunogens from uropathogenic Escherichia coli**

(57) Disclosed herein are various polypeptides that can be included in immunogenic compositions specific for pathogenic E. *coli* strains. The polypeptides have cellular locations which render them accessible to the immune system. The genes encoding the polypeptides were initially identified as being present in uropathogenic strain 536 but absent from non-pathogenic strains.

EP 2 586 790 A2

SEQUENCE LISTING

<110> Novartis AG

<120> Immunogens from Uropathogenic Escherichia Coli

<130> P060716EP

<150> US60/838975
<151> 2006-08-16

<150> PCT/IB2007/003306
<151> 2007-08-15

<150> EP07825559.3
<151> 2007-08-15

<160> 168

<170> SeqWin99, version 1.02

<210> 1
<211> 71
<212> PRT
<213> Escherichia coli

<400> 1
Met Thr Met Lys Leu Ile Lys Thr Leu Val Ala Val Ser Ala Leu Ser
1 5 10 15

Met Met Ser Phe Gly Val Phe Ala Gln Ser Val Ser Ala Thr Ala Ser
20 25 30

Thr Leu Asp Arg Ala Glu Ala Lys Ile Ala Ala Gln Ala Ala Glu Gln
35 40 45

Gly Ala Ser Tyr Lys Ile Thr Ser Ala Arg Val Glu Asn Arg Val Tyr
50 55 60

Met Thr Ala Glu Leu Leu Lys
65 70

<210> 2
<211> 274
<212> PRT
<213> Escherichia coli

<400> 2
Val Val Asn Gly His Thr Asp Val Ile Gly Ser Thr Ser Ile Arg His
1 5 10 15

Ile Leu Ala Val Arg Gln Ser Thr Leu Leu Gln Ile Asp Thr Leu Ile
20 25 30

Arg Gln Leu Ala Glu Ile Ser Ala Met Thr Val Ser Ile Gly Gly Lys
35 40 45

Thr Ala Leu Asp Trp Ala Met Lys Gln Asp Phe Arg Cys Gly Phe Trp
50 55 60

Leu Met Glu Lys Pro Glu Ile Ala Met Lys Ala Ile Thr Arg Asn Leu
65 70 75 80

Asp Arg Glu Leu Trp Arg Asp Leu Met Gln Arg Ser Gly Met Leu Ser
85 90 95

Leu Met Asp Ala Gln Ala Arg Glu Thr Trp Tyr Arg Ser Leu Glu Tyr
100 105 110

Asp Asn Val Pro Glu Ile Ser Glu Ala Asn Ile Leu Asn Thr Phe Lys
115 120 125

Gln Leu His Gln Asn Lys Asp Glu Val Phe Glu Arg Gly Val Ile Asn
130 135 140

Val Phe Arg Gly Leu Asn Trp Asn Tyr Lys Thr Asn Leu Pro Cys Lys
145 150 155 160

Phe Gly Ser Lys Ile Ile Val Asn Asn Leu Val Arg Trp Asp Arg Trp
165 170 175

Gly Phe His Ile Val Thr Gly Gln Gln Ala Asp Arg Val Ala Asp Leu
180 185 190

Glu Arg Met Leu His Leu Phe Ser Gly Trp Pro Ile Pro Asp Asn Arg
195 200 205

Glu Asn Ile Ile Ile Arg Leu Asp Asp His Ile Gln Ser Ala Gln Gly
210 215 220

Gln Glu Cys Tyr Glu Tyr Glu Asp Glu Met Phe Ser Ile Arg Tyr Phe
225 230 235 240

Lys Lys Gly Ser Ala His Ile Thr Phe Arg Lys Pro Glu Leu Ile Asp
245 250 255

Arg Leu Asn Asp Ile Ile Ala Lys Tyr Tyr Pro Glu Ile Ile Pro Tyr
260 265 270

Asn Ile

<210> 3
<211> 1024
<212> PRT
<213> Escherichia coli

<400> 3
Met Pro Thr Ile Thr Thr Ala Gln Ile Lys Ser Thr Leu Gln Ser Ala
1 5 10 15

Lys Gln Ser Ala Ala Asn Lys Leu His Ser Ala Gly Gln Ser Thr Lys
20 25 30

Asp Ala Leu Lys Lys Ala Ala Glu Gln Thr Arg Asn Ala Gly Asn Arg
35 40 45

Leu Ile Leu Leu Ile Pro Lys Asp Tyr Lys Gly Gln Gly Ser Ser Leu
50 55 60

Asn Asp Leu Val Arg Thr Ala Asp Glu Leu Gly Ile Glu Val Gln Tyr
65 70 75 80

Asp Glu Lys Asn Gly Thr Ala Ile Thr Lys Gln Val Phe Gly Thr Ala
85 90 95

Glu Lys Leu Ile Gly Leu Thr Glu Arg Gly Val Thr Ile Phe Ala Pro
100 105 110

Gln Leu Asp Lys Leu Leu Gln Lys Tyr Gln Lys Ala Gly Asn Lys Leu

115 120 125

Gly Gly Ser Ala Glu Asn Ile Gly Asp Asn Leu Gly Lys Ala Gly Ser
130 135 140

Val Leu Ser Thr Phe Gln Asn Phe Leu Gly Thr Ala Leu Ser Ser Met
145 150 155 160

Lys Ile Asp Glu Leu Ile Lys Lys Gln Lys Ser Gly Ser Asn Val Ser
165 170 175

Ser Ser Glu Leu Ala Lys Ala Ser Ile Glu Leu Ile Asn Gln Leu Val
180 185 190

Asp Thr Ala Ala Ser Ile Asn Asn Asn Val Asn Ser Phe Ser Gln Gln
195 200 205

Leu Asn Lys Leu Gly Ser Val Leu Ser Asn Thr Lys His Leu Asn Gly
210 215 220

Val Gly Asn Lys Leu Gln Asn Leu Pro Asn Leu Asp Asn Ile Gly Ala
225 230 235 240

Gly Leu Asp Thr Val Ser Gly Ile Leu Ser Val Ile Ser Ala Ser Phe
245 250 255

Ile Leu Ser Asn Ala Asp Ala Asp Thr Gly Thr Lys Ala Ala Ala Gly
260 265 270

Val Glu Leu Thr Thr Lys Val Leu Gly Asn Val Gly Lys Gly Ile Ser
275 280 285

Gln Tyr Ile Ile Ala Gln Arg Ala Ala Gln Gly Leu Ser Thr Ser Ala
290 295 300

Ala Ala Ala Gly Leu Ile Ala Ser Ala Val Thr Leu Ala Ile Ser Pro
305 310 315 320

Leu Ser Phe Leu Ser Ile Ala Asp Lys Phe Lys Arg Ala Asn Lys Ile
325 330 335

Glu Glu Tyr Ser Gln Arg Phe Lys Lys Leu Gly Tyr Asp Gly Asp Ser
340 345 350

Leu Leu Ala Ala Phe His Lys Glu Thr Gly Ala Ile Asp Ala Ser Leu
355 360 365

Thr Thr Ile Ser Thr Val Leu Ala Ser Val Ser Ser Gly Ile Ser Ala
370 375 380

Ala Ala Thr Thr Ser Leu Val Gly Ala Pro Val Ser Ala Leu Val Gly
385 390 395 400

Ala Val Thr Gly Ile Ile Ser Gly Ile Leu Glu Ala Ser Lys Gln Ala
405 410 415

Met Phe Glu His Val Ala Ser Lys Met Ala Asp Val Ile Ala Glu Trp
420 425 430

Glu Lys Lys His Gly Lys Asn Tyr Phe Glu Asn Gly Tyr Asp Ala Arg
435 440 445

His Ala Ala Phe Leu Glu Asp Asn Phe Lys Ile Leu Ser Gln Tyr Asn
450 455 460

```
Lys Glu Tyr Ser Val Glu Arg Ser Val Leu Ile Thr Gln Gln His Trp
465                 470                 475                 480

Asp Thr Leu Ile Gly Glu Leu Ala Gly Val Thr Arg Asn Gly Asp Lys
                485                 490                 495

Thr Leu Ser Gly Lys Ser Tyr Ile Asp Tyr Tyr Glu Glu Gly Lys Arg
            500                 505                 510

Leu Glu Lys Lys Pro Asp Glu Phe Gln Lys Gln Val Phe Asp Pro Leu
        515                 520                 525

Lys Gly Asn Ile Asp Leu Ser Asp Ser Lys Ser Ser Thr Leu Leu Lys
    530                 535                 540

Phe Val Thr Pro Leu Leu Thr Pro Gly Glu Glu Ile Arg Glu Arg Arg
545                 550                 555                 560

Gln Ser Gly Lys Tyr Glu Tyr Ile Thr Glu Leu Leu Val Lys Gly Val
                565                 570                 575

Asp Lys Trp Thr Val Lys Gly Val Gln Asp Lys Gly Ser Val Tyr Asp
            580                 585                 590

Tyr Ser Asn Leu Ile Gln His Ala Ser Val Gly Asn Asn Gln Tyr Arg
        595                 600                 605

Glu Ile Arg Ile Glu Ser His Leu Gly Asp Gly Asp Asp Lys Val Phe
    610                 615                 620

Leu Ala Ala Gly Ser Ala Asn Ile Tyr Ala Gly Lys Gly His Asp Val
625                 630                 635                 640

Val Tyr Tyr Asp Lys Thr Asp Thr Gly Tyr Leu Thr Ile Asp Gly Thr
                645                 650                 655

Lys Ala Thr Glu Ala Gly Asn Tyr Thr Val Thr Arg Val Leu Gly Gly
            660                 665                 670

Asp Val Lys Val Leu Gln Glu Val Val Lys Glu Gln Glu Val Ser Val
        675                 680                 685

Gly Lys Arg Thr Glu Lys Thr Gln Tyr Arg Ser Tyr Glu Phe Thr His
    690                 695                 700

Ile Asn Gly Thr Asp Leu Thr Glu Thr Asp Asn Leu Tyr Ser Val Glu
705                 710                 715                 720

Glu Leu Ile Gly Thr Asn Arg Ala Asp Lys Phe Phe Gly Ser Lys Phe
                725                 730                 735

Thr Asp Ile Phe His Gly Ala Asp Gly Asp Asp His Ile Glu Gly Asn
            740                 745                 750

Asp Gly Asn Asp Arg Leu Tyr Gly Asp Lys Gly Asn Asp Thr Leu Arg
        755                 760                 765

Gly Gly Asn Gly Asp Asp Gln Leu Tyr Gly Gly Asp Gly Asn Asp Lys
    770                 775                 780

Leu Thr Gly Gly Val Gly Asn Asn Tyr Leu Asn Gly Gly Asp Gly Asp
785                 790                 795                 800

Asp Glu Leu Gln Val Gln Gly Asn Ser Leu Ala Lys Asn Val Leu Ser
                805                 810                 815
```

Gly Gly Lys Gly Asn Asp Lys Leu Tyr Gly Ser Glu Gly Ala Asp Leu
820 825 830

Leu Asp Gly Gly Glu Gly Asn Asp Leu Leu Lys Gly Gly Tyr Gly Asn
835 840 845

Asp Ile Tyr Arg Tyr Leu Ser Gly Tyr Gly His His Ile Ile Asp Asp
850 855 860

Asp Gly Gly Lys Asp Asp Lys Leu Ser Leu Ala Asp Ile Asp Phe Arg
865 870 875 880

Asp Val Ala Phe Lys Arg Glu Gly Asn Asp Leu Ile Met Tyr Lys Ala
885 890 895

Glu Gly Asn Val Leu Ser Ile Gly His Lys Asn Gly Ile Thr Phe Arg
900 905 910

Asn Trp Phe Glu Lys Glu Ser Gly Asp Ile Ser Asn His Gln Ile Glu
915 920 925

Gln Ile Phe Asp Lys Asp Gly Arg Val Ile Thr Pro Asp Ser Leu Lys
930 935 940

Lys Ala Phe Glu Tyr Gln Gln Ser Asn Asn Gln Ala Asn Tyr Val Tyr
945 950 955 960

Gly Glu Tyr Ala Ser Thr Tyr Ala Asp Leu Asp Asn Leu Asn Pro Leu
965 970 975

Ile Asn Glu Ile Ser Lys Ile Ile Ser Ala Ala Gly Asn Phe Asp Val
980 985 990

Lys Glu Glu Arg Ser Ala Ala Ser Leu Leu Gln Leu Ser Gly Asn Ala
995 1000 1005

Ser Asp Phe Ser Tyr Gly Arg Asn Ser Ile Thr Leu Thr Ala Ser Ala
1010 1015 1020

<210> 4
<211> 409
<212> PRT
<213> Escherichia coli

<400> 4
Met Glu Leu Ile Met Pro Leu Ser Arg Arg Asn Phe Ile Gln Asn Ala
1 5 10 15

Val Leu Gly Ile Ser Ala Ala Gly Leu Ser Ala Ala Pro Ala Leu Ala
20 25 30

Lys Asn Ile Ser Ser Ser Thr Ala His Ile Ile Ser Lys Thr Ser Gly
35 40 45

His Ala Asp Thr Ser Thr Ser Lys Ser Leu His Ile Ile Ser Leu Asp
50 55 60

Arg Leu Glu Ala Ser Ala Lys Asp Val Met Thr Glu Ala Ala Tyr Ala
65 70 75 80

Tyr Ile Ala His Gly Ala Gly Asp Glu Trp Thr Tyr His Glu Asn Arg
85 90 95

Arg Ala Phe Ser Asp Tyr Pro Leu Leu Pro His Arg Leu Ser Gly Val
100 105 110

Ala Ala His Ser Ile Asp Ile Arg Thr Asp Leu Leu Gly His His Leu
115 120 125

Glu His Pro Leu Leu Ile Ala Pro Met Gly Ala His Met Phe Val His
130 135 140

Pro Glu Gly Glu Val Ile Ala Ala Ala Gly Ala Glu Lys Ala Gly Ala
145 150 155 160

Leu Tyr Glu Ser Ser Gly Ala Ser Asn Arg Ser Leu Glu Asp Ile Ala
165 170 175

Lys Ala Ser Lys Gly Pro Lys Trp Phe Gln Leu Tyr Phe Asn Ala Asp
180 185 190

Ala Gly Val Thr Arg Ser Leu Leu Glu Arg Ala Lys Ala Ala Gly Tyr
195 200 205

Ser Ala Ile Ile Ile Thr Ala Asp Ala Leu Gly Pro Gly Thr Ser Asp
210 215 220

Ala Phe Leu Ser Met Ser Ser Pro Phe Pro Ala Gly Ala Thr Phe Gly
225 230 235 240

Asn His Asp Pro Arg Tyr Gly Gly Lys Gly Asp Phe Phe Asn Gln Lys
245 250 255

Val Glu Leu Thr Pro Ala Asp Ile Glu Phe Val Lys Lys Ile Thr Gly
260 265 270

Leu Pro Val Ile Val Lys Gly Ile Leu Arg Gly Glu Asp Ala Val Val
275 280 285

Ala Ile Asp Ala Gly Ala Asp Ala Ile Gln Val Ser Asn His Gly Gly
290 295 300

Arg Gln Ile Asp Gly Val Pro Ser Ala Ile Ser Gln Leu Gln Glu Val
305 310 315 320

Ala Ala Arg Val Gly His Lys Val Pro Val Ile Phe Asp Ser Gly Ile
325 330 335

Arg Arg Gly Ile Asp Val Val Arg Ala Ile Ser Leu Gly Ala Thr Ala
340 345 350

Val Ala Val Gly Arg Pro Val Leu Tyr Gly Ile Ala Ala Gly Gly Val
355 360 365

Gly Gly Val Ala Ser Val Ile Glu His Leu Lys Thr Glu Leu Arg Thr
370 375 380

Ala Met Leu Leu Ser Gly Ala Arg Thr Leu Lys Asp Leu Ser Gln Gly
385 390 395 400

Phe Ile Arg Asn Lys Glu Thr Glu His
405

<210> 5
<211> 555
<212> PRT
<213> Escherichia coli

<400> 5

```
Met Ala Gln Val Ile Asn Thr Asn Ser Leu Ser Leu Ile Thr Gln Asn
1               5                   10                  15

Asn Ile Asn Lys Asn Gln Ser Ala Leu Ser Ser Ser Ile Glu Arg Leu
            20                  25                  30

Ser Ser Gly Leu Arg Ile Asn Ser Ala Lys Asp Asp Ala Ala Gly Gln
        35                  40                  45

Ala Ile Ala Asn Arg Phe Thr Ser Asn Ile Lys Gly Leu Thr Gln Ala
    50                  55                  60

Ala Arg Asn Ala Asn Asp Gly Ile Ser Val Ala Gln Thr Thr Glu Gly
65                  70                  75                  80

Ala Leu Ser Glu Ile Asn Asn Asn Leu Gln Arg Ile Arg Glu Leu Thr
                85                  90                  95

Val Gln Ala Ser Thr Gly Thr Asn Ser Asp Ser Asp Leu Asp Ser Ile
            100                 105                 110

Gln Asp Glu Ile Lys Ser Arg Leu Asp Glu Ile Asp Arg Val Ser Gly
        115                 120                 125

Gln Thr Gln Phe Asn Gly Val Asn Val Leu Ala Lys Asp Gly Ser Met
    130                 135                 140

Lys Ile Gln Val Gly Ala Asn Asp Gly Gln Thr Ile Thr Ile Asp Leu
145                 150                 155                 160

Lys Lys Ile Asp Ser Asp Thr Leu Gly Leu Ser Gly Phe Asn Val Asn
                165                 170                 175

Gly Lys Gly Ala Val Ala Asn Thr Ala Ala Thr Lys Asp Asp Leu Val
            180                 185                 190

Ala Ala Ser Val Ser Ala Ala Val Gly Asn Glu Tyr Thr Val Ser Ala
        195                 200                 205

Gly Leu Ser Lys Ser Thr Ala Ala Asp Val Ile Ala Ser Leu Thr Asp
    210                 215                 220

Gly Ala Thr Val Thr Ala Ala Gly Val Ser Asn Gly Phe Ala Ala Gly
225                 230                 235                 240

Ala Thr Gly Asn Ala Tyr Lys Phe Asn Gln Ala Asn Asn Thr Phe Thr
                245                 250                 255

Tyr Asn Thr Thr Ser Thr Ala Ala Glu Leu Gln Ser Tyr Leu Thr Pro
            260                 265                 270

Lys Ala Gly Asp Thr Ala Thr Phe Ser Val Glu Ile Gly Ser Thr Lys
        275                 280                 285

Gln Asp Val Val Leu Ala Ser Asp Gly Lys Ile Thr Ala Lys Asp Gly
    290                 295                 300

Ser Lys Leu Tyr Ile Asp Thr Thr Gly Asn Leu Thr Gln Asn Gly Gly
305                 310                 315                 320

Gly Thr Leu Glu Glu Ala Thr Leu Asn Gly Leu Ala Phe Asn His Ser
                325                 330                 335

Gly Pro Ala Ala Ala Val Gln Ser Thr Ile Thr Thr Ala Asp Gly Thr
```

340 345 350

Ser Ile Val Leu Ala Gly Ser Gly Asp Phe Gly Thr Thr Lys Thr Ala
355 360 365

Gly Ala Ile Asn Val Thr Gly Ala Val Ile Ser Ala Asp Ala Leu Leu
370 375 380

Ser Ala Ser Lys Ala Thr Gly Phe Thr Ser Gly Ala Tyr Thr Val Gly
385 390 395 400

Thr Asp Gly Val Val Lys Ser Gly Gly Asn Asp Val Tyr Asn Lys Ala
405 410 415

Asp Gly Thr Gly Leu Thr Thr Asp Asn Thr Thr Lys Tyr Tyr Leu Gln
420 425 430

Asp Asp Gly Ser Val Thr Asn Gly Ser Gly Lys Ala Val Tyr Val Asp
435 440 445

Ala Thr Gly Lys Leu Thr Thr Asp Ala Glu Thr Lys Ala Ala Thr Thr
450 455 460

Ala Asp Pro Leu Lys Ala Leu Asp Glu Ala Ile Ser Ser Ile Asp Lys
465 470 475 480

Phe Arg Ser Ser Leu Gly Ala Val Gln Asn Arg Leu Asp Ser Ala Val
485 490 495

Thr Asn Leu Asn Asn Thr Thr Thr Asn Leu Ser Glu Ala Gln Ser Arg
500 505 510

Ile Gln Asp Ala Asp Tyr Ala Thr Glu Val Ser Asn Met Ser Lys Ala
515 520 525

Gln Ile Ile Gln Gln Ala Gly Asn Ser Val Leu Ala Lys Ala Asn Gln
530 535 540

Val Pro Gln Gln Val Leu Ser Leu Leu Gln Gly
545 550 555

<210> 6
<211> 660
<212> PRT
<213> Escherichia coli

<400> 6
Met Ser Arg Pro Gln Phe Thr Ser Leu Arg Leu Ser Leu Leu Ala Leu
1 5 10 15

Ala Val Ser Ala Thr Leu Pro Thr Phe Ala Phe Ala Thr Glu Thr Met
20 25 30

Thr Val Thr Ala Thr Gly Asn Ala Arg Ser Ser Phe Glu Ala Pro Met
35 40 45

Met Val Ser Val Ile Asp Thr Ser Ala Pro Glu Asn Gln Thr Ala Thr
50 55 60

Ser Ala Thr Asp Leu Leu Arg Tyr Val Pro Gly Ile Thr Leu Asp Gly
65 70 75 80

Thr Gly Arg Thr Asn Gly Gln Asp Val Asn Met Arg Gly Tyr Asp His
85 90 95

```
Arg Gly Val Leu Val Leu Val Asp Gly Val Arg Gln Gly Thr Asp Thr
            100                 105                 110

Gly His Leu Asn Gly Thr Phe Leu Asp Pro Ala Leu Ile Lys Arg Val
        115                 120                 125

Glu Ile Val Arg Gly Pro Ser Ala Leu Leu Tyr Gly Ser Gly Ala Leu
    130                 135                 140

Gly Gly Val Ile Ser Tyr Asp Thr Val Asp Ala Lys Asp Leu Leu Gln
145                 150                 155                 160

Glu Gly Gln Ser Ser Gly Phe Arg Val Phe Gly Thr Gly Gly Thr Gly
                165                 170                 175

Asp His Ser Leu Gly Leu Gly Ala Ser Ala Phe Gly Arg Thr Glu Asn
            180                 185                 190

Leu Asp Gly Ile Val Ala Trp Ser Ser Arg Asp Arg Gly Asp Leu Arg
        195                 200                 205

Gln Ser Asn Gly Glu Thr Ala Pro Asn Asp Glu Ser Ile Asn Asn Met
    210                 215                 220

Leu Ala Lys Gly Thr Trp Gln Ile Asp Ser Ala Gln Ser Leu Ser Gly
225                 230                 235                 240

Leu Val Arg Tyr Tyr Asn Asn Asp Ala Arg Glu Pro Lys Asn Pro Gln
                245                 250                 255

Thr Val Glu Ala Ser Asp Ser Ser Asn Pro Met Val Asp Arg Ser Thr
            260                 265                 270

Ile Gln Arg Asp Ala Gln Leu Ser Tyr Lys Leu Ala Pro Gln Gly Asn
        275                 280                 285

Asp Trp Leu Asn Ala Asp Ala Lys Ile Tyr Trp Ser Glu Val Arg Ile
    290                 295                 300

Asn Ala Gln Asn Thr Gly Ser Ser Gly Glu Tyr Arg Glu Gln Ile Thr
305                 310                 315                 320

Lys Gly Ala Arg Leu Glu Asn Arg Ser Thr Leu Phe Ala Asp Ser Phe
                325                 330                 335

Ala Ser His Leu Leu Thr Tyr Gly Gly Glu Tyr Tyr Arg Gln Glu Gln
            340                 345                 350

His Pro Gly Gly Ala Thr Thr Gly Phe Pro Gln Ala Lys Ile Asp Phe
        355                 360                 365

Ser Ser Gly Trp Leu Gln Asp Glu Ile Thr Leu Arg Asp Leu Pro Ile
    370                 375                 380

Thr Leu Leu Gly Gly Thr Arg Tyr Asp Ser Tyr Arg Gly Ser Ser Asp
385                 390                 395                 400

Gly Tyr Lys Asp Val Asp Ala Asp Lys Trp Ser Ser Arg Ala Gly Met
                405                 410                 415

Thr Ile Asn Pro Thr Asn Trp Leu Met Leu Phe Gly Ser Tyr Ala Gln
            420                 425                 430

Ala Phe Arg Ala Pro Thr Met Gly Glu Met Tyr Asn Asp Ser Lys His
        435                 440                 445
```

Phe Ser Ile Gly Arg Phe Tyr Thr Asn Tyr Trp Val Pro Asn Pro Asn
    450                 455                 460

Leu Arg Pro Glu Thr Asn Glu Thr Gln Glu Tyr Gly Phe Gly Leu Arg
465                 470                 475                 480

Phe Asp Asp Leu Met Leu Ser Asn Asp Ala Leu Glu Phe Lys Ala Ser
                485                 490                 495

Tyr Phe Asp Thr Lys Ala Lys Asp Tyr Ile Ser Thr Thr Val Asp Phe
            500                 505                 510

Ala Ala Ala Thr Thr Met Ser Tyr Asn Val Pro Asn Ala Lys Ile Trp
        515                 520                 525

Gly Trp Asp Val Met Thr Lys Tyr Thr Thr Asp Leu Phe Ser Leu Asp
    530                 535                 540

Val Ala Tyr Asn Arg Thr Arg Gly Lys Asp Thr Asp Thr Gly Glu Tyr
545                 550                 555                 560

Ile Ser Ser Ile Asn Pro Asp Thr Val Thr Ser Thr Leu Asn Ile Pro
                565                 570                 575

Ile Ala His Ser Gly Phe Ser Val Gly Trp Val Gly Thr Phe Ala Asp
            580                 585                 590

Arg Ser Thr His Ile Ser Ser Ser Tyr Ser Lys Gln Pro Gly Tyr Gly
        595                 600                 605

Val Asn Asp Phe Tyr Val Ser Tyr Gln Gly Gln Gln Ala Leu Lys Gly
    610                 615                 620

Met Thr Thr Thr Leu Val Leu Gly Asn Ala Phe Asp Lys Glu Tyr Trp
625                 630                 635                 640

Ser Pro Gln Gly Ile Pro Gln Asp Gly Arg Asn Gly Lys Ile Phe Val
                645                 650                 655

Ser Tyr Gln Trp
            660

<210> 7
<211> 1024
<212> PRT
<213> Escherichia coli

<400> 7
Met Pro Thr Ile Thr Thr Ala Gln Ile Lys Ser Thr Leu Gln Ser Ala
1               5                   10                  15

Lys Gln Ser Ala Ala Asn Lys Leu His Ser Ala Gly Gln Ser Thr Lys
            20                  25                  30

Asp Ala Leu Lys Lys Ala Ala Glu Gln Thr Arg Asn Ala Gly Asn Arg
        35                  40                  45

Leu Ile Leu Leu Ile Pro Lys Asp Tyr Lys Gly Gln Gly Ser Ser Leu
    50                  55                  60

Asn Asp Leu Val Arg Thr Ala Asp Glu Leu Gly Ile Glu Val Gln Tyr
65                  70                  75                  80

Asp Glu Lys Asn Gly Thr Ala Ile Thr Lys Gln Val Phe Gly Thr Ala

85 90 95

Glu Lys Leu Ile Gly Leu Thr Glu Arg Gly Val Thr Ile Phe Ala Pro
100 105 110

Gln Leu Asp Lys Leu Leu Gln Lys Tyr Gln Lys Ala Gly Asn Lys Leu
115 120 125

Gly Gly Ser Ala Glu Asn Ile Gly Asp Asn Leu Gly Lys Ala Gly Ser
130 135 140

Val Leu Ser Thr Phe Gln Asn Phe Leu Gly Thr Ala Leu Ser Ser Met
145 150 155 160

Lys Ile Asp Glu Leu Ile Lys Lys Gln Lys Ser Gly Gly Asn Val Ser
165 170 175

Ser Ser Glu Leu Ala Lys Ala Ser Ile Glu Leu Ile Asn Gln Leu Val
180 185 190

Asp Thr Ala Ala Ser Leu Asn Asn Asn Val Asn Ser Phe Ser Gln Gln
195 200 205

Leu Asn Lys Leu Gly Ser Val Leu Ser Asn Thr Lys His Leu Asn Gly
210 215 220

Val Gly Asn Lys Leu Gln Asn Leu Pro Asn Leu Asp Asn Ile Gly Ala
225 230 235 240

Gly Leu Asp Thr Val Ser Gly Ile Leu Ser Ala Ile Ser Ala Ser Phe
245 250 255

Ile Leu Ser Asn Ala Asp Ala Asp Thr Gly Thr Lys Ala Ala Ala Gly
260 265 270

Val Glu Leu Thr Thr Lys Val Leu Gly Asn Val Gly Lys Gly Ile Ser
275 280 285

Gln Tyr Ile Ile Ala Gln Arg Ala Ala Gln Gly Leu Ser Thr Ser Ala
290 295 300

Ala Ala Ala Gly Leu Ile Ala Ser Val Val Thr Leu Ala Ile Ser Pro
305 310 315 320

Leu Ser Phe Leu Ser Ile Ala Asp Lys Phe Lys Arg Ala Asn Lys Ile
325 330 335

Glu Glu Tyr Ser Gln Arg Phe Lys Lys Leu Gly Tyr Asp Gly Asp Ser
340 345 350

Leu Leu Ala Ala Phe His Lys Glu Thr Gly Ala Ile Asp Ala Ser Leu
355 360 365

Thr Thr Ile Ser Thr Val Leu Ala Ser Val Ser Ser Gly Ile Ser Ala
370 375 380

Ala Ala Thr Thr Ser Leu Val Gly Ala Pro Val Ser Ala Leu Val Gly
385 390 395 400

Ala Val Thr Gly Ile Ile Ser Gly Ile Leu Glu Ala Ser Lys Gln Ala
405 410 415

Met Phe Glu His Val Ala Ser Lys Met Ala Asp Val Ile Ala Glu Trp
420 425 430

```
Glu Lys Lys His Gly Lys Asn Tyr Phe Glu Asn Gly Tyr Asp Ala Arg
        435                 440                 445

His Ala Ala Phe Leu Glu Asp Asn Phe Glu Ile Leu Ser Gln Tyr Asn
    450                 455                 460

Lys Glu Tyr Ser Val Glu Arg Ser Val Leu Ile Thr Gln Gln His Trp
465                 470                 475                 480

Asp Thr Leu Ile Gly Glu Leu Ala Gly Val Thr Arg Asn Gly Asp Lys
                485                 490                 495

Thr Leu Ser Gly Lys Ser Tyr Ile Asp Tyr Tyr Glu Glu Gly Lys Arg
            500                 505                 510

Leu Glu Lys Glu Pro Asp Glu Phe Gln Lys Gln Val Phe Asp Pro Leu
        515                 520                 525

Lys Gly Asn Ile Asp Leu Ser Val Ile Lys Ser Ser Thr Leu Leu Lys
    530                 535                 540

Phe Ile Thr Pro Leu Leu Thr Pro Gly Lys Glu Ile Arg Glu Arg Arg
545                 550                 555                 560

Gln Ser Gly Lys Tyr Glu Tyr Ile Thr Glu Leu Leu Val Lys Gly Val
                565                 570                 575

Asp Lys Trp Thr Val Lys Gly Val Gln Asp Lys Gly Ser Val Tyr Asp
            580                 585                 590

Tyr Ser Asn Leu Ile Gln His Ala Ser Val Gly Asn Asn Gln Tyr Arg
        595                 600                 605

Glu Ile Arg Ile Glu Ser His Leu Gly Asp Gly Asp Asp Lys Val Phe
    610                 615                 620

Leu Ser Ala Gly Ser Ala Asn Ile Tyr Ala Gly Lys Gly His Asp Val
625                 630                 635                 640

Val Tyr Tyr Asp Lys Thr Asp Thr Gly Tyr Leu Thr Ile Asp Gly Thr
                645                 650                 655

Lys Ala Thr Glu Ala Gly Asn Tyr Thr Val Thr Arg Val Leu Gly Gly
            660                 665                 670

Asp Val Lys Val Leu Gln Glu Val Val Lys Glu Gln Glu Val Ser Val
        675                 680                 685

Gly Lys Arg Thr Glu Lys Thr Gln Tyr Arg Ser Tyr Glu Phe Thr His
    690                 695                 700

Ile Asn Gly Thr Asp Leu Thr Glu Thr Asp Asn Leu Tyr Ser Val Glu
705                 710                 715                 720

Glu Leu Ile Gly Thr Asn Arg Ala Asp Lys Phe Phe Gly Ser Lys Phe
                725                 730                 735

Thr Asp Ile Phe His Gly Ala Asp Gly Asp Asp His Ile Glu Gly Asn
            740                 745                 750

Asp Gly Asn Asp Arg Leu Tyr Gly Asp Lys Gly Asn Asp Thr Leu Arg
        755                 760                 765

Gly Gly Asn Gly Asp Asp Gln Leu Tyr Gly Gly Asp Gly Asn Asp Lys
    770                 775                 780
```

Leu Thr Gly Gly Val Gly Asn Asn Tyr Leu Asn Gly Gly Asp Gly Asp
785 790 795 800

Asp Glu Leu Gln Val Gln Gly Asn Ser Leu Ala Lys Asn Val Leu Ser
805 810 815

Gly Gly Lys Gly Asn Asp Lys Leu Tyr Gly Ser Glu Gly Ala Asp Leu
820 825 830

Leu Asp Gly Gly Glu Gly Asn Asp Leu Leu Lys Gly Gly Tyr Gly Asn
835 840 845

Asp Ile Tyr Arg Tyr Leu Ser Gly Tyr Gly His His Ile Ile Asp Asp
850 855 860

Asp Gly Gly Lys Asp Asp Lys Leu Ser Leu Ala Asp Ile Asp Phe Arg
865 870 875 880

Asp Val Ala Phe Lys Arg Glu Gly Asn Asp Leu Ile Met Tyr Lys Ala
885 890 895

Glu Gly Asn Val Leu Ser Ile Gly His Lys Asn Gly Ile Thr Phe Arg
900 905 910

Asn Trp Phe Glu Lys Glu Ser Gly Asp Ile Ser Asn His Gln Ile Glu
915 920 925

Gln Ile Phe Asp Lys Asp Gly Arg Val Ile Thr Pro Asp Ser Leu Lys
930 935 940

Lys Ala Phe Glu Tyr Gln Gln Ser Asn Asn Gln Ala Asn Tyr Val Tyr
945 950 955 960

Gly Glu Tyr Ala Ser Thr Tyr Ala Asp Leu Asp Asn Leu Asn Pro Leu
965 970 975

Ile Asn Glu Ile Ser Lys Ile Ile Ser Ala Ala Gly Asn Phe Asp Val
980 985 990

Lys Glu Glu Arg Ser Ala Ala Ser Leu Leu Gln Leu Ser Gly Asn Ala
995 1000 1005

Ser Asp Phe Ser Tyr Gly Arg Asn Ser Ile Thr Leu Thr Ala Ser Ala
1010 1015 1020

<210> 8
<211> 948
<212> PRT
<213> Escherichia coli

<400> 8
Met Lys Arg His Leu Asn Thr Ser Tyr Arg Leu Val Trp Asn His Ile
1 5 10 15

Thr Gly Thr Leu Val Val Ala Ser Glu Leu Ala Arg Ser Arg Gly Lys
20 25 30

Arg Ala Gly Val Ala Val Ala Leu Ser Leu Ala Ala Val Thr Ser Val
35 40 45

Pro Ala Leu Ala Ala Asp Thr Val Val Gln Ala Gly Glu Thr Val Asn
50 55 60

```
Asp Gly Thr Leu Thr Asn His Asp Asn Gln Ile Val Leu Gly Thr Ala
65                  70                  75                  80

Asn Gly Met Thr Ile Ser Thr Gly Leu Glu Tyr Gly Pro Asp Asn Glu
                85                  90                  95

Ala Asn Thr Gly Gly Gln Trp Ile Gln Asn Gly Gly Ile Ala Asn Asn
            100                 105                 110

Thr Thr Val Thr Gly Gly Gly Leu Gln Arg Val Asn Ala Gly Gly Ser
        115                 120                 125

Val Ser Asp Thr Val Ile Ser Ala Gly Gly Gly Gln Ser Leu Gln Gly
    130                 135                 140

Gln Ala Val Asn Thr Thr Leu Asn Gly Gly Glu Gln Trp Val His Glu
145                 150                 155                 160

Gly Gly Ile Ala Thr Gly Thr Val Ile Asn Glu Lys Gly Trp Gln Ala
                165                 170                 175

Val Lys Ser Gly Ala Met Ala Thr Asp Thr Val Val Asn Thr Gly Ala
            180                 185                 190

Glu Gly Gly Pro Asp Ala Glu Asn Gly Asp Thr Gly Gln Phe Val Arg
        195                 200                 205

Gly Asn Ala Val Arg Thr Thr Ile Asn Glu Asn Gly Arg Gln Ile Val
    210                 215                 220

Ala Ala Glu Gly Thr Ala Asn Thr Thr Val Val Tyr Ala Gly Gly Asp
225                 230                 235                 240

Gln Thr Val His Gly Tyr Ala Leu Asp Thr Thr Leu Asn Gly Gly Asn
                245                 250                 255

Gln Tyr Val His Asn Gly Gly Thr Ala Ser Gly Thr Val Val Asn Ser
            260                 265                 270

Asp Gly Trp Gln Ile Val Lys Glu Gly Gly Leu Ala Asp Phe Thr Ile
        275                 280                 285

Val Asn Gln Lys Gly Lys Leu Gln Val Asn Ala Gly Gly Thr Ala Thr
    290                 295                 300

Asn Val Thr Leu Lys Gln Gly Gly Ala Leu Val Thr Ser Thr Ala Ala
305                 310                 315                 320

Thr Val Thr Gly Ser Asn Arg Leu Gly Asn Phe Thr Val Glu Asn Gly
                325                 330                 335

Asn Ala Asp Gly Val Val Leu Glu Ser Gly Gly Arg Leu Asp Val Leu
            340                 345                 350

Glu Gly His Ser Ala Trp Lys Thr Leu Val Asp Asp Gly Gly Thr Leu
        355                 360                 365

Ala Val Ser Ala Gly Gly Lys Ala Thr Asp Val Thr Met Thr Ser Gly
    370                 375                 380

Gly Ala Leu Ile Ala Asp Ser Gly Ala Thr Val Glu Gly Thr Asn Ala
385                 390                 395                 400

Ser Gly Lys Phe Ser Ile Asp Gly Ile Ser Gly Gln Ala Ser Gly Leu
                405                 410                 415
```

```
Leu Leu Glu Asn Gly Gly Ser Phe Thr Val Asn Ala Gly Gly Gln Ala
            420                 425                 430

Gly Asn Thr Thr Val Gly His Arg Gly Thr Leu Thr Leu Ala Ala Gly
        435                 440                 445

Gly Ser Leu Ser Gly Arg Thr Gln Leu Ser Lys Gly Ala Ser Met Val
    450                 455                 460

Leu Asn Gly Asp Val Val Ser Thr Gly Asp Ile Val Asn Ala Gly Glu
465                 470                 475                 480

Ile His Phe Asp Asn Gln Thr Thr Pro Asp Ala Ala Leu Ser Arg Ala
                485                 490                 495

Val Ala Lys Gly Asp Ser Pro Val Thr Phe His Lys Leu Thr Thr Ser
            500                 505                 510

Asn Leu Thr Gly Gln Gly Gly Thr Ile Asn Met Arg Val Arg Leu Asp
        515                 520                 525

Gly Ser Asn Thr Ser Asp Gln Leu Val Ile Asn Gly Gly Gln Ala Thr
    530                 535                 540

Gly Lys Thr Trp Leu Ala Phe Thr Asn Val Gly Asn Ser Asn Leu Gly
545                 550                 555                 560

Val Ala Thr Thr Gly Gln Gly Ile Arg Val Val Asp Ala Gln Asn Gly
                565                 570                 575

Ala Thr Thr Glu Glu Gly Ala Phe Ala Leu Ser Arg Pro Leu Gln Ala
            580                 585                 590

Gly Ala Phe Asn Tyr Thr Leu Asn Arg Asp Ser Asp Glu Asp Trp Tyr
        595                 600                 605

Leu Arg Ser Glu Asn Ala Tyr Arg Ala Glu Val Pro Leu Tyr Ala Ser
    610                 615                 620

Met Leu Thr Gln Ala Met Asp Tyr Asp Arg Ile Leu Ala Gly Ser Arg
625                 630                 635                 640

Ser His Gln Thr Gly Val Asn Gly Glu Asn Asn Ser Phe Arg Leu Ser
                645                 650                 655

Ile Gln Gly Gly His Leu Gly His Val Asn Asn Gly Gly Ile Ala Arg
            660                 665                 670

Gly Ala Thr Pro Glu Ser Ser Gly Ser Tyr Gly Leu Val Arg Leu Glu
        675                 680                 685

Gly Asp Leu Leu Arg Thr Glu Val Ala Gly Met Ser Leu Thr Thr Gly
    690                 695                 700

Val Tyr Gly Ala Ala Gly His Ser Ser Val Asp Val Lys Asp Asp Asp
705                 710                 715                 720

Gly Ser Arg Ala Gly Thr Val Arg Asp Asp Ala Gly Ser Leu Gly Gly
                725                 730                 735

Tyr Leu Asn Leu Val His Thr Ser Ser Gly Leu Trp Ala Asp Ile Val
            740                 745                 750

Ala Gln Gly Thr Arg His Ser Met Lys Ala Ser Ser Asp Asn Asn Asp
```

755 760 765

Phe Arg Ala Arg Gly Trp Gly Trp Leu Gly Ser Leu Glu Thr Gly Leu
770 775 780

Pro Phe Ser Ile Thr Asp Asn Leu Met Leu Glu Pro Gln Leu Gln Tyr
785 790 795 800

Thr Trp Gln Gly Leu Ser Leu Asp Asp Gly Gln Asp Asn Ala Gly Tyr
805 810 815

Val Lys Phe Gly His Gly Ser Ala Gln His Val Arg Ala Gly Phe Arg
820 825 830

Leu Gly Ser His Asn Asp Met Asn Phe Gly Lys Gly Thr Ser Ser Arg
835 840 845

Asp Thr Leu Arg Asp Ser Ala Lys His Ser Val Arg Glu Leu Pro Val
850 855 860

Asn Trp Trp Val Gln Pro Ser Val Ile Arg Thr Phe Ser Ser Arg Gly
865 870 875 880

Asp Met Ser Met Gly Thr Ala Ala Ala Gly Ser Asn Met Thr Phe Ser
885 890 895

Pro Ser Gln Asn Gly Thr Thr Leu Asp Leu Gln Ala Gly Leu Glu Ala
900 905 910

Arg Val Arg Glu Asn Ile Thr Leu Gly Val Gln Ala Gly Tyr Ala His
915 920 925

Ser Val Ser Gly Ser Ser Ala Glu Gly Tyr Asn Gly Gln Ala Thr Leu
930 935 940

Asn Val Thr Phe
945

<210> 9
<211> 725
<212> PRT
<213> Escherichia coli

<400> 9
Met Arg Ile Asn Lys Ile Leu Trp Ser Leu Thr Val Leu Leu Val Gly
1 5 10 15

Leu Asn Ser Gln Val Ser Val Ala Lys Tyr Ser Asp Asp Asp Asn Asp
20 25 30

Glu Thr Leu Val Val Glu Ala Thr Ala Glu Gln Val Leu Lys Gln Gln
35 40 45

Pro Gly Val Ser Val Ile Thr Ser Glu Asp Ile Lys Lys Thr Pro Pro
50 55 60

Val Asn Asp Leu Ser Asp Ile Ile Arg Lys Met Pro Gly Val Asn Leu
65 70 75 80

Thr Gly Asn Ser Ala Ser Gly Thr Arg Gly Asn Asn Arg Gln Ile Asp
85 90 95

Ile Arg Gly Met Gly Pro Glu Asn Thr Leu Ile Leu Ile Asp Gly Val
100 105 110

```
Pro Val Thr Ser Arg Asn Ser Val Arg Tyr Ser Trp Arg Gly Glu Arg
        115                 120                 125

Asp Thr Arg Gly Asp Thr Asn Trp Val Pro Pro Glu Gln Val Glu Arg
    130                 135                 140

Ile Glu Val Ile Arg Gly Pro Ala Ala Ala Arg Tyr Gly Ser Gly Ala
145                 150                 155                 160

Ala Gly Gly Val Val Asn Ile Ile Thr Lys Arg Pro Thr Asn Asp Trp
                165                 170                 175

His Gly Ser Leu Ser Leu Tyr Thr Asn Gln Pro Glu Ser Ser Glu Glu
            180                 185                 190

Gly Ala Thr Arg Arg Ala Asn Phe Ser Leu Ser Gly Pro Leu Ala Gly
        195                 200                 205

Asp Ala Leu Thr Thr Arg Leu Tyr Gly Asn Leu Asn Lys Thr Asp Ala
    210                 215                 220

Asp Ser Trp Asp Ile Asn Ser Pro Val Gly Thr Lys Asn Ala Ala Gly
225                 230                 235                 240

His Glu Gly Val Arg Asn Lys Asp Ile Asn Gly Val Ile Ser Trp Lys
                245                 250                 255

Leu Asn Pro Gln Gln Ile Leu Asp Phe Glu Ala Gly Tyr Ser Arg Gln
            260                 265                 270

Gly Asn Ile Tyr Ala Gly Asp Thr Gln Asn Ser Ser Ser Ser Ala Val
        275                 280                 285

Thr Glu Ser Leu Ala Lys Ser Gly Lys Glu Thr Asn Arg Leu Tyr Arg
    290                 295                 300

Gln Asn Tyr Gly Ile Thr His Asn Gly Ile Trp Asp Trp Gly Gln Ser
305                 310                 315                 320

Arg Phe Gly Ile Tyr Tyr Glu Lys Thr Asn Asn Thr Arg Met Asn Glu
                325                 330                 335

Gly Leu Ser Gly Gly Gly Glu Gly Arg Ile Leu Ala Gly Glu Lys Phe
            340                 345                 350

Thr Thr Asn Arg Leu Ser Ser Trp Arg Thr Ser Gly Glu Leu Asn Ile
        355                 360                 365

Pro Leu Asn Val Met Val Asp Gln Thr Leu Thr Val Gly Ala Glu Trp
    370                 375                 380

Asn Arg Asp Lys Leu Asp Asp Pro Ser Ser Thr Ser Leu Thr Val Asn
385                 390                 395                 400

Asp Arg Asp Ile Ser Gly Ile Ser Gly Ser Ala Ala Asp Arg Ser Ser
                405                 410                 415

Lys Asn His Ser Gln Ile Ser Ala Leu Tyr Ile Glu Asp Asn Ile Glu
            420                 425                 430

Pro Val Pro Gly Thr Asn Ile Ile Pro Gly Leu Arg Phe Asp Tyr Leu
        435                 440                 445

Ser Asp Ser Gly Gly Asn Phe Ser Pro Ser Leu Asn Leu Ser Gln Glu
    450                 455                 460
```

Leu Gly Asp Tyr Phe Lys Val Lys Ala Gly Val Ala Arg Thr Phe Lys
465 470 475 480

Ala Pro Asn Leu Tyr Gln Ser Ser Glu Gly Tyr Leu Leu Tyr Ser Lys
485 490 495

Gly Asn Gly Cys Pro Lys Asp Ile Thr Ser Gly Gly Cys Tyr Leu Ile
500 505 510

Gly Asn Lys Asp Leu Asp Pro Glu Ile Ser Val Asn Lys Glu Ile Gly
515 520 525

Leu Glu Phe Thr Trp Glu Asp Tyr His Ala Ser Val Thr Tyr Phe Arg
530 535 540

Asn Asp Tyr Gln Asn Lys Ile Val Ala Gly Asp Asn Val Ile Gly Gln
545 550 555 560

Thr Ala Ser Gly Ala Tyr Ile Leu Lys Trp Gln Asn Gly Gly Lys Ala
565 570 575

Leu Val Asp Gly Ile Glu Ala Ser Met Ser Phe Pro Leu Val Lys Asp
580 585 590

Arg Leu Asn Trp Asn Thr Asn Ala Thr Trp Met Ile Thr Ser Glu Gln
595 600 605

Lys Asp Thr Gly Asn Pro Leu Ser Val Ile Pro Lys Tyr Thr Ile Asn
610 615 620

Asn Ser Leu Asn Trp Thr Ile Thr Gln Ala Phe Ser Ala Asn Val Asn
625 630 635 640

Trp Thr Leu Tyr Gly Arg Gln Lys Pro Arg Thr His Ala Glu Thr Arg
645 650 655

Ser Glu Asp Thr Gly Gly Leu Ser Gly Lys Glu Leu Gly Ala Tyr Ser
660 665 670

Leu Val Gly Thr Asn Phe Asn Tyr Asp Ile Asn Lys Asn Leu Arg Leu
675 680 685

Asn Val Gly Val Ser Asn Ile Leu Asn Lys Gln Ile Phe Arg Ser Ser
690 695 700

Glu Gly Ala Asn Thr Tyr Asn Glu Pro Gly Arg Ala Tyr Tyr Ala Gly
705 710 715 720

Val Thr Ala Ser Phe
725

<210> 10
<211> 558
<212> PRT
<213> Escherichia coli

<400> 10
Met Lys Leu Phe Lys Ser Ile Leu Leu Ile Ala Ala Cys His Ala Ala
1 5 10 15

Gln Ala Ser Ala Ala Ile Asp Ile Asn Ala Asp Pro Asn Leu Thr Gly
20 25 30

Ala Ala Pro Leu Thr Gly Ile Leu Asn Gly Gln Gln Ser Asp Thr Gln

```
        35                  40                  45

Asn Met Ser Gly Phe Asp Asn Thr Pro Pro Pro Ala Pro Pro Val Val
    50                  55                  60

Met Ser Arg Met Phe Gly Ala Gln Leu Phe Asn Gly Thr Ser Ala Asp
65                  70                  75                  80

Ser Gly Ala Thr Val Gly Phe Asn Pro Asp Tyr Ile Leu Asn Pro Gly
                85                  90                  95

Asp Ser Ile Gln Val Arg Leu Trp Gly Ala Phe Thr Phe Asp Gly Ala
            100                 105                 110

Leu Gln Ile Asp Pro Lys Gly Asn Ile Phe Leu Pro Asn Val Gly Pro
        115                 120                 125

Val Lys Val Ala Gly Val Ser Asn Ser Gln Leu Asn Thr Leu Val Thr
    130                 135                 140

Ser Lys Val Lys Glu Val Tyr Gln Ser Asn Val Asn Val Tyr Ala Ser
145                 150                 155                 160

Leu Leu Gln Ala Gln Pro Val Lys Val Tyr Val Thr Gly Phe Val Arg
                165                 170                 175

Asn Pro Gly Leu Tyr Gly Gly Val Thr Ser Asp Ser Leu Leu Asn Tyr
            180                 185                 190

Leu Ile Lys Ala Gly Gly Val Asp Pro Glu Arg Gly Ser Tyr Val Asp
        195                 200                 205

Ile Val Val Lys Arg Gly Asn Arg Val Arg Ser Asn Val Asn Leu Tyr
    210                 215                 220

Asp Phe Leu Leu Asn Gly Lys Leu Gly Leu Ser Gln Phe Ala Asp Gly
225                 230                 235                 240

Asp Thr Ile Ile Val Gly Pro Arg Gln His Thr Phe Ser Val Gln Gly
                245                 250                 255

Asp Val Phe Asn Ser Tyr Asp Phe Glu Phe Arg Glu Ser Ser Ile Pro
            260                 265                 270

Val Thr Glu Ala Leu Ser Trp Ala Arg Pro Lys Pro Gly Ala Thr His
        275                 280                 285

Ile Thr Ile Met Arg Lys Gln Gly Leu Gln Lys Arg Ser Glu Tyr Tyr
    290                 295                 300

Pro Ile Ser Ser Ala Pro Gly Arg Met Leu Gln Asn Gly Asp Thr Leu
305                 310                 315                 320

Ile Val Ser Thr Asp Arg Tyr Ala Gly Thr Ile Gln Val Arg Val Glu
                325                 330                 335

Gly Ala His Ser Gly Glu His Ala Met Val Leu Pro Tyr Gly Ser Thr
            340                 345                 350

Met Arg Ala Val Leu Glu Lys Val Arg Pro Asn Ser Met Ser Gln Met
        355                 360                 365

Asn Ala Val Gln Leu Tyr Arg Pro Ser Val Ala Gln Arg Gln Lys Glu
    370                 375                 380
```

Met Leu Asn Leu Ser Leu Gln Lys Leu Glu Glu Ala Ser Leu Ser Ala
385 390 395 400

Gln Ser Ser Thr Lys Glu Glu Ala Ser Leu Arg Met Gln Glu Ala Gln
405 410 415

Leu Ile Ser Arg Phe Val Ala Lys Ala Arg Thr Val Val Pro Lys Gly
420 425 430

Glu Val Ile Leu Asn Glu Ser Asn Ile Asp Ser Val Leu Leu Glu Asp
435 440 445

Gly Asp Val Ile Asn Ile Pro Glu Lys Thr Ser Leu Val Met Val His
450 455 460

Gly Glu Val Leu Phe Pro Asn Ala Val Ser Trp Gln Lys Gly Met Thr
465 470 475 480

Thr Glu Asp Tyr Ile Glu Lys Cys Gly Gly Leu Thr Gln Lys Ser Gly
485 490 495

Asn Ala Arg Ile Ile Val Ile Arg Gln Asn Gly Ala Ala Val Asn Ala
500 505 510

Glu Asp Val Asp Ser Leu Lys Pro Gly Asp Glu Ile Met Val Leu Pro
515 520 525

Lys Tyr Glu Ser Lys Asn Ile Glu Val Thr Arg Gly Ile Ser Thr Ile
530 535 540

Leu Tyr Gln Leu Ala Val Gly Ala Lys Val Ile Leu Ser Leu
545 550 555

<210> 11
<211> 140
<212> PRT
<213> Escherichia coli

<400> 11
Met Ser Lys Tyr Ile Tyr Ile Leu Leu Ser Phe Leu Val Leu Phe Phe
1 5 10 15

Ile Phe Phe Tyr Ala Tyr Ile Ser Leu Met Ser Lys Glu His His Tyr
20 25 30

Thr Gln His Glu Leu Ser Pro Phe Phe Leu Tyr Thr Pro Glu Ser Leu
35 40 45

Arg Asn Leu Pro Asn Ile Ser Asn Val Ala Glu Tyr Ser Tyr Tyr Tyr
50 55 60

Asn Val Asp Asp Met Gln Thr Arg Val Ile Val Thr Trp Arg Asn Ile
65 70 75 80

Asp Asn Ile Phe Leu Gln Lys Ala Lys Leu Ile Asp Phe Leu Lys Arg
85 90 95

Met Gly Pro Ser Leu Gln Asn Asp Cys Ile Trp Phe Phe His Asp Lys
100 105 110

Ser Asp Tyr Ala Asn Asn Phe Gln Arg Tyr Cys Ile Ile Glu His Arg
115 120 125

Asp Ser Leu Gln Val Glu Tyr Phe Glu Thr Ile Glu
130 135 140

<210> 12
<211> 188
<212> PRT
<213> Escherichia coli

<400> 12
Met Ile Lys Ile Thr Pro His Lys Ile Thr Ile Leu Met Gly Leu Leu
1 5 10 15

Leu Ser Pro Ser Val Phe Ala Thr Asp Val Asn Val Asp Phe Thr Ala
20 25 30

Thr Val Lys Ala Thr Thr Cys Asn Ile Thr Leu Thr Gly Thr Asn Val
35 40 45

Thr Asp Asn Gly Asn Asp Lys Tyr Thr Leu Val Ile Pro Ser Met Gly
50 55 60

Met Asp Lys Ile Ala Asn Lys Thr Ala Gln Ser Glu Ala Asn Phe Lys
65 70 75 80

Leu Val Ala Asn Gly Cys Ser Ser Gly Ile Ser Trp Ile Asp Thr Thr
85 90 95

Leu Thr Gly Asn Gln Ser Gly Ser Ser Pro Ala Leu Ile Ile Pro Leu
100 105 110

Ala Ser Asp Thr Thr Ser Thr Thr Ser Tyr Ile Gly Met Gly Phe Lys
115 120 125

Arg Lys Ala Thr Ser Gly Asp Thr Phe Leu Lys Pro Asn Ser Ala Glu
130 135 140

Tyr Ile Arg Trp Ser Ala Ser Glu Ile Ser Thr Asp Gly Leu Glu Met
145 150 155 160

Thr Val Ala Leu Arg Glu Thr Ser Val Gly Lys Gly Val Pro Gly Lys
165 170 175

Phe Arg Ala Leu Ala Thr Phe Asn Phe Ser Tyr Gln
180 185

<210> 13
<211> 149
<212> PRT
<213> Escherichia coli

<400> 13
Val Asp His Trp Pro Asn Leu Leu Ala Phe His Phe Thr Leu Tyr Ser
1 5 10 15

Ala Glu Gly Asn Ile Asn Gly Gln Gln Ile His Ala Phe Cys Thr Ala
20 25 30

Phe Tyr Arg Gln Val Gln Glu His Ile Thr Glu Arg Asn His Thr Ala
35 40 45

Ser Pro Ala Pro Pro Val Val Leu Arg Trp Leu Arg Glu Gln His Gly
50 55 60

Gly Ala Thr Ile Arg Cys Leu Leu Leu Leu Ser Gln Ala Ser Ile Cys
65 70 75 80

His Leu Arg Val Ser Ala Thr Val Asp Glu Glu Cys Ser Gln Val Val

85 90 95

Asp Leu Leu Gln Gln Ala Trp Arg Gly Ile Asn Ala Gly Gly Gln Cys
100 105 110

Arg Val Glu Arg Cys Phe Arg Val Thr Trp Pro Asp Thr Ser Glu Gln
115 120 125

Tyr Val Ala Leu Lys Thr Ala Val Gln Ser Leu Ile Pro Leu Val Ile
130 135 140

Ala Thr Ile Ile Arg
145

<210> 14
<211> 164
<212> PRT
<213> Escherichia coli

<400> 14
Val Thr Leu Asn Thr Ser Gln Val Ser Tyr Tyr Ile Thr Gln Arg Lys
1 5 10 15

Lys Gly Ile Thr Gln His Ile Ser Ala Met Lys Ala Gly Ile Ser Val
20 25 30

Arg Ser Gly Arg Arg Ile Glu Lys Gly Gln Arg Ala Lys Asn Ser Val
35 40 45

Arg His Trp Ser Thr Arg Lys Asp Pro Leu Glu Ala Val Trp Asp Ser
50 55 60

Met Leu Val Pro Leu Leu Lys Glu Arg Pro Val Leu Thr Pro Thr Thr
65 70 75 80

Leu Leu Glu Met Leu Gln Asp Lys Tyr Pro Gly Gln Tyr Pro Asn Ser
85 90 95

Phe Arg Arg Thr Met Gln Arg Arg Gly Cys Glu Trp Lys Leu Gln Ser
100 105 110

Gly Ala Glu Gln Glu Val Met Phe Arg Gln Trp His Gln Pro Gly Leu
115 120 125

Arg Gly Leu Leu Asp Phe Thr Lys Leu Lys Gly Val Val Val Thr Ile
130 135 140

Ala Gly Lys Leu Leu Val His Met Leu Tyr Asn Phe Arg Leu Glu Trp
145 150 155 160

Ser His Trp Ser

<210> 15
<211> 840
<212> PRT
<213> Escherichia coli

<400> 15
Val Met Arg Val Met Lys Asp Arg Ile Pro Phe Ala Val Asn Asn Ile
1 5 10 15

Thr Cys Val Ile Leu Leu Ser Leu Phe Cys Asn Ala Ala Ser Ala Val
20 25 30

```
Glu Phe Asn Thr Asp Val Leu Asp Ala Ala Asp Lys Lys Asn Ile Asp
        35                  40                  45

Phe Thr Arg Phe Ser Glu Ala Gly Tyr Val Leu Pro Gly Gln Tyr Leu
    50                  55                  60

Leu Asp Val Ile Val Asn Gly Gln Ser Ile Ser Pro Ala Ser Leu Gln
65                  70                  75                  80

Ile Ser Phe Val Glu Pro Gln Ser Ser Gly Asp Lys Ala Glu Lys Lys
                85                  90                  95

Leu Pro Gln Ala Cys Leu Thr Ser Asp Met Val Arg Leu Met Gly Leu
            100                 105                 110

Thr Ala Glu Ser Leu Asp Lys Val Val Tyr Trp His Asp Gly Gln Cys
        115                 120                 125

Ala Asp Phe His Gly Leu Pro Gly Val Asp Ile Arg Pro Asp Thr Gly
    130                 135                 140

Ala Gly Val Leu Arg Ile Asn Met Pro Gln Ala Trp Leu Glu Tyr Ser
145                 150                 155                 160

Asp Ala Thr Trp Leu Pro Pro Ser Arg Trp Asp Asp Gly Ile Pro Gly
                165                 170                 175

Leu Met Leu Asp Tyr Asn Leu Asn Gly Thr Val Ser Arg Asn Tyr Gln
            180                 185                 190

Gly Gly Asp Ser His Gln Phe Ser Tyr Asn Gly Thr Val Gly Gly Asn
        195                 200                 205

Leu Gly Pro Trp Arg Leu Arg Ala Asp Tyr Gln Gly Ser Gln Glu Gln
    210                 215                 220

Ser Arg Tyr Asn Gly Glu Lys Thr Thr Asn Arg Asn Phe Thr Trp Ser
225                 230                 235                 240

Arg Phe Tyr Leu Phe Arg Ala Ile Pro Arg Trp Arg Ala Asn Leu Thr
                245                 250                 255

Leu Gly Glu Asn Asn Ile Asn Ser Asp Ile Phe Arg Ser Trp Ser Tyr
            260                 265                 270

Thr Gly Ala Ser Leu Glu Ser Asp Asp Arg Met Leu Pro Pro Arg Leu
        275                 280                 285

Arg Gly Tyr Ala Pro Gln Ile Thr Gly Ile Ala Glu Thr Asn Ala Arg
    290                 295                 300

Val Val Val Ser Gln Gln Gly Arg Val Leu Tyr Asp Ser Met Val Pro
305                 310                 315                 320

Ala Gly Pro Phe Ser Ile Gln Asp Leu Asp Ser Ser Val Arg Gly Arg
                325                 330                 335

Leu Asp Val Glu Val Ile Glu Gln Asn Gly Arg Lys Lys Thr Phe Gln
            340                 345                 350

Val Asp Thr Ala Ser Val Pro Tyr Leu Thr Arg Pro Gly Gln Val Arg
        355                 360                 365

Tyr Lys Leu Val Ser Gly Arg Ser Arg Gly Tyr Gly His Glu Thr Glu
    370                 375                 380
```

```
Gly Pro Val Phe Ala Thr Gly Glu Ala Ser Trp Gly Leu Ser Asn Gln
385                 390                 395                 400

Trp Ser Leu Tyr Gly Gly Ala Val Leu Ala Gly Asp Tyr Asn Ala Leu
                405                 410                 415

Ala Ala Gly Ala Gly Trp Asp Leu Gly Val Pro Gly Thr Leu Ser Ala
            420                 425                 430

Asp Ile Thr Gln Ser Val Ala Arg Ile Glu Gly Glu Arg Thr Phe Gln
        435                 440                 445

Gly Lys Ser Trp Arg Leu Ser Tyr Ser Lys Arg Phe Asp Asn Ala Asp
    450                 455                 460

Ala Asp Ile Thr Phe Ala Gly Tyr Arg Phe Ser Glu Arg Asn Tyr Met
465                 470                 475                 480

Thr Met Glu Gln Tyr Leu Asn Ala Arg Tyr Arg Asn Asp Tyr Ser Ser
                485                 490                 495

Arg Glu Lys Glu Met Tyr Thr Val Thr Leu Asn Lys Asn Val Ala Asp
            500                 505                 510

Trp Asn Thr Ser Phe Asn Leu Gln Tyr Ser Arg Gln Thr Tyr Trp Asp
        515                 520                 525

Ile Arg Lys Thr Asp Tyr Tyr Thr Val Ser Val Asn Arg Tyr Phe Asn
    530                 535                 540

Val Phe Gly Leu Gln Gly Val Ala Val Gly Leu Ser Ala Ser Arg Ser
545                 550                 555                 560

Lys Tyr Leu Gly Arg Asp Asn Asp Ser Ala Tyr Leu Arg Ile Ser Val
                565                 570                 575

Pro Leu Gly Thr Gly Thr Ala Ser Tyr Ser Gly Ser Met Ser Asn Asp
            580                 585                 590

Arg Tyr Val Asn Met Ala Gly Tyr Thr Asp Thr Phe Asn Asp Gly Leu
        595                 600                 605

Asp Ser Tyr Ser Leu Asn Ala Gly Leu Asn Ser Gly Gly Gly Leu Thr
    610                 615                 620

Ser Gln Arg Gln Ile Asn Ala Tyr Tyr Ser His Arg Ser Pro Leu Ala
625                 630                 635                 640

Asn Leu Ser Ala Asn Ile Ala Ser Leu Gln Lys Gly Tyr Thr Ser Phe
                645                 650                 655

Gly Val Ser Ala Ser Gly Gly Ala Thr Ile Thr Gly Lys Gly Ala Ala
            660                 665                 670

Leu His Ala Gly Gly Met Ser Gly Gly Thr Arg Leu Leu Val Asp Thr
        675                 680                 685

Asp Gly Val Gly Gly Val Pro Val Asp Gly Gly Gln Val Val Thr Asn
    690                 695                 700

Arg Trp Gly Thr Gly Val Val Thr Asp Ile Ser Ser Tyr Tyr Arg Asn
705                 710                 715                 720

Thr Thr Ser Val Asp Leu Lys Arg Leu Pro Asp Asp Val Glu Ala Thr
```

725 730 735

Arg Ser Val Val Glu Ser Ala Leu Thr Glu Gly Ala Ile Gly Tyr Arg
740 745 750

Lys Phe Ser Val Leu Lys Gly Lys Arg Leu Phe Ala Ile Leu Arg Leu
755 760 765

Ala Asp Gly Ser Gln Pro Pro Phe Gly Ala Ser Val Thr Ser Glu Lys
770 775 780

Gly Arg Glu Leu Gly Met Val Ala Asp Glu Gly Leu Ala Trp Leu Ser
785 790 795 800

Gly Val Thr Pro Gly Glu Thr Leu Ser Val Asn Trp Asp Gly Lys Ile
805 810 815

Gln Cys Gln Val Asn Val Pro Glu Thr Ala Ile Ser Asp Gln Gln Leu
820 825 830

Leu Leu Pro Cys Thr Pro Gln Lys
835 840

<210> 16
<211> 239
<212> PRT
<213> Escherichia coli

<400> 16
Met Asn Asn Val Lys Lys Ile Leu Leu Met Glu Asp Asp Tyr Asp Ile
1 5 10 15

Ala Ala Leu Leu Arg Leu Asn Leu Gln Asp Glu Gly Tyr Gln Ile Val
20 25 30

His Glu Ala Asp Gly Ala Arg Ala Arg Leu Leu Leu Asp Lys Gln Thr
35 40 45

Trp Asp Ala Val Ile Leu Asp Leu Met Leu Pro Asn Val Asn Gly Leu
50 55 60

Glu Ile Cys Arg Tyr Ile Arg Gln Met Thr Ser Tyr Leu Pro Val Ile
65 70 75 80

Ile Ile Ser Ala Arg Thr Ser Glu Thr His Arg Val Leu Gly Leu Glu
85 90 95

Met Gly Ala Asp Asp Tyr Leu Pro Lys Pro Phe Ser Ile Pro Glu Leu
100 105 110

Ile Ala Arg Ile Lys Ala Leu Phe Arg Arg Gln Glu Ala Met Gly Gln
115 120 125

Asn Ile Leu Leu Ala Ser Gly Leu Ile Cys Cys His Gly Leu Cys Ile
130 135 140

Asn Pro Phe Ser Arg Glu Val His Leu His Asn Lys Gln Val Asp Leu
145 150 155 160

Thr Pro Arg Glu Phe Asp Leu Leu Leu Trp Phe Ala Arg His Pro Gly
165 170 175

Glu Val Phe Ser Arg Leu Ser Leu Leu Asp Asn Val Trp Gly Tyr Gln
180 185 190

```
His Glu Gly Tyr Glu His Thr Val Asn Thr His Ile Asn Arg Leu Arg
        195                 200                 205

Ala Lys Ile Glu Gln Asp Ala Ala Glu Pro Lys Met Ile Gln Thr Val
    210                 215                 220

Trp Gly Lys Gly Tyr Arg Phe Ser Val Asp Asn Ala Gly Met Arg
225                 230                 235

<210>   17
<211>   122
<212>   PRT
<213>   Escherichia coli

<400>   17
Met Asn Tyr Ala Leu Ile Ile Ala Ile Ile Ile Ser Phe Pro Cys Ala
1               5                   10                  15

Leu Leu Ser Val Phe Leu Val Leu Lys Gly Trp Ala Leu Ile Gly Asp
            20                  25                  30

Ala Met Asn His Ala Met Phe Pro Gly Ile Val Leu Ala Trp Ile Ile
        35                  40                  45

Gly Met Pro Leu Gly Ile Gly Ala Phe Ile Val Gly Leu Ser Cys Ala
    50                  55                  60

Ile Ala Thr Gly Tyr Leu Lys Asp Asn Ser Ile Ile Lys Lys Asp Thr
65                  70                  75                  80

Ile Ile Gly Ile Val Phe Ser Gly Ile Phe Val Leu Thr Ile Asn Arg
                85                  90                  95

His Lys Ser Ala Asp Tyr Arg Tyr His Tyr Tyr Ile Thr Val Tyr Ser
            100                 105                 110

Ala Trp Tyr Arg Leu Pro Phe Leu Pro His
        115                 120

<210>   18
<211>   283
<212>   PRT
<213>   Escherichia coli

<400>   18
Met Asn Cys Ser Leu Thr Leu Ser Gln Arg Leu Ser Leu Val Phe Thr
1               5                   10                  15

Val Val Leu Leu Phe Cys Ala Ala Val Thr Cys Gly Val His Ile Tyr
            20                  25                  30

Ser Ser Asn Leu Tyr Gly Asn Ala Met Val Gln Arg Leu Ser Ala Gly
        35                  40                  45

Leu Ala Gln Gln Ile Val Ile Thr Glu Pro Leu Leu Asp Asn Arg Gly
    50                  55                  60

Gln Val Asn His Arg Thr Leu Lys Gly Leu Phe Glu Arg Leu Met Thr
65                  70                  75                  80

Leu Asn Pro Ser Val Glu Leu Tyr Ile Val Ser Pro Glu Gly Arg Leu
                85                  90                  95

Leu Val Glu Ala Ala Pro Pro Gly His Ile Lys Arg Arg Tyr Ile Asn
            100                 105                 110
```

Ile Ala Pro Leu Lys Lys Phe Leu Ser Gly Ala Val Trp Pro Val Tyr
115 120 125

Gly Asp Asp Pro Arg Ser Val Asn Lys Lys Lys Val Phe Ser Thr Ala
130 135 140

Pro Leu Tyr Leu Arg Asp Asp Leu Lys Gly Tyr Leu Tyr Ile Ile Leu
145 150 155 160

Gln Gly Glu Glu Leu Asn Ala Leu Thr Asp Ala Ala Trp Thr Lys Ala
165 170 175

Leu Trp Asn Ala Leu Tyr Trp Ser Leu Phe Leu Val Val Ile Cys Gly
180 185 190

Leu Leu Ser Gly Met Leu Val Trp Tyr Trp Val Thr Arg Pro Ile Gln
195 200 205

Gln Leu Thr Glu Asn Val Ser Gly Ile Glu Gln Asp Ser Ile Ser Ala
210 215 220

Ile Lys Gln Leu Ala Ile Gln Arg Pro Ala Thr Pro Leu Ala Thr Arg
225 230 235 240

Ser Arg Tyr Tyr Thr Met Pro Ser Leu Asn Trp Pro Val Lys Tyr Pro
245 250 255

Val Ser Gly Ile Asn Phe Gln Lys Val Ile Asn Ser Ala Val Asn Leu
260 265 270

Leu Pro Ile Ser Pro Met Ile Tyr Gly Arg His
275 280

<210> 19
<211> 1042
<212> PRT
<213> Escherichia coli

<400> 19
Met Leu Met Lys Arg His Leu Asn Thr Cys Tyr Arg Leu Val Trp Asn
1 5 10 15

His Ile Thr Gly Ala Phe Val Val Ala Ser Glu Leu Ala Arg Ala Arg
20 25 30

Gly Lys Arg Gly Gly Val Ala Val Ala Leu Ser Leu Ala Ala Val Thr
35 40 45

Ser Leu Pro Val Leu Ala Ala Asp Ile Val Val His Pro Gly Glu Thr
50 55 60

Val Asn Gly Gly Thr Leu Val Asn His Asp Asn Gln Phe Val Ser Gly
65 70 75 80

Thr Ala Asp Gly Val Thr Val Ser Thr Gly Leu Glu Leu Gly Pro Asp
85 90 95

Ser Asp Glu Asn Thr Gly Gly Gln Trp Ile Lys Ala Gly Gly Thr Gly
100 105 110

Arg Asn Thr Thr Val Thr Ala Asn Gly Arg Gln Ile Val Gln Ala Gly
115 120 125

Gly Thr Ala Ser Asp Thr Val Ile Arg Asp Gly Gly Gly Gln Ser Leu

130 135 140

Asn Gly Leu Ala Val Asn Thr Thr Leu Asp Asn Arg Gly Glu Gln Trp
145 150 155 160

Val His Gly Gly Gly Lys Ala Ala Gly Thr Ile Ile Asn Gln Asp Gly
165 170 175

Tyr Gln Thr Ile Lys His Gly Gly Leu Ala Thr Gly Thr Ile Val Asn
180 185 190

Thr Gly Ala Glu Gly Gly Pro Glu Ser Glu Asn Val Ser Thr Gly Gln
195 200 205

Met Val Gly Gly Thr Ala Glu Ser Thr Thr Ile Asn Asn Asn Gly Arg
210 215 220

Gln Val Ile Trp Ser Ser Gly Val Ser Arg Asp Thr Leu Ile Tyr Thr
225 230 235 240

Gly Gly Asp Gln Thr Val His Gly Glu Ala His Asn Thr Arg Leu Glu
245 250 255

Gly Gly Asn Gln Tyr Val His Lys Tyr Gly Leu Ala Leu Asn Thr Val
260 265 270

Ile Asn Glu Gly Gly Trp Gln Val Val Lys Ala Gly Gly Thr Ala Gly
275 280 285

Asn Thr Thr Ile Asn Gln Asn Gly Glu Leu Lys Val His Ala Gly Gly
290 295 300

Glu Ala Ser Asp Val Thr Gln Asn Thr Gly Gly Ala Leu Val Thr Ser
305 310 315 320

Thr Ala Ala Thr Val Thr Gly Thr Asn Arg Leu Gly Ala Phe Ser Val
325 330 335

Val Glu Gly Lys Ala Asp Asn Val Val Leu Glu Asn Gly Gly Arg Leu
340 345 350

Asp Val Leu Ser Gly His Thr Ala Thr Asn Thr Arg Val Asp Asp Gly
355 360 365

Gly Thr Leu Asp Val Arg Asn Gly Gly Thr Ala Thr Thr Val Ser Met
370 375 380

Gly Asn Gly Gly Val Leu Leu Ala Asp Ser Gly Ala Ala Val Ser Gly
385 390 395 400

Thr Arg Ser Asp Gly Thr Ala Phe Arg Ile Gly Gly Gly Gln Ala Asp
405 410 415

Ala Leu Met Leu Glu Lys Gly Ser Ser Phe Thr Leu Asn Ala Gly Asp
420 425 430

Thr Ala Thr Asp Thr Thr Val Asn Gly Gly Leu Phe Thr Ala Arg Gly
435 440 445

Gly Ser Leu Ala Gly Thr Thr Thr Leu Asn Asn Gly Ala Ile Leu Thr
450 455 460

Leu Ser Gly Lys Thr Val Asn Asn Asp Thr Leu Thr Ile Arg Glu Gly
465 470 475 480

```
Asp Ala Leu Leu Gln Gly Gly Ser Leu Thr Gly Asn Gly Ser Val Glu
                485                 490                 495

Lys Ser Gly Ser Gly Thr Leu Thr Val Ser Asn Thr Thr Leu Thr Gln
            500                 505                 510

Lys Ala Val Asn Leu Asn Glu Gly Thr Leu Thr Leu Asn Asp Ser Thr
        515                 520                 525

Val Thr Thr Asp Val Ile Ala Gln Arg Gly Thr Ala Leu Lys Leu Thr
    530                 535                 540

Gly Ser Thr Val Leu Asn Gly Ala Ile Asp Pro Thr Asn Val Thr Leu
545                 550                 555                 560

Ala Ser Gly Ala Thr Trp Asn Ile Pro Asp Asn Ala Thr Val Gln Ser
                565                 570                 575

Val Val Asp Asp Leu Ser His Ala Gly Gln Ile His Phe Thr Ser Thr
            580                 585                 590

Arg Thr Gly Lys Phe Val Pro Ala Thr Leu Lys Val Lys Asn Leu Asn
        595                 600                 605

Gly Gln Asn Gly Thr Ile Ser Leu Arg Val Arg Pro Asp Met Ala Gln
    610                 615                 620

Asn Asn Ala Asp Arg Leu Val Ile Asp Gly Gly Arg Ala Thr Gly Lys
625                 630                 635                 640

Thr Ile Leu Asn Leu Val Asn Ala Gly Asn Ser Ala Ser Gly Leu Ala
                645                 650                 655

Thr Ser Gly Lys Gly Ile Gln Val Val Glu Ala Ile Asn Gly Ala Thr
            660                 665                 670

Thr Glu Glu Gly Ala Phe Val Gln Gly Asn Arg Leu Gln Ala Gly Ala
        675                 680                 685

Phe Asn Tyr Ser Leu Asn Arg Asp Ser Asp Glu Ser Trp Tyr Leu Arg
    690                 695                 700

Ser Glu Asn Ala Tyr Arg Ala Glu Val Pro Leu Tyr Ala Ser Met Leu
705                 710                 715                 720

Thr Gln Ala Met Asp Tyr Asp Arg Ile Leu Ala Gly Ser Arg Ser His
                725                 730                 735

Gln Thr Gly Val Asn Val Lys Asn Asn Ser Val Arg Leu Ser Ile Gln
            740                 745                 750

Gly Gly His Leu Gly His Asp Asn Asn Gly Gly Ile Ala Arg Gly Ala
        755                 760                 765

Thr Pro Glu Ser Ser Gly Ser Tyr Gly Phe Val Arg Leu Glu Gly Asp
    770                 775                 780

Leu Leu Arg Thr Glu Val Ala Gly Met Ser Val Thr Ala Gly Val Tyr
785                 790                 795                 800

Gly Ala Ala Gly His Ser Ser Val Asp Val Lys Asp Asp Asp Gly Ser
                805                 810                 815

Arg Ala Gly Thr Val Arg Asp Asp Ala Gly Ser Leu Gly Gly Tyr Leu
            820                 825                 830
```

Asn Leu Thr His Thr Ser Ser Gly Leu Trp Ala Asp Ile Val Ala Gln
        835                 840                 845

Gly Thr Arg His Ser Met Lys Ala Ser Ser Asp Asn Asn Asp Phe Arg
    850                 855                 860

Ala Arg Gly Trp Gly Trp Leu Gly Ser Leu Glu Thr Gly Leu Pro Phe
865                 870                 875                 880

Ser Ile Thr Asp Asn Leu Met Leu Glu Pro Gln Leu Gln Tyr Thr Trp
                885                 890                 895

Gln Gly Leu Ser Leu Asp Asp Gly Gln Asp Asn Ala Gly Tyr Val Lys
            900                 905                 910

Phe Gly His Gly Ser Ala Gln His Val Arg Ala Gly Phe Arg Leu Gly
        915                 920                 925

Ser His Ser Asp Met Thr Phe Gly Glu Gly Thr Ser Ser Arg Asp Thr
    930                 935                 940

Leu Arg Asp Ser Ala Lys His Ser Val Arg Glu Leu Pro Val Asn Trp
945                 950                 955                 960

Trp Val Gln Pro Ser Val Ile Arg Thr Phe Ser Ser Arg Gly Asp Met
                965                 970                 975

Ser Met Gly Thr Ala Ala Ala Gly Ser Asn Met Thr Phe Ser Pro Ser
            980                 985                 990

Gln Asn Gly Thr Ser Leu Asp Leu Gln Ala Gly Leu Glu Ala Arg Val
        995                 1000                1005

Arg Glu Asn Ile Thr Leu Gly Val Gln Ala Gly Tyr Ala His Ser Val
    1010                1015                1020

Ser Gly Ser Ser Ala Glu Gly Tyr Asn Gly Gln Ala Thr Leu Asn Val
1025                1030                1035                1040

Thr Phe

<210> 20
<211> 352
<212> PRT
<213> Escherichia coli

<400> 20
Met Lys Arg Lys Val Leu Ala Met Leu Val Pro Ala Leu Leu Val Ala
1               5                   10                  15

Gly Ala Ala Asn Ala Ala Glu Ile Tyr Asn Lys Asn Gly Asn Lys Val
            20                  25                  30

Glu Leu Tyr Gly Lys Met Val Gly Glu Arg Ile Leu Thr Asp Arg Glu
        35                  40                  45

Ser Gly Glu Lys Gly Asp Asn Ser Gln Asp Thr Ser Tyr Ala Arg Val
    50                  55                  60

Gly Val Lys Gly Glu Thr Gln Ile Asn Pro Glu Leu Thr Gly Tyr Gly
65                  70                  75                  80

Gln Phe Glu Leu Asp Leu Glu Ala Ser Asn Arg His Asn Pro Asp Gln

85 90 95

Thr Arg Leu Ala Tyr Ala Gly Leu Ser Tyr Lys Asp Phe Gly Ser Phe
100 105 110

Asp Tyr Gly Arg Asn Val Gly Val Ala Tyr Asp Ala Glu Ala Phe Thr
115 120 125

Asp Met Phe Val Glu Trp Gly Gly Asp Ser Trp Ala Gly Thr Asp Leu
130 135 140

Phe Met Thr Asn Arg Thr Asn Gly Val Ala Thr Tyr Arg Asn Thr Asp
145 150 155 160

Phe Phe Gly Met Val Glu Gly Leu Asn Phe Ala Leu Gln Tyr Gln Gly
165 170 175

Lys Asn Glu Gly Thr Gly Asn Tyr Lys Ala Asn Gly Asp Gly His Gly
180 185 190

Leu Ser Ala Thr Tyr Thr Ile Asp Gly Phe Ser Phe Ala Gly Ala Tyr
195 200 205

Ala Asn Ser Asp Arg Thr Asp Trp Gln Ser Gly Asp Gly Lys Gly Glu
210 215 220

Arg Ala Glu Val Trp Ala Leu Ser Thr Lys Tyr Asp Ala Asn Asn Val
225 230 235 240

Tyr Ala Ala Val Met Tyr Gly Glu Ser His Asn Met Asn Ser Asp Asp
245 250 255

Gly Asp Val Val Asn Lys Thr Gln Asn Phe Glu Ala Val Leu Gln Tyr
260 265 270

Gln Phe Asp Phe Gly Leu Arg Pro Ser Ile Gly Tyr Ser Tyr Ser Lys
275 280 285

Ala Leu Asp Val Ala Gly Tyr Lys Asp Ser Asp Arg Leu Asn Tyr Ile
290 295 300

Glu Ile Gly Thr Trp Tyr Tyr Phe Asn Lys Asn Met Asn Val Tyr Thr
305 310 315 320

Ala Tyr Gln Ile Asn Leu Leu Asp Lys Ser Asp Tyr Val Leu Ala His
325 330 335

Gly Leu Asn Thr Asp Asp Gln Leu Ala Val Gly Ile Val Tyr Gln Phe
340 345 350

<210> 21
<211> 487
<212> PRT
<213> Escherichia coli

<400> 21
Met Ile Lys Lys Lys Gly Phe Thr Leu Leu Glu Val Thr Ile Val Leu
1 5 10 15

Gly Ile Gly Thr Leu Ile Ala Phe Met Lys Phe Gln Asp Met Arg Asn
20 25 30

Asp Gln Glu Ala Val Leu Ala Asp Asn Val Gly Thr Gln Ile Lys Gln
35 40 45

```
Leu Gly Glu Ala Val Asn Arg Tyr Ile Ser Ile Arg Tyr Asp Lys Ile
    50                  55                  60

Ser Thr Leu Ser Ser Ser Asn Asn Gln Ser Ser Asp Pro Gly Pro Arg
65                  70                  75                  80

Thr Cys Ser Ala Ala Gly Cys Glu Ile Thr Tyr Gln Thr Leu Ile Asn
                85                  90                  95

Glu Gly Leu Leu Pro Val Gly Tyr Thr Gly Thr Asn Ala Gln Lys Ala
            100                 105                 110

Ser Tyr Lys Ile Val Leu Lys Arg Ser Gly Thr Ala Pro Asp Tyr Val
        115                 120                 125

Ile Asn Gly Leu Ile Thr Thr Ala Ser Pro Trp Glu Glu Gly Gly Arg
    130                 135                 140

Ile Arg Tyr Asp Leu Leu Gly Lys Ala Val Gln Ala Ala Gly Val Asp
145                 150                 155                 160

Gly Gly Met Ser Arg Asn Thr Lys Ile Ala Ser Gly Tyr Gly Gly Gln
                165                 170                 175

Trp Ser Glu Asn Ser Asn Ser Tyr Ser Asn Ile Thr Gly Gly Gly Leu
            180                 185                 190

Leu Ala Tyr Arg Val Gly Tyr Asn Ser Ser Met Tyr Ser Val Tyr Leu
        195                 200                 205

Arg Arg Asp Gly Thr Leu Pro Met Thr Gly Asp Leu Asn Leu Gly Gly
    210                 215                 220

Lys Ser Ile Lys Asn Ile Lys Asp Met Thr Ala Ser Gly Thr Thr Thr
225                 230                 235                 240

Thr Gly Ala Leu Lys Thr Thr Gly Asn Ala Ser Val Ala Ser Asp Leu
                245                 250                 255

Ser Val Gly Gly Thr Ser Thr Leu Asn Gly Pro Val Asn Ile Asn Asn
            260                 265                 270

Asn Leu Lys Val Lys Ser Asp Thr Tyr Leu Asn Thr Leu Ser Thr Thr
        275                 280                 285

Gly Leu Ala Lys Phe Gly Ser Arg Ile Ala Thr Asn Gly Leu Asn Pro
    290                 295                 300

Asn Asp Leu Pro Ala Gly Trp Ala Gly Gly Val Arg Thr Tyr Asp Leu
305                 310                 315                 320

Tyr Ala Ser Gly Thr Val Gly Val Gly Thr Gly Lys Thr Val Lys Ala
                325                 330                 335

Tyr Met Asn Asn Ala Gly Asn Ile Tyr Ala Ser Gly Asn Leu Thr Ala
            340                 345                 350

Gly Thr Ile Lys Ser Asn Gly Thr Ile Glu Ser Thr Gly Arg Ile Lys
        355                 360                 365

Ala Gly Glu Tyr Leu His Leu Asn Gly Gln Ala Thr Leu Asn Ala Lys
    370                 375                 380

Cys Thr Pro Asn Gly Leu Val Gly Arg Asp Ser Thr Gly Arg Val Leu
```

385 390 395 400

Ser Cys Val Ser Gly Lys Trp Gln Thr Ala Ser Gly Asp Gly Leu Lys
405 410 415

Gly Ile Phe Ile Thr Ile Thr Asp Gln Thr Ser Gly Tyr Lys Cys Val
420 425 430

Ile Pro Asn Ser Asp Thr Gly Ala Cys Ala Cys Pro Gly Ser Met Tyr
435 440 445

Asp Ser Arg Tyr Gly Phe Leu Ser Gly Thr Leu Ile Ala Glu Tyr Asp
450 455 460

Glu Arg Arg Cys Ser Gly Ser Lys Asn Glu His Cys Tyr Ser Asn Phe
465 470 475 480

Arg Arg Leu Tyr Ala Cys Lys
485

<210> 22
<211> 185
<212> PRT
<213> Escherichia coli

<400> 22
Met Val Leu Leu Asn Asn Lys Arg Lys Ser Lys Lys Gly Phe Ser Leu
1 5 10 15

Leu Glu Leu Leu Leu Val Leu Gly Ile Ile Ala Ala Leu Val Val Ala
20 25 30

Ala Phe Ile Val Tyr Pro Lys Val Gln Ala Ser Gln Arg Ala Gln Ala
35 40 45

Glu Ser Asn Asn Ile Ala Thr Ile Gln Ala Gly Val Lys Ala Leu Tyr
50 55 60

Thr Ser Ala Ser Ser Phe Thr Gly Leu Thr Asn Thr Val Ala Val Gln
65 70 75 80

Ala Lys Ile Phe Pro Asp Asn Met Leu Ser Gly Ser Gly Thr Ala Ala
85 90 95

Lys Pro Ile Asn Ala Phe Lys Gly Asn Val Thr Leu Ala Ala Thr Ala
100 105 110

Thr Gly Pro Ser Ser Ala Thr Gly Ser Ser Phe Thr Ile Thr Tyr Asp
115 120 125

Asn Val Pro Ala Ala Glu Cys Val Lys Ile Ala Thr Ala Ala Ala Gly
130 135 140

Asn Phe Tyr Ile Thr Thr Val Gly Thr Lys Val Val Lys Ala Ala Gly
145 150 155 160

Gly Thr Leu Asp Val Ala Ala Thr Ala Ala Ala Cys Thr Asn Ala Thr
165 170 175

Ser Asn Thr Leu Val Phe Thr Ser Ile
180 185

<210> 23
<211> 287
<212> PRT

<213> Escherichia coli

<400> 23

Met Tyr Asn Ile Leu Phe Phe Ile Phe Leu Ser Ile Ala Ile Pro Phe
1 5 10 15

Leu Leu Phe Leu Ala Trp Lys Gln His Leu Lys Thr Lys Glu Ile Arg
20 25 30

Ser Tyr Leu Leu Lys Glu Gly Tyr Asn Ile Ile Phe Asn Gly Glu Gly
35 40 45

Asn Ser Tyr Leu Ala Phe Asn Ile Ser Asn Ala Thr Phe Arg Ala Gly
50 55 60

Asn Leu Thr Ser Asn Asp Tyr Phe Gln Ala Ser Ile Ser Tyr Ile His
65 70 75 80

Asp Tyr Arg Trp Glu Trp Lys Glu Val Glu Ala Lys Lys Ile Asn Asn
85 90 95

Ile Phe Ile Ile Tyr Ile Ser Asn Ile Asp Phe Pro Ser Gln Lys Leu
100 105 110

Phe Tyr Arg Asn Asn Lys Ser Leu Ala Glu Ile Asp Trp Ala Lys Leu
115 120 125

Gln Ala Ile Phe His Gln Pro Tyr Glu Ile Gln Asn Asp Val Met Gln
130 135 140

Asp Asn Asn Asn Thr His Tyr Asp Phe Phe Ile Ser His Ala Lys Glu
145 150 155 160

Asp Lys Asp Thr Phe Val Arg Pro Leu Val Asp Glu Leu Asn Arg Leu
165 170 175

Gly Val Ile Ile Trp Tyr Asp Glu Gln Thr Leu Glu Val Gly Asp Ser
180 185 190

Leu Arg Arg Asn Ile Asp Leu Gly Leu Arg Lys Ala Asn Tyr Gly Ile
195 200 205

Val Ile Leu Ser His Asn Phe Leu Asn Lys Lys Trp Thr Gln Tyr Glu
210 215 220

Leu Asp Ser Leu Ile Asn Arg Ala Val Tyr Asp Asp Asn Lys Ile Ile
225 230 235 240

Leu Pro Ile Trp His Asn Ile Asn Ala Gln Glu Val Ser Lys Tyr Ser
245 250 255

His Tyr Leu Ala Asp Lys Met Ala Leu Gln Thr Ser Leu Tyr Ser Val
260 265 270

Lys Glu Ile Ala Arg Glu Leu Ala Glu Ile Ala Tyr Arg Arg Arg
275 280 285

<210> 24
<211> 176
<212> PRT
<213> Escherichia coli

<400> 24

Met Lys Lys Ile Leu Leu Val Ala Gly Ala Ala Leu Ala Leu Ala Gly
1 5 10 15

Cys Gly Glu Lys Gly Asp Phe Glu Lys Ala Ile Asn Ala Lys Ile Gly
20 25 30

Gln Asp Lys Tyr Cys Phe Ser Leu Asp Asn Asn Asn Thr Ser Phe Pro
35 40 45

Ile Arg Leu Ala Lys Pro Arg Leu Asp Ser Thr Gly Thr Gly Thr Asn
50 55 60

Ser Val Ile Leu Asp Gly Phe Val Glu Gln Gly Met Met Val Phe Glu
65 70 75 80

Gln Gly Tyr Asp Ser Asn Val Leu Gly Ile Thr Glu Glu Gly Lys Lys
85 90 95

Ala Lys Val Trp Ser Thr Thr Asp Gly Ala Cys Val Gly Arg Arg Ala
100 105 110

Val Asp Gly Ile Lys Glu Trp Thr Glu Pro Gly Asn Gly Asn Gln Lys
115 120 125

Val Val Arg Val Thr Tyr Thr Trp Lys Leu Val Asp Val Pro Gly Trp
130 135 140

Ile Asp Lys Lys Ala Phe Ala Ser Val Lys Gly Met Asn Glu Pro Ala
145 150 155 160

Asp Gly Ala Met Asn Leu Phe Lys Thr Ser Asn Gly Trp Lys Ala Asn
165 170 175

<210> 25
<211> 157
<212> PRT
<213> Escherichia coli

<400> 25
Leu Glu Ala Val Gly Ile Val Phe Leu Val Val Leu Phe Ile Ile Ile
1 5 10 15

Met Thr Ala Val Asp Ile Gln Lys Lys Lys His Tyr Asn Ser Phe Thr
20 25 30

Glu Val Leu Asp Gly Asp Ile Leu Ser Tyr Glu Cys Gln Gln Thr Gly
35 40 45

Ile Val Ile Asp Thr Gln Lys His Thr Val Arg Ile Phe Asn Lys Asp
50 55 60

Lys Asp Ser Thr Tyr Thr Phe Asp Gln Ile Arg Glu Ile Asn Tyr Thr
65 70 75 80

Leu Ser Glu Gly Gly Lys Phe Tyr Gly Asn Gly Thr Leu Arg Gly Met
85 90 95

Asn Asn Ala Ala Ile Ala Asn Trp Arg Glu Gln Leu Ser Ala Asn Lys
100 105 110

Arg Ser Gly Leu Asn Ile Leu Thr Asp Asp Ile Lys Asn Pro Met Trp
115 120 125

Lys Val Asn Val Pro Leu Lys Asn Leu Ser Thr Ser Asn His Glu Leu
130 135 140

Cys Glu Arg Trp Met Leu Val Phe Lys Lys Tyr Val Phe
145 150 155

<210> 26
<211> 320
<212> PRT
<213> Escherichia coli

<400> 26
Met Glu Gly Lys Leu Ile Phe Cys Ser Asp Ala Ile Leu Arg Phe Gln
1 5 10 15

Ser Asp Tyr Asp Glu Thr Ser Ala Val Pro Leu Leu Ser Ile Gln Asn
20 25 30

Thr Ile Ala Asn Thr Asp Pro Phe Phe Leu Leu Arg Phe Phe His His
35 40 45

Thr Val Leu Ile Glu Glu Gly Thr Ser Leu Ala Ser Ile Phe Leu Ala
50 55 60

Ile Glu Leu Trp Lys Ala Leu Leu Ala Ala Tyr Leu Asp Arg Asp Val
65 70 75 80

Gly Ala Tyr Ile Asp Glu Val Arg Lys Pro Ser Gly Pro Thr Thr Trp
85 90 95

Asp Ile Glu Trp Ile Gly Ile Asp Arg Arg Ser Ser Val Tyr Arg Ala
100 105 110

Tyr Lys Arg Gln Asp Met Glu Glu Gly Glu Asp Phe Ser Thr Tyr Phe
115 120 125

Asn Arg Glu Arg Phe Pro Thr Asp Glu Phe Asp Ile Glu Ser Ser Cys
130 135 140

Asp Ala Ser Gly Phe Ile Lys Gly Asp Lys Glu Arg Trp Ser Ile Ser
145 150 155 160

Gly Asp Val His Glu Ile Lys Asn Leu Pro Val Ile Leu Tyr Ser Lys
165 170 175

Gln Ala Leu Met Thr Ser Ala Lys Asp Gly Leu Leu Lys Lys Asn Val
180 185 190

Ser Gly Val Lys Ser Ser Lys His Gly Cys Phe Val Tyr Gly Asp Thr
195 200 205

Ser Phe Ser Phe Ser Glu Val Met Glu Ala Ile Phe Ile Ser Gly Leu
210 215 220

Phe Phe Tyr Ala Pro Ile Asp Ala Ala Ser Ser Leu Asp Glu Leu Lys
225 230 235 240

Val Ser Leu Ala Gly Leu Glu Glu Glu Leu Ala Glu Val Pro Lys Val
245 250 255

Asp Ser Asn Gly Asn Glu Thr Asp Lys Glu Pro Thr Ile Val Val Ala
260 265 270

Glu Gly Ala Phe Asp Ser Val Ala Ala His Met Glu Thr Glu Ala Glu
275 280 285

Glu Trp Gln Ser Ile Lys Asn Leu Cys Gln Arg Glu Gly Glu Leu Pro
290 295 300

Ile Arg Ile Gly Gly Ile Lys Met Ala Glu Pro Pro Glu Phe His Phe
305 310 315 320

<210> 27
<211> 483
<212> PRT
<213> Escherichia coli

<400> 27
Met Arg Asn Tyr Met Asn Asn Asn Lys Ile Ile Thr Pro Ile Ile Met
1 5 10 15

Ala Gly Gly Ser Gly Ser Arg Leu Trp Pro Leu Ser Arg Ile Leu Tyr
20 25 30

Pro Lys Gln Phe Leu Ser Leu Ile Gly Ser His Thr Met Leu Gln Thr
35 40 45

Thr Ala Asn Arg Leu Asp Gly Leu Asp Cys Thr Asn Pro Tyr Val Ile
50 55 60

Cys Asn Glu Gln Tyr Arg Phe Ile Val Ala Glu Gln Leu Arg Lys Ile
65 70 75 80

Asp Arg Leu Thr Ser Lys Asn Ile Ile Leu Glu Pro Val Gly Arg Asn
85 90 95

Thr Ala Pro Ala Ile Ala Leu Ala Ala Leu Leu Met Ser Lys Ser Asp
100 105 110

Lys Ser Ala Asp Asp Leu Met Leu Val Leu Ala Ala Asp His Val Ile
115 120 125

His Asp Glu Glu Lys Phe Cys Asn Ala Val Arg Ser Ala Ile Pro Tyr
130 135 140

Ala Ala Asp Gly Lys Leu Val Thr Phe Gly Ile Ile Pro Asp Lys Ala
145 150 155 160

Glu Thr Gly Tyr Gly Tyr Ile His Arg Gly Gln Tyr Ile Asn Gln Glu
165 170 175

Asp Ser Asp Ala Phe Ile Val Ser Ser Phe Val Glu Lys Pro Asn His
180 185 190

Glu Thr Ala Thr Lys Tyr Leu Ala Ser Gly Glu Tyr Tyr Trp Asn Ser
195 200 205

Gly Met Phe Leu Phe Ser Ala Asn Arg Tyr Ile Glu Glu Leu Lys Gln
210 215 220

Phe Arg Pro Asp Ile Leu Ser Ala Cys Glu Lys Ala Ile Ala Ser Ala
225 230 235 240

Asn Phe Asp Leu Asp Phe Val Arg Leu Asp Glu Ser Ser Phe Ser Lys
245 250 255

Cys Pro Glu Glu Ser Ile Asp Tyr Ala Val Met Glu Lys Thr Lys Asp
260 265 270

Ala Ile Val Ile Pro Met Asp Ala Gly Trp Ser Asp Val Gly Ser Trp
275 280 285

```
Ser Ser Leu Trp Glu Ile Asn Asp Lys Asp Ser Asp Gly Asn Val Ile
    290                 295                 300

Val Gly Asp Ile Phe Ser His Glu Thr Lys Asn Ser Phe Ile Tyr Ala
305                 310                 315                 320

Glu Ser Gly Ile Val Ala Thr Val Gly Val Glu Asn Leu Val Val Val
                325                 330                 335

Gln Thr Lys Asp Ala Val Leu Val Ser Glu Arg Asn Lys Val Gln Asp
            340                 345                 350

Val Lys Lys Ile Val Glu Gln Ile Lys Asn Ser Gly Arg Ser Glu His
        355                 360                 365

Tyr Val His Arg Glu Val Tyr Arg Pro Trp Gly Lys Tyr Asp Ser Ile
    370                 375                 380

Asp Thr Gly Glu Arg Tyr Gln Val Lys Arg Ile Thr Val Asn Pro Gly
385                 390                 395                 400

Glu Gly Leu Ser Leu Gln Met His His His Arg Ala Glu His Trp Ile
                405                 410                 415

Ile Val Ser Gly Thr Ala Arg Val Thr Ile Gly Ser Glu Thr Lys Ile
            420                 425                 430

Leu Ser Glu Asn Glu Ser Val Tyr Ile Pro Leu Gly Val Ile His Cys
        435                 440                 445

Leu Glu Asn Pro Gly Lys Ile Pro Leu Asp Leu Ile Glu Val Arg Ser
    450                 455                 460

Gly Ser Tyr Leu Glu Glu Asp Asp Val Ile Arg Phe Gln Asp Arg Tyr
465                 470                 475                 480

Gly Arg Ser


<210>   28
<211>   381
<212>   PRT
<213>   Escherichia coli

<400>   28
Met Lys Lys Asn Lys Lys Leu Cys Leu Ile Ser Ile Asn Ser Tyr Asn
1               5                   10                  15

Glu Leu Thr Gly Gly Gly Val Tyr Leu Arg Thr Leu Val Ser Phe Leu
            20                  25                  30

Gln Lys Gln Asn Val Asn Leu Thr Leu Ile Asp Lys Lys Ser Ser Gly
        35                  40                  45

Lys Leu Phe Glu Asp Asn Thr Phe Gln His Ile Ser Phe Ile Lys Gly
    50                  55                  60

Lys Arg Gln Asp Ile Ile Ser Arg Leu Phe Phe Ile Pro Ser Phe Tyr
65                  70                  75                  80

Val Pro Tyr Ile Phe Ser Ile Ile Lys Ile Leu Arg Lys Gln Asp Ile
                85                  90                  95

Leu Ala Phe His Asn Ser Arg Leu Gly Leu Leu Cys Leu Leu Phe Arg
            100                 105                 110
```

Ile Leu Met Pro His Lys Lys Ile Ile Leu Phe Thr Asp Asn Phe Glu
115 120 125

Tyr Asp Leu Ile Arg Gln Lys Asp Lys Asn Ile Thr Thr Phe Ile Glu
130 135 140

Lys Leu Ile Val Tyr Leu Asn Glu Phe Ile Gly Leu Lys Asn Ser Asp
145 150 155 160

Leu Val Ser Tyr Ile Thr Arg Gln Asp Lys Asn Ala Met Asp Lys Phe
165 170 175

Tyr Gly Ile Lys Lys Ser Arg Asn Leu Ile Leu Pro Val Ile Phe Ser
180 185 190

Arg Glu Lys Pro Thr Asp Val Leu Ser Ala His Phe Ile Asn Glu Tyr
195 200 205

Asn Arg Leu Asn Asn Asp Asn Arg Lys Lys Val Val Phe Thr Ala Ser
210 215 220

Phe Asp Phe Phe Pro Asn Ile Asp Ala Ala Asn Tyr Val Leu Asn Ala
225 230 235 240

Ala Lys Ser Asn Asn Asp Tyr Cys Tyr Ile Leu Ala Gly Arg Lys Ser
245 250 255

Thr Thr Leu Asn Leu Pro Asp Leu Asp Asn Leu Phe Phe Phe Asp Asn
260 265 270

Leu Ser Asn Ser Glu Met Ser Tyr Leu Leu Ser Ala Cys Asp Val Phe
275 280 285

Tyr Ser Pro Ile Val Leu Gly Ser Gly Met Lys Thr Lys Ile Ala Glu
290 295 300

Ala Leu Ser Tyr Gly Leu Tyr Ile Tyr Ala Thr Glu His Ser Leu Ile
305 310 315 320

Gly Tyr Asp Glu Ile Ile Asn Asn Lys Glu Cys Val Lys Lys Ile Ser
325 330 335

His Leu Asp Glu Glu Phe Pro Lys Asp Phe Lys Met Lys Ser Ile Asn
340 345 350

Lys Gln Leu Ile Met Ser Tyr Gln Gln Lys Tyr Tyr Ser His Tyr Arg
355 360 365

Phe Asn Gly His Glu Leu Asp Ile Ile Asn Phe Asp Asp
370 375 380

<210> 29
<211> 343
<212> PRT
<213> Escherichia coli

<400> 29
Met Ile Ile Tyr Ile Ala Ala Tyr Asn Gly Ser Gly Gly Gln Gly Gly
1 5 10 15

Val Glu Arg Val Val Ala Gln Gln Cys Asn Ile Leu Lys Asn Leu Gly
20 25 30

Val Lys Val Ile Ile Leu Asp Lys Thr Tyr Phe Lys Met Ser Asn Lys

35 40 45

Ile Arg Asn Lys Lys Ile Gln Val Ala Leu Tyr Pro Ile Leu Val Ser
50 55 60

Leu Tyr Leu Thr Leu Gln Lys Leu Arg Gly Val Thr Phe Lys Val Ile
65 70 75 80

Ala His Gly Tyr Cys Ser Pro Phe Tyr Arg Asn Asp Ile Leu Ile Ala
85 90 95

His Gly Asn Met Lys Cys Tyr Phe Gln Thr Val Met Asn Lys Lys Pro
100 105 110

Asn Arg Leu Ser Gly Ser Gly Leu Leu Ser Phe Tyr Glu Arg Trp Ala
115 120 125

Gly Ala Phe Ser Lys Asn Ile Trp Ala Val Ser Asn Lys Val Lys Ser
130 135 140

Glu Trp Asn Glu Leu Tyr Asn Ile Asn Ser His Lys Ile Lys Val Val
145 150 155 160

Arg Asn Phe Ile Asn Leu Ala Gln Phe Asp Tyr Thr Asp Val Asn Glu
165 170 175

Ala Glu Tyr Val Thr Phe Val Gly Arg Leu Glu Lys Gly Lys Gly Ile
180 185 190

Asp Asp Leu Tyr Tyr Ile Cys Lys Asn Leu Pro Asp Thr Ser Phe His
195 200 205

Leu Val Ser Ser Ile Pro Ala Pro Gln Asn Phe Ala Ser Leu Asn Asn
210 215 220

Val Leu Thr Ser Ile Ala Val Pro Tyr Ala Lys Met Pro Glu Ile Phe
225 230 235 240

Lys Lys Ser Arg Val Leu Ile Leu Pro Ser Tyr Tyr Glu Gly Tyr Glu
245 250 255

Leu Val Thr Ile Glu Ala Leu Cys Cys Gly Cys Pro Val Ile Gly Tyr
260 265 270

Asn Val Gly Ala Ile Arg Glu Leu Tyr Ala Glu Ser Phe Pro Gly Val
275 280 285

Phe Ile Ala Asn Asn Lys Glu Asp Leu Ala Gln Val Ala Tyr Lys Leu
290 295 300

Ile Ser Leu Asp Asn Glu Lys Tyr Tyr His Leu Arg Gln Thr Ile Tyr
305 310 315 320

Ser Lys Arg Glu Leu Phe Ser Glu Glu Arg Tyr Ala Glu Ile Leu Thr
325 330 335

Ala Ala Phe Asn Glu Lys Lys
340

<210> 30
<211> 253
<212> PRT
<213> Escherichia coli

<400> 30

```
Met Lys Lys Thr Asn Leu Leu Phe Arg Leu Leu Ile Leu Met Val Phe
1               5                   10                  15

Met Leu Gly Ser Arg Ala Gly Met Gly Gln Thr Phe Arg Asn Val Tyr
            20                  25                  30

Ile Tyr Tyr Ala Thr Phe Gly Trp Asn Ser Gln Val Glu Leu Tyr Leu
        35                  40                  45

Asp Val Ala Glu Val Ser Ser Val Tyr Tyr Gly Thr Tyr Tyr Gln Ser
    50                  55                  60

Asn Tyr Val Met Thr Leu Gly Asn Leu Pro Asn Arg Thr Trp Thr Gly
65                  70                  75                  80

Thr Gly Ser Ser Pro Ala Pro Thr Ile Thr Val Lys Asp Gly Thr Thr
                85                  90                  95

Val Asn Asn Ser Phe Cys Pro Gly Met Glu Ala Leu Glu Ala Lys Asp
            100                 105                 110

Ser Arg Trp Glu Cys Leu Lys Leu Pro Leu Asp Ile Thr Val Asp Gly
        115                 120                 125

Asp Val Gly Gly Cys Pro Trp Leu Val Ser Ile Arg Ala Thr Ser Gln
    130                 135                 140

Val Val Val Gly Ser Val Asp Lys Asp Ile Tyr Tyr Gly Pro Asn Ala
145                 150                 155                 160

Thr Val Ser Val Cys Pro Thr Val Ser Val Thr Pro Tyr Asp Val Ser
                165                 170                 175

Trp Asp Glu Asn Tyr Ile Asn Lys Thr Lys Thr Leu Ser Leu Gln Ser
            180                 185                 190

Thr Gly Gly Val Ile Glu Lys Thr Leu Ser Thr Tyr Leu Met Lys Asp
        195                 200                 205

Gly Lys Leu Cys Asp Gly Ser Gln Met Asn Glu Glu Gly Ala Tyr Cys
    210                 215                 220

Arg Trp Val Ala Gln Met Ile Thr Phe Ser Ser Ser Gly Cys Asp Asn
225                 230                 235                 240

Ala Asn Val Thr Val Ala Pro Lys Gln Ala Ser His His
                245                 250

<210>   31
<211>   351
<212>   PRT
<213>   Escherichia coli

<400>   31
Met Val Tyr Val Leu Ile Arg Leu Val Thr Tyr Gly Tyr Tyr Arg Gln
1               5                   10                  15

Ile Ser Glu Glu Thr Leu Val Arg Gly Ala Arg Ala Ser Ser Tyr Phe
            20                  25                  30

Met Glu Ser Leu Tyr Gly Ile Ala Thr Val Lys Ile Gln Gly Met Val
        35                  40                  45

Gly Ile Arg Gly Thr His Trp Leu Asn Leu Lys Ile Asp Ala Ile Asn
    50                  55                  60
```

Ser Gly Ile Lys Leu Thr Arg Met Asp Leu Leu Phe Gly Gly Ile Asn
65 70 75 80

Thr Phe Val Ala Ala Cys Asp Gln Met Ala Ile Leu Trp Leu Gly Ala
85 90 95

Ser Leu Val Ile Asp Asn Gln Met Thr Ile Gly Met Phe Val Ala Phe
100 105 110

Gly Ser Phe Arg Gly Gln Phe Ser Asp Arg Val Ala Ser Leu Thr Ser
115 120 125

Phe Leu Leu Gln Leu Arg Ile Met Ser Leu His Asn Glu Arg Ile Ala
130 135 140

Asp Ile Ala Leu His Glu Lys Glu Glu Lys Lys Pro Glu Ile Glu Ile
145 150 155 160

Val Ala Asp Met Ser Pro Val Ser Leu Glu Thr Thr Asp Leu Ser Tyr
165 170 175

Arg Tyr Asp Ser Gln Ser Ala Gln Val Phe Ser Gly Leu Asn Leu Ser
180 185 190

Val Ala Pro Gly Glu Ser Val Ala Ile Thr Gly Ala Ser Gly Ala Gly
195 200 205

Lys Thr Thr Leu Met Lys Val Leu Cys Gly Leu Phe Glu Pro Asp Ser
210 215 220

Gly Lys Val Leu Val Asn Gly Thr Asp Ile Arg Gln Leu Gly Ile Asn
225 230 235 240

Asn Tyr His Arg Met Ile Ala Cys Val Met Gln Asp Asp Arg Leu Phe
245 250 255

Ser Gly Ser Ile Arg Glu Asn Ile Cys Gly Phe Ala Glu Glu Thr Asp
260 265 270

Asp Glu Trp Met Thr Glu Cys Ala Arg Ala Ser His Ile His Asp Val
275 280 285

Ile Met Lys Met Pro Met Gly Tyr Glu Thr Leu Ile Gly Glu Leu Gly
290 295 300

Glu Gly Leu Ser Gly Gly Gln Lys Gln Arg Ile Phe Ile Ala Arg Ala
305 310 315 320

Leu Tyr Arg Lys Pro Gly Ile Leu Phe Met Asp Glu Ala Thr Ser Ser
325 330 335

Leu Asp Thr Glu Ser Glu Arg Phe Val Asn Ala Ala Ile Lys Lys
340 345 350

<210> 32
<211> 278
<212> PRT
<213> Escherichia coli

<400> 32
Met Ala Lys Val Asn Phe Phe Asp Lys Arg Ile Leu Lys Lys Phe Ser
1 5 10 15

Asp Tyr Thr Ser Thr Ile Ser Thr Ile Phe Ser Leu Phe Leu Ile Phe

```
            20                  25                  30

Val Asp Ile Pro Thr Glu Asn Lys Leu Thr Leu Gly Ile Ile Phe Leu
        35                  40                  45

Ile Ile Leu Phe Leu Leu Tyr Phe Gly Ile Trp Phe Lys Ser Asn Asn
    50                  55                  60

Leu Ser Glu Ile Asn Leu Asp Val Glu Gly Ser Ile Val Thr Val Lys
65                  70                  75                  80

Ala Gly Asp Leu Phe Arg Gln Asp Gly Phe Lys Val Ile Ala Phe Asn
                85                  90                  95

Glu Tyr Phe Asp Thr Gln Val Asp Asp Val Ile Ile Ser His Asn Ser
            100                 105                 110

Leu Asn Gly Leu Tyr Ile Asp Asn Tyr Leu Ala Gly Ser Val Ser Asp
        115                 120                 125

Leu Asp His Arg Ile Ser Asn His Gln Phe Glu Glu Asp Glu Leu Leu
    130                 135                 140

Glu Val Asn His Lys Arg Lys Val Gly Lys Thr Gln Lys Tyr Ser Leu
145                 150                 155                 160

Gly Thr Ile Phe Val Asn Asn Asp Tyr Leu Leu Thr Ala Phe Ser Lys
                165                 170                 175

Phe Asp Asp Lys Asn Arg Ala Phe Leu Thr Met Pro Asp Tyr Leu Ala
            180                 185                 190

Phe Leu Ile Asn Phe Trp Asp Lys Val Asn Arg Ile Tyr Ala Gln Lys
        195                 200                 205

Ser Val Ser Val Pro Ile Phe Gly Ser Gly Ile Thr Arg Ile Lys Glu
    210                 215                 220

His Lys Asn Ile Ser Asp Glu Asp Leu Leu Lys Ile Met Leu Trp Thr
225                 230                 235                 240

Phe Arg Ile Ser Glu Met Arg Phe Lys Phe Pro Ala Lys Leu Thr Ile
                245                 250                 255

Val Ile His Lys Asp Lys Ile Asp Lys Ile Asn Leu Leu Asp Ile Lys
            260                 265                 270

Ser Ala Arg Asn Gly Leu
        275

<210>  33
<211>  447
<212>  PRT
<213>  Escherichia coli

<400>  33
Met Lys Val Lys Ala Val Pro Ala Ile Thr Phe Tyr Leu Ser Leu Met
1               5                   10                  15

Leu Thr Ile Leu Val Leu Leu Phe Gly Asn Glu Pro Asn Lys Ser Gln
            20                  25                  30

Tyr Ile Leu Val Ile Ala Thr Ile Thr Val Phe Tyr Ile Ala Tyr Ile
        35                  40                  45
```

```
Thr Asn Lys Ile Thr Ser Pro Ala Ser Leu Leu Val Ile Ser Ser Phe
    50                  55                  60

Val Phe Leu Gly Cys Arg Pro Leu Leu Ser Leu Phe Ala Asn Tyr Asp
65                  70                  75                  80

Tyr Arg Ile Ala Asp Trp Phe Ile Glu Gly Tyr Met Asp Asp Asp Val
                85                  90                  95

Ile Leu Ala Asn Tyr Ala Ile Thr Leu Met Tyr Tyr Gly Tyr Thr Leu
            100                 105                 110

Gly Leu Ile Leu Cys Lys Asn Thr Glu Lys Phe Tyr Pro His Gly Pro
        115                 120                 125

Tyr Pro Glu Lys Gln Leu Leu Lys Ile Lys Phe Leu Leu Thr Leu Phe
    130                 135                 140

Phe Leu Gly Ser Ile Gly Met Val Val Lys Gly Ile Phe Phe Phe Asn
145                 150                 155                 160

Phe Ile Glu Ser Asn Ser Tyr Val Asp Ile Tyr Gln Ser Asn Ile Thr
                165                 170                 175

Thr Pro Ile Gly Tyr Asp Phe Leu Ser Tyr Leu Phe Tyr Cys Ser Phe
            180                 185                 190

Phe Leu Ile Cys Ala Phe His Ile Gln Phe Arg Thr Asn Lys Lys Phe
        195                 200                 205

Leu Phe Ile Ala Ile Cys Ile Ala Ala Phe Ser Thr Leu Lys Gly Ser
    210                 215                 220

Arg Ser Glu Ala Ile Thr Phe Leu Leu Thr Val Thr Cys Ile Tyr Phe
225                 230                 235                 240

Asn Glu Val Lys Thr Arg Asn Leu Arg Leu Leu Ile Thr Met Ile Phe
                245                 250                 255

Val Phe Ser Val Ile Phe Val Ile Ser Glu Phe Ile Ser Met Trp Arg
            260                 265                 270

Thr Gly Gly Ser Phe Phe Gln Leu Met Gln Gly Asn Asn Pro Val Ile
        275                 280                 285

Asn Phe Val Tyr Gly Met Gly Val Ser Tyr Leu Ser Ile Tyr Gln Ser
    290                 295                 300

Val Lys Leu Gln Leu Leu Ser Gly Gly Tyr Asn Val Thr Tyr Leu Phe
305                 310                 315                 320

Ser Gln Leu Ile Ile Thr Cys Ser Ser Ile Phe Asn Val Lys Leu Ser
                325                 330                 335

Leu Pro Glu Ile Ser Tyr Ser His Leu Ala Ser Tyr Thr Ala Asn Pro
            340                 345                 350

Glu Leu Tyr Asn Leu Gly Phe Gly Leu Gly Gly Ser Tyr Leu Ala Glu
        355                 360                 365

Ser Phe Leu Ala Phe Gly Leu Ile Gly Cys Phe Ile Ile Pro Phe Leu
    370                 375                 380

Leu Leu Leu Asn Leu Asn Val Leu Glu Lys Tyr Thr Lys Asn Lys Pro
385                 390                 395                 400
```

```
Ile Ile Tyr Phe Val Tyr Tyr Ser Val Leu Pro Pro Ile Leu Phe Thr
                405                 410                 415

Pro Arg Glu Thr Leu Phe Tyr Phe Phe Pro Tyr Leu Val Lys Ser Ile
            420                 425                 430

Phe Val Ala Phe Leu Val Thr Leu Tyr Ile Gln Tyr Lys Lys Asp
        435                 440                 445

<210>   34
<211>   412
<212>   PRT
<213>   Escherichia coli

<400>   34
Val His Asn Tyr Leu Arg Leu Lys Asp Lys Ile Lys Asn Val Phe Leu
1               5                   10                  15

Gly Leu Tyr Ile Leu Gln Leu Phe Asn Tyr Ile Ala Pro Leu Val Ile
            20                  25                  30

Ile Pro Ile Leu Ile Lys Tyr Ile Gly Leu Gly Glu Tyr Gly Glu Leu
        35                  40                  45

Val Tyr Ile Thr Ser Ile Tyr Gln Ile Val Ala Leu Ile Ile Asp Phe
    50                  55                  60

Gly Phe Thr Tyr Thr Gly Pro Val Val Ala Ala Arg His Arg Cys Glu
65                  70                  75                  80

Thr Gln Asn Leu Gln Arg Tyr Tyr Ser Ile Val Val Leu Leu Lys Ser
                85                  90                  95

Leu Leu Phe Ile Ile Ala Leu Thr Cys Val Phe Leu Leu Cys Arg Leu
            100                 105                 110

Asn Ile Val His Leu Ser Phe Phe Gly Phe Leu Ser Ile Phe Leu Cys
        115                 120                 125

Thr Ile Gly Asn Ile Leu Ser Pro Asn Trp Phe Leu Gln Gly Ile Gly
    130                 135                 140

Asp Phe Lys Lys Leu Ser Tyr Ser Gln Val Ile Val Arg Ile Thr Leu
145                 150                 155                 160

Phe Ile Ile Leu Leu Val Tyr Val Cys Ser Gly Gly Asp Asn Val Phe
                165                 170                 175

Ile Leu Ser Phe Leu Gln Asn Ala Thr Leu Leu Ile Cys Cys Ile Tyr
            180                 185                 190

Leu Trp Pro Asn Ile His Ile Ser His Val Val His Leu Lys Pro Asn
        195                 200                 205

Glu Cys Ile Val Glu Phe Lys Lys Ala Gly Asn Val Phe Ile Gly Val
    210                 215                 220

Ile Gly Thr Ile Gly Tyr Asn Gly Leu Ile Pro Val Leu Ile Gly Asn
225                 230                 235                 240

Leu Cys Gly Asn Thr Ser Leu Gly Val Phe Ser Ile Val Gln Lys Met
                245                 250                 255

Thr Thr Ala Cys Gln Ser Leu Ile Asn Pro Ile Ser Gln Tyr Met Leu
```

260 265 270

Ser Gln Val Ser Glu Ile Lys Pro Gln Asp Lys Leu Phe Tyr Tyr Arg
275 280 285

Ile Lys Lys Ser Phe Phe Val His Leu Thr Ile Ser Ile Ile Ala Cys
290 295 300

Leu Cys Tyr Met Gly Leu Gly Gln Tyr Val Ala Thr Phe Ile Gly Lys
305 310 315 320

Val Asp Val Ser Phe Val Ile Ile Leu Phe Ala Ser Ile Ile Thr Ile
325 330 335

Phe Ser Ser Leu Asn Asn Val Leu Gly Ile Gln Phe Leu Ile Pro Thr
340 345 350

Asp Asn Val Lys Ile Leu Arg Ser Ile Asn Val Met Ala Gly Ile Ile
355 360 365

Val Val Ser Leu Ser Trp Leu Leu Ile Ser Arg Phe Asp Ile Leu Gly
370 375 380

Gly Val Leu Leu Asn Leu Ile Gly Glu Phe Leu Val Phe Ser Met Leu
385 390 395 400

Ala Phe Ile Ala His Arg Lys Trp Gly Ala Arg Val
405 410

<210> 35
<211> 320
<212> PRT
<213> Escherichia coli

<400> 35
Met Thr Ser Met Pro Ala Ser Leu Leu His Phe Asn Asn Lys Thr His
1 5 10 15

Arg Leu Met Tyr Lys Lys Ser Leu Arg Asp Ile Val Cys Lys Lys Gly
20 25 30

Ala Asp Met Arg Thr His Pro His Leu Ile Lys Gln Thr Ile Asp Pro
35 40 45

Arg Leu Ile Asn Glu Ile Asn Lys Arg Thr Gln Ser Asn Ser Glu Lys
50 55 60

Leu Ser Asn Ser Asp Lys Leu Thr Tyr Ile Asn Asp Asn Tyr Cys Glu
65 70 75 80

Ile Thr Arg Asn Tyr Thr Ser Phe Tyr Gly Met Asn Met Leu Ser Ile
85 90 95

Val Leu Phe Leu Pro Ile Val Ile Leu Cys Phe Ala Ser Val Leu Phe
100 105 110

Phe Ile Tyr Asp Ile Thr Phe Asn Phe Glu Arg Tyr Leu Asp Thr Trp
115 120 125

Arg Tyr Arg Ala Asp Ser Glu Leu Met Leu His Ile Ala Tyr Cys Ala
130 135 140

Ile Phe Phe Met Ser Phe Val Phe Ala Ala Ile Leu Asn Tyr Phe Ile
145 150 155 160

Phe Ala Pro Arg His Tyr Pro Ile Arg Phe Asn Arg Lys Thr Gly Lys
165 170 175

Val Tyr Ile Cys Asp Tyr Ile Leu Asn Asp Met Ser His Lys Ser Thr
180 185 190

Tyr Thr Phe Trp Trp His Tyr Pro Phe Tyr Lys His Thr Lys Pro Glu
195 200 205

Tyr Lys Glu Tyr Asp Trp Glu Gln Ile Gln Gly Ile Ala Phe Phe Ala
210 215 220

Gln Asn Arg His Ser Ser Tyr Ser Thr Ile Arg Cys Ile Val Tyr Glu
225 230 235 240

Ser Leu Asp Ser Thr Asn Ile Ile Asp Thr Phe Asn Leu Ile Thr Thr
245 250 255

Glu Ser Ala Ile Asp Ala Leu His Met Thr Asn His Lys Leu Trp Leu
260 265 270

Trp Ile Asn Ser Phe Met Asn Phe Asp Asp Glu Phe Leu Glu Lys Ser
275 280 285

Ile Asn Thr Ala Pro Gly Ile Trp Gly Arg Glu Val Asn Trp Pro Glu
290 295 300

Asp Met Asn Lys Lys Ser Met Leu Ser Ser Met Asp Glu Tyr Ile Leu
305 310 315 320

<210> 36
<211> 199
<212> PRT
<213> Escherichia coli

<400> 36
Met Gln Pro Leu Pro Leu Lys Gln Ile His Pro Leu Trp Leu Arg Cys
1 5 10 15

Cys His Trp Ile Asn Ala Val Thr Val Ala Gly Met Ile Cys Ser Gly
20 25 30

Trp Arg Ile Tyr Asn Ala Ser Pro Leu Phe Ser Phe Asn Tyr Pro Ala
35 40 45

Ala Leu Thr Leu Gly Gly Trp Leu Gly Gly Ala Leu Leu Trp His Phe
50 55 60

Ala Leu Met Trp Ile Leu Leu Ile Asn Ala Thr Ile Tyr Leu Thr Leu
65 70 75 80

Gly Ile Cys Ser Gly Arg Phe Arg Ser Lys Phe Phe Pro Leu Ser Trp
85 90 95

Thr Ser Leu Lys Lys Ser Leu Thr Gln Ala Leu Tyr Gly Lys Leu His
100 105 110

His Thr Asn Met Gln Glu Tyr Asn Met Val Gln Lys Phe Ala Tyr Ile
115 120 125

Leu Ile Ile Leu Asp Gly Ala Ala Leu Leu Leu Ser Gly Leu Val Leu
130 135 140

Trp Lys Pro Val Gln Phe Ser Leu Leu Thr Ile Leu Phe Gly Gly Tyr

145 150 155 160

Asp Thr Ala Arg Tyr Ile His Phe Leu Cys Met Asn Ile Met Val Leu
165 170 175

Phe Ile Ile Ile His Leu Val Met Val Leu Leu Val Pro Arg Ala Leu
180 185 190

Leu Ser Met Ile Arg Gly His
195

<210> 37
<211> 181
<212> PRT
<213> Escherichia coli

<400> 37
Met Lys Leu Lys Phe Ile Ser Met Ala Val Phe Ser Ala Leu Thr Leu
1 5 10 15

Gly Val Ala Thr Asn Ala Ser Ala Val Thr Thr Val Asn Gly Gly Thr
20 25 30

Val His Phe Lys Gly Glu Val Val Asp Ala Ala Cys Ala Val Asn Thr
35 40 45

Asn Ser Ala Asn Gln Thr Val Leu Leu Gly Gln Val Arg Ser Ala Lys
50 55 60

Leu Ala Asn Asp Gly Glu Lys Ser Ser Pro Val Gly Phe Ser Ile Glu
65 70 75 80

Leu Asn Asp Cys Ser Ser Ala Thr Ala Gly His Ala Ser Ile Ile Phe
85 90 95

Ala Gly Asn Val Ile Ala Thr His Asn Asp Val Leu Ser Leu Gln Asn
100 105 110

Ser Ala Ala Gly Ser Ala Thr Asn Val Gly Ile Gln Ile Leu Asp His
115 120 125

Thr Gly Thr Ala Val Gln Phe Asp Gly Val Thr Ala Ser Thr Gln Phe
130 135 140

Thr Leu Thr Asp Gly Thr Asn Lys Ile Pro Phe Gln Ala Val Tyr Tyr
145 150 155 160

Ala Thr Gly Lys Ser Thr Pro Gly Ile Ala Asn Ala Asp Ala Thr Phe
165 170 175

Lys Val Gln Tyr Gln
180

<210> 38
<211> 476
<212> PRT
<213> Escherichia coli

<400> 38
Met Gly Thr Gly Tyr Phe Leu Val Arg Gly Asp Lys Thr Thr Cys Gly
1 5 10 15

Gly Lys Ile Ile Glu Gly Ala Asp Asp His Thr Ile Met Gly Ile Pro
20 25 30

Gln Ala Arg Asp Met Asp Arg Val Thr Cys Gly Arg Tyr Pro Gly Met
35 40 45

Phe Ile Ile Val Gly Gly Val Pro Glu Thr Asp Ile His Gly Arg Leu
50 55 60

Met Ala Gly Thr Leu Asp Ser Gln Ser Ser Cys Pro Cys Lys Ala Arg
65 70 75 80

Phe Ile Ala Ser Met Met Asp Asp Thr Tyr Glu Thr Asp Asp Gly Gly
85 90 95

Asn Glu Pro Glu Gln His Ala Gln Ser Ala Lys Lys Asp Leu Thr Ser
100 105 110

Gly Ser Asp Ser Ser Ser Asp Asp Val Lys Leu Asp Tyr Arg Ile Lys
115 120 125

Leu Ser Gly Asn Lys Ile Leu Thr Pro Leu Asn Ile Pro Asp Tyr Lys
130 135 140

Glu Met Ile Ser Gly Gly Ser Thr Lys Asn Thr Glu Lys Ile Asp Phe
145 150 155 160

Thr Ile Thr Asn Lys Gly Asp Glu Thr Glu Ala Leu Ser Leu Glu Val
165 170 175

Leu Asp Gly Asn Glu Val Ile Tyr Ser Glu Arg Gln Thr Gly Lys Tyr
180 185 190

Cys Asp Lys Gly Glu His Ala Trp Gln Trp Asp Gly Tyr Ser Asn Gln
195 200 205

Gly Ile Leu Asp Thr Thr Lys Ile Lys Ser Lys Ser Leu Leu Val Arg
210 215 220

Leu Ile Ala Leu Cys Gly Asp Met Met Ile Lys Val Asp Tyr Pro Leu
225 230 235 240

Lys Thr Ser Ala Asn Glu Glu Asp Trp Val Asp Val Lys Val Asp Lys
245 250 255

Asn Gln Lys Ile Val Asp Val Ser Trp Arg Val Glu Phe Glu Asp Asp
260 265 270

Gly Val Ser His Lys Asp Asp Arg Ile Ala Pro Val Gly Phe Glu Lys
275 280 285

Leu Lys Gln Leu Ala Lys Asp Gly Met Glu Tyr Tyr Trp Ala Arg Asn
290 295 300

Gly Met Arg Asn Pro Gly Ile Gly Asn Asn Ile Thr Thr Pro His Gly
305 310 315 320

Lys Tyr Asn Val Asn Ile Ser Val Ser Ile Asn Ile Asp Pro Ala Met
325 330 335

Asp Ser Phe Asp Leu Ile Glu Glu Gln Asp Leu Glu Ser Val Arg Ser
340 345 350

Ser Ala Phe Met Gly Arg Met Asn Ile Tyr Phe Asn Arg Gly Tyr Tyr
355 360 365

Glu Tyr Gly Arg Gly Tyr Lys Lys Gly Ala Asp Pro Val Phe Lys Ala
370 375 380

Gly Leu Glu Phe Lys Leu Asp Val Ala His Glu Thr Gly His Met Ile
385 390 395 400

Leu Lys Ser Tyr Gly Gly Asn Thr Tyr Ser Trp Glu His Lys Gly Thr
405 410 415

Ser Asn Leu Val Pro Pro Gln Tyr Ala Leu Pro His Ser Val Tyr Pro
420 425 430

Gly Thr Gly Glu Ile Asp Leu Met Lys Tyr Tyr Asp Gly His Ile Tyr
435 440 445

Thr Ser Asp Leu Tyr Ala Arg Ser Val Ala Val Glu Asn Asp Val Cys
450 455 460

Ala Met Ile Trp Leu Ser Arg Val Lys Phe His Asp
465 470 475

<210> 39
<211> 182
<212> PRT
<213> Escherichia coli

<400> 39
Met Lys His Phe Ile Ala Thr Ala Val Met Ala Ser Ser Leu Leu Phe
1 5 10 15

Gly Ser Asn Ala Leu Ala Ala Ser Ser Ser Ala Ala Gln Lys Glu Ser
20 25 30

Ser Pro Glu Gln Ser Gly Gly Thr Phe Lys Tyr Ser Thr Asp Ser Lys
35 40 45

Ile Ala Thr Glu Gln Lys Asn Val Tyr Asp Thr Ile Ile Lys Tyr Gln
50 55 60

Asn Ala Leu Asn Ala Gly Asp Thr Lys Thr Ile Leu Gly Leu Phe Ala
65 70 75 80

Asp Glu Ser Tyr Ser Gln Trp Asn Asp Lys Leu Thr Ala Asp Asn Thr
85 90 95

Glu Lys Arg Gln Gly Gln Tyr Asp Asp Leu Phe Lys Arg Glu Lys Phe
100 105 110

Glu Thr Asp Phe Ala Phe Asp Ser Ile Trp Ile Asn Gly Asp Thr Ala
115 120 125

Val Val Arg Thr His His His Val Gly Ser Val Val Thr Asn Phe Lys
130 135 140

Glu Gln Lys Thr Ile Ile Asp Leu Asn Arg Glu Val Phe Val Leu Ser
145 150 155 160

Lys Ile Asn Gly Glu Trp Lys Ile Phe Leu Tyr Thr Phe Asn Thr Asn
165 170 175

Pro Leu Gln Gly Val Ala
180

<210> 40
<211> 102
<212> PRT
<213> Escherichia coli

<400> 40
Val Leu Phe Ser Asp Arg His Ile Thr Val Ser Ile Met Thr Met Pro
1 5 10 15

Ser Gly Gly Gly Ile Pro Val Ile Pro Cys His Asp Pro Val Thr Ser
20 25 30

Pro Gly Cys Leu Lys Pro Glu Arg Pro Gly Pro Thr Thr Leu Pro Val
35 40 45

Thr Gln Ser Arg Cys Ser Thr Pro Cys Ile Arg Leu Phe Cys Ala Glu
50 55 60

Gly Tyr Arg Lys Asp Asn Asn Val Thr Ala Arg Met His Gln Ile Lys
65 70 75 80

Gln Cys Ile Leu Ser Val Ser Ser His Trp Ser Gly Leu Trp Gly Glu
85 90 95

Ile Gln Val Leu Ile Leu
100

<210> 41
<211> 246
<212> PRT
<213> Escherichia coli

<400> 41
Met Asn Lys Val Phe Val Val Ser Val Val Ala Ala Ala Cys Val Phe
1 5 10 15

Ala Ala Asn Ala Gly Ala Lys Glu Gly Lys Ser Gly Phe Tyr Leu Thr
20 25 30

Gly Lys Ala Gly Ala Ser Val Val Ser Leu Ser Asp Gln Arg Phe Leu
35 40 45

Ser Gly Asp Glu Glu Glu Thr Ser Lys Tyr Lys Gly Gly Asp Asp His
50 55 60

Asp Thr Val Phe Ser Gly Gly Ile Ala Ala Gly Tyr Asp Phe Tyr Pro
65 70 75 80

Gln Phe Ser Ile Pro Val Arg Thr Glu Leu Glu Phe Tyr Ala Arg Gly
85 90 95

Lys Ala Asp Ser Lys Tyr Asn Val Asp Lys Asp Ser Trp Ser Gly Gly
100 105 110

Tyr Trp Arg Asp Asp Leu Lys Asn Glu Val Ser Val Asn Thr Leu Met
115 120 125

Leu Asn Ala Tyr Tyr Asp Phe Arg Asn Asp Ser Ala Phe Thr Pro Trp
130 135 140

Val Ser Ala Gly Ile Gly Tyr Ala Arg Ile His Gln Lys Thr Thr Gly
145 150 155 160

Ile Ser Thr Trp Asp Tyr Glu Tyr Gly Ser Ser Gly Arg Glu Ser Leu
165 170 175

Ser Arg Ser Gly Ser Ala Asp Asn Phe Ala Trp Ser Leu Gly Ala Gly
180 185 190

Val Arg Tyr Asp Val Thr Pro Asp Ile Ala Leu Asp Leu Ser Tyr Arg
195 200 205

Tyr Leu Asp Ala Gly Asp Ser Ser Val Ser Tyr Lys Asp Glu Trp Gly
210 215 220

Asp Lys Tyr Lys Ser Glu Val Asp Val Lys Ser His Asp Ile Met Leu
225 230 235 240

Gly Met Thr Tyr Asn Phe
245

<210> 42
<211> 335
<212> PRT
<213> Escherichia coli

<400> 42
Met Lys Lys Trp Phe Pro Ala Phe Leu Phe Leu Ser Leu Ser Gly Cys
1 5 10 15

Asn Asp Ala Leu Ala Ala Asn Gln Ser Thr Ile Phe Tyr Ser Phe Asn
20 25 30

Asp Asn Ile Tyr His Pro Gln Leu Ser Val Lys Val Thr Asp Ile Val
35 40 45

Gln Phe Ile Val Asp Ile Asn Ser Ala Ser Ser Thr Ala Thr Leu Ser
50 55 60

Tyr Val Ala Cys Asn Gly Phe Thr Trp Thr His Gly Leu Tyr Trp Ser
65 70 75 80

Glu Tyr Phe Ala Trp Leu Val Val Pro Lys His Val Ser Tyr Asn Gly
85 90 95

Tyr Asn Ile Tyr Leu Glu Leu Gln Ser Lys Gly Gly Phe Ser Leu Asp
100 105 110

Ala Glu Asp Asn Asp Asn Tyr Tyr Leu Thr Lys Gly Phe Ala Trp Asp
115 120 125

Glu Val Asn Ser Ser Gly Arg Val Cys Phe Asp Ile Gly Glu Lys Arg
130 135 140

Ser Leu Ala Trp Ser Phe Gly Gly Val Thr Leu Asn Ala Arg Leu Pro
145 150 155 160

Val Asp Leu Pro Lys Gly Asp Tyr Thr Phe Pro Val Lys Phe Leu Arg
165 170 175

Gly Ile Gln Arg Asn Asn Tyr Asp Tyr Ile Gly Gly Arg Tyr Lys Ile
180 185 190

Pro Ser Ser Leu Met Lys Thr Phe Pro Phe Asn Gly Thr Leu Asn Phe
195 200 205

Ser Ile Lys Asn Thr Gly Gly Cys Arg Pro Ser Ala Gln Ser Leu Glu
210 215 220

Ile Asn His Gly Asp Leu Ser Ile Asn Ser Ala Asn Asn His Tyr Ala
225 230 235 240

Ala Gln Thr Leu Ser Val Ser Cys Asp Val Pro Thr Asn Ile Arg Phe
245 250 255

Phe Leu Leu Ser Asn Thr Thr Pro Ala Tyr Ser His Gly Gln Gln Phe
260 265 270

Ser Val Gly Leu Gly His Gly Trp Asp Ser Ile Val Ser Ile Asn Gly
275 280 285

Val Asp Thr Gly Glu Thr Thr Met Arg Trp Tyr Arg Ala Gly Thr Gln
290 295 300

Asn Leu Thr Ile Gly Ser Arg Leu Tyr Gly Glu Ser Ser Lys Ile Gln
305 310 315 320

Pro Gly Val Leu Ser Gly Ser Ala Thr Leu Leu Met Ile Leu Pro
325 330 335

<210> 43
<211> 158
<212> PRT
<213> Escherichia coli

<400> 43
Val Leu Met Ser Gln His Ala His Ala Ala Asp Asn Leu Thr Phe Lys
1 5 10 15

Gly Lys Leu Ile Ile Pro Ala Cys Thr Val Gln Asn Ala Glu Val Asp
20 25 30

Trp Gly Asp Ile Glu Ile Gln Asn Leu Val Gln Asn Gly Gly Asn Gln
35 40 45

Lys Asp Phe Thr Val Asp Met Asn Cys Pro Tyr Ser Leu Gly Thr Met
50 55 60

Lys Val Thr Ile Thr Ser Asn Gly Gln Thr Gly Asn Ser Ile Leu Val
65 70 75 80

Pro Asn Thr Ser Thr Ala Ser Gly Asp Gly Leu Leu Ile Tyr Leu Tyr
85 90 95

Asn Ser Asn Asn Ser Gly Ile Gly Asn Ala Val Thr Leu Gly Ser Gln
100 105 110

Phe Thr Pro Gly Lys Ile Thr Gly Thr Ala Pro Ala Arg Lys Ile Thr
115 120 125

Leu Tyr Ala Lys Leu Gly Tyr Lys Gly Asn Met Gln Ser Leu Gln Ala
130 135 140

Gly Thr Phe Ser Ala Thr Ala Thr Leu Val Ala Ser Tyr Ser
145 150 155

<210> 44
<211> 182
<212> PRT
<213> Escherichia coli

<400> 44
Val Gln Ile Thr Glu Ala Leu Ile Ser Glu Pro Gly Asp Ile Arg Arg
1 5 10 15

Phe Ile Gln His Ala Val Asp His Trp Pro Arg Leu Leu Ala Val His
20 25 30

Phe Ile Leu His Ser Thr Glu Gly Asn Ile Tyr Gly Gln Gln Ile His

35 40 45

Ala Phe Cys Thr Ser Phe Tyr Arg Gln Leu His Glu Arg Ile Thr Glu
50 55 60

Ser Asn His Thr Ala Ser Pro Ser Ser Ser Val Val Leu Arg Trp Leu
65 70 75 80

Arg Glu Gln His Gly Gly Ala Thr Ile Arg Cys Leu Leu Leu Leu Ser
85 90 95

Gln Thr Ser Ile Cys His Pro Arg Ala Ser Val Thr Val Asp Glu Gln
100 105 110

Cys Ser Gln Val Val Asp Leu Leu Gln His Ser Trp Gln Val Ile Ser
115 120 125

Ala Gly Gly Gln Cys Arg Val Glu Arg Cys Phe Arg Val Ala Arg Gly
130 135 140

Asp Thr Ser Gly Gln Tyr Val Ala Leu Lys Thr Val Ala Leu Ser Leu
145 150 155 160

Gly Leu Pro Val Val Thr Ala Ile Thr His Arg Pro Val Gln Arg Cys
165 170 175

Thr Leu Ile Thr Ala Gln
180

<210> 45
<211> 145
<212> PRT
<213> Escherichia coli

<400> 45
Met Leu Leu Ser Gly Leu Asp Leu Ser Arg Trp Cys Glu Tyr His Leu
1 5 10 15

Met Gln Trp Ser Leu Ser Arg Gln Met Thr Ser Asn Arg Ala Arg Tyr
20 25 30

Leu Asn Gln Asp Leu Ile Val Leu Val Arg Cys Arg Lys Pro Val Ile
35 40 45

Phe Arg Val Gly Ile Cys Val Gly Ile Gly Trp Cys Cys Leu Leu Leu
50 55 60

Thr Ile Leu Asn His Glu Val Lys Ser His Val Arg Val Arg Pro Ser
65 70 75 80

Thr Thr Pro Tyr Ala Asn Arg Val Ala Gln Glu Arg Leu Phe Leu Cys
85 90 95

Leu Lys Ser Pro Ile Ser Val Gln Tyr Ile Gly Leu Pro Glu Lys Gln
100 105 110

Phe Ser Ile Leu His Phe Val Val Asp Gly Pro Lys Phe Ala Leu Ala
115 120 125

Gly Ser Cys Ile Val His Ile Thr Trp Ala Gly Trp Phe Arg Tyr Ala
130 135 140

Thr
145

```
<210>   46
<211>   109
<212>   PRT
<213>   Escherichia coli

<400>   46
Val Pro Gly Ser Asn Asn Asn Asn Leu Pro Arg Gln Ile Ser Lys Lys
1               5                   10                  15

Asn Ser Asp Thr Leu Ile Leu Glu Ser Cys Leu Phe Tyr Tyr Val Cys
            20                  25                  30

Arg Lys Lys Leu Arg Lys Arg Arg Thr Ile Asn Thr Gln Leu Ser His
        35                  40                  45

Thr Asp Trp Gln Arg Lys Cys Leu Gln Glu Gln Val Ile Cys His Gly
    50                  55                  60

Lys Ala Asn Gln Lys Tyr Arg Leu Ile Leu Val Phe Lys Pro Phe Leu
65                  70                  75                  80

Leu Val Ser Met Ile Gln Arg Leu Ser Lys Lys Leu Gly Asn Ser Thr
                85                  90                  95

Thr Leu Gly Asn Tyr Gly Leu Cys Pro Ala Ala Leu Ile
            100                 105

<210>   47
<211>   1624
<212>   PRT
<213>   Escherichia coli

<400>   47
Met Asn Lys Ile Phe Lys Val Ile Trp Asn Pro Ala Thr Gly Ser Tyr
1               5                   10                  15

Thr Val Ala Ser Glu Thr Ala Lys Ser Arg Gly Lys Lys Ser Gly Arg
            20                  25                  30

Ser Lys Leu Leu Ile Ser Ala Leu Val Val Gly Gly Leu Leu Ser Ser
        35                  40                  45

Gly Val Gln Ala Ser Thr Ala Leu Asp Gly Gly Gly Ala Ser Glu Ser
    50                  55                  60

Thr Pro Leu Thr Asp Thr Trp Ile Ala Ile Gly Gln Gly Ala Lys Ala
65                  70                  75                  80

Ser Ser Val Asp Thr Ser Pro Ser Gly Gly Pro Gly Thr Ser Ser Ile
                85                  90                  95

Ala Ile Gly Gln Glu Ala Gln Ser Gly Ile Gly Ser Thr Ser Leu Gly
            100                 105                 110

Phe Lys Ser Gln Ala Thr Gly Glu Arg Ser Val Ala Leu Gly Gln Thr
        115                 120                 125

Ala Asp Ala Thr Gly Asn Arg Ala Ile Ala Ile Gly Ser Gly Ala Lys
    130                 135                 140

Ser Thr Ala Asp Tyr Ser Leu Thr Leu Gly Asn Gly Ser Lys Ala Phe
145                 150                 155                 160

Ala Glu Asn Ala Thr Ala Ile Gly Lys Asp Ala Arg Ala Glu Gly Lys
                165                 170                 175
```

Gly Ser Gln Ala Phe Gly Lys Val Ala Leu Ala Thr Gly Glu Asn Ala
180 185 190

Ile Ala Leu Gly Ser Asn Ser Asn Ala Ser Gly Asp Asn Ala Val Ala
195 200 205

Leu Gly Ser Thr Ser Gln Ala Tyr Gly Asp Ser Ser Leu Ala Ile Gly
210 215 220

Glu Leu Ser Glu Thr Asn Ala Glu Asn Ala Ile Ala Leu Gly Lys Leu
225 230 235 240

Ser Asn Ala Ser Lys Ile Asn Ser Ile Ala Leu Gly Ser Asn Ser Thr
245 250 255

Ala Ser Gly Glu Gly Ser Val Ala Leu Gly Glu Asn Ser Phe Ala Gly
260 265 270

Gly Ile Asn Ser Leu Ala Leu Gly Ser Gln Ser Asn Ala Asn Gly Asp
275 280 285

Asn Ala Val Ala Leu Gly Val Gly Ser Val Ala Glu Gln Asp Asn Thr
290 295 300

Val Ser Val Gly Asn Ser Thr Thr Gln Arg Lys Ile Thr Asn Met Ala
305 310 315 320

Ala Gly Leu Ile Gln Pro Gly Ser Thr Glu Ala Ile Asn Gly Ser Gln
325 330 335

Leu His Ala Leu Ser Ala Ser Val Ala Ala Arg Leu Gly Ala Gly Ala
340 345 350

Gly Val Asn Gly Asp Gly Ser Val Asn Ala Pro Ser Tyr Lys Leu Lys
355 360 365

Ser Gly Ile Tyr Asn Asn Val Gly Glu Ala Leu Leu Gly Ile Asp Asn
370 375 380

Asp Thr Leu His Trp Asp Lys Thr Glu Gly Ala Phe Ser Ala Ser Tyr
385 390 395 400

Ile Val Lys Asn Ala Asp Gly Ser Pro Lys Ser Ser Ile Ala Gln Asn
405 410 415

Lys Ile Ile Asn Val Ala Lys Gly Thr Ile Ser Ala Thr Ser Thr Asp
420 425 430

Val Val Asn Gly Ser Gln Leu Tyr Asp Leu Gln Gln Asp Ala Leu Leu
435 440 445

Trp Asn Gly Thr Ala Phe Ser Ala Ala His Gly Ser Asp Thr Thr Ser
450 455 460

Lys Ile Thr Asn Val Ala Lys Gly Thr Val Ser Ala Thr Ser Thr Asp
465 470 475 480

Val Val Asn Gly Ser Gln Leu Tyr Asp Leu Gln Gln Asp Ala Leu Leu
485 490 495

Trp Asn Gly Thr Ala Phe Ser Ala Ala His Gly Thr Glu Ala Thr Ser
500 505 510

Lys Ile Thr Asn Val Glu Asp Gly Thr Ile Ser Asp Thr Ser Lys Asp

```
        515                 520                 525

Ala Val Asn Gly Ser Gln Leu Lys Glu Thr Lys Asp Asp Val Ala Thr
    530                 535                 540

Asn Thr Ala Asn Ile Ala Asp Asn Thr Ala Asp Ile Ala Thr Asn Thr
545                 550                 555                 560

Ala Asn Ile Ala Asp Asn Thr Thr Asp Ile Ala Thr Asn Thr Ala Asn
                565                 570                 575

Ile Ala Thr Asn Thr Thr Asp Ile Ala Thr Asn Thr Ala Asn Ile Ala
            580                 585                 590

Asp Asn Thr Ala Asp Ile Ala Thr Asn Thr Thr Asp Ile Ala Thr Asn
        595                 600                 605

Thr Ala Asn Ile Ala Asp Asn Thr Ala Asn Ile Ala Thr Asn Thr Thr
    610                 615                 620

Asp Ile Ala Thr Asn Thr Ala Asp Ile Ala Thr Asn Ala Ser Ser Ile
625                 630                 635                 640

Thr Thr Leu Asn Ala Asp Val Asp Thr Leu Glu Lys Asp Ala Leu Leu
                645                 650                 655

Trp Asn Gly Thr Ala Phe Ser Ala Lys His Gly Thr Glu Ala Thr Ser
            660                 665                 670

Lys Ile Thr Asn Val Thr Ala Gly Asp Leu Thr Ala Gly Ser Thr Asp
        675                 680                 685

Ala Val Asn Gly Ser Gln Leu Lys Thr Thr Asn Asp Asn Val Ser Thr
    690                 695                 700

Asn Thr Thr Asn Ile Thr Asn Leu Thr Asp Ser Val Gly Asp Leu Lys
705                 710                 715                 720

Asp Asp Ser Leu Leu Trp Asn Lys Thr Ala Gly Ala Phe Ser Ala Ala
                725                 730                 735

His Gly Thr Glu Ala Thr Ser Lys Ile Thr Asn Leu Leu Ala Gly Lys
            740                 745                 750

Val Ser Ser Asp Ser Thr Asp Ala Ile Asn Gly Ser Gln Leu Tyr Gly
        755                 760                 765

Val Ala Asp Ser Phe Thr Ser Tyr Leu Gly Gly Gly Ala Asp Ile Ser
    770                 775                 780

Asp Ala Gly Val Leu Thr Gly Pro Thr Tyr Thr Ile Gly Gly Thr Asp
785                 790                 795                 800

Tyr Thr Asn Val Gly Asp Ala Leu Ala Ala Ile Asn Thr Ser Phe Ser
                805                 810                 815

Ser Ser Leu Gly Asp Ala Leu Leu Trp Asp Ala Thr Ala Gly Lys Phe
            820                 825                 830

Ser Ala Lys His Gly Ile Asn Asn Asp Pro Ser Val Ile Thr Asp Val
        835                 840                 845

Ala Asn Gly Ala Val Ser Ser Thr Ser Ser Asp Ala Ile Asn Gly Ser
    850                 855                 860
```

```
Gln Leu Tyr Gly Val Ser Asp Tyr Ile Ala Asp Ala Leu Gly Gly Asn
865                 870                 875                 880

Ala Val Val Asn Ala Asp Gly Ser Ile Thr Thr Pro Thr Tyr Ala Ile
                885                 890                 895

Ala Gly Gly Ser Tyr Asp Asn Val Gly Asp Ala Leu Glu Ala Ile Asp
            900                 905                 910

Thr Thr Leu Asp Asp Ala Leu Leu Trp Asp Glu Thr Ala Asn Gly Gly
        915                 920                 925

Asn Gly Ala Phe Ser Ala Ala His Gly Lys Asp Lys Thr Ala Ser Val
    930                 935                 940

Ile Thr Asn Val Ala Asn Gly Ala Val Ser Ala Thr Ser Ser Asp Ala
945                 950                 955                 960

Ile Asn Gly Ser Gln Leu Tyr Ser Thr Asn Lys Tyr Ile Ala Asp Ala
                965                 970                 975

Leu Gly Gly Asp Ala Glu Val Asn Ala Asp Gly Thr Ile Thr Ala Pro
            980                 985                 990

Thr Tyr Thr Ile Ala Asn Thr Asp Tyr Asn Asn Val Gly Glu Ala Leu
        995                 1000                1005

Asp Ala Leu Asp Asn Asn Ala Leu Leu Trp Asp Glu Asp Ala Gly Ala
    1010                1015                1020

Tyr Asn Ala Ser His Asp Gly Asn Ala Ser Lys Ile Thr Asn Val Ala
1025                1030                1035                1040

Ala Gly Asp Leu Ser Thr Thr Ser Thr Asp Ala Val Asn Gly Ser Gln
                1045                1050                1055

Leu Asn Ala Thr Asn Ile Leu Val Thr Gln Asn Ser Gln Met Ile Asn
            1060                1065                1070

Gln Leu Ala Gly Asn Thr Ser Glu Thr Tyr Ile Glu Asp Asn Gly Ala
        1075                1080                1085

Ser Ile Asn Tyr Val Arg Thr Asn Asp Asn Gly Leu Ala Phe Asn Asp
    1090                1095                1100

Ala Ser Ala Ser Gly Ile Gly Ala Thr Ala Val Gly Tyr Asn Ala Val
1105                1110                1115                1120

Ala Ser Gly Glu Ser Ser Val Ala Ile Gly Gln Gly Ser Ser Ser Thr
                1125                1130                1135

Val Asp Thr Gly Ile Ala Leu Gly Ser Ser Ser Val Ser Ser Arg Val
            1140                1145                1150

Ile Thr Lys Gly Ser Arg Asp Thr Ser Val Ser Glu Glu Gly Val Val
        1155                1160                1165

Ile Gly Tyr Asp Thr Thr Asp Gly Glu Leu Leu Gly Ala Leu Ser Ile
    1170                1175                1180

Gly Asp Asp Gly Lys Tyr Arg Gln Ile Ile Asn Val Ala Asp Gly Ser
1185                1190                1195                1200

Glu Ala His Asp Ala Val Thr Val Arg Gln Leu Gln Asn Ala Ile Gly
                1205                1210                1215
```

Ala Val Ala Thr Thr Pro Thr Lys Tyr Tyr His Ala Asn Ser Thr Ala
1220 1225 1230

Glu Asp Ser Leu Ala Val Gly Glu Asp Ser Leu Ala Met Gly Ala Lys
1235 1240 1245

Thr Ile Val Asn Gly Asn Ala Ser Ile Gly Ile Gly Leu Asn Thr Leu
1250 1255 1260

Val Leu Ala Asp Ala Ile Asn Gly Ile Ala Ile Gly Ser Asn Ala Arg
1265 1270 1275 1280

Ala Asn His Ala Asp Ser Ile Ala Met Gly Asn Gly Ser Gln Thr Thr
1285 1290 1295

Arg Gly Ala Gln Thr Asn Tyr Thr Ala Tyr Asn Met Asp Ala Pro Gln
1300 1305 1310

Asn Ser Val Gly Glu Phe Ser Val Gly Ser Glu Asp Gly Gln Arg Gln
1315 1320 1325

Ile Thr Asn Val Ala Ala Gly Ser Ala Asp Thr Asp Ala Val Asn Val
1330 1335 1340

Ser Gln Leu Lys Val Thr Asp Glu Arg Val Ala Gln Asn Thr Gln Asn
1345 1350 1355 1360

Ile Ser Asn Leu Asn Asn Gln Val Thr Asn Leu Asp Thr Arg Val Thr
1365 1370 1375

Asn Ile Glu Asn Gly Ile Gly Asp Ile Val Thr Thr Gly Ser Thr Lys
1380 1385 1390

Tyr Phe Lys Thr Asn Thr Asp Gly Ala Asp Ala Asn Ala Gln Gly Lys
1395 1400 1405

Asp Ser Val Ala Ile Gly Ser Gly Ser Ile Ala Ala Ala Asp Asn Ser
1410 1415 1420

Val Ala Leu Gly Thr Gly Ser Val Ala Asp Glu Glu Asn Thr Ile Ser
1425 1430 1435 1440

Val Gly Ser Ser Thr Asn Gln Arg Arg Ile Thr Asn Val Ala Ala Gly
1445 1450 1455

Val Asn Ala Thr Asp Ala Val Asn Val Ser Gln Leu Lys Ser Ser Glu
1460 1465 1470

Ala Gly Gly Val Arg Tyr Asp Thr Lys Ala Asp Gly Ser Ile Asp Tyr
1475 1480 1485

Ser Asn Ile Thr Leu Gly Gly Gly Asn Ser Gly Thr Thr Arg Ile Ser
1490 1495 1500

Asn Val Ser Ala Gly Val Asn Asn Asn Asp Ala Val Asn Tyr Ala Gln
1505 1510 1515 1520

Leu Lys Gln Ser Val Gln Glu Thr Lys Gln Tyr Thr Asp Gln Arg Met
1525 1530 1535

Val Glu Met Asp Asn Lys Leu Ser Lys Thr Glu Ser Lys Leu Ser Gly
1540 1545 1550

Gly Ile Ala Ser Ala Met Ala Met Thr Gly Leu Pro Gln Ala Tyr Thr

1555 1560 1565

Pro Gly Ala Ser Met Ala Ser Ile Gly Gly Gly Thr Tyr Asn Gly Glu
1570 1575 1580

Ser Ala Val Ala Leu Gly Val Ser Met Val Ser Ala Asn Gly Arg Trp
1585 1590 1595 1600

Val Tyr Lys Leu Gln Gly Ser Thr Asn Ser Gln Gly Glu Tyr Ser Ala
1605 1610 1615

Ala Leu Gly Ala Gly Ile Gln Trp
1620

<210> 48
<211> 306
<212> PRT
<213> Escherichia coli

<400> 48
Met Tyr Glu Val Cys Val Thr Phe Tyr Met Arg Val Lys Gln Val Ile
1 5 10 15

Thr Thr Phe Ser Lys Gly Tyr Ser Gln Val Leu Leu Gln Asn Asn Val
20 25 30

Val Ser Gly Leu Phe Phe Phe Leu Ala Thr Gly Ile Ala Ser Phe Asn
35 40 45

Met Gly His Pro Glu Ile Leu Tyr Phe Ser Ala Ile Ser Ala Ala Leu
50 55 60

Ser Pro Phe Phe Ala Trp Cys Leu Arg Tyr Pro Asp Glu Glu Ile Asn
65 70 75 80

Glu Gly Ile Trp Gly Tyr Asn Ala Val Leu Tyr Gly Ile Ala Cys Gly
85 90 95

Met Val Val Pro Val Ser Val Ser Gly Ile Thr Val Leu Ile Val Gly
100 105 110

Ile Leu Gly Ile Val Leu Phe Thr Pro Leu Leu Ser Ser Val Leu Arg
115 120 125

Gly Leu Pro Val Leu Thr Ala Pro Phe Ile Ile Ala Thr Trp Leu Leu
130 135 140

His Ser Gly Ile Met Pro His Asp Met Ser Val Pro Ser Gly Glu Gly
145 150 155 160

Ala Gly Ser Val Leu Leu Lys Asn Tyr Pro Val Ala Phe Ser Gly Asn
165 170 175

Phe Met Glu Ile Phe Met Phe Pro Asp Ala Val Gly Gly Ile Leu Val
180 185 190

Met Leu Gly Leu Leu Ala Gly Ser Arg Lys Leu Leu Met Ile Thr Val
195 200 205

Val Ala Ser Phe Leu Ser Val Val Val Ser Phe Cys Ile Pro Glu Leu
210 215 220

Ser Pro Asp Lys Ile Ser Ala Gly Leu Tyr Gly Tyr Asn Ala Ile Leu
225 230 235 240

```
Thr Ala Val Ala Val Tyr Ala Phe Ala Gly Asp Asn Gln Phe Asp Asn
                245                 250                 255

Ile Phe Leu Gly Cys Gly Ala Ile Ile Leu Ala Phe Ile Ile Pro Val
            260                 265                 270

Leu Ile Gly Pro Leu Ile Pro Val Pro Leu Leu Thr Ser Pro Phe Val
        275                 280                 285

Ile Ser Thr Trp Leu Tyr Ile Leu Ala Gln Arg Arg Cys Met Ser Ser
    290                 295                 300

His Ile
305

<210>   49
<211>   133
<212>   PRT
<213>   Escherichia coli

<400>   49
Met Ser Ser Lys Thr Lys Cys Trp Leu Trp Met Leu Leu Val Ile Leu
1               5                   10                  15

Ser Glu Thr Ser Ala Thr Ser Thr Leu Lys Met Phe Asp Asn Ser Glu
            20                  25                  30

Gly Met Thr Lys Thr Leu Leu Leu Ala Leu Ile Val Val Leu Tyr Cys
        35                  40                  45

Ile Cys Tyr Tyr Ser Leu Ser Arg Ala Val Lys Asp Ile Pro Val Gly
    50                  55                  60

Leu Ala Tyr Ala Thr Trp Ser Gly Thr Gly Ile Leu Met Val Ser Thr
65                  70                  75                  80

Leu Gly Ile Leu Phe Tyr Gly Gln His Pro Asp Thr Ala Ala Ile Ile
                85                  90                  95

Gly Met Val Ile Ile Ala Ser Gly Ile Ile Ile Met Asn Leu Phe Ser
            100                 105                 110

Lys Met Gly Ser Glu Glu Ala Glu Glu Thr Pro Val Thr Asn Leu Asp
        115                 120                 125

Lys Lys Ile Ala Asn
    130

<210>   50
<211>   386
<212>   PRT
<213>   Escherichia coli

<400>   50
Met Ser Ile Lys Leu Arg Gly Gly Thr Trp His Cys Asp Phe Val Ala
1               5                   10                  15

Pro Asp Gly Ser Arg Val Arg Arg Ser Leu Glu Thr Ser Asp Lys Arg
            20                  25                  30

Gln Ala Gln Glu Leu His Asp Arg Leu Lys Ala Glu Ala Trp Arg Val
        35                  40                  45

Lys Asn Leu Gly Glu Ser Pro Lys Lys Leu Phe Lys Glu Ala Cys Ile
    50                  55                  60
```

```
Arg Trp Leu Arg Glu Lys Ser Asp Lys Lys Ser Ile Asp Asp Asp Lys
65                  70                  75                  80

Ser Ile Ile Ser Phe Trp Met Leu His Phe Arg Glu Thr Ile Leu Ser
                85                  90                  95

Asp Ile Thr Thr Glu Lys Ile Met Glu Ala Val Asp Gly Met Glu Asn
            100                 105                 110

Arg Arg His Arg Leu Asn Trp Glu Met Ser Arg Asp Arg Cys Leu Arg
        115                 120                 125

Leu Gly Lys Pro Val Pro Glu Tyr Lys Pro Lys Leu Ala Ser Lys Gly
    130                 135                 140

Thr Lys Thr Arg His Leu Ala Ile Leu Arg Ala Ile Leu Asn Met Ala
145                 150                 155                 160

Val Glu Trp Gly Trp Leu Asp Arg Thr Pro Lys Ile Ser Thr Pro Arg
                165                 170                 175

Val Lys Asn Gly Arg Ile Arg Trp Leu Thr Glu Glu Glu Ser Lys Arg
            180                 185                 190

Leu Phe Ala Glu Ile Ala Pro His Phe Phe Pro Val Val Met Phe Ala
        195                 200                 205

Ile Thr Thr Gly Leu Arg Arg Ser Asn Val Thr Asp Leu Glu Trp Ser
    210                 215                 220

Gln Val Asp Leu Asp Lys Lys Met Ala Trp Met His Pro Asp Glu Thr
225                 230                 235                 240

Lys Ala Gly Asn Ala Ile Gly Val Pro Leu Asn Glu Thr Ala Cys Gln
                245                 250                 255

Ile Leu Arg Lys Gln Gln Gly Leu His Lys Arg Trp Val Phe Val His
            260                 265                 270

Thr Lys Pro Ala Tyr Arg Ser Asp Gly Thr Lys Thr Ala Ala Val Arg
        275                 280                 285

Lys Met Arg Thr Asp Ser Asn Lys Ala Trp Lys Gly Ala Leu Lys Arg
    290                 295                 300

Ala Gly Ile Ser Asn Phe Arg Phe His Asp Leu Arg His Thr Trp Ala
305                 310                 315                 320

Ser Trp Leu Val Gln Ser Gly Val Ser Leu Leu Ala Leu Lys Glu Met
                325                 330                 335

Gly Gly Trp Glu Thr Leu Glu Met Val Gln Arg Tyr Ala His Leu Ser
            340                 345                 350

Ala Gly His Leu Thr Glu His Ala Ser Lys Ile Asp Ala Ile Ile Ser
        355                 360                 365

Arg Asn Gly Thr Asn Thr Ala Gln Glu Glu Asn Val Val Tyr Leu Asn
    370                 375                 380

Val Arg
385
```

<210> 51

<211> 583
<212> PRT
<213> Escherichia coli

<400> 51

```
Met Tyr Phe Pro Leu Ile His Tyr Gly Ile Ile Tyr Cys Asp Thr Leu
1               5                   10                  15

Leu Ser His Pro Gln Lys His Leu Pro Lys Arg Lys Leu Thr Ala Gly
            20                  25                  30

Gln Ser Phe Ala Gly Leu Ala Trp Lys Gln Tyr His Val Thr Asp Thr
        35                  40                  45

Ala Gln His Arg Leu His Leu Arg Arg Phe Val Asn Met Asn Gln Pro
    50                  55                  60

His Ala Asp Asn Leu Gln Gly Gln His Pro Asp Arg Ala Ile Cys Ser
65                  70                  75                  80

Ala Asp Glu Asp Gln Tyr Gly Phe Ile His Ile Ala Thr Gln Leu Ala
                85                  90                  95

Val Ala Val Lys Gly Ile Gly Arg Glu Gly Ser Ala Val Ile Gly Ile
            100                 105                 110

Glu Gly Pro Trp Gly Ser Gly Lys Thr Ser Leu Leu Asn Leu Leu Arg
        115                 120                 125

Asn Ala Leu Val Glu Gln Ile Glu Glu Cys Thr Phe Val Leu Thr Ile
    130                 135                 140

Ser Pro Trp Leu Asp Gly Ser Asn Thr Ser Leu Val Ala Ser Leu Leu
145                 150                 155                 160

Leu Pro Val Ala Asn Ile Ile Ala Ala Glu Glu Glu Gln Arg Leu Ala
                165                 170                 175

Pro Glu Glu Arg Ala Ala Leu Arg Arg Arg Lys Ser Leu Thr Arg Thr
            180                 185                 190

Ala Lys Thr Met Ile Asp Tyr Thr Arg Ala Thr Ala Arg Asn Leu Ala
        195                 200                 205

Thr Val Ala Ser Ala Ala Ala Val Ile Pro Gly Val Pro Asp Ala Ser
    210                 215                 220

Gly Ala Leu Lys Ala Leu Ser Glu Thr Arg Trp Leu Lys Glu Lys Glu
225                 230                 235                 240

Lys Thr Thr Ala Glu Met Arg Thr Glu Ile Ala Lys Lys Ile Asp Glu
                245                 250                 255

Leu Asp Leu Ser Phe Ile Val Leu Leu Asp Asp Leu Asp Arg Leu Glu
            260                 265                 270

Pro Ala Gln Ala Val Glu Val Ile Arg Leu Val Lys Ser Val Gly Asp
        275                 280                 285

Phe Pro Arg Phe Arg Tyr Leu Leu Cys Tyr Asp Lys Ala Ile Leu Ser
    290                 295                 300

Gln Ala Ile Ser Leu Gly Leu Gly Val Pro Asp Gly Asn Leu Tyr Leu
305                 310                 315                 320
```

Gln Lys Ile Val Gln Ile Ser Phe Cys Leu Pro Arg Pro Glu Thr Phe
325 330 335

Val Leu Arg Arg Lys Phe Arg Asp Ala Ala Ala Ala Leu Tyr Arg Lys
340 345 350

Val Asn Gly His Ala Pro Asp Arg Ile Val Leu Glu Gln Leu Thr Gln
355 360 365

Val Ala Asp Val Tyr Gly Ala Ala Leu Lys Thr Pro Arg Glu Val Gln
370 375 380

Met Val Leu Asn Ala Leu Thr Phe Leu Tyr Pro Gly Met Arg Asp Tyr
385 390 395 400

Val Tyr Phe Pro Asp Leu Cys Phe Leu Gln Leu Leu Arg Thr Thr Asn
405 410 415

Ala Gly Leu Tyr Asp Trp Val Glu Glu Tyr Leu Ser Glu Arg Ala Val
420 425 430

Val Ala Ala Gly Asp Gly His Val Ser Glu Leu Glu Gln Ala Glu Met
435 440 445

Ala Glu Ser Leu Lys Ser His Leu Ala Arg Tyr Phe Pro Ala Glu Ala
450 455 460

His Ala Val His Ala Leu Ala Arg Trp Val Pro Gly Ile Thr Gly Trp
465 470 475 480

Lys Thr Asp Ser Pro Ile Lys Leu Phe Val Pro Thr Pro Glu Gln Asp
485 490 495

Ser Ala Leu Leu Thr Ala Gly Lys Arg Leu Gly Ser Gln Ala Tyr Trp
500 505 510

Leu Tyr Tyr Phe Ala Phe Ser Ala Pro Gln Asn Val Leu Ser Pro Glu
515 520 525

Met Leu Asn Glu Ile Phe Asp Ile Ala Gly Tyr Pro Glu Gln Gln His
530 535 540

Arg Leu Ala Glu Arg Leu Leu Gly Tyr Ile Gln Ser Lys Gly Leu Ser
545 550 555 560

Ser Arg Thr Trp Phe Glu His Ile Leu Thr Gln Leu Thr Thr Gln Gln
565 570 575

Ile Ala Thr Val Asn Asp Gly
580

<210> 52
<211> 240
<212> PRT
<213> Escherichia coli

<400> 52
Leu Arg Leu Tyr Met Thr Val Phe Ser Phe Ile Lys Glu Val Leu Ser
1 5 10 15

Pro His Ile Arg Leu Leu Tyr Ser Pro Gln Glu Leu Gly Phe Asn Asp
20 25 30

Ala Gly Leu Ile Val Leu Asn His Phe Gly Tyr Asp Asn Asn Ile Tyr
35 40 45

Tyr Ser Val Thr Leu Thr Arg Lys Glu Ile Thr Ser Phe Gln Asn Thr
    50                  55                  60

Ser Glu Asn Thr His Tyr Phe Ala Tyr Ser Lys Thr Ile Lys Lys Thr
65                  70                  75                  80

Ile Asp Arg Ile Phe Lys Ile Thr Gly Glu Lys Ile Thr Ile Gly Asn
                85                  90                  95

Ile Thr Pro Ser Ile Asn Gly Lys Val Ser Val Thr Leu Ile Leu Ser
            100                 105                 110

Leu Leu Glu Met Lys Leu Ile Asp Phe Ile Ala Asp Asp Asn Thr Asp
        115                 120                 125

Val Arg Val Arg Leu Thr Val Leu Asn Met Leu Asn Asn Asn Thr Thr
    130                 135                 140

Thr Ile Arg Glu Leu Ala Ala Ser Val Tyr Leu Gln Glu Asp Tyr Leu
145                 150                 155                 160

Lys Arg Ile Phe Arg Arg Tyr Thr Gly Thr Gly Leu Tyr Gln Phe Tyr
                165                 170                 175

Asn Trp Ile Lys Leu Leu Lys Ala Ile Ser Lys Ile Gln Asn Asn Asp
            180                 185                 190

Lys Ile Lys Lys Ile Ala Ile Glu Leu Gly Tyr Ser Ser Ser Ser Ser
        195                 200                 205

Phe Asn Tyr Ser Phe Lys Lys Glu Phe Ser Ile Gly Pro Arg Asp Tyr
    210                 215                 220

Ile Lys Glu Tyr Leu His Asn Lys Ile Ser Cys Met Ala Val His Glu
225                 230                 235                 240

<210> 53
<211> 197
<212> PRT
<213> Escherichia coli

<400> 53
Leu Asn Ile Leu Ala Pro Ile Phe His Asp Cys Ser Ser Leu Ser Leu
1               5                   10                  15

Gly Leu Leu Cys Ala Arg Ser Gly Leu Leu Thr Gly Phe Asp Val Arg
            20                  25                  30

Tyr Trp Ala Ile Arg Ile Ala Ala Thr Lys Met Arg Ala Arg Leu Met
        35                  40                  45

Asn Tyr Phe Ile Arg Pro Ala Thr Pro Asp Asp Ile Asn Lys Ile Ile
    50                  55                  60

Glu Phe Thr Thr Gln Glu Ala Gln Glu Ser Glu Gly Ser Val Ile Asp
65                  70                  75                  80

Leu Val Ala Val Glu Arg Gly Val Ser Ser Ala Phe Ser Glu Asn Pro
                85                  90                  95

Arg Ser Lys Tyr Trp Val Met Glu Asp Ser Ser Gly Thr Val Ile Ala
            100                 105                 110

```
Asn Thr Ser Val Val Lys Glu Trp Ser Asp Phe His Gly Asn Asp Tyr
        115                 120                 125

Trp Trp Ile Lys Ser Phe Tyr Ile Ala Pro Glu His Arg Gly Met Gly
    130                 135                 140

Leu Ala Asp Glu Leu Ile Lys His Leu Ile Lys Glu Ala Lys Ser Glu
145                 150                 155                 160

Lys Ala Leu Glu Leu Arg Leu Tyr Val His Gly Asp Asn Gly Arg Ala
                165                 170                 175

Ile Arg Ala Tyr Glu Arg Cys Gly Phe Ile Glu Ser Pro Tyr Thr Ile
            180                 185                 190

Met Lys Val Gly Leu
        195

<210>   54
<211>   340
<212>   PRT
<213>   Escherichia coli

<400>   54
Met Ile Arg Tyr Ser Gly Ile Phe Leu Ser Leu Leu Met Leu Ser Gly
1               5                   10                  15

Cys Ala Thr Glu Ala Leu Lys Asn Glu Gln Ala Glu Thr Val Ile Thr
            20                  25                  30

Ala Gly Asn Lys Ala Val Ala Ala Asn Ala Ser Phe Phe Lys Gln Tyr
        35                  40                  45

Tyr Ala Ser Arg Glu Asn Phe Leu Val Asn Phe Tyr Ala Thr Asn Pro
    50                  55                  60

Asp Cys Ala Leu Arg Pro Asp Tyr Glu Thr Ile Met Glu Asn Asn Thr
65                  70                  75                  80

Asn Lys Glu Ser Leu Cys Leu Gln Ser Glu Lys Gly Gly Lys Thr Ser
                85                  90                  95

Tyr Lys Asp Ile Thr Lys Ile Gln Leu Ala Thr Thr Glu Lys Gln Lys
            100                 105                 110

Leu Leu Ala Ser Leu Glu Val Val Asn Ser Met Ser Ser Tyr Val Glu
        115                 120                 125

Leu Leu Thr Val Gly Met Asp Val Glu Asp Ser Lys Gln Ile Ile Leu
    130                 135                 140

Leu Asn Gln Ala Leu Asp His Ala Asn Asn Ala Gln Lys Leu Leu Gly
145                 150                 155                 160

Phe Ser Glu Gly Glu Glu Leu Thr Lys Ser Ala Val Ala Val Lys Asp
                165                 170                 175

Leu Val Gln Tyr Phe Asn Gly Leu Tyr Lys Glu Lys Ile Lys Ser Glu
            180                 185                 190

Asn Val Gly Lys Ala Val Gly Ser Asp Trp Pro Arg Leu Gln Arg Asn
        195                 200                 205

Leu Gln Asp Leu Leu Asp Asp Ile Ala Thr Lys Gln Gln Gln Met Asp
    210                 215                 220
```

Arg Asn Asn Phe Ala Asn Ile Ser Asn Asn Phe Tyr Tyr Tyr Asn Lys
225 230 235 240

Ala Arg Ala Asn Thr Lys Asn Ile Glu Phe Lys Thr Leu Lys Ser Arg
245 250 255

Gln Glu Phe Leu Ser Ala Val Ile Asn Leu Asn Glu Ser Arg Leu Arg
260 265 270

Ser Ser Asp Pro Ala Ala Pro Ala Ile Gln Ala Ile Asn Ala Phe Met
275 280 285

Asp Lys Gly Asn His Leu Gln Asp Leu Tyr Ser Asn Lys Ser Leu Ser
290 295 300

Lys Ala Asp Leu Glu Arg Arg Asn Glu Ile Leu Arg Ala Gln Val Ile
305 310 315 320

Ser Gly Leu Gln Ala Ser Glu Glu Leu Ala Lys Leu Leu Ile Ala Ser
325 330 335

Gly Val Met Leu
340

<210> 55
<211> 102
<212> PRT
<213> Escherichia coli

<400> 55
Met Asn Asp Val Gln Asn Arg Arg Ile Leu Ala Leu Leu Asn Met Leu
1 5 10 15

Tyr Tyr Ala Leu Gly Asn Arg Thr Thr Cys Asp Ile Cys His Lys Tyr
20 25 30

Phe Ile Leu Val Leu Ser Val Met Asn Asn Leu Thr Ile Asn Tyr Gly
35 40 45

Ser Gln Thr Ser Pro Glu Leu Thr Gly Ile Ser Tyr Glu Asp Ala Asn
50 55 60

Asp Leu Ile Thr Glu Leu Ile Met Phe Ile Glu Asp Val Asn Ala Asp
65 70 75 80

Asn Asp Met Glu Tyr Pro Ala His Leu Asn Lys Ile Gln Val Leu Leu
85 90 95

Asn Phe Ala Val Ala Asn
100

<210> 56
<211> 2458
<212> PRT
<213> Escherichia coli

<400> 56
Met Gln Arg Lys Thr Leu Leu Ser Ala Cys Ile Ala Leu Ala Leu Ser
1 5 10 15

Gly Gln Gly Trp Ala Ala Asp Ile Thr Glu Ile Glu Thr Thr Thr Gly
20 25 30

Glu Lys Lys Asn Thr Asn Val Thr Cys Pro Ala Asp Pro Gly Lys Leu

```
        35                  40                  45

Ser Pro Glu Glu Leu Lys Arg Leu Pro Ser Glu Cys Ser Ser Val Val
    50                  55                  60

Glu Gln Asn Leu Met Pro Trp Leu Val Thr Gly Ala Ala Thr Ala Leu
65                  70                  75                  80

Ile Thr Thr Leu Ala Ile Val Glu Leu Asn Asp Asp Asp Asp His His
                85                  90                  95

Arg Asn Asn Ser Pro Leu Pro Pro Thr Pro Pro Asp Asp Asp Ser Asp
            100                 105                 110

Asp Thr Pro Val Pro Pro Thr Pro Gly Gly Asp Glu Ile Ile Pro Asp
        115                 120                 125

Asp Gly Pro Asp Asp Thr Pro Ala Pro Pro Lys Pro Ile Ala Phe Asn
    130                 135                 140

Asn Asp Val Thr Leu Asp Lys Thr Ala Lys Thr Leu Thr Ile Arg Asp
145                 150                 155                 160

Ser Val Phe Ser Tyr Thr Glu Asn Ala Asp Gly Thr Ile Ser Leu Gln
                165                 170                 175

Asp Ser Asn Gly Arg Lys Ala Thr Ile Asn Leu Trp Gln Ile Asp Glu
            180                 185                 190

Thr Asn Asn Thr Val Ala Leu Glu Gly Met Ser Ala Asp Gly Ala Thr
        195                 200                 205

Lys Trp Gln Tyr Asn His Asn Gly Glu Leu Val Ile Thr Gly Asp Asn
    210                 215                 220

Thr Thr Val Asn Asn Thr Gly Lys Thr Ile Val Asp Gly Lys Gly Ala
225                 230                 235                 240

Thr Gly Thr Glu Ile Ala Gly Asn Asn Ala Val Val Asn Gln Asp Gly
                245                 250                 255

Glu Leu Asp Val Ser Gly Gly Gly His Gly Ile Asp Ile Thr Gly Asp
            260                 265                 270

Ser Ala Thr Val Asp Asn Lys Gly Gly Met Thr Val Thr Asp Pro Asp
        275                 280                 285

Ser Ile Gly Ile Gln Ile Asp Gly Asp Lys Ala Val Val Asn Asn Asp
    290                 295                 300

Gly Asp Asn Ala Ile Ser Asn Gly Gly Thr Gly Thr Gln Val Asn Gly
305                 310                 315                 320

Asp Glu Ala Thr Val Asn Asn Asn Gly Ser Thr Thr Val Asp Gly Lys
                325                 330                 335

Asp Ser Thr Gly Thr Glu Ile Asn Gly Asp Lys Ala Ile Val Asn Asn
            340                 345                 350

Asp Gly Asp Ser Thr Ile Leu Asp Gly Gly Thr Glu Thr Arg Ile Thr
        355                 360                 365

Gly Asp Asp Ala Thr Ala Asn Asn Ser Gly Asn Thr Thr Val Asp Gly
    370                 375                 380
```

Gln Gly Ser Thr Gly Thr Glu Ile Ala Gly Asn Asn Ala Val Val Asn
385 390 395 400

Gln Asp Gly Glu Leu Asp Val Ser Gly Gly Gly His Gly Ile Asp Ile
405 410 415

Thr Gly Asp Ser Ala Thr Val Asp Asn Lys Gly Gly Met Thr Val Ile
420 425 430

Asp Pro Asp Ser Ile Gly Ile Gln Ile Asp Gly Asp Lys Ala Val Val
435 440 445

Asn Asn Asp Gly Asp Asn Ala Ile Ser Asn Gly Gly Thr Gly Thr Gln
450 455 460

Ile Asn Gly Asp Glu Ala Thr Val Asn Asn Asn Gly Ser Thr Thr Val
465 470 475 480

Asp Gly Lys Asp Ser Thr Gly Thr Glu Ile Asn Gly Asp Lys Ala Ile
485 490 495

Val Asn Asn Asp Gly Asp Ser Thr Ile Leu Asp Gly Gly Thr Gly Thr
500 505 510

Arg Ile Thr Gly Asp Asp Ala Thr Ala Asn Asn Ser Gly Asn Thr Thr
515 520 525

Val Asp Gly Gln Gly Ser Thr Gly Thr Glu Ile Ala Gly Asn Asn Ala
530 535 540

Val Val Asn Gln Asp Gly Glu Leu Asp Val Ser Gly Gly Gly His Gly
545 550 555 560

Ile Asp Ile Thr Gly Asp Ser Ala Thr Val Asp Asn Lys Gly Gly Met
565 570 575

Thr Val Thr Asp Pro Asp Ser Ile Gly Ile Gln Ile Asp Gly Asp Lys
580 585 590

Ala Val Val Asn Asn Asp Gly Asp Asn Ala Ile Ser Asn Gly Gly Thr
595 600 605

Gly Thr Gln Val Asn Gly Asp Glu Ala Thr Val Asn Asn Asn Gly Asn
610 615 620

Thr Thr Val Asp Gly Lys Asp Ser Thr Gly Thr Glu Ile Asn Gly Asp
625 630 635 640

Lys Ala Ile Val Asn Asn Asp Gly Asp Ser Thr Ile Leu Asp Gly Gly
645 650 655

Thr Gly Thr Arg Ile Thr Gly Asp Asp Ala Thr Ala Asn Asn Ser Gly
660 665 670

Asn Thr Thr Val Asp Gly Gln Gly Ser Thr Gly Thr Glu Ile Ala Gly
675 680 685

Asn Asn Ala Val Val Asn Gln Asp Gly Glu Leu Asp Val Ser Gly Gly
690 695 700

Gly His Gly Ile Asp Ile Thr Gly Asp Ser Ala Thr Val Asp Asn Lys
705 710 715 720

Gly Gly Met Thr Val Ala Asp Ala Asp Ser Ile Gly Ile Gln Ile Asp
725 730 735

Gly Asp Lys Ala Val Val Asn Asn Asp Gly Asp Asn Ala Ile Ser Asn
            740                 745                 750

Gly Gly Thr Gly Thr Gln Val Asn Gly Asp Glu Ala Ile Val Asn Asn
        755                 760                 765

Asn Gly Asn Thr Thr Val Asp Gly Lys Asp Ser Thr Gly Thr Glu Ile
    770                 775                 780

Asn Gly Asp Lys Ala Ile Val Asn Asn Asp Gly Asp Ser Thr Ile Leu
785                 790                 795                 800

Asp Gly Gly Thr Gly Thr Arg Ile Thr Gly Asp Asp Ala Thr Ala Asn
                805                 810                 815

Asn Ser Gly Asn Thr Thr Val Asp Gly Gln Gly Ser Thr Gly Thr Glu
            820                 825                 830

Ile Ala Gly Asn Asn Ala Val Val Asn Gln Asp Gly Glu Leu Asp Val
        835                 840                 845

Ser Gly Gly Gly His Gly Ile Asp Ile Thr Gly Asp Ser Ala Thr Val
    850                 855                 860

Asp Asn Lys Gly Gly Met Thr Val Ala Asp Ala Asp Ser Ile Gly Ile
865                 870                 875                 880

Gln Ile Asp Gly Asp Lys Ala Val Val Asn Asn Asp Gly Asp Ser Thr
                885                 890                 895

Ile Leu Asp Gly Gly Thr Gly Thr Arg Ile Thr Gly Asp Asp Ala Thr
            900                 905                 910

Ala Asn Asn Ser Gly Asn Thr Thr Val Asp Gly Gln Gly Ser Thr Gly
        915                 920                 925

Thr Glu Ile Ala Gly Asn Asn Ala Val Val Asn Gln Asp Gly Lys Leu
    930                 935                 940

Asp Val Ser Gly Gly Gly His Gly Ile Asp Ile Thr Gly Asp Ser Ala
945                 950                 955                 960

Thr Val Asp Asn Lys Gly Gly Met Thr Val Ala Asp Ala Asp Ser Ile
                965                 970                 975

Gly Ile Gln Ile Asp Gly Asp Lys Ala Val Val Asn Asn Asp Gly Asp
            980                 985                 990

Asn Ala Ile Ser Asn Gly Gly Thr Gly Thr Gln Val Asn Gly Asp Glu
        995                 1000                1005

Ala Thr Val Asn Asn Asn Gly Asn Thr Thr Val Asp Gly Lys Asp Ser
    1010                1015                1020

Thr Gly Thr Glu Ile Ala Gly Asn Asn Ala Thr Val Thr Gln Glu Gly
1025                1030                1035                1040

Glu Leu Thr Val Ser Ser Gly Gly Arg Gly Ile Asp Ile Thr Gly Asn
                1045                1050                1055

Asn Ala Lys Val Asp Thr Lys Gly Lys Met Thr Ile Thr Gly Thr Asp
            1060                1065                1070

Ser Val Gly Val Ser Ile Asn Gly Asp Ser Ala Thr Leu Thr Asn Thr

1075 1080 1085

Gly Asp Ile Asp Val Ser Asn Ser Ala Thr Gly Phe Ser Leu Val Thr
1090 1095 1100

Asn Glu Gly Ile Ile Ser Leu Ala Gly Ser Met Lys Val Gly Asp Phe
1105 1110 1115 1120

Ser Thr Gly Met Ala Leu Ser Gly Asp Asn Asn Ser Val Thr Leu Ala
1125 1130 1135

Ala Lys Asp Ile Asn Val Thr Gly Gln Lys Ala Thr Gly Val Asn Ile
1140 1145 1150

Ser Gly Asp Ser Asn Thr Val Asp Ile Thr Gly Asn Ile Leu Val Asp
1155 1160 1165

Lys Asp Gln Thr Ala Asp Asn Ala Val Asp Tyr Phe Tyr Asp Pro Ser
1170 1175 1180

Val Gly Val Asn Ile Ser Gly Asn Ser Asn Thr Val Ser Leu Asp Gly
1185 1190 1195 1200

Lys Leu Thr Val Ile Ala Asp Ser Glu Leu Thr Ser Arg Lys Tyr Met
1205 1210 1215

Glu Phe Asp Gly Ser Gln Glu Asn Ile Ser Gly Leu Thr Val Ser Gly
1220 1225 1230

Asp Gly Asn Thr Val Asn Leu Asn Gly Gly Ile Gln Phe Val Gly Glu
1235 1240 1245

Lys Asn Ala Leu Ala Asp Gly Ser Thr Ile Ala Asp Lys Arg Ser Tyr
1250 1255 1260

Phe Gly Lys Thr Pro Leu Val Ser Val Asp Gly Gln Ser Lys Val Tyr
1265 1270 1275 1280

Leu Asn Gly Asp Ser Thr Ile Ser Gly Ser Leu Pro Leu Gly Tyr Ala
1285 1290 1295

Asn Ile Leu Gln Leu Ser Asn Lys Ala Ala Leu Glu Ile Gly Ser Asp
1300 1305 1310

Ala Thr Phe Ser Met Gln Asp Ile Ser Val Tyr Glu His Tyr Phe Thr
1315 1320 1325

Gln Thr Pro Gln Ile Ile Lys Val Asp Thr Gly Ser Gln Val Val Asn
1330 1335 1340

Asn Gly Asp Val Asp Ile Trp Asn Ile Ser Phe Ala Gly Ile Trp Gly
1345 1350 1355 1360

Glu Asn Ser Thr Gly Ile Asn Asn Gly Asn Ile Thr Leu Ser Gln Tyr
1365 1370 1375

Asp Tyr Ser Ser Pro Glu Thr Ser Phe Ser Glu Pro Asp His Met Ala
1380 1385 1390

Phe Leu Ser Ser Ser Gly Gly Ser Ala Val Asn Asn Gly Thr Ile Thr
1395 1400 1405

Ala Lys Val Met Glu Gln His Ser Val Leu Asn Met Gly Ser Ala Ala
1410 1415 1420

Gly Val Ala Asp Pro Arg Val Phe Asn Asn Ser Val Ser Ser Met Met
1425 1430 1435 1440

Gly Met Glu Ala Tyr Gly Lys Gly Thr Val Leu Asn Ser Glu Ser Gly
1445 1450 1455

Val Ile Asp Met His Gly Arg Gly Asn Ile Gly Met Leu Ala Val Asp
1460 1465 1470

Asp Ser Ala Ala Asp Asn Ala Gly Lys Ile Thr Leu Asp Thr Leu Trp
1475 1480 1485

Val Asp Gln Asn Asp Thr Thr Thr Leu Arg Thr Asp Leu Pro Ser Ser
1490 1495 1500

Thr Ala Ile Asp Tyr Gly Val Gly Met Ala Thr Gly Thr Asn Ser Gly
1505 1510 1515 1520

Gly Gly Ala Arg Ser Asn Gly Val Ala Thr Asn Gln Gln Gly Gly Val
1525 1530 1535

Ile Thr Val Tyr Asn Ala Gly Ala Ala Met Ala Ala Tyr Gly Ala Ser
1540 1545 1550

Asn Met Val Ile Asn Gln Gly Ile Ile Asn Leu Glu Lys Asn Gly Asn
1555 1560 1565

Tyr Asp Gly Gly Leu Gly Ala Asn Met Leu Val Gly Met Ala Val Tyr
1570 1575 1580

Asn Arg Gly Thr Ala Ile Asn Asp Lys Thr Gly Val Ile Asn Ile Asn
1585 1590 1595 1600

Val Asp Thr Gly Gln Ala Phe Tyr Asn Asp Gly Thr Gly Thr Ile Leu
1605 1610 1615

Asn Tyr Gly Glu Ile Asn Leu Leu Gly Ser Pro Met Asp Ser Ala Asp
1620 1625 1630

Ser His Met Gly Ala Ile Pro Glu Asn Leu Asp Leu Leu Thr Ala Leu
1635 1640 1645

Thr Gly Ser Gly Glu Thr Asp Met Arg Thr Ala Ser Ser Gly Gly Phe
1650 1655 1660

Val Thr Thr Lys Ala Leu Ala Asn Tyr Gly Asn Glu Thr Leu Asn Ser
1665 1670 1675 1680

Asn Val Ala Ala Lys Ala Trp Leu Tyr Asn Gln Asp Lys Ala Asn Leu
1685 1690 1695

Thr Ile Asn Gly Glu Leu Ser Ile Gly Gln Gly Leu Glu Asn Ser Gly
1700 1705 1710

Leu Leu Asp Ser Asp Thr Ile Ser Ala Ala Ala Asn Val Tyr Asn Arg
1715 1720 1725

Ala Ser Gly Ser Ile Ile Thr Asp Gln Leu Ser Leu Thr Gly Ser Asn
1730 1735 1740

Ser Phe Phe Asn Glu Gly Asn Phe Ser Gly Ser Val Ala Gly Ser Ser
1745 1750 1755 1760

Tyr Lys Gln Asn Val Val Asn Thr Gly Thr Met Ala Val Met Ala Asp
1765 1770 1775

Gly Lys Ser Leu Ile Ser Gly Ser Phe Leu Leu Tyr Asn Glu Ala Gly
1780 1785 1790

Ala Thr Leu Ser Asn Ser Ser Ser Ala Val Ser Gly Gly Glu Asn Ala
1795 1800 1805

Ile Val Asn Val Thr Arg Thr Gly Asp Ser Leu Ala Gln Val Asn Arg
1810 1815 1820

Gly Thr Ile Thr Ala Ile Asn Gly Tyr Ser Ala Ile Lys Thr Ala Ser
1825 1830 1835 1840

Thr Gly Ser Asn Ser Asn Gly Lys Trp Ile Trp Asn Thr Asp Thr Gly
1845 1850 1855

Val Ile Ser Gly Val Asn Pro Asn Ala Pro Leu Ile Asp Leu Gly Arg
1860 1865 1870

Gly Tyr Asn Phe Ala Asn Ala Gly Thr Ile Asn Val Gln Gly Asp Gly
1875 1880 1885

Ala Val Ala Ile Ser Gly Gly Thr Thr Ser Tyr Thr Val Gln Leu Val
1890 1895 1900

Asn Ser Gly Thr Ile Asn Val Gly Ala Ala Gln Gly Lys Ala Asp Gly
1905 1910 1915 1920

Thr Asn Gly Thr Gly Leu Ile Gly Ile Lys Gly Asn Gly Ser Asp Thr
1925 1930 1935

Thr Ile Asn Asn Ala Gln Ser Gly Val Ile Asn Val Tyr Ala Asp Asn
1940 1945 1950

Ser Trp Ala Phe Gly Gly Lys Thr Lys Ala Ile Ile Asn Asn Gly Glu
1955 1960 1965

Ile Asn Leu Leu Cys Asp Thr Gly Cys Asp Ile Tyr Ala Pro Gly Thr
1970 1975 1980

Thr Gly Thr Leu Asn Asp His Asn Ser Thr Thr Asp Ile Ile Val Pro
1985 1990 1995 2000

Ala Ala Thr Ser Thr Pro Thr Gln Gly Ser Val Pro Thr Val Pro Ala
2005 2010 2015

Asp Ser Ser Ala Gln Gln Lys Leu Thr Asn Tyr Thr Ile Gly Thr Asn
2020 2025 2030

Ser Asp Gly Thr Ser Gly Met Leu Lys Ala Asn Asn Leu Val Ile Ser
2035 2040 2045

Asp Asn Val Lys Val Asn Thr Gly Phe Ser Ala Gly Thr Ala Asp Thr
2050 2055 2060

Thr Val Val Ile Asn Asp Val Phe Lys Gly Glu Asn Ile Ser Gly Ala
2065 2070 2075 2080

Glu Asn Ile Ser Ser Ser Thr Val Met Trp Asn Ala Gln Gly Ser Thr
2085 2090 2095

Asp Ala Ser Gly Asn Val Asp Val Thr Met Thr Lys Asn Ala Tyr Thr
2100 2105 2110

Asp Val Val Thr Asp Ser Ser Val Asn Asn Val Ala Gln Val Leu Asp

2115 2120 2125

Ser Gly Tyr Thr Asn Asn Asp Leu Tyr Thr Ser Leu Asn Val Gly Thr
2130 2135 2140

Thr Ala Glu Leu Asn Ser Ala Leu Lys Gln Ile Ser Gly Ser Gln Ala
2145 2150 2155 2160

Thr Thr Val Phe Asn Glu Ala Arg Val Leu Ser Asn Arg Phe Ser Met
2165 2170 2175

Leu Ser Asp Ala Ala Pro Glu Val Ala Asn Gly Leu Ala Phe Asn Val
2180 2185 2190

Val Ala Lys Gly Asp Pro Arg Ala Glu Leu Gly Asn Asp Thr Gln Tyr
2195 2200 2205

Asp Met Met Ala Leu Arg Lys Ser Leu Thr Leu Thr Glu His Gln Asn
2210 2215 2220

Leu Ser Leu Glu Tyr Gly Ile Ala Arg Leu Glu Gly Asn Gly Ser Asp
2225 2230 2235 2240

Thr Ala Gly Asp Asn Gly Val Thr Gly Gly Tyr Ser Gln Phe Phe Gly
2245 2250 2255

Leu Lys His Gln Met Ala Phe Asp Asn Gly Met Ser Trp Asn Asn Ala
2260 2265 2270

Leu Arg Tyr Asp Val His Asn Leu Asp Ser Ser Arg Ser Ile Ala Tyr
2275 2280 2285

Gly Asp Val Asn Lys Thr Ala Asp Ala Asn Val Lys Gln Gln Tyr Leu
2290 2295 2300

Glu Phe Arg Ser Glu Gly Ala Lys Thr Phe Glu Leu Arg Glu Gly Leu
2305 2310 2315 2320

Asn Val Thr Pro Tyr Ala Gly Val Lys Leu Arg His Thr Leu Glu Asn
2325 2330 2335

Gly Tyr Gln Glu Arg Asn Ala Gly Asp Phe Asn Leu Ser Met Asn Ser
2340 2345 2350

Gly Ser Glu Thr Ala Val Asp Ser Ile Val Gly Leu Lys Leu Asp Tyr
2355 2360 2365

Ala Gly Lys Glu Gly Trp Ser Ala Asn Ala Thr Leu Glu Gly Gly Pro
2370 2375 2380

Asn Leu Ser Tyr Val Lys Ser Gln Arg Thr Ala Ser Ile Ser Gly Ala
2385 2390 2395 2400

Gly Ser Gln Arg Phe Asn Ile Asp Asp Gly Gln Ser Gly Gly Gly Phe
2405 2410 2415

Asn Ser Leu Ala Thr Met Gly Val Lys Tyr Ser Ser Gln Glu Ser Ala
2420 2425 2430

Leu Gln Leu Asp Ala Phe His Trp Lys Glu Asp Gly Ile Ser Asp Lys
2435 2440 2445

Gly Val Met Leu Asn Phe Lys Lys Thr Phe
2450 2455

<210> 57
<211> 505
<212> PRT
<213> Escherichia coli

<400> 57
Met Tyr Lys Lys Thr Thr Leu Ala Val Leu Ile Ala Leu Leu Thr Gly
1 5 10 15

Ala Thr Thr Val His Ala Gln Thr Asp Ile Ser Ser Ile Glu Ser Arg
20 25 30

Leu Ala Ala Leu Glu Gln Arg Leu Lys Asn Ala Glu Ser Arg Ala Gln
35 40 45

Ala Ala Glu Ala Arg Ala Lys Thr Ala Glu Leu Gln Val Gln Lys Leu
50 55 60

Ala Glu Thr Gln Gln Gln Asn Gln Leu Thr Thr Gln Glu Val Ala Gln
65 70 75 80

Arg Thr Val Gln Leu Glu Gln Lys Ser Ala Glu Asn Ser Gly Phe Glu
85 90 95

Phe His Gly Tyr Ala Arg Ser Gly Leu Leu Met Asn Asp Ala Ala Ser
100 105 110

Ser Ser Lys Ser Gly Pro Tyr Leu Thr Pro Ala Gly Glu Thr Gly Gly
115 120 125

Ala Val Gly Arg Leu Gly Asn Glu Ala Asp Thr Tyr Val Glu Leu Asn
130 135 140

Val Glu His Lys Gln Thr Leu Asp Asn Gly Ala Thr Thr Arg Phe Lys
145 150 155 160

Ala Met Leu Ala Asp Gly Gln Arg Asp Tyr Asn Asp Trp Thr Gly Gly
165 170 175

Ser Ser Asn Leu Asn Ile Arg Gln Ala Phe Ala Glu Leu Gly Ala Leu
180 185 190

Pro Ser Phe Thr Gly Ala Phe Lys Asp Ser Thr Val Trp Ala Gly Lys
195 200 205

Arg Phe Asp Arg Asp Asn Phe Asp Ile His Trp Leu Asp Ser Asp Val
210 215 220

Val Phe Leu Ala Gly Thr Gly Gly Gly Ile Tyr Asp Val Lys Trp Asn
225 230 235 240

Asp Thr Phe Arg Ser Asn Phe Ser Leu Tyr Gly Arg Asn Phe Gly Asp
245 250 255

Leu Asp Asp Ile Asp Asn Asn Val Gln Asn Tyr Ile Leu Thr Met Asn
260 265 270

His Tyr Ala Gly Pro Phe Gln Leu Met Val Ser Gly Leu Arg Ala Lys
275 280 285

Asp Asn Asp Asp Arg Lys Asp Ala Asn Gly Asp Leu Ile Gln Thr Asp
290 295 300

Ala Ala Asn Thr Gly Val His Ala Leu Val Gly Leu His Asn Asp Thr
305 310 315 320

```
Phe Tyr Gly Leu Arg Glu Gly Thr Ala Lys Thr Ala Leu Leu Tyr Gly
                325                 330                 335

His Gly Leu Gly Ala Glu Val Lys Gly Ile Gly Ser Asp Gly Ala Leu
            340                 345                 350

Leu Ser Glu Ala Asn Thr Trp Arg Phe Ala Ser Tyr Gly Thr Thr Pro
        355                 360                 365

Leu Gly Ser Gly Trp Tyr Val Ala Pro Ala Ile Leu Ala Gln Ser Ser
    370                 375                 380

Lys Asp Arg Tyr Val Lys Gly Asp Ser Tyr Glu Trp Val Thr Phe Asn
385                 390                 395                 400

Thr Arg Leu Ile Lys Glu Val Thr Gln Asn Phe Ala Leu Ala Phe Glu
                405                 410                 415

Gly Ser Tyr Gln Tyr Met Asp Leu Lys Pro Lys Gly Tyr Gln Asn His
            420                 425                 430

Asn Ala Val Asn Gly Ser Phe Tyr Lys Leu Thr Phe Ala Pro Thr Leu
        435                 440                 445

Lys Ala Asn Asp Ile Asn Asn Phe Phe Ser Arg Pro Glu Leu Arg Leu
    450                 455                 460

Phe Ala Thr Trp Met Asp Trp Ser Ser Lys Leu Asp Asp Phe Ala Ser
465                 470                 475                 480

Asn Asp Ala Phe Gly Ser Ser Gly Phe Asn Thr Gly Gly Glu Trp Asn
                485                 490                 495

Phe Gly Val Gln Met Glu Thr Trp Phe
            500                 505

<210>   58
<211>   272
<212>   PRT
<213>   Escherichia coli

<400>   58
Met Pro Ala Arg Gly Phe Phe Phe Ile Val Ile Asp Glu Pro Gly Lys
1               5                   10                  15

Asp Gly Phe Leu Val Arg Lys Ile Tyr Ala Ser Pro Asn Ser Pro Tyr
            20                  25                  30

Leu Thr Lys Met Asn Leu Asp Pro Leu Arg Leu Leu Ser Glu Thr Glu
        35                  40                  45

Pro Glu Arg Tyr Gly Leu Met Pro Lys Asp Tyr Ser Ala Pro Thr Thr
    50                  55                  60

Val Ala Glu His Val Met Gly Asn Asn Lys Ser Ser Leu Ile Ser Thr
65                  70                  75                  80

Ser Ser Ile Phe Pro Asp Gly Ser Pro Arg Phe Glu Gly Lys Thr Ile
                85                  90                  95

Phe Ile Asp Val His Lys Ala Lys Asn Ala Gly Leu Lys Leu Ile Ser
            100                 105                 110

Thr Ala Glu Ile Ile Thr Ala Leu Glu Glu Tyr Lys Lys Gln Tyr Pro
```

115 120 125

His Leu Ala Lys Arg Val Glu Lys Ile Ser Gly Tyr Val Asp Asp Ile
130 135 140

Asp Lys Glu Val Leu Ile Glu Gly Arg Lys Val Pro Ala Gln Thr Ile
145 150 155 160

Phe Thr Pro Glu Ser Leu Lys Thr Thr Gln Asn Ile Val Lys Ala Gly
165 170 175

Arg Val Val Gln Val Phe Ser Ile Phe Phe Thr Ala Tyr Asp Leu Glu
180 185 190

Gln Ala Thr Glu Lys Ser Phe Glu Thr Lys Ser Ile Lys Pro Ile Gly
195 200 205

Ala Glu Ala Ile Arg Gln Ile Gly Gly Trp Gly Gly Ala Val Ala Gly
210 215 220

Ala Lys Ile Gly Thr Val Ile Gly Ala Ser Leu Gly Val Thr Thr Gly
225 230 235 240

Pro Gly Ala Val Ala Thr Gly Ile Ile Gly Gly Ile Val Phe Gly Ala
245 250 255

Ala Gly Tyr Phe Gly Ala Asp Trp Val Ala Asp Tyr Ile Asp Glu Asn
260 265 270

<210> 59
<211> 306
<212> PRT
<213> Escherichia coli

<400> 59
Met Phe Leu Thr Asn Thr Ser Arg Phe Leu Phe Leu Phe Leu Phe Leu
1 5 10 15

Met His Lys Asn Lys Leu Tyr Ala Tyr Asp Asn Phe Ser Phe Asn Ala
20 25 30

Asp Val His Tyr Gly Tyr Gly Lys Leu Leu Trp His Asp Lys Lys Pro
35 40 45

Val Ile Tyr Pro Leu Ala Ser Leu Ser Asp Tyr Thr Tyr Tyr Asn Glu
50 55 60

Phe Asp Pro Phe Glu Thr Ile Tyr Tyr Val Gly Gln Tyr Tyr Lys Ile
65 70 75 80

Ser Leu Asn Glu Ser Lys Arg Ile Gln Glu Gln Lys Asp Ile Tyr Ala
85 90 95

His Leu Gln Leu Lys His Asn Lys Ser Leu Ile Thr Ala Asn Val Thr
100 105 110

Leu His Asn Lys Ser Asn Ser Ser Tyr Tyr Val His Arg Met Asn Leu
115 120 125

Pro Ala Ser Ile His Glu Ser Pro Tyr Ser Pro Leu Cys Ser Gly Ser
130 135 140

Phe Leu Ile Val Thr Asn Asn Ile Arg Leu Asp Tyr Ile Arg Lys Ile
145 150 155 160

Thr Cys Arg Phe Asp Leu Trp Met Lys Lys Ser Asp Trp Ile Glu Ile
165 170 175

Ser Pro Gly Glu Lys Leu Ser Tyr Thr Val Asn Leu Asn Asp Tyr Tyr
180 185 190

Ala Phe Leu Pro Ala Lys His Gln Tyr Asp Ile Gly Thr Val Glu Phe
195 200 205

Thr Leu Val Asn Ser Asn Trp Phe Leu Glu Gln His Ile Tyr Asp Leu
210 215 220

Phe Phe Ala Ile Val Asp Glu Arg His Tyr Lys Gln Cys Lys Asn Thr
225 230 235 240

Ser Gly Ile Val Arg Lys Tyr Ile Tyr Lys Ser Trp Gln Leu Cys Glu
245 250 255

Thr Tyr Arg Gly Arg Trp Ser Pro Leu Gln Glu Leu Met Asn Arg Phe
260 265 270

His Tyr Glu Gly Gly Ser Glu Asn Ile Phe Gln Ile Arg Thr Asn Gln
275 280 285

Glu Asn Ile Asn Ile Asn Gly Asp Lys Ile Lys Ser Leu Tyr Ser Ser
290 295 300

Met Gln
305

<210> 60
<211> 330
<212> PRT
<213> Escherichia coli

<400> 60
Met Lys Tyr Val Lys Lys Ile Leu Gly Ile Ser Phe Phe Ser Leu Leu
1 5 10 15

Pro Leu Ser Ala Glu Ala Glu Ser Tyr Cys Asn Leu Leu Trp Ala Ser
20 25 30

Asn Thr Leu Pro Ser Ala Ser Val Asn Val Ser Phe Asp Gly Asn Thr
35 40 45

Ser Gly Ile Phe Pro Leu Asn Leu Gln Ala Gly Gly Leu Ser Thr Val
50 55 60

Ile Lys Arg Ala Ala Lys Asn Met Asp Thr Phe Val Thr Leu Asp Gly
65 70 75 80

Thr Tyr Arg Tyr Trp Ile Gln Tyr Pro Glu Ala Trp Gln Thr Thr Pro
85 90 95

Asp Gly Leu Lys Tyr Arg Ile Thr Ser Glu Leu Glu Val Ser Gly Thr
100 105 110

Gln Thr Ala Gly Val Lys Thr Val Val Thr Ser Val Gly Tyr His Thr
115 120 125

Trp Val Asn Thr Tyr Gly Cys Arg Asp Val Gly Gly Thr Tyr Asp Phe
130 135 140

Gly Val Ala Ser Val Ser Gly Val Asn Ile Glu Ile Asp Arg Gly Thr

145 150 155 160

Ala Trp Pro Gly Val Tyr Ser Ile Gln Leu Pro Val Lys Val Ala Tyr
165 170 175

Glu Glu Asn Lys Gly Asn Tyr Asp Gly Lys Asn Gly Gly Gly Trp Arg
180 185 190

Glu Phe Pro Val Ser Met Lys Ser Phe Ser Pro Val Asp Ser Lys Gly
195 200 205

Ile Ser Ile Thr Ile Ser Ser Lys Cys Asn Val Gly Glu Gln Ser Leu
210 215 220

Ser Val Asn Met Gly Asp Asn Ile Thr Pro Asp Glu Ala Lys Ser Gly
225 230 235 240

Val Glu Lys Lys Val Asn Phe Ser Leu Thr Cys Asn Ala Pro Ala Lys
245 250 255

Val Ser Leu Ser Leu Lys Gly Thr Asp Ile Val Asp Gly Val Asn Asn
260 265 270

Lys Thr Lys Cys Gly Ser Gly Ser Cys Ser Leu Asn Phe Asp Asn Asp
275 280 285

Ser Ser Ser Lys Ile Leu Glu Val Asn Gln Gly Thr Tyr Gln Val Pro
290 295 300

Ile Thr Val Arg Phe Gln Asp Ala Asn Pro Val Ala Gly Gly Phe Asp
305 310 315 320

Gly Ser Ala Val Leu Ser Val Asp Ile Leu
325 330

<210> 61
<211> 172
<212> PRT
<213> Escherichia coli

<400> 61
Leu Arg Ile Thr Val Phe Leu Leu Thr Phe Leu Ser Phe Leu Ser Asp
1 5 10 15

Leu Trp Ala Val Asp Ile Pro Ile Asn Ile Thr Gly Thr Ile Ile Ile
20 25 30

Pro Pro Cys Gln Ile Asn Asn Ser Asn Pro Val Asp Val Asp Phe Gly
35 40 45

Asn Ile Arg Val Ser Glu Leu Asp Thr Lys Glu His Ile Lys Val Val
50 55 60

Ser Phe Pro Val Tyr Cys Pro Tyr His Gln Gly Glu Ala Tyr Val Lys
65 70 75 80

Met Thr Gly Gln Ser Met Thr Gly Lys Asp Asn Val Leu Ala Thr Asn
85 90 95

Ile Asp Gly Leu Gly Ile Glu Leu Tyr Gln Gly Gly Glu Gly Thr Gly
100 105 110

Asn His Leu Ile Leu Gly Ser Gly Ser Ser Gly Tyr Gly Tyr Glu Val
115 120 125

```
Ile Asn Ala Leu Ser Glu Lys Asn Val Glu Arg Thr Thr Phe Thr Phe
    130                 135                 140

Thr Ala Lys Ile Tyr Lys Ala Glu Gly Val Thr Ile Asn Ser Gly Glu
145                 150                 155                 160

Phe Ser Ala Ser Ala Leu Ile Asn Ile Val Tyr Leu
                165                 170
```

<210> 62
<211> 187
<212> PRT
<213> Escherichia coli

<400> 62

```
Met Asn Arg Ala Thr Ile Gly Phe Tyr Leu Ala Val Val Leu Gly Ser
1               5                   10                  15

Cys Ser Met Asn Gly Val Ser Gln Ala Asp Glu Leu Leu Thr Arg Asp
            20                  25                  30

Asp Phe Phe Val Ala Asp Glu Ser Arg His Gln Trp Val Thr Glu His
        35                  40                  45

Asn Gly Arg Thr Gly Ala Leu Asn Val Lys Gly Ala Leu Val Ser Ser
    50                  55                  60

Pro Cys Ile Leu Asp Thr Pro Glu Val Asn Phe Pro Leu Gln Lys Asp
65                  70                  75                  80

Lys Gly Arg Tyr Val Leu Asn Leu Lys Val Ser Arg Cys Gly Asp Gly
                85                  90                  95

His Ser Asp Ile Pro Glu Arg Gln Ser Thr Glu His Gly Asn Ile Val
            100                 105                 110

Val Lys Gln Ser Ala Val Leu Lys Thr Gly Lys Asp Gln Ile Leu Leu
        115                 120                 125

Ser Ala Trp Arg Asn Gly Gly Leu Ser Arg Lys Asn Leu Gln His Gly
    130                 135                 140

Asp Asn Gln Ile Thr Tyr Tyr Ile Asn Glu Ala Gln Tyr Gln Lys Ile
145                 150                 155                 160

Ala Lys Thr Gln Met Pro Val Thr Ala His Lys Thr Ser Asp Ser Asn
                165                 170                 175

Arg Gly Val Met His Leu Asn Ile Met Tyr Glu
            180                 185
```

<210> 63
<211> 290
<212> PRT
<213> Escherichia coli

<400> 63

```
Val Cys Gly Gly His Pro Gly Thr Ser Gly Pro Gly Thr Thr Val Ala
1               5                   10                  15

Ala Ala Leu Ser Ser Gly Glu Val Thr Leu Tyr Thr Pro Ala Ile Val
            20                  25                  30

Cys Ile Ser Arg Gln Lys Asn Val Lys Lys Gln Arg Ala Glu Asn Met
        35                  40                  45
```

```
Gln Lys Met Lys Pro Ala Leu Lys Lys Thr Leu Met Ala Val Ala Cys
    50                  55                  60

Leu Ser Ala Val Pro Ala Ala Gln Ala Ala Val Ser Leu Asp Arg Thr
65                  70                  75                  80

Arg Ala Ile Phe Asn Gly Asp Glu Lys Ser Met Thr Leu Asn Ile Ala
                85                  90                  95

Asn Asp Asn Lys Gln Leu Pro Tyr Leu Ala Gln Ala Trp Val Glu Asn
            100                 105                 110

Glu Lys Lys Glu Lys Ile Thr Thr Gly Pro Ile Ile Ala Thr Pro Pro
        115                 120                 125

Val Gln Arg Leu Glu Pro Gly Ser Lys Ser Met Val Arg Leu Thr Ser
    130                 135                 140

Thr Pro Asp Ile Ser Arg Leu Pro Gln Asp Arg Glu Ser Leu Phe Tyr
145                 150                 155                 160

Phe Ser Leu Arg Glu Ile Pro Pro Lys Ser Asp Lys Ala Asn Val Leu
                165                 170                 175

Gln Ile Ala Leu Gln Thr Lys Ile Lys Leu Phe Tyr Arg Pro Glu Ser
            180                 185                 190

Ile Lys Ala Lys Pro Asn Ala Val Trp Gln Asp Gln Leu Val Leu Asn
        195                 200                 205

Lys Thr Ser Gly Gly Tyr Arg Ile Asp Asn Pro Thr Pro Tyr Tyr Val
    210                 215                 220

Thr Val Ile Gly Ile Gly Gly Ser Glu Lys Gln Ala Arg Glu Gly Glu
225                 230                 235                 240

Phe Asp Ala Val Met Leu Ala Pro Lys Ser Thr Gln Met Val Lys Ser
                245                 250                 255

Gly Thr Tyr Asn Thr Pro Tyr Leu Ser Tyr Ile Asn Asp Tyr Gly Gly
            260                 265                 270

Arg Pro Val Leu Gln Phe Ser Cys Gly Gly Ser Arg Cys Thr Ala Val
        275                 280                 285

Lys Lys
    290

<210>   64
<211>   856
<212>   PRT
<213>   Escherichia coli

<400>   64
Met Leu Lys Arg Val Gly Cys Ala Leu Gln Ser Val Phe Val Leu Asn
1               5                   10                  15

Arg Leu His Ile Met Lys Lys Asn Lys Ser Thr Phe Thr Ile Asn Phe
            20                  25                  30

Ile Thr Tyr Ser Leu Met Leu Ser Leu Ala Gly Val Pro Val Tyr Ala
        35                  40                  45

Val Asp Phe Asn Thr Asp Val Leu Asp Ala Ala Asp Arg Gln Asn Ile
```

50 55 60

Asp Phe Ser Arg Phe Ser Arg Ala Gly Tyr Ile Met Pro Gly Gln Tyr
65 70 75 80

Gln Met Glu Ile Arg Val Asn Gly Gln Asp Ile Ser Pro Ser Ala Phe
85 90 95

Gln Ile Ala Phe Leu Glu Pro Pro Phe Ser Asp Ser Asp Asn Glu Lys
100 105 110

Pro Leu Pro Glu Pro Cys Leu Thr Pro Glu Ile Val Ser Arg Met Gly
115 120 125

Leu Thr Glu Ala Ser Gln Glu Lys Val Thr Tyr Trp Asn Asn Gly Gln
130 135 140

Cys Ala Asp Phe Arg Gln Leu Ser Gly Val Glu Ile Arg Pro Asn Pro
145 150 155 160

Ala Glu Gly Met Leu Tyr Ile Asn Met Pro Gln Ala Trp Leu Glu Tyr
165 170 175

Ser Asp Ala Ser Trp Leu Pro Pro Ser Arg Trp Asp Asn Gly Ile Pro
180 185 190

Gly Leu Leu Phe Asp Tyr Asn Ile Asn Gly Thr Val Asn Lys Pro His
195 200 205

Gln Gly Lys Gln Ser Gln Ser Leu Asn Tyr Asn Gly Thr Ala Gly Ala
210 215 220

Asn Phe Gly Ala Trp Arg Leu Arg Ala Asp Tyr Gln Gly Asn Leu Asn
225 230 235 240

His Thr Thr Gly Ser Ala Gln Gly Thr Asp Ser Gln Phe Thr Trp Ser
245 250 255

Arg Phe Tyr Met Tyr Arg Ala Ile Pro Arg Trp Arg Ala Asn Leu Thr
260 265 270

Leu Gly Glu Asn Tyr Ile Asn Ser Glu Ile Phe Ser Ser Trp Arg Tyr
275 280 285

Thr Gly Ala Ser Leu Glu Ser Asp Asp Arg Met Leu Pro Pro Lys Leu
290 295 300

Arg Gly Tyr Ala Pro Gln Val Ser Gly Ile Ala Asp Thr Asn Ala Arg
305 310 315 320

Val Val Ile Ser Gln Gln Gly Arg Ile Leu Tyr Asp Ser Thr Val Pro
325 330 335

Ala Gly Pro Phe Thr Ile Gln Asp Leu Asp Ser Ser Val Arg Gly Arg
340 345 350

Leu Asp Val Glu Val Ile Glu Gln Asp Gly Arg Lys Lys Thr Phe Gln
355 360 365

Val Asp Thr Ala Tyr Val Pro Tyr Leu Thr Arg Pro Gly Gln Val Arg
370 375 380

Tyr Lys Leu Val Ser Gly Arg Ser Arg Thr Tyr Glu His Thr Met Glu
385 390 395 400

Gly Pro Val Phe Ala Ala Gly Glu Ala Ser Trp Gly Ile Ser Asn Thr
405 410 415

Trp Ser Leu Tyr Gly Gly Ser Ile Val Ala Gly Asp Tyr Asn Ala Leu
420 425 430

Ala Val Gly Leu Gly Arg Asp Leu Ser Lys Phe Gly Thr Val Ser Ala
435 440 445

Asp Val Thr Gln Ser Val Ala Arg Ile Pro Gly Tyr Asp Thr Lys Gln
450 455 460

Gly Lys Ser Trp Arg Leu Ser Tyr Ser Lys Arg Phe Asp Glu Val Asn
465 470 475 480

Thr Asp Ile Thr Phe Ala Gly Tyr Arg Phe Ser Glu Arg Asn Tyr Met
485 490 495

Thr Met Asp Gln Tyr Leu Asn Ala Arg Tyr Arg Asn Asp Phe Thr Gly
500 505 510

Arg Glu Lys Glu Leu Tyr Thr Val Thr Leu Asn Lys Asn Phe Glu Asp
515 520 525

Trp Lys Ala Ser Val Asn Leu Gln Tyr Ser His Gln Thr Tyr Trp Asp
530 535 540

Arg Arg Thr Ser Asp Tyr Tyr Thr Leu Ser Val Asn Arg Tyr Phe Asp
545 550 555 560

Ala Phe Ser Phe Lys Asn Ile Ala Leu Gly Ile Ser Ala Ser Arg Ser
565 570 575

Lys Tyr Leu Asn Arg Asp Asn Asp Ser Ala Phe Val Arg Leu Ser Val
580 585 590

Pro Trp Gly Thr Gly Thr Ala Ser Tyr Ser Gly Ser Met Ser Asn Asp
595 600 605

Arg Tyr Thr Asn Thr Val Gly Tyr Ser Asp Thr Leu Asn Asn Gly Leu
610 615 620

Ser Ser Tyr Ser Leu Asn Ala Gly Val Asn Ser Gly Gly Gly Gln Pro
625 630 635 640

Ser Gln Arg Gln Met Ser Ala Tyr Tyr Asn His Asn Gly Ser Leu Thr
645 650 655

Asn Leu Ser Ala Ser Phe Ser Ala Val Glu Asn Gly Tyr Ser Ser Phe
660 665 670

Gly Met Ser Ala Ser Gly Gly Ala Thr Val Thr Met Lys Gly Ala Ala
675 680 685

Leu His Ala Gly Gly Met Asn Gly Gly Thr Arg Leu Leu Val Asp Thr
690 695 700

Asp Gly Val Gly Gly Val Pro Val Asp Gly Gly Arg Val Tyr Thr Asn
705 710 715 720

Arg Trp Gly Ile Gly Val Val Thr Asp Val Ser Ser Tyr Tyr Arg Asn
725 730 735

Thr Thr Ser Val Asp Leu Asn Lys Leu Pro Glu Asp Met Glu Ala Thr
740 745 750

Arg Ser Val Val Glu Ser Val Leu Thr Glu Gly Ala Ile Gly Tyr Arg
755 760 765

Glu Phe Glu Val Leu Lys Gly Ser Arg Leu Phe Ala Val Leu Arg Met
770 775 780

Ser Asp Asn Ser Tyr Pro Pro Phe Gly Ala Ser Val Thr Asn Ala Lys
785 790 795 800

Gly Arg Glu Leu Gly Met Val Ala Asp Ser Gly Leu Ala Trp Leu Ser
805 810 815

Gly Val Asn Pro Gly Glu Thr Leu Asn Val Gly Trp Asp Gly Arg Thr
820 825 830

Gln Cys Val Val Asp Ile Pro Ala His Pro Asp Pro Ala Gln Gln Leu
835 840 845

Leu Leu Pro Cys Arg Gln Val Lys
850 855

<210> 65
<211> 195
<212> PRT
<213> Escherichia coli

<400> 65
Met Leu Arg Arg Met Ile Leu Ser Val Leu Phe Leu Trp Cys Ala Val
1 5 10 15

Ala Gln Ala Leu Pro Ser Ala Ser Phe Pro Pro Pro Gly Met Thr Leu
20 25 30

Pro Glu Tyr Trp Gly Glu Glu His Val Trp Trp Asp Gly Arg Ala Ser
35 40 45

Phe Lys Gly Gln Val Ile Ala Pro Ala Cys Thr Leu Ser Met Glu Asp
50 55 60

Ala Trp Gln Glu Ile Asp Met Gly Thr Thr Pro Leu Arg Asp Leu Gln
65 70 75 80

Asn Ser Pro Ala Gly Pro Glu Lys Lys Phe Arg Leu Arg Leu Arg Asn
85 90 95

Cys Glu Leu Thr Gly Ala Gly Lys Gln Val Tyr Thr Ala Thr Arg Val
100 105 110

Arg Val Thr Phe Asp Gly Ile Pro Gly Glu Thr Pro Asp Lys Phe Ser
115 120 125

Leu Thr Gly Gln Ala Glu Gly Ile Asn Leu Gln Ile Met Asp Asn Tyr
130 135 140

Gly Tyr Pro Ala Arg Ala Gly Lys Ser Met Pro Pro Leu Ile Leu Arg
145 150 155 160

Gly Asn Glu Asp Gly Leu Asp Tyr Ser Leu Arg Ile Val Arg Asn Gly
165 170 175

Tyr Pro Leu Lys Ala Gly Asp Tyr Tyr Ala Ala Leu Arg Phe Lys Leu
180 185 190

Asp Tyr Glu

195

<210> 66
<211> 177
<212> PRT
<213> Escherichia coli

<400> 66
Met Leu Lys Ser Thr Leu Gly Ile Ala Ile Ala Phe Ser Leu Cys Ala
1 5 10 15

Val Ala His Ala Ala Asn Gln Gly Gln Gly Val Val Asn Phe Lys Gly
20 25 30

Thr Val Ile Asp Ala Pro Cys Gly Ile Ala Pro Glu Ser Ala Asp Gln
35 40 45

Thr Ile Asp Phe Gly Gln Ile Ser Lys Ser His Leu Lys Asn Asp Gly
50 55 60

Ile Ser Val Lys Lys Asp Leu Asp Ile Lys Leu Val Asn Cys Asp Phe
65 70 75 80

Thr Asp Pro Thr Ala Lys Lys Thr Val Ser Val Thr Phe Ser Gly Thr
85 90 95

Pro Val Asn Gly His Thr Asp Glu Leu Gly Thr Ala Gly Asp Thr Gly
100 105 110

Thr Ala Val Val Val Ser Ala Ser Asp Gly Ser Met Val Lys Phe Asp
115 120 125

Gly Thr Thr Ser Ser Asn Ala Thr Gln Leu Gln Asp Gly Asp Asn Thr
130 135 140

Leu Lys Tyr Thr Thr Trp Val Lys Lys Ser Ser Ala Ala Gly Thr Glu
145 150 155 160

Val Lys Glu Gly Asp Phe Thr Ala Val Ala Asn Phe Asn Leu Ser Tyr
165 170 175

Gln

<210> 67
<211> 128
<212> PRT
<213> Escherichia coli

<400> 67
Met Cys Asn Ala Thr Leu Ile Ala Ile Ser Gly Tyr Gly Phe Ile Pro
1 5 10 15

Ser Val Leu Lys Ile Phe Phe Thr Gly Glu Thr Gly Lys Trp Lys Asp
20 25 30

Phe Arg Asp Asp Val Arg Ile Arg Tyr Ser Asp Gly Ile Met Leu Thr
35 40 45

Leu Ile Thr Ala Gln Asn Asn Glu Arg Ile Met Glu Val Lys Thr Lys
50 55 60

Glu Asp Trp Leu Tyr Gln Phe Arg Arg Cys Ser Ser Arg Glu Thr Leu
65 70 75 80

Glu Lys Val Ile Ser His Thr Arg Tyr Lys Leu Thr Pro Ala Glu Met
85 90 95

Glu Ala Phe Asn Ser Ala Val Asp His Arg Leu Ala Glu Leu Thr Met
100 105 110

Asn Lys Leu Tyr Asp Arg Val Pro Ala Ser Val Trp Lys Tyr Val Thr
115 120 125

<210> 68
<211> 224
<212> PRT
<213> Escherichia coli

<400> 68
Met Arg Ala Tyr Lys Phe Arg Ser Ala Ser Gln Ile Ala Phe Thr Leu
1 5 10 15

Asp Ile Ile Phe Asn Arg Arg Leu Phe Cys Ser Asp Trp Lys Met Leu
20 25 30

Asn Asp Pro Met Glu Gly Met Phe Val Phe Ser His Gln Thr Ser Asp
35 40 45

Gly Tyr Asp Tyr Lys Lys Glu Ile Gln Gln Ile Ile Glu Glu Lys Lys
50 55 60

Arg Leu Lys Val Cys Ser Leu Ser Ala Thr Tyr Glu Ser His Leu Leu
65 70 75 80

Trp Ala His Tyr Ala Gly Gly Val Asp Gly Cys Ala Ile Glu Ile Glu
85 90 95

Ile Pro Asp Asn His His Ser Ile Lys Gln Ile Glu Tyr Arg Gly Val
100 105 110

Phe Ala Asn Leu Ala Met Pro Asn Ile Tyr Ser Pro Asp Glu Leu Ala
115 120 125

Asn Gln Val Leu Ser Ser Lys Tyr Arg Glu Trp Glu Tyr Glu Gln Glu
130 135 140

Val Arg Ile Leu Gln Thr Asn Glu Trp Phe Tyr Leu Asp Lys Pro Val
145 150 155 160

Arg Arg Val Ile Ala Gly His Arg Met Pro Ser Ala Leu Phe Glu Ala
165 170 175

Met Asn Ile Ile Cys Asn Ser Met Gly Ile Glu Phe Cys Arg Thr Gly
180 185 190

Ile Gly Asp Glu Gly Ile Asp Ala Asp Tyr Val Glu Pro Ser Lys Ile
195 200 205

Leu Thr Pro Asn Asn His Leu Asp Trp Asp Val Leu Lys Asn Asp Arg
210 215 220

<210> 69
<211> 360
<212> PRT
<213> Escherichia coli

<400> 69

```
Met Arg Asn Arg Leu Ile Ala Val Ile Leu Cys Leu Phe Gly Met Val
1               5                   10                  15

Ala Gly Gly His Ala Thr Pro Asn Val Thr Ala Glu Ile Thr Tyr Asp
            20                  25                  30

Leu Ala Ala Gly Arg Ala Asp Tyr Tyr Phe Trp Asn Glu Glu Glu Ser
        35                  40                  45

Pro Gly Asn Asn Ala Tyr Met Trp Tyr Glu Cys Ser Tyr Pro Asn Ile
    50                  55                  60

Gln Gln Thr Cys Ser Asp Ser Gly Asn Phe Ser Thr Val Gln Ile Tyr
65                  70                  75                  80

Leu Thr Glu Gln Arg Ser Gly Met Arg Trp Pro Ile Thr Leu Lys Gly
                85                  90                  95

Phe Lys Lys Ala Tyr Val Asp Asp Gly Ile Pro Gly Cys Glu Gly Tyr
            100                 105                 110

Val Ala Gln Thr Asp Leu Arg Asp Ser Asn Arg Tyr Lys Cys Tyr Val
        115                 120                 125

Ser Gly Asp Ser Thr Pro His Tyr Ala Asp Lys Lys Phe Leu Thr Leu
    130                 135                 140

Tyr Leu Glu Gln Ala Glu Met Lys Lys Leu Pro Ile Gly Gly Val Trp
145                 150                 155                 160

Lys Gly Gln Val Lys Leu His Ser Asn Ser Pro Ser Val Asp Tyr Phe
                165                 170                 175

Ala Asp Ile Thr Leu Asn Thr Leu Asp Pro Asn His Ile Asp Val Phe
            180                 185                 190

Phe Pro Glu Phe Ala His Ala Thr Pro Arg Val Gln Leu Asp Leu His
        195                 200                 205

Pro Thr Gly Ser Val Asn Gly Asn Asn Tyr Ala Gln Asp Leu Thr Met
    210                 215                 220

Leu Asp Met Cys Leu Tyr Asp Gly Phe Asn Gly Asn Gly Leu Ser Tyr
225                 230                 235                 240

Glu Ile Leu Leu Lys Asp Glu Gly Lys Thr Ala Ala Gly Arg Ser Asn
                245                 250                 255

Gly Ala Phe Ser Ile Tyr Arg Gln Gly Ala Ser Ser Thr Asp Glu Gly
            260                 265                 270

Glu Arg Ile Asp Tyr Arg Val Lys Met Tyr Asp Pro Glu Ser Gly Gly
        275                 280                 285

Gln Ile Asp Val Arg Asn Asn Glu Ser Met Val Trp Thr Asn Ile Asn
    290                 295                 300

Leu Lys His Val Arg Pro Val Val Leu Pro Gly Ile Arg Tyr Ala Val
305                 310                 315                 320

Met Cys Val Pro Thr Pro Leu Thr Leu Val Val Asp Lys Phe Asn Val
                325                 330                 335

Thr Ala Lys Gln Ala Gly Tyr Tyr Met Gly Lys Leu Ser Val Ile Phe
            340                 345                 350
```

Thr Pro Ser Leu Pro Thr Ile Asn
355 360

<210> 70
<211> 119
<212> PRT
<213> Escherichia coli

<400> 70
Met Asp Tyr Arg Thr Ala Met Asp Lys Lys Leu Leu Ala Leu Leu Ile
1 5 10 15

Leu Ala Ser Leu Ser Pro Ala Lys Ala Thr Leu Thr Lys Ile Pro Ala
20 25 30

Gly Phe Glu Val Ile Ala Gln Gly Gln Gln Glu Tyr Ile Glu Val Tyr
35 40 45

Phe Ala Gly Lys Ser Leu Gly Lys Tyr Tyr Ala Met Val Asn Leu Asp
50 55 60

Thr Val Thr Phe Leu Asp Ser Ser Ser Leu Tyr Ser Lys Leu Glu Leu
65 70 75 80

Ser Thr Asp Asp Gln Lys Ile Ala His Thr Val Lys Glu Lys Leu Ser
85 90 95

Gln Pro Leu Ala Arg His Gly Glu Leu Ala Cys Gly Phe Val Arg Thr
100 105 110

Asp Ser Gly Cys Gly Tyr Ser
115

<210> 71
<211> 166
<212> PRT
<213> Escherichia coli

<400> 71
Met Lys Lys Val Phe Ala Lys Ser Leu Leu Val Ala Ala Met Phe Ser
1 5 10 15

Val Ala Gly Ser Ala Leu Ala Val Gln Lys Asp Ile Thr Val Thr Ala
20 25 30

Asn Val Asp Ala Ala Leu Asp Met Thr Gln Thr Asp Asn Thr Ala Leu
35 40 45

Pro Lys Ala Val Glu Met Gln Tyr Leu Pro Gly Gln Gly Leu Gln Ser
50 55 60

Tyr Gln Leu Met Thr Lys Ile Trp Ser Asn Asp Thr Thr Lys Asp Val
65 70 75 80

Lys Met Gln Leu Val Ser Pro Ala Gln Leu Val Gln Ser Val Asp Ala
85 90 95

Ser Lys Ile Val Pro Leu Thr Val Thr Trp Gly Gly Glu Val Ile Ala
100 105 110

Ala Asp Ala Ala Thr Thr Phe Thr Ala Thr Lys Ile Phe Ala Ser Asp
115 120 125

Ala Leu Thr Asn Gly Ser Leu Ala Lys Pro Leu Met Phe Ser Gln Ala

130 135 140

Thr Lys Gly Val Leu Glu Thr Gly Ile Tyr Arg Gly Val Val Ser Ile
145 150 155 160

Tyr Leu Ser Gln Ala Leu
165

<210> 72
<211> 238
<212> PRT
<213> Escherichia coli

<400> 72
Met Val Ala Lys Ile Asn Arg Leu Leu Arg Leu Gly Pro Val Leu Leu
1 5 10 15

Leu Cys Thr Ser Phe Phe Ala Gln Ser Asn Met Thr Val Tyr Pro Met
20 25 30

Ser Val Ser Ile Asn Ser Gln Gly Glu Gly Ser Val Arg Val Ile Ser
35 40 45

Lys Thr Asn Glu Val Gln Tyr Ile Lys Ala Thr Val Phe Arg Ile Asp
50 55 60

Asn Pro Ser Thr Pro Gln Glu Lys Glu Ile Glu Val Thr Ser Gly Asp
65 70 75 80

Ala Asn Gln Leu Val Ile Met Pro Pro Lys Phe Ala Leu Pro Gly Gly
85 90 95

Ser Ser Lys Thr Val Arg Phe Val Ala Met Glu Pro Glu Gln Lys Glu
100 105 110

Lys Asn Tyr Arg Val Lys Phe Glu Ala Val Pro Ser Ile Asp Asp Ala
115 120 125

Thr Asp Asn Lys Lys Asp Leu Ser Met Gln Leu Thr Val Asn Leu Ile
130 135 140

Trp Gly Ile Val Val Ser Val Pro Pro Gln Gln Pro Val Ala Lys Leu
145 150 155 160

Asp Ile Asn Ala Asp Lys Lys Leu Ile Asn Asn Gly Asn Gln Arg Leu
165 170 175

Lys Ile Leu Thr Ile Ala Tyr Cys Lys Asn Ser Gly Ser Ala Glu Cys
180 185 190

Arg Thr Gln Thr Val Asn Lys Asn Ile Phe Pro Gly Gln Glu Arg Val
195 200 205

Leu Glu Ser Ile Ser Gly Tyr Asp Lys Ile Val Val Lys Tyr Asn Asn
210 215 220

Trp Ile Thr Lys Asp Asn Gly Glu Phe Glu Leu Ala Val His
225 230 235

<210> 73
<211> 183
<212> PRT
<213> Escherichia coli

<400> 73

Met Lys Ile Lys Val Ile Ala Leu Ala Thr Phe Val Ser Ala Val Phe
1               5                   10                  15

Val Gly Ser Ala Met Ala Tyr Asp Gly Thr Ile Thr Phe Thr Gly Lys
            20                  25                  30

Val Val Ala Gln Thr Cys Asn Val Thr Thr Gly Asn Asp Gly Asn Phe
        35                  40                  45

Thr Val Thr Leu Pro Thr Val Ser Asn Lys Ala Leu Ala Ser Pro Gly
    50                  55                  60

Glu Thr Thr Gly Leu Thr Pro Phe Ser Ile Asn Leu Thr Gly Cys Thr
65                  70                  75                  80

Ser Thr Val Asp Gly Ala Gly His Val Lys Ala Phe Phe Glu Pro Gly
                85                  90                  95

Ser Ser Thr Asp Phe Val Thr Asn Asn Leu Lys Asn Ile Ala Ser Asp
            100                 105                 110

Asn Ala Ala Gly Asp Val Gln Ile Gln Leu Leu Asn Ser Asp Gly Thr
        115                 120                 125

Ser Thr Ile Phe Leu Gly Lys Asp Phe Ala Gly Gln Asp Val His Ser
    130                 135                 140

Glu Thr Ile Gly Gly Asp Gly Asn Ala Thr Leu Arg Tyr Met Ala Gln
145                 150                 155                 160

Tyr Tyr Ala Thr Gly Ala Ser Thr Pro Gly Asn Val Lys Thr Thr Val
                165                 170                 175

Asn Tyr Thr Ile Ala Tyr Glu
            180

<210> 74
<211> 560
<212> PRT
<213> Escherichia coli

<400> 74
Val Ser Lys Pro Ala Phe Ile Ser Ser Gly Ala Val Pro Asp Ser Cys
1               5                   10                  15

Phe Met Leu Cys Met Asp Cys Val Ser Ser Leu Gln Gln Asn Lys Gly
            20                  25                  30

Val Arg Ala Met Tyr Pro Ser Thr Gly His Tyr Ser Gln Pro Asn Leu
        35                  40                  45

Pro Ala Leu Thr Ser Thr Ile Gln Ala Ile Lys Arg Ile Ser Pro Tyr
    50                  55                  60

Pro Thr Asp Thr Leu Pro Pro Val Leu Gly Asn Val Ile Arg Ser Leu
65                  70                  75                  80

His Asn Ser Thr Gly Ile Pro Thr Glu Leu Ile Gly Asn Val Val Leu
                85                  90                  95

Ala Thr Val Ser Leu Thr Cys Gln Ser Leu Val Asp Val Ile Gln Pro
            100                 105                 110

His Thr Asp Met Pro Glu Pro Cys Ser Leu Tyr Leu Met Thr Val Ala
        115                 120                 125

```
Glu Ser Gly Glu Gly Lys Thr Thr Ile Asn Lys Leu Val Met Lys Pro
    130                 135                 140

Cys Tyr Glu Phe Ala Ala Ser Leu Ile Gln Gln Tyr Gln Thr Gln Asn
145                 150                 155                 160

Asn Asp Tyr Lys Asn Gln Leu Asn Ile Trp Lys Ile Arg Gln Arg Val
                165                 170                 175

Leu Glu Gly Asn Phe Arg Gln Ala Ile Lys Lys Arg Cys Ser Gly Lys
            180                 185                 190

Asp Glu Arg Glu Glu Ile His Gln His Phe Leu Ser Arg Pro Gln Arg
        195                 200                 205

Pro Pro Tyr Pro Asn Phe Ile Tyr Glu Asp Thr Ser Leu Lys Ala Leu
    210                 215                 220

Val Glu Gly Leu Ser Asp Tyr Pro Trp Ala Gly Met Ile Ser Asp Glu
225                 230                 235                 240

Ala Ile Ile Phe Phe Lys Ser His Leu Lys Asn Asn Pro Gly Leu Leu
                245                 250                 255

Ser Lys Ala Trp Asp Gly Glu Ile Ile Asp Phe Arg Arg Ala Asp Gly
            260                 265                 270

Glu Cys Tyr His Phe Ile Pro His Leu Thr Phe Ser Leu Met Pro Gln
        275                 280                 285

Pro Gly Val Phe Ser Glu Tyr Ile Arg Lys Asn Thr Val Ser Ala Arg
    290                 295                 300

Glu Ser Gly Phe Leu Ser Arg Phe Leu Phe Val Cys Pro Asp Arg Asn
305                 310                 315                 320

Ser Ala Glu Cys Leu Asn Thr Gly Thr Gly Ala Tyr Thr Asp Tyr Asn
                325                 330                 335

Leu Asn Val Phe His Lys Arg Ile Asn Glu Leu Leu Asp Arg His Asn
            340                 345                 350

Asn Leu Asn Ser Gly Thr Glu Ile Arg Lys Lys Thr Leu Arg Leu Ser
        355                 360                 365

Asp Glu Ala Ile Ala Asp Trp Gln Glu Asn His Ala Val Ile Gln Arg
    370                 375                 380

Met Ile Ser Pro Gly Gly Glu Trp Glu His Ile Arg Asp Ile Ala Leu
385                 390                 395                 400

Lys Ala Ser Ala Asn Ile Leu Arg Leu Ser Ala Leu Phe Asn Tyr Val
                405                 410                 415

His Asp Asp Arg Glu Asp Ala Ile His Ser His Ser Leu Lys Ala Val
            420                 425                 430

Ile Tyr Leu Val Asn Trp Tyr Leu Gly Gln Ala Glu Lys Leu Phe Tyr
        435                 440                 445

Pro Met Ser Glu Arg Tyr Gln Phe Glu Asn Asp Val Arg Arg Leu Tyr
    450                 455                 460

Ala Tyr Ile Phe Asp Lys Val Asn Gln Asn Asn Gly Tyr Pro Phe Pro
```

465 470 475 480

Lys Asn His Leu Glu Lys Tyr Gly Pro His Ser Leu Arg Arg Ala Glu
485 490 495

Lys Leu Thr Pro Val Leu Asn Gln Leu Ile Lys Gln Arg Leu Val Cys
500 505 510

Val Ile Gln Ile Tyr Pro Gly Arg Ala Leu Tyr Val Ala Leu Cys Asn
515 520 525

Gln Asn Gly Ile Ile Ile Ala Pro Tyr Asn Val His Tyr Thr Val Ile
530 535 540

Gln Ser Gln Glu Asn Thr Trp Gly Arg Pro Tyr Asp Val Thr Ile Leu
545 550 555 560

<210> 75
<211> 217
<212> PRT
<213> Escherichia coli

<400> 75
Leu Leu Gln Asp Lys Arg Gly Gly Ile Leu Asp Ile Leu Phe Leu Ala
1 5 10 15

Ser Arg Asn Ser Gln Met Lys Lys Met Ile Met Pro Leu Thr Met Val
20 25 30

Ser Val Leu Met Ser Gly Ser Ala Leu Ala Ala Ile Gly Asp Asn Gly
35 40 45

Gln Ala Asn Asn Asn Asp Ala Ser Gln Ala Glu Leu His Phe Thr Gly
50 55 60

Lys Leu Thr Ser Ser Leu Cys Gln Val Ala Thr Ser Asp Val Lys Lys
65 70 75 80

Glu Ile Tyr Leu Gly Glu Leu Ser Gln Ala Ala Leu Thr Gln Gly Arg
85 90 95

Gly Arg Gly Pro Ser Gln Ser Phe Ser Val Ser Leu Val Asn Cys Asp
100 105 110

Pro Gln Val Thr Thr Ile Lys Tyr Ser Leu Gln Asp Lys Asn Gly Ser
115 120 125

Lys Gly Asp Tyr Leu Val Asn Gln Ser Gly Asp Thr Met Ala Lys Gly
130 135 140

Val Gly Val Tyr Ile Glu Asp Asn Leu Asn Gln Pro Leu Gln Val Asp
145 150 155 160

Gly Ala Glu Asn Thr Val Thr Val Gln Asn Asn Gly Thr Gly Ala Leu
165 170 175

Pro Asn Gln Val Ile Pro Leu Ala Ala Tyr Ile Gly Ser Thr Ser Gly
180 185 190

Lys Val Asp Asp Val Gly Thr Val Thr Met Gly Leu Val Asp Ala Ser
195 200 205

Ala Val Met Thr Ile Arg Ala Ala Gln
210 215

<210> 76
<211> 233
<212> PRT
<213> Escherichia coli

<400> 76
Met Arg Tyr Gln Tyr Ser Val Leu Phe Val Phe Leu Phe Ser Gly Val
1 5 10 15

Val Gln Ala Ala Glu Phe Phe Gly Pro Arg Glu Ser Arg Leu Leu Phe
20 25 30

Glu Glu Lys Lys Gly Asn Thr Phe Tyr Arg Ile Asp Asn Ser Asp Lys
35 40 45

Lys Leu Pro Trp Leu Val Gln Ala Trp Ile Glu Asp Ala Ser Glu Lys
50 55 60

Lys Thr Thr Ala Leu Thr Ala Thr Pro Met Val Phe Arg Val Glu Pro
65 70 75 80

Ser Ser Val Phe Thr Val Arg Val Val Lys Thr Gly Ala Leu Pro Glu
85 90 95

Asp Arg Glu Thr Leu Phe Trp Ala Val Ser Asn Ser Leu Pro Gly Gly
100 105 110

Ala Ser Thr Lys Gln Asp Asn Glu Glu Gly Lys Ile Ser Ala Lys Ile
115 120 125

Ser Leu Ala Tyr Arg Phe Lys Val Pro Leu Ile Tyr Arg Pro Ala Ala
130 135 140

Leu Asp Asn Phe Arg Gln Glu Pro Glu Lys Leu Glu Trp Thr Tyr Asn
145 150 155 160

Gly Lys Glu Gly Leu Lys Leu Tyr Asn Pro Thr Arg Tyr Val Val Gln
165 170 175

Leu His Asn Val Thr Ala Asn Gly Arg Glu Phe Lys Gly Lys Gly Val
180 185 190

Ser Phe Ile Leu Leu Pro Met Ser Gly Lys Asn Val Ser Ala Ala Val
195 200 205

Asn Lys Gly Thr Arg Ile Lys Tyr Gly Val Ile Asn Asp Tyr Gly Ala
210 215 220

Val Lys Glu Tyr Asp Gly Val Val Lys
225 230

<210> 77
<211> 143
<212> PRT
<213> Escherichia coli

<400> 77
Met Met Val Leu Leu Ser Asn Ala Leu Lys Lys Ala Val Leu Ser Leu
1 5 10 15

Leu Ala Ser Cys Ser Ile Val Val Phe Ala Ser Glu Ser Glu Leu Gly
20 25 30

Val Ala Val Glu Pro Met Arg Leu Ser Ile Lys Pro Gly Glu Met Thr

35 40 45

Tyr Phe Thr Val Ile Asn Glu Thr Glu Arg Glu Tyr Ile Val Thr Thr
50 55 60

Arg Val Val Ser Asp Ser Gly Ile Asp Asp Lys Asp Lys Val Phe Val
65 70 75 80

Phe Ser Pro Pro Leu Lys His Leu Lys Lys Arg Glu Gln Ser Val Met
85 90 95

Gly Val Val Tyr Leu Lys Asn Gly Arg Glu Asn Lys Met Lys Tyr Tyr
100 105 110

Leu Ser Val Ser Phe Val Pro Lys Leu Ser Glu Asp Lys Val Lys Ile
115 120 125

Ser Ile Pro Val Val Leu Val His Gln Ile Pro Leu Met Phe Glu
130 135 140

<210> 78
<211> 835
<212> PRT
<213> Escherichia coli

<400> 78
Met Asn Lys Tyr Tyr Leu Leu Ile Ile Leu Phe Phe Val Arg Ser Thr
1 5 10 15

Lys Val Phe Ala Glu Asp Tyr Phe Asp Pro Ser Leu Leu Ala Thr Asp
20 25 30

Ile Ile Gly Glu Gly Asn Ile Asp Leu Ser Ala Phe Ser Arg Pro Gly
35 40 45

Gly Gly Met Glu Gly Glu Gln Glu Val Ala Ile Tyr Val Asn Asp Glu
50 55 60

Phe Tyr Ser Arg Asn Thr Leu Phe Phe Lys Asn Thr Leu Asp Lys Gly
65 70 75 80

Leu Leu Pro Glu Phe Thr Pro Gly Phe Phe Asp Glu Leu Leu Ser Gly
85 90 95

Asp Phe Leu Val Ser Glu Glu Asp Lys Thr Ile Ser Ser Ser Asp Phe
100 105 110

Leu Lys Lys Val Pro Tyr Ser Asp Ile Asn Phe Asn Gln Gly Met Ser
115 120 125

Arg Val Asn Val Ser Ile Pro Gln Ala Tyr Leu Gly Asp Gly Ala Lys
130 135 140

Leu Ile Ser Ser Pro Asp Thr Trp Glu Tyr Gly Gly Pro Ala Phe Leu
145 150 155 160

Leu Asp Tyr Asn Ile Ser Gly Asn Arg Asn Asp Ser Gly Asn Tyr Asp
165 170 175

Ser Arg Ser Leu Tyr Ile Ser Ser Gln Met Gly Val Asn Leu Met Lys
180 185 190

Trp Arg Leu Arg Thr Ser Ser Ser Tyr Ser Asn Tyr Lys Thr Asn Ser
195 200 205

Val Trp Gly Gly Ala Arg Ser Glu Gln Asn Ser Phe Tyr Asn Thr Tyr
210 215 220

Ala Glu Arg Asp Ile Ser Ser Leu Arg Ala Ile Leu Arg Leu Gly Glu
225 230 235 240

Val Ser Thr Ala Gly Leu Ile Leu Asp Ser Val Pro Phe Arg Gly Met
245 250 255

Lys Leu Ser Ser Ser Asp Asp Met Leu Gly Met Arg Leu Arg Asn Tyr
260 265 270

Thr Pro Thr Val Arg Gly Met Ala Ser Ser Gln Ala Val Val Thr Ile
275 280 285

Thr Gln Asn Gly Arg Gln Val Tyr Gln Thr Asn Val Pro Ala Gly Pro
290 295 300

Phe Glu Leu Asn Asp Phe Tyr Leu Ser Gly Tyr Ser Gly Asp Met Leu
305 310 315 320

Val Thr Val Arg Glu Ala Asp Gly Ser Glu His Ser Phe Leu Gln Pro
325 330 335

Tyr Ser Thr Leu Pro Glu Met Lys Arg Glu Gly Val Ser Gly Phe Glu
340 345 350

Val Ser Val Gly Arg Tyr Asp Asn Asn Gly Ala Glu His Tyr Tyr Asp
355 360 365

Ala Glu Ser Phe Val Tyr Gly Asn Trp Ser Arg Gly Phe Ala Arg Gly
370 375 380

Val Thr Phe Phe Ala Glu Thr Leu Gln Ala Glu Lys Tyr Gln Ser Leu
385 390 395 400

Gly Gly Gly Ser Thr Leu Ser Leu Gly Arg Leu Gly Ala Ala Ser Ala
405 410 415

Asp Ile Ser Leu Ser Arg Ala Asp Lys Tyr Gly Asp Ile Arg Ile Gly
420 425 430

Gln Ser Tyr Gly Phe Lys Tyr Ser Lys Ser Gln Ile Glu Thr Gly Thr
435 440 445

Thr Val Thr Leu Ala Thr Tyr Arg Tyr Ser Thr Glu Asn Phe Tyr Thr
450 455 460

Phe Arg Asp Phe Val Ser Lys Thr Asp Thr Ala Arg Tyr Ile Trp Glu
465 470 475 480

Asn Lys Leu Lys Ser Arg Met Thr Phe Ser Leu Ser Gln Ser Leu Gly
485 490 495

Glu Tyr Gly Tyr Leu Ser Ala Asn Ala Ser Gln Gln Asp Tyr Trp Asn
500 505 510

Ser Arg Glu Val Ser Arg Asn Tyr Ser Leu Thr His Ser Phe Ser Trp
515 520 525

Asn Asp Ile Tyr Phe Ser Thr Thr Leu Ser Met Asp Asp Gln Arg Gly
530 535 540

Arg Glu Thr Gly His Leu Ser Asn Lys Gln Ala Gly Ile Tyr Ala Ser
545 550 555 560

Val Pro Leu Ser Lys Leu Leu Pro Arg Thr Asp Pro Thr Ser Ser Ser
565 570 575

Leu Thr Trp Ser Thr Ser His Ala Asp His Lys Val Arg Asn Ser Val
580 585 590

Thr Leu Asp Gly Lys Val Pro Glu Ser Asp Val Arg Tyr Arg Val Gly
595 600 605

Gly Ser Trp Gly Asn Gly Thr Thr Glu Gly Ser Arg Met Ala Ser Val
610 615 620

Ser Trp Thr Gly Asp His Ala Ser Thr Ser Leu Gly Tyr Thr Arg Val
625 630 635 640

Gly Lys Tyr Arg Thr Leu Asp Tyr Ser Met Ser Gly Ala Ala Val Met
645 650 655

Tyr Pro Trp Gly Ile Ala Val Gly Asn Ser Ser Val Thr Gly Asp Gly
660 665 670

Ala Ile Val Val Glu Thr Pro Gly Ala Lys Gly Val Arg Thr Ser Thr
675 680 685

Gly Tyr Lys Thr Ser Trp Leu Gly Thr Ala Leu Ile Ser Ser Pro Gln
690 695 700

Lys Tyr Thr Glu Asn Arg Ile Asn Leu Tyr Pro Asp Gly Leu Pro Ser
705 710 715 720

Asp Thr Val Leu Gly Glu Thr Ser Lys Thr Ala Val Pro Ala Lys Gly
725 730 735

Ala Val Val Val Leu Asp Tyr Thr Val Phe Arg Gly Ser Gln Val Val
740 745 750

Phe Thr Leu Arg Gln Thr Asp Gly Asn Pro Leu Pro Phe Gly Thr Val
755 760 765

Ile Thr Leu Asp Gly Val Ser Arg Gly Lys Glu Asn Ser Gly Ile Val
770 775 780

Gly Glu Glu Gly Arg Val Tyr Met Ala Gly Ile Pro Glu Lys Gly Thr
785 790 795 800

Leu Thr Ala Ser Trp Gly Leu Asn Lys Thr Cys Ser Ile Pro Phe Arg
805 810 815

Ile Asn Gln His Lys Ala Glu Ala Val Ile Arg Glu Val Gln Gly Val
820 825 830

Cys Arg Val
835

<210> 79
<211> 150
<212> PRT
<213> Escherichia coli

<400> 79
Met Pro Cys Met Met Leu Gln Ile Phe Ile Thr Ile Leu Leu Leu Leu
1 5 10 15

Pro Leu Ser Val Thr Lys Ala Asp Ile Leu Tyr Pro Trp Pro Ser Asp

20 25 30

Thr Glu Val Arg Leu Lys Ile Ser Asp Glu Lys Gly Gln Ser Arg Gly
35 40 45

Asp Leu Arg Val Thr Asn Pro Gly Asp Ala Leu Trp Leu Val Gln Ala
50 55 60

Trp Ala Glu Asp Glu Lys Tyr Arg Arg Tyr Ser Val Ile Tyr Pro Ser
65 70 75 80

Val Tyr Arg Leu Glu Pro Phe Ser Ala Tyr Ala Leu Asn Ile Tyr Pro
85 90 95

Gly Asn Asn Ile Met Pro Glu Arg Leu Lys Trp Phe Leu Ile Ser Phe
100 105 110

Ile Pro Ser Ile Val Glu Lys Asn Lys Asn Gln Leu Ile Ile Pro Val
115 120 125

Thr Tyr Arg Leu Lys Ile Ile Asp Asp Val Val Cys Lys Gly Ile Glu
130 135 140

Lys Val Ala Gly Glu Cys
145 150

<210> 80
<211> 182
<212> PRT
<213> Escherichia coli

<400> 80
Val Ser Val Lys Ser Met Leu Lys Lys Ile Pro Cys Ile Ile Leu Met
1 5 10 15

Ser Leu Pro Gly Leu Ile Ser Ala Ala Glu Ile Thr Lys Gln Ile Glu
20 25 30

Leu Thr Leu Lys Val Asn Val Leu Lys Pro Val Cys Lys Leu Ser Ser
35 40 45

Gly Gln Gln Thr Ile Asn Phe Gly Asp Phe Asp Val Leu Asp Val Ile
50 55 60

Thr Lys Ser Ser Lys Val Asn Gly Ser Ala Thr Phe Arg Phe Thr Glu
65 70 75 80

Cys Ser Ala Val Asn Asn Ile Lys Ile Lys Phe Lys Gln Ala Gly Gln
85 90 95

Ser Pro Val Pro Asp Ile Glu Asn Asn Tyr Ile Pro Asn Ala Lys Gly
100 105 110

Asp Ile Met Ala Lys Gly Val Ala Val Lys Leu Leu Asp Asp Gln Lys
115 120 125

Lys Glu Val Glu Leu Asp Lys Ile Met Ser Val Ser Val Gly Glu Asn
130 135 140

Gln Ile Ser Lys Asp Leu Thr Leu Asn Ala Gln Val Ile Ser Val Asn
145 150 155 160

Lys Thr Gly Glu Gly Ile Ser Pro Gly Met Leu Gln Thr Ala Ile Gly
165 170 175

```
Met Glu Ile Ser Tyr Glu
            180

<210>   81
<211>   421
<212>   PRT
<213>   Escherichia coli

<400>   81
Met Leu Lys Cys Leu Cys Phe Gly Tyr Phe Thr Lys Ser Leu Met Gln
1               5                   10                  15

Arg Ile Ile Lys Cys Thr Tyr Gly Ala Gly Glu Tyr Ala Thr Ile Phe
            20                  25                  30

Thr Val Leu Phe Phe Ser Ala His Arg Cys Ala Ile Ala Val Glu Asn
        35                  40                  45

Asn Tyr Thr Ile Ser Ile Asp Lys Thr Phe Asp Ile Ser Ser Ile Thr
    50                  55                  60

Arg Asp Trp Ile Glu Leu Gly Glu Phe Pro Tyr Pro Lys Ala Ala Ser
65                  70                  75                  80

Asn Asn Glu Thr His Phe Arg Cys Thr Glu Gly Ala Gly Lys Gly Leu
                85                  90                  95

Cys Ala His Thr Glu Phe Arg Val Asn Gly Gly Leu Gln Ser Asn Pro
            100                 105                 110

Ala Ala Tyr Trp Asn Gly Trp Met Arg Met Glu Pro Lys Glu Val Asn
        115                 120                 125

Ile Gly Asp Gly Glu Lys Leu Ile Phe Ser Ala Tyr Trp Lys Gly Ser
    130                 135                 140

Pro Ser Ile Arg Trp Glu Glu Thr Asn Arg Arg Asn Met Lys Ser Ile
145                 150                 155                 160

Phe His Gln Tyr Thr Ile Gly Val Ala Ser Ile Gly Ser Ser Ala Gly
                165                 170                 175

Ser Ile Thr Lys Trp Ser Ser Gly Glu Gln Gly Pro Ser Leu Arg Cys
            180                 185                 190

Gly Asp Leu Gly Gly Cys Thr Ile Asp Thr His Thr Tyr Phe Asp Asn
        195                 200                 205

Arg Ser Gly Gly Ala Leu Lys Leu Ala Val Lys Leu Pro Ala Ala Phe
    210                 215                 220

Arg Lys Gly Thr Tyr Thr Phe Ser Asn Val Glu Val Leu Asp Leu Gly
225                 230                 235                 240

His Thr Ala Arg Asn Ala Ser Gly Thr Thr Gln Gln Ser Val Ser Ala
                245                 250                 255

Lys Val Tyr Ile Ser Gly Lys Ile Thr Val Pro Glu Arg Cys Tyr Ile
            260                 265                 270

Asp Thr Gly Ser Gly Ser Glu Ile Lys Phe Asn Asp Val Ser Ala Gly
        275                 280                 285

Ala Ser Asn Gly Glu Ile Asp Lys Arg Ser Phe Thr Leu Lys Thr Thr
    290                 295                 300
```

Cys Lys Tyr Ile Asn Leu Lys Leu Asn Gln Tyr Ile Lys Val Asn Gly
305 310 315 320

Ser Asn Gly Leu Ser Glu Tyr Glu Ile Phe Ser Arg Asp Asn Ser Asn
325 330 335

Glu Lys Ala Leu Ala Leu Val Met Arg Ile Val Arg Gly Asp Asn Gly
340 345 350

Glu Asp Phe Ser Ala Asn Cys Glu Pro Asp His Ser Gly Arg Val Tyr
355 360 365

Phe Ser Lys Glu Tyr Leu Leu Arg Glu Ile Asn Gly Asn Gly Leu Asn
370 375 380

Ile His Thr Tyr Asn Asp Ile Ile Arg Phe Ser Leu Cys Lys Tyr Gly
385 390 395 400

Ile Pro Lys Asp Tyr Gly Glu Lys Ile Ile Pro Leu Thr Ile Val Ser
405 410 415

Arg Trp Ser Asp Ser
420

<210> 82
<211> 278
<212> PRT
<213> Escherichia coli

<400> 82
Val Leu Tyr Tyr Asn Asn Phe Asp Gly Gly Tyr Cys Gly Gln Ser Leu
1 5 10 15

Cys Phe Phe Arg Asn Ser Pro Thr Gly Tyr Arg Arg Cys Ala Ala Gly
20 25 30

Thr Val Met Asp Ser Tyr Phe Leu Gly Gly Gly Thr Gly Met Ile Ala
35 40 45

Leu His Ser Leu Ala Phe Gly Tyr Ser Gly Gln His Pro Leu Gly Thr
50 55 60

Leu Asp Gly Cys Phe Asp Thr Gly Ser Leu Thr Ala Ile Ile Gly Ala
65 70 75 80

Asn Gly Thr Gly Lys Ser Thr Leu Leu Lys Thr Leu Ala Gly Leu Leu
85 90 95

Pro Pro Leu Gly Gly Cys Phe Cys Met Val Pro Gln Gly Gln Arg Gln
100 105 110

Leu Gly Tyr Leu Pro Gln Leu Thr Glu Phe Asp Arg Gln Phe Pro Leu
115 120 125

Ser Val Asn Asp Leu Val Leu Met Gly Cys Ile Pro His Ser Gly Met
130 135 140

Phe Gly Arg Ile Ser Cys Leu Trp Arg Lys Lys Ala Ile Glu Ala Leu
145 150 155 160

Asp Thr Val Gly Met Thr Glu Phe Ser Gln Met His Ile Gly Thr Leu
165 170 175

Ser Gly Gly Gln Leu Gln Arg Val Leu Phe Ala Arg Leu Leu Val Met

180 185 190

Gln Pro Ser Val Ile Leu Leu Asp Glu Pro Phe Thr Gly Ile Asp Val
195 200 205

Gln Thr Ile Arg Thr Leu Leu Val Val Ile Arg Gln Leu His Leu Glu
210 215 220

Gly Arg Thr Ile Leu Ala Val Leu His Asp Met Glu Gln Val Glu Lys
225 230 235 240

Tyr Phe Ser His Val Leu Met Leu Ser Ala Glu Gly His Arg Trp Gly
245 250 255

Arg Ser Ala Asp Ile Leu His Ser Leu Thr Ala Ala Val Thr Pro Pro
260 265 270

Gln Gln Gly Leu Leu Pro
275

<210> 83
<211> 292
<212> PRT
<213> Escherichia coli

<400> 83
Met Lys Arg Ser Ile Leu Val Val Ala Leu Ser Ser Leu Leu Val Ser
1 5 10 15

Pro Leu Val Ile Ala Lys Glu Leu Asn Val Val Ala Ser Phe Ser Val
20 25 30

Leu Gly Asp Met Val Ser Gln Ile Gly Gly Pro Tyr Val His Val Thr
35 40 45

Asp Leu Val Gln Pro Asp Gly Asp Pro His Glu Phe Glu Pro Ser Pro
50 55 60

Lys Asp Ser Lys Thr Leu Ala Gln Ala Asp Val Val Phe Val Asn Gly
65 70 75 80

Leu Gly Leu Glu Gly Trp Leu Asp Arg Leu Met Lys Ala Ser Gly Tyr
85 90 95

Arg Gly Glu Val Ile Thr Ala Ser Asn Gly Ile Asp Thr Leu Lys Met
100 105 110

Lys Glu Asp Gly Thr Thr Ile Thr Asp Pro His Ala Trp Asn Ser Met
115 120 125

Lys Asn Gly Ile Val Tyr Ala His Asn Ile Val Asn Gly Leu Ser Lys
130 135 140

Ala Asp Pro Glu His Ala Ser Asp Tyr Arg Lys Gln Gly Asp Ser Tyr
145 150 155 160

Ile Gln Gln Leu Gln Gln Leu Asp Asn Tyr Ala Thr Gln Thr Phe Ala
165 170 175

Ala Ile Pro Arg Glu Lys Arg Lys Val Leu Thr Ser His Asp Ala Phe
180 185 190

Gly Tyr Phe Ala Ala Ala Tyr Gly Val Arg Phe Leu Ser Pro Val Gly
195 200 205

Tyr Ser Thr Glu Ser Glu Ala Ser Ser Lys Asn Val Ala Lys Leu Ile
    210                 215                 220

Asn Gln Ile Lys Arg Glu His Val Lys Leu Tyr Phe Ile Glu Asn Gln
225                 230                 235                 240

Thr Asp Pro Arg Leu Val Lys Gln Ile Ala Asn Ala Ser Gly Ala Gln
                245                 250                 255

Ala Gly Gly Glu Leu Tyr Pro Glu Ala Leu Thr Asp Ser Ser Gly Leu
            260                 265                 270

Ala Ala Thr Tyr Thr Ala Ala Phe Lys His Asn Val Asp Thr Leu Ala
        275                 280                 285

Ala Gly Met Lys
    290

<210> 84
<211> 271
<212> PRT
<213> Escherichia coli

<400> 84
Met Lys Glu Ile Thr Ser Pro Ser Ser Leu Asn Thr Phe Ser Pro Ile
1               5                   10                  15

Ser Ile Ser Ile Arg Asn Ile Ala Thr Asn Lys Phe Leu Ile Ile His
            20                  25                  30

Thr Asp Asn Cys Asp Leu His Ile Glu Asn Glu His Gly Asn Phe Phe
        35                  40                  45

Cys Thr Arg Gly Ser Phe Val Phe Met Ser Arg Asn Met Asn Phe Ser
    50                  55                  60

Cys Arg Ile His Lys Ile Glu Thr Gly Arg Pro Pro Tyr Lys Thr Ile
65                  70                  75                  80

Arg Leu Gly Arg Glu Glu Leu Thr Met Leu Thr Asn Ile Leu His Ser
                85                  90                  95

Thr His Val Tyr Cys Leu Asn Lys His Ile Thr Glu Gly Lys Ser His
            100                 105                 110

Asn Gly Ile Val Cys Ile Gly Gly Asn Lys Asp Trp Gly Tyr Ile Phe
        115                 120                 125

Asn Lys Ile Glu Thr Ser Lys Asp Ser Ala Leu Lys Met Leu Lys Leu
    130                 135                 140

Ser Tyr Leu Ile Ser Arg Met Gly Ile Ala Pro Asp Ile Ile Ile Ser
145                 150                 155                 160

Leu Met Ala Ser Thr Ala Val Thr Phe Thr Glu Lys Ile Arg Asn Gln
                165                 170                 175

Ile Glu Asn Asn Pro Ser Lys Arg Trp Cys Leu Asn Met Ile Ala Asp
            180                 185                 190

Glu Phe Asn Ile Ser Glu Ile Ser Val Arg Lys Arg Leu Glu Ser Glu
        195                 200                 205

Gly Thr Ser Phe Ser Asn Leu Phe Leu Lys Val Arg Met Glu Lys Ser
    210                 215                 220

Met Gln Leu Leu Leu Gln Asn Glu Leu Gln Ile Cys Gln Ile Ser Lys
225 230 235 240

Ser Val Gly Phe Arg Ser Thr Ser Tyr Phe Ile Lys Ile Phe Lys Asp
245 250 255

Tyr Phe Gly Ile Thr Pro Lys Gln Leu Ile Ile Tyr Phe Arg Asn
260 265 270

<210> 85
<211> 205
<212> PRT
<213> Escherichia coli

<400> 85
Val Cys Ser Val Met Lys Lys Arg Leu Leu Phe Met Ile Lys Ser Val
1 5 10 15

Ile Ala Gly Ala Val Ala Met Ala Val Val Ser Phe Gly Val Asn Ala
20 25 30

Ala Ala Thr Val Pro Gln Gly Gln Gly Lys Val Thr Phe Ser Gly Thr
35 40 45

Val Val Asp Ala Pro Cys Gly Ile Asp Ala Gln Ser Ala Asp Gln Ser
50 55 60

Val Asp Phe Gly Gln Ile Ser Lys Val Phe Leu Asp Asn Asp Gly Gln
65 70 75 80

Thr Thr Pro Lys Ala Phe Asp Ile Lys Leu Val Asn Cys Asp Ile Thr
85 90 95

Asn Tyr Lys Lys Pro Ala Thr Gly Ala Ala Pro Gly Gly Ala Pro Lys
100 105 110

Ala Gly Thr Val Ser Met Thr Phe Ser Gly Val Thr Ala Gly Ala Gly
115 120 125

Asn Ala Asn Asp Met Leu Gln Thr Asn Gly Glu Thr Asn Thr Ala Ile
130 135 140

Val Val Ile Asp Pro His Gly Lys Arg Val Lys Phe Asp Gly Arg Thr
145 150 155 160

Ser Thr Gly Val Ser Asn Leu Ile Asn Gly Asp Asn Thr Ile His Phe
165 170 175

Thr Ala Ala Val Met Lys Asp Gly Ser Gly Asn His Val Lys Glu Gly
180 185 190

Ala Phe Ser Ala Val Ala Asn Phe Asn Leu Thr Tyr Gln
195 200 205

<210> 86
<211> 568
<212> PRT
<213> Escherichia coli

<400> 86
Met Asn Val Glu Lys Leu Ile Val Asp His Met Glu Thr Trp Thr Ser
1 5 10 15

Ala Leu Gln Thr Arg Ser Thr Ala Gly Arg Gly Ser Ser Gly Lys Ile

20 25 30

Asp Leu Tyr Gly Ile Lys Lys Leu Arg Glu Leu Ile Leu Glu Leu Ala
35 40 45

Val Arg Gly Lys Leu Val Pro Gln Asp Pro Asn Asp Glu Pro Ala Ser
50 55 60

Glu Leu Leu Lys Arg Ile Ala Ala Glu Lys Ala Glu Leu Val Lys Gln
65 70 75 80

Ala Lys Ile Lys Lys Gln Lys Pro Leu Pro Glu Ile Ser Glu Glu Glu
85 90 95

Lys Leu Phe Glu Leu Pro Glu Gly Trp Glu Trp Val Arg Phe Gly Asn
100 105 110

Ile Tyr Glu Met Glu Tyr Gly Asn Asn Leu Pro Gln Glu Lys Arg Ser
115 120 125

Asn Ser Gly Glu Tyr Asn Val Tyr Gly Ser Asn Gly Val Val Gly Thr
130 135 140

His Asn Glu Ala Cys Ile Lys Ser Pro Cys Ile Ile Ile Gly Arg Lys
145 150 155 160

Gly Ser Ala Gly Ala Leu Asn Leu Ser Asn Gln Pro Ala Cys Trp Val
165 170 175

Thr Asp Val Ala Tyr Ser Thr Ile Pro Pro Ile Ala Met Val Leu Glu
180 185 190

Phe Val Phe Ile Gln Phe His Thr Leu Gly Leu Asp Lys Leu Gly Lys
195 200 205

Gly Ile Lys Pro Gly Leu Asn Arg Asn Asp Ala Tyr Ser Leu Val Ile
210 215 220

Ala Ile Pro Pro Arg Ser Glu Gln Lys Ala Ile Val Ser Lys Val Asn
225 230 235 240

Glu Leu Met Ser Leu Cys Asp Gln Leu Glu Gln Gln Ser Leu Thr Ser
245 250 255

Leu Asp Ala His Gln Gln Leu Val Glu Thr Leu Leu Gly Thr Leu Ala
260 265 270

Asp Ser Gln Asn Ala Glu Glu Leu Ala Glu Asn Trp Ala Arg Ile Ser
275 280 285

Glu His Phe Asp Thr Leu Phe Thr Thr Glu Ala Ser Val Asp Ala Leu
290 295 300

Lys Gln Thr Ile Leu Gln Leu Ala Val Met Gly Lys Leu Val Pro Gln
305 310 315 320

Asp Pro Asn Asp Glu Pro Ala Ser Glu Leu Leu Lys Arg Ile Ala Gln
325 330 335

Glu Lys Ala Gln Leu Val Lys Glu Gly Lys Ile Lys Lys Gln Lys Pro
340 345 350

Leu Leu Pro Ile Ser Asp Glu Glu Lys Pro Phe Glu Leu Pro Asn Gly
355 360 365

```
Trp Glu Trp Cys Arg Leu Gly Glu Leu Ile Asp Ser Ile Asp Ala Gly
    370                 375                 380

Trp Ser Pro Ala Cys Ser Ser Glu Pro Ala Ala Pro Gly Glu Trp Gly
385                 390                 395                 400

Val Leu Lys Thr Thr Ala Val Gln Ser Leu Glu Tyr Arg Glu Tyr Glu
                405                 410                 415

Asn Lys Ala Leu Pro Lys Asn Lys Ala Pro Arg Pro Gln Leu Glu Val
            420                 425                 430

Lys Ala Gly Asp Ile Leu Ile Thr Arg Ala Gly Pro Lys Asn Arg Val
        435                 440                 445

Gly Ile Ser Cys Leu Val Glu Asn Thr Arg Glu Asn Leu Met Ile Ser
    450                 455                 460

Asp Lys Ile Ile Arg Phe His Leu Ile Ser Glu Asp Ile Ser Glu Lys
465                 470                 475                 480

Tyr Ile Ser Leu Cys Leu Asn Tyr Gly Phe Thr Ser Thr Tyr Leu Glu
                485                 490                 495

Asn Ser Lys Ser Gly Met Ala Glu Ser Gln Met Asn Ile Ser Gln Asp
            500                 505                 510

Ile Leu Lys Met Ala Pro Ile Ala Ile Pro Thr Thr His Glu Gln Leu
        515                 520                 525

Lys Ile Thr Asp Lys Ile Asn Glu Met Met Asp Tyr Phe Ile Thr Leu
    530                 535                 540

Lys Ser Gln Ile Gln Ser Ala Gln Gln Thr Gln Leu His Leu Ala Asp
545                 550                 555                 560

Ala Leu Thr Asn Ala Ala Ile Asn
                565

<210>   87
<211>   134
<212>   PRT
<213>   Escherichia coli

<400>   87
Met Ile Arg Leu Ile Phe Ser Phe Leu Leu Cys Thr Leu Cys Ala Gly
1               5                   10                  15

Cys Leu Ser Gln Pro Phe His Ser Cys Ser Ile Val Met Val Asn Thr
            20                  25                  30

Cys Lys Val Ser Ile Ser Ile Ser Thr Val Asn Asn Ser Thr His Tyr
        35                  40                  45

Asp Gly Lys Val Arg Glu Asn Phe His Leu Asp Val Gly Glu Arg Lys
    50                  55                  60

Pro Ile Val Tyr Phe Ile Tyr Thr Gly Arg Glu Ser Asp Val Phe Asn
65                  70                  75                  80

Lys Asn Pro Val Asn Ile Val Ser Glu Leu Ser Ile Leu Ile Ser Asp
                85                  90                  95

Ser Asn Asn Ser Lys Thr Leu Ser Gly Tyr His Ile Ser Ser Leu Leu
            100                 105                 110
```

```
Lys Lys Ser Ser Ser Ser Met Glu Ser Asp Phe Ser Phe Glu Ile Asn
        115                 120                 125

Asp Pro Ser Ile Cys Pro
    130

<210>   88
<211>   129
<212>   PRT
<213>   Escherichia coli

<400>   88
Leu Gln Phe Gln Thr Val Arg Arg Tyr Lys Phe Ala Gln Asn Asn Gln
1               5                   10                  15

Pro Glu Leu Gln Tyr Arg Gln Cys Thr Arg Pro Ala Ser Ala Phe Ala
            20                  25                  30

Leu Pro Ser Lys Ser Leu Leu Ala Gln Tyr Val Asp Val Leu Ser Asp
        35                  40                  45

Val Arg Thr Ser Arg Lys Gln His Val Gly Trp Phe Glu Thr Ile Gln
    50                  55                  60

Met Val Ile Tyr Arg Leu Ile Ser Leu Ala Leu Thr Pro Arg Arg Ser
65                  70                  75                  80

Ser Ser Ser Ile Arg Gly Glu His Pro Ser Arg Arg Gly Asn Lys Ala
                85                  90                  95

Leu Lys Arg Ser Leu Phe Leu Thr Ala Phe Ala Ala Leu Ile Ala Pro
            100                 105                 110

Ile Ser Lys Ala Tyr Tyr Thr His Lys Met Arg Gln Gly Lys Arg His
        115                 120                 125

Asn


<210>   89
<211>   436
<212>   PRT
<213>   Escherichia coli

<400>   89
Met Ser Gly Pro Lys Lys Ile Gly Leu Ile Thr Cys Ile Ala Val Val
1               5                   10                  15

Ala Gly Asn Met Met Gly Ser Gly Ile Ala Leu Leu Pro Ala Ser Leu
            20                  25                  30

Ala Lys Ile Gly Ser Val Ala Ile Trp Gly Trp Ile Ile Ala Ala Ile
        35                  40                  45

Gly Ala Leu Ser Leu Ala Tyr Val Phe Ala Arg Leu Ala Thr Asn Asn
    50                  55                  60

Pro Gln Thr Gly Gly Pro Ile Ala Tyr Ala Gly Glu Val Ala Pro Ile
65                  70                  75                  80

Phe Gly His Gln Thr Ser Ile Leu Tyr Tyr His Ala Asn Trp Ile Gly
                85                  90                  95

Asn Leu Ala Ile Ala Ile Thr Ala Val Ala Tyr Leu Ser Val Phe Phe
```

```
            100                 105                 110

Pro Val Leu Asn Asn Pro Val Pro Ala Ala Ile Thr Ser Ile Ala Met
        115                 120                 125

Val Trp Leu Phe Thr Phe Val Asn Leu Leu Gly Gly Ala Trp Val Ser
    130                 135                 140

Arg Leu Thr Ser Ile Gly Leu Val Leu Val Leu Ile Pro Val Val Gly
145                 150                 155                 160

Thr Ala Leu Phe Gly Trp Gly Ser Phe Asp Ile Glu Val Tyr Arg Gln
                165                 170                 175

Asn Trp Met Ala Gln Pro Asp Gly Asn Val His Gln Ala Val Ile Asn
            180                 185                 190

Ser Val Leu Leu Cys Leu Trp Ala Phe Val Gly Val Glu Ser Ala Ala
        195                 200                 205

Val Ser Ser Asp Leu Val His Asn Pro Ser Arg Thr Val Pro Leu Ala
    210                 215                 220

Thr Leu Gly Gly Thr Ala Leu Ala Gly Ile Val Tyr Ile Ala Ala Ser
225                 230                 235                 240

Gln Val Ile Ala Gly Met Phe Pro Asn Glu Val Val Ala Asn Ser Gly
                245                 250                 255

Ala Pro Phe Ala Leu Ser Ala Ser Arg Ile Val Gly Asp Trp Ala Ala
            260                 265                 270

Pro Phe Val Ser Ala Phe Thr Ala Ile Ala Cys Leu Thr Ser Leu Gly
        275                 280                 285

Ser Trp Met Met Leu Val Gly Gln Ala Gly Arg Arg Ala Ala Met Asp
    290                 295                 300

Gly Asn Phe Pro Ala Val Phe Gly Glu Thr Asp Asn Asn Gly Val Pro
305                 310                 315                 320

Lys Lys Gly Leu Leu Ile Ala Ser Thr Met Met Ser Ile Leu Met Val
                325                 330                 335

Leu Leu Thr Ile Leu Ser Ala Ser Gly Ser Asn Ala Ala Asp Leu Phe
            340                 345                 350

Thr Gln Leu Thr Ser Ile Ala Val Leu Leu Thr Met Phe Pro Tyr Phe
        355                 360                 365

Tyr Ser Ala Ile Asp Leu Ile Arg Phe Glu Ser Ala Thr Ser Lys Ser
    370                 375                 380

Ile Leu Ser Leu Ile Ala Ser Ala Phe Ala Met Leu Phe Cys Phe Ala
385                 390                 395                 400

Ala Leu Ala Gly Ala Glu His Tyr Glu Ile Thr Ala Thr Ile Ile Ile
                405                 410                 415

Ser Leu Leu Ile Phe Ala Phe Tyr Ala Arg Lys Leu Gly Gly Ile Gln
            420                 425                 430

His Lys Asn Ile
        435
```

<210> 90
<211> 404
<212> PRT
<213> Escherichia coli

<400> 90

```
Met Pro Val Arg Leu Asn Thr Ile Pro Asp Ala Glu Pro Tyr Pro Ala
1               5                   10                  15

Pro Pro Ala His Ile Lys Trp Leu Ser Ala Leu Leu Leu Met Leu Ser
            20                  25                  30

Val Gly Val Ala Leu Thr Ser Leu Phe Ala Ser Asp Glu Leu Ala Lys
        35                  40                  45

Asn Gly Pro His Phe Trp Gly Leu Ala Cys Gly Val Pro Ala Phe Ile
    50                  55                  60

Trp Ser Leu Val Ala Ser Val Arg Trp Leu Phe Phe Ile Thr Gln Tyr
65                  70                  75                  80

Ile Arg Ala Asp Ala Trp Asn Lys Arg Arg Glu Glu Val Ile Leu Gln
                85                  90                  95

Glu Thr Arg Arg Gly Arg Arg Ala Leu Gln Ile Leu Ser Phe Ser Val
            100                 105                 110

Gln Thr Ala Leu Asn Gly Asp Ser Val Ala Glu Thr Thr Thr Ala Phe
        115                 120                 125

Leu Ala Arg Gln Gln Val Leu Asn Thr Tyr Ala Asp Leu Arg Gly Glu
    130                 135                 140

Asp Thr Val Arg Arg Ser Val Ile Pro Val Leu Thr Asn Lys Pro Val
145                 150                 155                 160

Thr Gly Arg Leu Ser Glu Ile Ile Ser Arg Leu Phe Phe Asp Ile Arg
                165                 170                 175

Pro Gln Leu Phe Leu Leu Pro Pro Asp Phe His Ile Asn Val Leu Leu
            180                 185                 190

Glu Ile Asp Ala Pro Leu Ser Gly Ala Ser Val Arg Thr Ile Trp Gln
        195                 200                 205

Asp Glu Trp Gln Lys Ala Gly Leu Pro Glu Ala Arg Leu Phe Ser Ala
    210                 215                 220

Pro Glu Pro Gly Leu Ala Ala Val Asp Asp Trp Leu Asp Asn Phe Ile
225                 230                 235                 240

Gln Glu Lys Ala Val Leu Leu Val Ile Ser Val Arg Leu Glu Pro Lys
                245                 250                 255

Asn Pro Glu Arg Thr Ala Glu Ser Ala Thr Ala Leu Leu Leu Ala Asn
            260                 265                 270

Arg Leu Thr Gln Thr Ala Leu Thr Pro Leu Ala Leu Leu His Arg Pro
        275                 280                 285

Glu Arg Ile Thr Asp Thr Glu Met Met Ala Ser Gly Ile Ala Gln Ala
    290                 295                 300

Leu Asp Trp Met Pro Val Gln Pro Asp Ala Ile Ser Gly Met Trp Thr
305                 310                 315                 320
```

```
Ala Glu Leu Asp Arg Glu Gln Arg Ala Ala Leu Leu Ser Leu Asn Gln
                325                 330                 335

Pro Phe Ala Gln Glu Ala Leu Met Tyr Glu Leu Asp Ala Phe Leu Gly
            340                 345                 350

Arg Ser Gly Pro Ala Ala Pro Trp Leu Ser Val Ala Ala Ala Thr Leu
        355                 360                 365

Ala Ala Ile Gln Ser Gln His Pro Gln Leu Thr Leu Ser Gly Val Gln
    370                 375                 380

Gly Gly His Tyr Ser Trp Ala Thr Val Val Ser Pro Phe Val Ser Pro
385                 390                 395                 400

Gln Glu Ala Ser


<210>   91
<211>   208
<212>   PRT
<213>   Escherichia coli

<400>   91
Met Arg Arg Phe Cys Thr Val Leu Val Leu Leu Ser Met Ala Tyr Phe
1               5                   10                  15

Ile Ile Gly Gly Leu Phe Ala Tyr Leu Asn Lys Leu Glu Leu Pro Ile
            20                  25                  30

Tyr Leu Asn Gly Met Ala Ile Val Gly Phe Val Met Thr Ile Val Cys
        35                  40                  45

Ile Tyr Ser Leu Ser Lys Pro Ala Ile Thr Gln Ser Asp Leu Glu Gln
    50                  55                  60

Leu Glu Ala Thr Ser Leu Glu Ser Ile Thr Lys Thr Ile Ser Glu Leu
65                  70                  75                  80

Lys Ser Leu Lys Thr Asn Lys Asn Ser Thr Gln Glu Glu Ile Leu Asp
                85                  90                  95

Leu Glu Lys Lys Arg Lys Glu Met Glu Leu Leu Val Lys Lys Ala Ser
            100                 105                 110

Met Ala Leu Phe Phe Arg Glu Gln Leu Asn Tyr His Glu Arg Arg Ile
        115                 120                 125

Leu Ser Glu Ile Lys Gly Ser Glu Thr Leu Asn His Ser Leu Ser Glu
    130                 135                 140

Ile Lys Glu Ile Lys Gly Lys Leu Lys Ala Leu Asp Glu Glu Ile Ala
145                 150                 155                 160

Cys Asp Pro Glu Val Glu Phe Leu Arg Ser Ile Leu Leu Glu Ala Ser
                165                 170                 175

Arg Arg Thr Pro Thr Leu Asp Glu Leu Thr Glu Gly Met Pro Phe Ile
            180                 185                 190

Phe Arg Ile Met Ala Arg Ser Thr Gln Leu Ile Leu Gly Asn Lys Ile
        195                 200                 205
```

<210> 92
<211> 258
<212> PRT
<213> Escherichia coli

<400> 92
Met Ala Arg Ser Gly Phe Glu Val Gln Lys Ala Ala Val His Ala Leu
1 5 10 15

Phe Leu Arg Glu Leu Arg Thr Arg Phe Gly Lys Tyr Arg Leu Gly Tyr
20 25 30

Leu Trp Ala Val Leu Glu Pro Ala Ala His Leu Leu Ile Met Leu Ala
35 40 45

Ile Phe Gly Phe Phe Met His Arg Thr Met Pro Asp Ile Ser Phe Pro
50 55 60

Val Phe Leu Ile Asn Gly Ile Ile Pro Tyr Phe Ile Phe Ser Asn Ile
65 70 75 80

Ala Thr Arg Ser Ile Gly Ala Ile Glu Ala Asn Gln Gly Leu Phe Asn
85 90 95

Tyr Arg Pro Val Arg Pro Ile Asp Thr Ile Ile Ala Arg Ala Ile Leu
100 105 110

Glu Val Leu Ile Tyr Ser Ile Val Tyr Leu Val Leu Met Ser Leu Leu
115 120 125

Leu Ile Ile Gly Glu Gln Phe Lys Ile Tyr Asn Ile Leu Leu Leu Phe
130 135 140

Ser Ala Trp Ile Leu Leu Ala Leu Phe Ser Cys Gly Ile Gly Leu Ile
145 150 155 160

Phe Met Val Ile Gly Lys Thr Phe Pro Glu Thr Glu Lys Phe Leu Pro
165 170 175

Ile Ile Leu Lys Pro Leu Tyr Phe Val Ser Cys Ile Met Leu Pro Leu
180 185 190

His Ala Ile Pro Lys Gly Tyr Trp Gly Tyr Ile Leu Trp Asn Pro Ile
195 200 205

Val His Val Val Glu Leu Cys Arg Glu Ser Val Val Thr Gly Tyr Val
210 215 220

Ser Glu Gly Val Ser Leu Tyr Tyr Leu Phe Ile Ser Thr Leu Ile Leu
225 230 235 240

Leu Phe Leu Gly Leu Ala Leu Tyr Ser Ser Arg Glu Glu Tyr Met Leu
245 250 255

Thr Ser

<210> 93
<211> 308
<212> PRT
<213> Escherichia coli

<400> 93
Met Gly Glu Lys Cys Arg Tyr Thr Pro Phe Pro His Gly Cys Ser His
1 5 10 15

Ala Thr Ala Ala Gly Ala Ala Ala Met Met Leu Leu His Leu Leu Cys
20 25 30

Glu Pro Phe Gly Asp Phe Gly Phe Met Arg Arg Ala Leu Val Gly Cys
35 40 45

Leu Ala Leu Thr Leu Ser Ala Ala Pro Leu Gly Cys Phe Leu Leu Leu
50 55 60

Arg Arg Met Ser Leu Ile Gly Asp Ala Leu Ser His Ala Val Leu Pro
65 70 75 80

Gly Val Ala Ile Gly Tyr Leu Val Ser Gly Met Ser Leu Val Ala Met
85 90 95

Gly Val Gly Gly Phe Ile Ala Gly Leu Ser Val Ala Met Leu Ser Gly
100 105 110

Val Val Ser Arg Arg Thr Gly Leu Arg Glu Asp Ala Ser Phe Ala Gly
115 120 125

Phe Tyr Leu Gly Ser Leu Ala Leu Gly Val Thr Leu Val Ser Leu Arg
130 135 140

Gly Ser Ser Val Asp Leu Leu His Val Leu Phe Gly Ser Ile Leu Ala
145 150 155 160

Ile Asp Ala Asn Ala Leu Ile Thr Ile Gly Ile Ile Ser Ser Gly Ser
165 170 175

Val Leu Val Leu Ala Leu Ile Tyr Arg Val Leu Val Ile Glu Ser Phe
180 185 190

Asp Val Thr Phe Leu Lys Val Leu Ser Arg Arg Ser Arg Ala Leu Ile
195 200 205

His Cys Leu Phe Leu Ser Met Val Val Leu Asn Leu Val Ala Gly Phe
210 215 220

Gln Leu Leu Gly Thr Leu Met Thr Val Gly Ile Met Met Leu Pro Ala
225 230 235 240

Ala Ser Ala Arg Phe Trp Ser Gln Arg Leu Ser Ile Met Leu Leu Ala
245 250 255

Ala Val Gly Ile Gly Thr Cys Ala Ser Leu Val Gly Leu Thr Trp Ser
260 265 270

Tyr Tyr Ala Asp Leu Pro Ala Gly Pro Ala Val Ile Leu Thr Ser Thr
275 280 285

Leu Phe Phe Cys Phe Ser Val Leu Phe Gly Ser Asn Gly Gly Met Leu
290 295 300

Cys Arg Cys Arg
305

<210> 94
<211> 142
<212> PRT
<213> Escherichia coli

<400> 94
Met Ile Asn Ile Thr Thr Lys Asp Gly Thr Gln Val Ser Gly Glu Val

```
1               5                   10                  15

Arg Asp Leu Val Ile Leu Leu Glu Thr Asp Gly Lys Thr Val Ser Asp
            20                  25                  30

Tyr Leu Gln Pro Ile Pro Pro Lys Lys Asp Thr His Trp Ser Met Ile
        35                  40                  45

Phe Ile Pro Val Ala Ile Tyr Ile Leu Leu Asn Leu Val Tyr Ile Ile
    50                  55                  60

Ala His Pro Leu Phe Pro Glu Lys Tyr Leu Lys Thr Ser Thr Ser Ile
65                  70                  75                  80

Phe Leu Phe Ile Ser Phe Ile Leu Ser Ala Ile Val Ala Tyr Phe Val
                85                  90                  95

Phe His Lys His Lys Ser Ala Thr Leu Ser Ile Cys Ser Leu Val Phe
            100                 105                 110

Leu Val Leu Ile Val Gly Ala Asn Val Gly Phe Ile Ser Tyr Ser Asp
        115                 120                 125

Leu Thr Gln Lys Ala Thr Gln Lys Leu Asp Ser Leu Ser Lys
    130                 135                 140

<210>   95
<211>   401
<212>   PRT
<213>   Escherichia coli

<400>   95
Met Asn Gln Ser Glu Gln Arg Lys Lys Ile Leu Val Leu Thr Pro Arg
1               5                   10                  15

Phe Pro Tyr Pro Val Ile Gly Gly Asp Arg Leu Arg Val Tyr Met Leu
            20                  25                  30

Cys Lys Glu Leu Ser Lys Lys Tyr Asp Leu Ile Leu Leu Ser Leu Cys
        35                  40                  45

Asp Gln Pro Leu Glu Leu Glu Ile Asn Ile Asn Asp Ser Val Phe Lys
    50                  55                  60

Glu Ile His Arg Val Tyr Leu Pro Lys Tyr Lys Ser Tyr Tyr Asn Val
65                  70                  75                  80

Leu Lys Ala Leu Val Thr Gln Lys Pro Leu Gln Ile Ala Tyr Tyr Gln
                85                  90                  95

Ser Asp Thr Phe Lys Asn Lys Tyr Asn Lys Leu Ile Lys Gln Cys Asp
            100                 105                 110

Ala Val Phe Cys His Leu Ile Arg Val Ala Asp Tyr Val Lys Asp Thr
        115                 120                 125

Asp Lys Phe Lys Ile Leu Asp Met Thr Asp Ala Ile Ser Leu Asn Tyr
    130                 135                 140

Ser Arg Val Lys Lys Leu Ala Ser Lys Lys Ser Leu Arg Ala Ile Ile
145                 150                 155                 160

Tyr Ser Leu Glu Gln Lys Arg Leu Glu Ser Tyr Glu Arg Ser Val Ala
                165                 170                 175
```

```
Asn Leu Phe Asp Leu Thr Thr Phe Ile Ser Ser Val Asp Arg Asp Tyr
            180                 185                 190

Leu Tyr Pro Asn Pro Gly Ser Asn Ile His Ile Val Asn Asn Gly Val
        195                 200                 205

Asp Thr Ser Ala Leu Arg Tyr Ile Lys Arg Glu Ile Lys Ile Asp Lys
    210                 215                 220

Pro Val Glu Leu Ile Phe Ile Gly Asn Met Tyr Ser Leu Gln Asn Met
225                 230                 235                 240

Asp Ala Ala Lys His Phe Ala Lys Asn Ile Leu Pro Cys Leu Tyr Asp
                245                 250                 255

Glu Phe Asn Ile Ile Phe Lys Val Ile Gly Lys Ile Ser Glu Thr Asn
            260                 265                 270

Lys Asn Ile Leu Asn Ser Phe Lys Asn Thr Ile Ala Leu Gly Thr Val
        275                 280                 285

Asp Asp Ile Asn Ser Ser Ala Ser Thr Gly His Ile Gly Ile Cys Pro
    290                 295                 300

Val Arg Leu Gly Ala Gly Val Gln Asn Lys Ile Leu Glu Tyr Met Ala
305                 310                 315                 320

Leu Gly Leu Pro Cys Ile Thr Ser Ser Ile Gly Tyr Glu Gly Ile Asn
                325                 330                 335

Ala Lys Ser Gly Ser Glu Ile Phe Val Ala Asp Thr Val Glu Gln Tyr
            340                 345                 350

Lys Asn Val Leu Arg Glu Ile Ile Tyr Asp Tyr Asn Arg Tyr Thr Glu
        355                 360                 365

Val Ala Glu Asn Ala Arg Ser Phe Val Glu Asn Asn Phe Ser Trp Glu
    370                 375                 380

Ser Lys Val Ala Asn Leu Met Asn Thr Leu Asp Glu Lys Leu Tyr Glu
385                 390                 395                 400

Gln


<210>   96
<211>   129
<212>   PRT
<213>   Escherichia coli

<400>   96
Met Gly Gly Ala Val Asn Gln Thr Thr Ile Asn Asn Gly Val Leu Gln
1               5                   10                  15

Val Tyr Gly Ala Ala Thr Asp Pro Thr Ile Lys Gly Gly Arg Leu Ile
            20                  25                  30

Val Glu Lys Asp Gly Ile Ala Val Phe Ala Val Ile Glu Lys Gly Gly
        35                  40                  45

Leu Leu Glu Val Lys Ala Thr Gly Asp Phe Lys Ile Phe Val Thr Asp
    50                  55                  60

Thr Gly Ala Ser Pro Ala Ala Gly Asp Asn Leu Thr Leu Val Thr Thr
65                  70                  75                  80
```

```
Gly Ala Val Met Arg His Leu Arg Trp Ala Met Pro Glu Ala Leu Leu
                85                  90                  95

Ile Ser Val Arg Met Asn Ile Pro Cys Trp Ile Met Ala Thr Ile Ala
            100                 105                 110

Gly Val Ser Gln Arg Ile Ala Tyr Lys Cys Pro Leu Gln Pro Arg Met
        115                 120                 125

Cys


<210>   97
<211>   2683
<212>   PRT
<213>   Escherichia coli

<400>   97
Met Ser Leu Thr Leu Asn Lys Ile Leu Val Leu Cys Ala Leu Leu Ile
1               5                   10                  15

Ser Ala Met Leu Pro Gly Trp Ser Trp Ala Glu Ser Ala Trp Gln Asp
            20                  25                  30

Ser Ser Asp Thr Val Gly Glu Phe Asn Gly Thr Val Pro Thr Ala Asp
        35                  40                  45

Ser Ala Ser Ile Pro Val Tyr Gln Gly Ser Val Phe Leu Asp Pro Ala
    50                  55                  60

Lys Thr His Asp Val Ala Phe Thr Ala Lys Pro Ser Glu Phe Ser Ala
65                  70                  75                  80

Asp Val Ser Val Ser Lys Leu Leu Val Thr Asn Pro Gln Asp Arg Glu
                85                  90                  95

Gly Asp Ile Ile Ala Thr Pro Arg Trp Glu Asn Gln Thr Pro Pro Ala
            100                 105                 110

Ile Ser Leu Val Trp Ala Asp Ala Ala Thr Pro Gly Thr Leu Leu Asp
        115                 120                 125

Pro Gln Pro Val Ala Asp Arg Ser Phe Cys Ala Gln Gly Leu Ala Gly
    130                 135                 140

Arg Ser Leu Val Ala Trp Ala Gln Pro Asp Pro Gln Gln Thr Met Pro
145                 150                 155                 160

Leu Leu Tyr Leu Leu Thr Ser Thr Gly Tyr Pro Tyr Glu Ser Val Leu
                165                 170                 175

Thr Leu Ala Asp Gln Lys Val Thr Leu Lys Ile Ala Pro Ala Gln Gly
            180                 185                 190

Asp Leu Ile Ser Val Ser Ala Ala Gly Tyr Asp Glu Ser Ser Gly Ala
        195                 200                 205

Ala Lys Met Thr Val Gly Gly Ser Ile Thr Leu Thr Val Thr Thr Lys
    210                 215                 220

Asp Cys Val Gly Asn Val Val Gly Asn Ile Pro Phe Val Ile Lys Arg
225                 230                 235                 240

Lys Asp Ala Glu Asn Arg Gln Gly Val Val Asn Asn Thr Ala Pro Val
```

```
                245                 250                 255

Lys Leu Gly Thr Thr Glu Leu Thr Thr Thr Ala Thr Glu Tyr Arg Gly
            260                 265                 270

Thr Thr Asp Ala Asn Gly Val Ala Thr Val Thr Val Thr Gln Ala Asn
        275                 280                 285

Gly Pro Gly Val Lys Thr Pro Leu Val Ala Ser Leu Ala Gly Ile Ala
    290                 295                 300

Gln Ala Ser Glu Thr Ala Val Ile Phe Thr Val Leu Thr Ser Pro Asp
305                 310                 315                 320

Val Pro Gln Ala Thr Met Trp Gly His Met Pro Asp Thr Leu Lys Ala
                325                 330                 335

Arg Asp Tyr Thr Phe Ser Arg Pro Lys Leu Ala Ala Glu Val Asp Asn
            340                 345                 350

Glu Asp Gly Thr Val Asn Asp His Asn Glu Thr Trp Ser Thr Phe Thr
        355                 360                 365

Trp Ser Gly Ala Asp Lys His Cys Asp Ile Leu Pro Gly Met Arg Gln
    370                 375                 380

Phe Gly Ala Leu Ala Thr Val Val Pro Thr Ser Val Gln Asp Val Ala
385                 390                 395                 400

Gly Trp Pro Met Gln Gly Asn Phe Tyr Trp Ser Ser Leu Ala Gly Met
                405                 410                 415

Ser Gly Gln His His Ala Ala Asp Val Ser Asn Arg Ser Glu Ala Gln
            420                 425                 430

Lys Pro Asp Asp Thr Thr Phe Ile Val Ser Cys Val Asp Lys Glu Ala
        435                 440                 445

Pro Asp Val Glu Pro Lys Leu Val Leu Thr Pro Gly Ser Tyr Asp Ser
    450                 455                 460

Thr Ile Lys Ala Met Lys Val Lys Val Gly Glu Glu Ala Ser Leu Arg
465                 470                 475                 480

Leu Thr Ile Thr Asp Ser Lys Asn Asn Asp Gln Pro Leu Ala Tyr Tyr
                485                 490                 495

Tyr Phe Ser Leu His Leu Asp Asp Gly Ile Asn Arg Lys Asn Gln Thr
            500                 505                 510

Asp Ala Ala Trp Glu Thr His Pro Val Gln Ile Asp Gly Gly Ser Asn
        515                 520                 525

Val Arg Lys Val Asp Ala His Thr Tyr Glu Gly Ile Thr Asp Ala Asn
    530                 535                 540

Gly Glu Ala Thr Leu Thr Leu Thr Gln Pro Gly Gly Val Gly Val Lys
545                 550                 555                 560

Thr His Ile Thr Ala Arg Met Arg Ser Asp Phe Thr Ala Ser Asp Glu
                565                 570                 575

Lys Asp Val Ile Phe Thr Val Ile Thr Ser Pro Asp Thr Asp Lys Ala
            580                 585                 590
```

Arg Met Trp Gly His Met Leu Gly Ile Ile Glu Ala Asn Asn Ile Phe
595 600 605

Lys Arg Pro Arg Leu Ala Asp Glu Thr Asp Asn Glu Leu Gly Ser Val
610 615 620

Arg Glu Asn Asn Glu Asp Trp Ala Leu Phe Asp Gln Asn Ser Ser Met
625 630 635 640

Gln Ala Glu Cys Gly Leu Gly His Ile Pro Ser Gln Ser Ser Leu His
645 650 655

Ser Leu Phe Ala Ala His Pro Ala Asn Ala Ile Gly Thr Glu Tyr Gly
660 665 670

Trp Pro Thr Leu Gln Lys Ala Tyr Leu Ser Ala Val Glu Glu Thr Ser
675 680 685

His Ala Ser Val Asn Leu Ala Thr Gly Asn Ile Asp Thr Tyr Ser Gly
690 695 700

Phe Lys Gln Asn Tyr Leu Ser Cys Ser Gly Asn Glu Met Val Ala Lys
705 710 715 720

Ile Ala Ala Thr Thr Asp Arg Asp Val Ser Ala Gly Ser Arg Ala Gln
725 730 735

Ala Lys Val Gly Asp Thr Ile Thr Met Thr Val Arg Thr Phe Asn Ala
740 745 750

Leu Asn Asn Ala Pro Val Pro Tyr Thr Ala Phe Thr Ile Thr Lys Asp
755 760 765

Met Gly Lys Asn Arg Gln Gly Gln Thr Thr Gly Phe Asp Asp Pro Thr
770 775 780

Arg Gly Ala Ile Glu Met Asn Gly Thr Leu Tyr Gly Thr Ser Gln Pro
785 790 795 800

Ser Leu Val Tyr Ala Gly Thr Thr Asp Ala Gln Gly Phe Ala Thr Val
805 810 815

Glu Ile Lys Gln Ser Gln Gly Val Gly Leu Ser Thr Pro Leu Asn Ile
820 825 830

Val Pro Val Asn Ser Tyr Ile Pro Asn Thr Val Asn Tyr Asn Val Ile
835 840 845

Phe Thr Thr Leu Thr Ser Pro Asp Ala Val Gly Ala Gln Met Trp Gly
850 855 860

His Met Asp Glu Thr Ile Thr Val Asp Ala Leu Thr Phe Ala Arg Pro
865 870 875 880

Arg Leu Ala Ala Glu Val Phe Ser Pro Asp Gly Thr Leu Thr Glu Asn
885 890 895

Asn Glu Val Trp Ser Arg Val Ser Gln Ala Asn Ala Ser Ser Thr Ser
900 905 910

Lys Gly Gly Cys Gly Ala Asn Met Leu Pro Arg Arg Ser Gln Leu Ser
915 920 925

Ala Leu Tyr Asp Ala Asn Asn Gly Asn Gly Val Gln Thr Val His Gly
930 935 940

Trp Pro Thr Gln Arg Gln Pro Tyr Trp Ser Ser Ser Pro Ala Asp Gln
945 950 955 960

Val Pro His Tyr Tyr Thr Ile Ala Leu Asn Asp Gly Ala Arg Thr Val
965 970 975

Gly Gly Ser Thr Ala Val Tyr Val Ser Cys Leu Thr Thr Ala Asn Asn
980 985 990

Pro Ala Ser Ser Ile Thr Leu Glu Val Val Asp Pro Ala Gln Trp Asn
995 1000 1005

Ala Ala Ala Asn Ala Ala Lys Leu Lys Lys Gly Glu Thr Leu Gln Val
1010 1015 1020

Lys Val Thr Val Lys Asp Ala Gln Gly Asn Pro Leu Gly Asp Ile Pro
1025 1030 1035 1040

Phe Thr Leu Lys Arg Gly Asp Gly Tyr Thr Arg Ser Glu Glu Lys His
1045 1050 1055

Val Ala Gly Ser Gly Asp Ala Leu Val Ala Pro Val Val Val Asn Gly
1060 1065 1070

Gly Leu Ala Asp Glu Thr Ser Leu Asn Asn Thr Ala Ala Ala Tyr Ser
1075 1080 1085

Ala Ile Thr Gly Ser Asp Gly Thr Lys Ile Leu Thr Ile Thr Arg Pro
1090 1095 1100

Asp Thr His Gly Thr Lys Thr Ser Leu Thr Ala Thr Leu Tyr Ser Asp
1105 1110 1115 1120

Thr Thr Lys Lys Ala Thr Leu Asp Thr Ile Phe Thr Val Val Thr Ser
1125 1130 1135

Pro Asp Ser Asp Lys Ala Lys Met Trp Gly His Met Pro Glu Thr Val
1140 1145 1150

Thr Ala Ala Asp Gly Thr Val Phe Lys Arg Pro Leu Leu Leu Lys Glu
1155 1160 1165

Leu Ser Ser Thr Ser Gly Arg Thr Ala Ile Ala Glu Glu Asn Glu Asp
1170 1175 1180

Trp Ala Gln Phe Thr Gln Thr Gln Ala Ile Ser Thr Ser Ser Asn Gly
1185 1190 1195 1200

Cys Gly Ser Glu Tyr Val Pro Ser Gln Ala Gly Leu Glu Ser Leu Tyr
1205 1210 1215

Glu Ala Asn Arg Gly Asn Ala Met Lys Thr Val Gln Gly Trp Pro Val
1220 1225 1230

Ala Ser Ser Tyr Leu Ser Ser Thr Thr Gly Ser Ser Ser Leu Glu Gln
1235 1240 1245

Arg Asp Phe Lys Ala Val Asn Leu Ser Ser Gly Thr Ser Ser Ile Ile
1250 1255 1260

Pro Ser Ala Thr Lys Glu Leu Leu Thr Cys Gln Thr Thr Pro Ile Val
1265 1270 1275 1280

Lys Ala Ser Gln Ile Val Leu Glu Ala Ala Asp Pro Thr Lys Phe Asp

1285 1290 1295

Ser Thr Asn Asn Val Val Lys Ala Lys Lys Gly Glu Glu Ala Val Leu
1300 1305 1310

Arg Val Thr Thr Lys Asp Ala Gln Gly Asn Pro Val Gly Asn Thr Ala
1315 1320 1325

Phe Thr Leu Lys Arg Asn Thr Ser Val Asn Arg Ala Asn Val Ser Thr
1330 1335 1340

Thr Thr Ser Ile Ala Ser Leu Ala Val Thr Asp Ala Trp Gly Asn Thr
1345 1350 1355 1360

Gln Asp Asp Phe Leu Ser Thr Thr Leu Val Ile Tyr Gly Val Thr Gly
1365 1370 1375

Ala Asp Gly Ile Thr Thr Phe Thr Leu Lys Gln Asp Gln Thr Thr Gly
1380 1385 1390

Leu Lys Thr Glu Leu Thr Ala Ala Leu Asp Ser Ser Ser Ser Thr Lys
1395 1400 1405

Ser Thr Leu Pro Val Val Phe Thr Val Leu Thr Ser Pro Asp Ser Pro
1410 1415 1420

Lys Ala Lys Phe Trp Gly His Met Ala Glu Thr Ala Thr Gly Asp Asp
1425 1430 1435 1440

Gly Leu Ile Tyr Arg Arg Pro Leu Leu Arg Asp Glu Asn Ser Ala Thr
1445 1450 1455

Thr Ser Ile Gly Thr Leu Val Glu Glu Gly Glu Ala Trp Ser Thr Phe
1460 1465 1470

Pro Ser Gly Gln Ala Asn Asp Thr Ser Ile Asn Gly Cys Gly Ala Glu
1475 1480 1485

Tyr Val Pro Thr Asp Asn Glu Leu Arg Ala Ile Tyr Ala His Gln Gly
1490 1495 1500

Ser Ser Ala Leu His Asp Ala Ile Gly Trp Pro Val Ser Arg Phe Tyr
1505 1510 1515 1520

Ile Ser Asn Thr Val Ala Asp Thr Phe Thr Gln Thr Phe Thr Tyr Asp
1525 1530 1535

Val Val Ser Leu Lys Thr Gly Asp Glu Thr Gln Met Pro Ser Ser Gly
1540 1545 1550

Gly Ala Leu Leu Ser Cys Arg Thr Thr Pro Val Ala Val Ala Ser Gln
1555 1560 1565

Ile Ile Val Glu Ala Asn Asp Thr Ala Gln Phe Val Lys Val Asp Asp
1570 1575 1580

Thr Leu Ser Ala Leu Lys Val Lys Lys Gly Glu Asp Ala Val Ile Arg
1585 1590 1595 1600

Val Val Thr Lys Asn Ala Gln Gly Asn Ser Val Pro Asn Val Pro Phe
1605 1610 1615

Ile Leu Arg Arg Glu Gly Ser Lys Asn Arg Gln Asn Ala Glu Met Ile
1620 1625 1630

Asn Lys Ser Ile Thr Val Ile Asn Ala Ala Gly Ala Ser Ala Arg Met
1635 1640 1645

Asn Ser Ser Ser Ser Leu Leu Tyr Gly Val Thr Gly Ala Asp Gly Thr
1650 1655 1660

Thr Ser Phe Thr Val Lys Gln Asp Asp Ser Met Gly Leu Val Thr Asn
1665 1670 1675 1680

Met Tyr Ala Gln Leu Tyr Gln Leu Thr Ile Glu Ser Asn Lys Leu Pro
1685 1690 1695

Val Met Phe Thr Val Ile Thr Ser Pro Asp Thr Pro Leu Ala Ser Tyr
1700 1705 1710

Trp Gly His Met Ser Glu Thr Phe Thr Thr Arg Ser Gly Ile Ala Phe
1715 1720 1725

Lys Arg Pro Leu Leu Thr Ala Glu His Pro Ala Gly Gln Ser Thr Met
1730 1735 1740

Ala Asn Asn Glu Ser Trp Leu Ser Leu Asn Thr Ala Ala Lys Asn Asp
1745 1750 1755 1760

Val Ser Lys Ser Asp Cys Gly Glu Pro Tyr Gln Pro Leu Leu Ser Glu
1765 1770 1775

Phe Gln Glu Leu Tyr Ser Glu His Pro Asn Gly Ala Ile Gly Thr Asp
1780 1785 1790

Leu Gly Leu Pro Leu Thr Asn Thr Trp Trp Ala Tyr Asp Lys Ile Ala
1795 1800 1805

Tyr Ala Asn Val Trp Tyr Asp Gln Ser Ile Asn Leu Ser Asn Gly Ser
1810 1815 1820

Ser Ser Arg Ala Leu Ser Asn Thr Val Ala Phe Val Ser Cys Leu Val
1825 1830 1835 1840

Asn Pro His Ala Val Ala Ala Ser Ile Lys Met Thr Ser Thr Ala Leu
1845 1850 1855

Asp Ala Glu Lys Thr Ala Ser Asn Asp Gly Arg Pro Ser Ala Thr Ala
1860 1865 1870

Ala Lys Gly Thr Ala Ile Arg Met Thr Val Ile Val Arg Asp Ser Gly
1875 1880 1885

Gly Asn Leu Leu Pro Gly Ala Asn Phe Asn Leu Ile Arg Gly Thr Ala
1890 1895 1900

Leu Asp Arg Ala Lys Asn Arg Leu Asp Ser Thr Tyr Asp Asp Leu Thr
1905 1910 1915 1920

Ile Val Pro Val Thr Pro Ala Gly Val Asn Met Ser Leu Tyr Asn Asn
1925 1930 1935

Gly Ala Gln Ala Leu Leu Thr Thr Gly Ser Asp Gly Lys Ala Thr Phe
1940 1945 1950

Asp Val Thr Gln Asn Glu Thr Tyr Gly Leu Ala Thr Pro Leu Thr Ala
1955 1960 1965

Thr Leu Met Arg Asp Thr Thr Lys Ser Ala Thr Met Asp Val Ile Phe
1970 1975 1980

Thr Val Ile Thr Ser Pro Asn Ser Pro Lys Ala Lys Tyr Trp Gly His
1985 1990 1995 2000

Met Pro Asp Thr Phe Thr Ser Arg Ala Gly Val Thr Phe Lys Arg Pro
2005 2010 2015

Leu Leu Ala Ala Glu Ala Thr Leu Gly Ser Ser Val Ser Asn Asn Asn
2020 2025 2030

Glu Ser Trp Ser Tyr Leu Phe Tyr Thr Asn Lys Val Thr Pro Asp Cys
2035 2040 2045

Pro Val Glu Tyr Gln Pro Arg Leu Asn Glu Leu Gln Gly Leu Tyr Asn
2050 2055 2060

Asp His Pro Gly Gly Thr Ile Leu Thr Asp Leu Gly Leu Pro Ile Thr
2065 2070 2075 2080

Ala Gly Ser Gly Asn Trp Trp Thr Tyr Glu Met Ser Thr Thr Asp Ala
2085 2090 2095

Leu Thr Trp Tyr Tyr Gly Val Ile Asn Leu Lys Thr Gly Gln Ser Thr
2100 2105 2110

Thr Thr Ile Asn Gly Tyr Ala Leu Met Leu Cys Leu Thr Gln Pro His
2115 2120 2125

Ser Ala Pro Ala Ser Leu Thr Leu Ser Ser Thr Ala Tyr Asp Glu Gly
2130 2135 2140

Arg Thr Ala Ser Asn Gly Gly Thr Pro Thr Ser Ser Val Lys Lys Gly
2145 2150 2155 2160

Glu Met Leu Pro Ile Val Val Thr Ile Lys Asp Ala Asn Gly Asn Pro
2165 2170 2175

Val Gly Gly Glu Gly Val Thr Leu Lys Arg Val Gln Ala Lys Ser Arg
2180 2185 2190

Ser Gly Ile Ser Val Ser Ser Asn Thr Val Asp Asp Leu Ile Leu Asp
2195 2200 2205

Glu Val Thr Pro Thr Ser Ala Arg Ile Ser Phe Asn Gln Asn Thr Ser
2210 2215 2220

Ala Trp Ser Gly Phe Thr Gly Ser Asp Gly Thr Ile Thr Phe Asn Val
2225 2230 2235 2240

Thr Gln Asn Asn Thr Val Gly Leu Val Thr Pro Phe Thr Ala Ser Leu
2245 2250 2255

Ala Arg Asn Pro Gln Val Thr Ala Asn Gln Asp Leu Ile Phe Thr Val
2260 2265 2270

Val Thr Ser Pro Asp Ser Ala Lys Ala Asn Tyr Trp Gly His Met Pro
2275 2280 2285

Ala Thr Leu Thr Ala Val Asn Gly Ala Val Phe Glu Arg Pro Lys Leu
2290 2295 2300

Trp Ser Glu Leu Thr Ser Thr Ser Gly Val Gly Lys Ile Asn Asn Asn
2305 2310 2315 2320

Asn Glu Asp Trp Pro Tyr Phe Thr Pro Thr Gln Lys Ser Asp Ala Ser

2325 2330 2335

Val Ser Pro Cys Glu Val Ala Arg Gln Pro Leu Phe Asn Asp Leu Ser
2340 2345 2350

Ser Leu Ser Ala Arg Tyr Pro Asn Asn Thr Phe Val Thr Glu Thr Gly
2355 2360 2365

Trp Pro Ala Tyr Tyr Thr Trp Trp Ala Glu Asp Lys Ser Ala Asp Gly
2370 2375 2380

Lys Asp Gln Ser Val Asp Leu Arg Asn Gly Thr Leu Tyr Thr Gly Ser
2385 2390 2395 2400

Thr Lys Ser Phe Gln Pro Cys Leu Ala Asn Ala Arg Ser Thr Val Ser
2405 2410 2415

Ser Val Thr Leu Thr Ser Thr Ala Phe Asp Ala Asp Ser Gln Ala Ala
2420 2425 2430

Lys Val Lys Lys Gly Glu Ala Met Ser Val Thr Val Thr Val Lys Asp
2435 2440 2445

Ser Ala Gly Asn Thr Val Pro Asn Val Glu Phe Thr Leu Lys Arg Gly
2450 2455 2460

Glu Ala Ser Pro Arg Asn Ala Gly Ala Thr Leu Tyr Gly Asn Val Val
2465 2470 2475 2480

Ala Met Asp Asp Leu Ile Val Gln Pro Leu Ser Gly Ser Ala Ile Thr
2485 2490 2495

Leu Ser Glu Ser Gly Asn Thr Ile Ser Gly Met Thr Gly Ala Asp Gly
2500 2505 2510

Thr Ala Ser Phe Ser Leu Arg Gln Asp Asn Thr Pro Gly Tyr Lys Met
2515 2520 2525

Pro Leu Thr Val Thr Leu Ala Asn Tyr Ala Ser Ala Thr Asp Thr Leu
2530 2535 2540

Asp Ala Ile Phe Thr Val Pro Thr Ser Pro Asn Val Ser Ser Ala His
2545 2550 2555 2560

Phe Trp Gly His Met Ala Asp Thr Val Val Val Asn Ser Lys Ser Leu
2565 2570 2575

His Arg Pro Leu Leu Thr Thr Glu Leu Pro Ser Gly Ala Asn Pro Val
2580 2585 2590

Ser Ser Pro Ile Ile Asn Tyr Glu Asn Trp Ala Ser Ala His Ile Ile
2595 2600 2605

Asp Ala Ser Lys Trp Asp Ile Ala Arg Gln Cys Gly Ser Ile Glu Asn
2610 2615 2620

Ala Pro Thr Tyr Asn Glu Leu Glu Leu Leu His Thr Val Phe Asn Ser
2625 2630 2635 2640

Leu Gly Trp Pro Ser Ser Pro Ser Phe Pro Tyr Leu Ser Ser Gln Gln
2645 2650 2655

Cys Gly Met Asp Glu Gly Thr Gly Ala Gln Asp Cys Ser Ile Thr Leu
2660 2665 2670

Ile Asn Lys Pro Gly Leu Val Thr Cys Phe Gln
2675 2680

<210> 98
<211> 193
<212> PRT
<213> Escherichia coli

<400> 98
Met Val Val Asn Lys Thr Thr Ala Val Leu Tyr Leu Ile Ala Leu Ser
1 5 10 15

Leu Ser Gly Phe Ile His Thr Phe Leu Arg Ala Glu Glu Arg Gly Ile
20 25 30

Tyr Asp Asp Val Phe Thr Ala Asp Glu Leu Arg His Tyr Arg Ile Asn
35 40 45

Glu Arg Gly Gly Arg Thr Gly Ser Leu Ala Ile Ser Gly Ala Leu Leu
50 55 60

Ser Ser Pro Cys Thr Leu Val Ser Asn Glu Val Pro Leu Ser Leu Arg
65 70 75 80

Pro Glu Asn His Ser Ala Ala Ala Gly Ala Pro Leu Met Leu Arg Leu
85 90 95

Ala Gly Cys Gly Asp Gly Gly Ala Leu Gln Pro Gly Lys Arg Gly Val
100 105 110

Ala Met Thr Val Ser Gly Ser Leu Val Thr Gly Pro Gly Ser Gly Ser
115 120 125

Ala Leu Leu Pro Asp Arg Lys Leu Ser Gly Cys Asp His Leu Val Ile
130 135 140

His Asp Gly Asp Thr Phe Leu Leu Cys Arg Pro Asp Arg Arg Gln Glu
145 150 155 160

Glu Met Leu Ala Ala Trp Arg Lys Arg Ala Thr Gln Glu Gly Glu Tyr
165 170 175

Ser Asp Ala Arg Ser Asn Pro Ala Met Leu Arg Leu Ser Ile Lys Tyr
180 185 190

Glu

<210> 99
<211> 159
<212> PRT
<213> Escherichia coli

<400> 99
Met Met Thr Leu Glu Ala Asp Ser Val Asn Val Gln Ala Leu Asp Met
1 5 10 15

Gly Arg Ile Val Ile Asp Val Asp Gly Val Thr Leu Ala Glu Leu Ile
20 25 30

Asn Val Val Cys Asp Asn Gly Tyr Ser Leu Arg Val Val Asp Glu Ser
35 40 45

Asp Arg Ala Ser Thr Asp Cys Thr Pro Pro Phe Ala Val Leu Thr Gly
50 55 60

Ile Arg Cys Ser Thr Ala His Ile Thr Ala Lys Asp Asn Ala Trp Leu
65 70 75 80

Tyr Ser Leu Ser His Gln Thr Asn Asp Thr Gly Glu Ser Glu Trp Ile
85 90 95

His Phe Thr Gly Ser Gly Tyr Leu Leu Arg Thr Asp Ala Arg Ser Tyr
100 105 110

Pro Val Leu Arg Leu Lys Arg Leu Gly Leu Ser Lys Thr Phe Arg Arg
115 120 125

Leu Ile Ala Thr Leu Ile Arg Arg Tyr Gly Val Ser Leu Ile His Leu
130 135 140

Asp Ala Ser Ala Gly Cys Leu Pro Gly Leu Pro Thr Phe Asp Trp
145 150 155

<210> 100
<211> 464
<212> PRT
<213> Escherichia coli

<400> 100
Met Lys Lys Ser Thr Leu Ser Leu Ala Ile Gly Leu Leu Leu Ala Cys
1 5 10 15

Ser Thr Gly Met Ala Lys Thr Gln His Leu Thr Leu Glu Gln Arg Met
20 25 30

Ala Leu Leu Glu Glu Arg Leu Glu Ala Ala Glu Met Arg Ala Ala Lys
35 40 45

Ala Glu Ser Gln Val Lys Gln Leu Gln Thr Gln Gln Ala Ala Glu Ile
50 55 60

Arg Glu Ile Lys Ala Ala Gln Gly Asn Thr Pro Val Asn Gly Gln Ala
65 70 75 80

Thr Ala Glu Ser Ala Lys Lys Asn Ser Thr Ser Pro Asn Leu Leu Leu
85 90 95

Ser Gly Tyr Gly Asp Leu Lys Ile Tyr Gly Asp Val Glu Phe Asn Met
100 105 110

Asp Ala Glu Ser Asn His Gly Leu Leu Ala Met Thr Asn Ala Asp Val
115 120 125

Asn Ser Asp Pro Thr Asn Glu Gln Trp Asn Leu Asn Gly Arg Ile Leu
130 135 140

Leu Gly Phe Asp Gly Met Arg Lys Leu Asp Asn Gly Tyr Phe Ala Gly
145 150 155 160

Phe Ser Ala Gln Pro Leu Gly Asp Met His Gly Ser Val Asn Ile Asp
165 170 175

Asp Ala Val Phe Phe Phe Gly Lys Glu Asn Asp Trp Lys Val Lys Val
180 185 190

Gly Arg Phe Glu Ala Tyr Asp Met Phe Pro Leu Asn Gln Asp Thr Phe
195 200 205

Val Glu His Ser Gly Asn Thr Ala Asn Asp Leu Tyr Asp Asp Gly Ser

210 215 220

Gly Tyr Ile Tyr Met Met Lys Glu Gly Arg Gly Arg Ser Asn Ala Gly
225 230 235 240

Gly Asn Phe Leu Val Ser Lys Gln Leu Asp Asn Trp Tyr Phe Glu Leu
245 250 255

Asn Thr Leu Leu Glu Asp Gly Thr Ser Leu Tyr Asn Asp Gly Asn Tyr
260 265 270

His Gly Arg Asp Met Glu Gln Gln Lys Asn Val Ala Tyr Leu Arg Pro
275 280 285

Val Ile Ala Trp Ser Pro Thr Glu Glu Phe Thr Val Ser Ala Ala Met
290 295 300

Glu Ala Asn Val Val Asn Asn Ala Tyr Gly Tyr Thr Asp Ser Lys Gly
305 310 315 320

Asn Phe Val Asp Gln Ser Asp Arg Thr Gly Tyr Gly Met Ser Met Thr
325 330 335

Trp Asn Gly Leu Lys Thr Asp Pro Glu Asn Gly Ile Val Val Asn Leu
340 345 350

Asn Thr Ala Tyr Leu Asp Ala Asn Asn Glu Lys Asp Phe Thr Ala Gly
355 360 365

Ile Asn Ala Leu Trp Lys Arg Phe Glu Leu Gly Tyr Ile Tyr Ala His
370 375 380

Asn Lys Ile Asp Glu Phe Ser Gly Val Val Cys Asp Asn Asp Cys Trp
385 390 395 400

Ile Asp Asp Glu Gly Thr Tyr Asn Ile His Thr Ile His Ala Ser Tyr
405 410 415

Gln Phe Ala Asn Val Met Asp Met Glu Asn Phe Asn Ile Tyr Leu Gly
420 425 430

Thr Tyr Tyr Ser Ile Leu Asp Ser Asp Gly Asp Lys Ile His Gly Asp
435 440 445

Asp Ser Asp Asp Arg Tyr Gly Ala Arg Val Arg Phe Lys Tyr Phe Phe
450 455 460

<210> 101
<211> 810
<212> PRT
<213> Escherichia coli

<400> 101
Met Ala Met Lys Lys Leu Leu Ile Ala Ser Leu Leu Phe Ser Ser Ala
1 5 10 15

Thr Val Tyr Gly Ala Glu Gly Phe Val Val Lys Asp Ile His Phe Glu
20 25 30

Gly Leu Gln Arg Val Ala Val Gly Ala Ala Leu Leu Ser Met Pro Val
35 40 45

Arg Thr Gly Asp Thr Val Asn Asp Glu Asp Ile Ser Asn Thr Ile Arg
50 55 60

```
Ala Leu Phe Ala Thr Gly Asn Phe Glu Asp Val Arg Val Leu Arg Asp
65                  70                  75                  80

Gly Asp Thr Leu Leu Val Gln Val Lys Glu Arg Pro Thr Ile Ala Ser
                85                  90                  95

Ile Thr Phe Ser Gly Asn Lys Ser Val Lys Asp Asp Met Leu Lys Gln
            100                 105                 110

Asn Leu Glu Ala Ser Gly Val Arg Val Gly Glu Ser Leu Asp Arg Thr
        115                 120                 125

Thr Ile Ala Asp Ile Glu Lys Gly Leu Glu Asp Phe Tyr Tyr Ser Val
    130                 135                 140

Gly Lys Tyr Ser Ala Ser Val Lys Ala Val Val Thr Pro Leu Pro Arg
145                 150                 155                 160

Asn Arg Val Asp Leu Lys Leu Val Phe Gln Glu Gly Val Ser Ala Glu
                165                 170                 175

Ile Gln Gln Ile Asn Ile Val Gly Asn His Ala Phe Thr Thr Asp Glu
            180                 185                 190

Leu Ile Ser His Phe Gln Leu Arg Asp Glu Val Pro Trp Trp Asn Val
        195                 200                 205

Val Gly Asp Arg Lys Tyr Gln Lys Gln Lys Leu Ala Gly Asp Leu Glu
    210                 215                 220

Thr Leu Arg Ser Tyr Tyr Leu Asp Arg Gly Tyr Ala Arg Phe Asn Ile
225                 230                 235                 240

Asp Ser Thr Gln Val Ser Leu Thr Pro Asp Lys Lys Gly Ile Tyr Val
                245                 250                 255

Thr Val Asn Ile Thr Glu Gly Asp Gln Tyr Lys Leu Ser Gly Val Glu
            260                 265                 270

Val Ser Gly Asn Leu Ala Gly His Ser Ala Glu Ile Glu Gln Leu Thr
        275                 280                 285

Lys Ile Glu Pro Gly Glu Leu Tyr Asn Gly Thr Lys Val Thr Lys Met
    290                 295                 300

Glu Asp Asp Ile Lys Lys Leu Leu Gly Arg Tyr Gly Tyr Ala Tyr Pro
305                 310                 315                 320

Arg Val Gln Ser Met Pro Glu Ile Asn Asp Ala Asp Lys Thr Val Lys
                325                 330                 335

Leu Arg Val Asn Val Asp Ala Gly Asn Arg Phe Tyr Val Arg Lys Ile
            340                 345                 350

Arg Phe Glu Gly Asn Asp Thr Ser Lys Asp Ala Val Leu Arg Arg Glu
        355                 360                 365

Met Arg Gln Met Glu Gly Ala Trp Leu Gly Ser Asp Leu Val Asp Gln
    370                 375                 380

Gly Lys Glu Arg Leu Asn Arg Leu Gly Phe Phe Glu Thr Val Asp Thr
385                 390                 395                 400

Asp Thr Gln Arg Val Pro Gly Ser Pro Asp Gln Val Asp Val Val Tyr
```

405 410 415

Lys Val Lys Glu Arg Asn Thr Gly Ser Phe Asn Phe Gly Ile Gly Tyr
420 425 430

Gly Thr Glu Ser Gly Val Ser Phe Gln Ala Gly Val Gln Gln Asp Asn
435 440 445

Trp Leu Gly Thr Gly Tyr Ala Val Gly Ile Asn Gly Thr Lys Asn Asp
450 455 460

Tyr Gln Thr Tyr Ala Glu Leu Ser Val Thr Asn Pro Tyr Phe Thr Val
465 470 475 480

Asp Gly Val Ser Leu Gly Gly Arg Leu Phe Tyr Asn Asp Phe Gln Ala
485 490 495

Asp Asp Ala Asp Leu Ser Asp Tyr Thr Asn Lys Ser Tyr Gly Thr Asp
500 505 510

Val Thr Leu Gly Phe Pro Ile Asn Glu Tyr Asn Ser Leu Arg Ala Gly
515 520 525

Leu Gly Tyr Val His Asn Ser Leu Ser Asn Met Gln Pro Gln Val Ala
530 535 540

Met Trp Arg Tyr Leu Tyr Ser Met Gly Glu His Pro Ser Thr Ser Asp
545 550 555 560

Gln Asp Asn Ser Phe Lys Thr Asp Asp Phe Thr Phe Asn Tyr Gly Trp
565 570 575

Thr Tyr Asn Lys Leu Asp Arg Gly Tyr Phe Pro Thr Asp Gly Ser Arg
580 585 590

Val Asn Leu Thr Gly Lys Val Thr Ile Pro Gly Ser Asp Asn Glu Tyr
595 600 605

Tyr Lys Val Thr Leu Asp Thr Ala Thr Tyr Val Pro Ile Asp Asp Asp
610 615 620

His Lys Trp Val Val Leu Gly Arg Thr Arg Trp Gly Tyr Gly Asp Gly
625 630 635 640

Leu Gly Gly Lys Glu Met Pro Phe Tyr Glu Asn Phe Tyr Ala Gly Gly
645 650 655

Ser Ser Thr Val Arg Gly Phe Gln Ser Asn Thr Ile Gly Pro Lys Ala
660 665 670

Val Tyr Phe Pro His Gln Ala Ser Asn Tyr Asp Pro Asp Tyr Asp Tyr
675 680 685

Glu Cys Ala Thr Gln Asp Gly Ala Lys Asp Leu Cys Lys Ser Asp Asp
690 695 700

Ala Val Gly Gly Asn Ala Met Ala Val Ala Ser Leu Glu Phe Ile Thr
705 710 715 720

Pro Thr Pro Phe Ile Ser Asp Lys Tyr Ala Asn Ser Val Arg Thr Ser
725 730 735

Phe Phe Trp Asp Met Gly Thr Val Trp Asp Thr Asn Trp Asp Ser Ser
740 745 750

Gln Tyr Ser Gly Tyr Pro Asp Tyr Ser Asp Pro Ser Asn Ile Arg Met
755 760 765

Ser Ala Gly Ile Ala Leu Gln Trp Met Ser Pro Leu Gly Pro Leu Val
770 775 780

Phe Ser Tyr Ala Gln Pro Phe Lys Lys Tyr Asp Gly Asp Lys Ala Glu
785 790 795 800

Gln Phe Gln Phe Asn Ile Gly Lys Thr Trp
805 810

<210> 102
<211> 346
<212> PRT
<213> Escherichia coli

<400> 102
Met Lys Lys Thr Ala Ile Ala Ile Ala Val Ala Leu Ala Gly Phe Ala
1 5 10 15

Thr Val Ala Gln Ala Ala Pro Lys Asp Asn Thr Trp Tyr Thr Gly Ala
20 25 30

Lys Leu Gly Trp Ser Gln Tyr His Asp Thr Gly Phe Ile Asn Asn Asn
35 40 45

Gly Pro Thr His Glu Asn Gln Leu Gly Ala Gly Ala Phe Gly Gly Tyr
50 55 60

Gln Val Asn Pro Tyr Val Gly Phe Glu Met Gly Tyr Asp Trp Leu Gly
65 70 75 80

Arg Met Pro Tyr Lys Gly Ser Val Glu Asn Gly Ala Tyr Lys Ala Gln
85 90 95

Gly Val Gln Leu Thr Ala Lys Leu Gly Tyr Pro Ile Thr Asp Asp Leu
100 105 110

Asp Val Tyr Thr Arg Leu Gly Gly Met Val Trp Arg Ala Asp Thr Lys
115 120 125

Ser Asn Phe Asp Gly Lys Asn His Asp Thr Gly Val Ser Pro Val Phe
130 135 140

Ala Gly Gly Val Glu Tyr Ala Ile Thr Pro Glu Ile Ala Thr Arg Leu
145 150 155 160

Glu Tyr Gln Trp Thr Asn Asn Ile Gly Asp Ala His Thr Ile Gly Thr
165 170 175

Arg Pro Asp Asn Gly Met Leu Ser Leu Gly Val Ser Tyr Arg Phe Gly
180 185 190

Gln Gly Glu Ala Ala Pro Val Val Ala Pro Ala Pro Ala Pro Ala Pro
195 200 205

Glu Val Gln Thr Lys His Phe Thr Leu Lys Ser Asp Val Leu Phe Thr
210 215 220

Phe Asn Lys Ala Thr Leu Lys Pro Glu Gly Gln Ala Ala Leu Asp Gln
225 230 235 240

Leu Tyr Ser Gln Leu Ser Asn Leu Asp Pro Lys Asp Gly Ser Val Val
245 250 255

Val Leu Gly Tyr Thr Asp Arg Ile Gly Ser Asp Ala Tyr Asn Gln Ala
260 265 270

Leu Ser Glu Arg Arg Ala Gln Ser Val Val Asp Tyr Leu Ile Ser Lys
275 280 285

Gly Ile Pro Ala Asp Lys Ile Ser Ala Arg Gly Met Gly Glu Ser Asn
290 295 300

Pro Val Thr Gly Asn Thr Cys Asp Asn Val Lys Gln Arg Ala Ala Leu
305 310 315 320

Ile Asp Cys Leu Ala Pro Asp Arg Arg Val Glu Ile Glu Val Lys Gly
325 330 335

Ile Lys Asp Val Val Thr Gln Pro Gln Ala
340 345

<210> 103
<211> 219
<212> PRT
<213> Escherichia coli

<400> 103
Met Lys Lys Arg Val Tyr Leu Ile Ala Ala Val Val Ser Gly Ala Leu
1 5 10 15

Ala Val Ser Gly Cys Thr Thr Asn Pro Tyr Thr Gly Glu Arg Glu Ala
20 25 30

Gly Lys Ser Ala Ile Gly Ala Gly Leu Gly Ser Leu Val Gly Ala Gly
35 40 45

Ile Gly Ala Leu Ser Ser Ser Lys Lys Asp Arg Gly Lys Gly Ala Leu
50 55 60

Ile Gly Ala Ala Ala Gly Ala Ala Leu Gly Gly Gly Val Gly Tyr Tyr
65 70 75 80

Met Asp Val Gln Glu Ala Lys Leu Arg Asp Lys Met Arg Gly Thr Gly
85 90 95

Val Ser Val Thr Arg Ser Gly Asp Asn Ile Ile Leu Asn Met Pro Asn
100 105 110

Asn Val Thr Phe Asp Ser Ser Ser Ala Thr Leu Lys Pro Ala Gly Ala
115 120 125

Asn Thr Leu Thr Gly Val Ala Met Val Leu Lys Glu Tyr Pro Lys Thr
130 135 140

Ala Val Asn Val Ile Gly Tyr Thr Asp Ser Thr Gly Gly His Asp Leu
145 150 155 160

Asn Met Arg Leu Ser Gln Gln Arg Ala Asp Ser Val Ala Ser Ala Leu
165 170 175

Ile Thr Gln Gly Val Asp Ala Ser Arg Ile Arg Thr Gln Gly Leu Gly
180 185 190

Pro Ala Asn Pro Ile Ala Ser Asn Ser Thr Ala Glu Gly Lys Ala Gln
195 200 205

Asn Arg Arg Val Glu Ile Thr Leu Ser Pro Leu

210 215

<210> 104
<211> 394
<212> PRT
<213> Escherichia coli

<400> 104
Val Ser Lys Glu Lys Phe Glu Arg Thr Lys Pro His Val Asn Val Gly
1 5 10 15

Thr Ile Gly His Val Asp His Gly Lys Thr Thr Leu Thr Ala Ala Ile
20 25 30

Thr Thr Val Leu Ala Lys Thr Tyr Gly Gly Ala Ala Arg Ala Phe Asp
35 40 45

Gln Ile Asp Asn Ala Pro Glu Glu Lys Ala Arg Gly Ile Thr Ile Asn
50 55 60

Thr Ser His Val Glu Tyr Asp Thr Pro Thr Arg His Tyr Ala His Val
65 70 75 80

Asp Cys Pro Gly His Ala Asp Tyr Val Lys Asn Met Ile Thr Gly Ala
85 90 95

Ala Gln Met Asp Gly Ala Ile Leu Val Val Ala Ala Thr Asp Gly Pro
100 105 110

Met Pro Gln Thr Arg Glu His Ile Leu Leu Gly Arg Gln Val Gly Val
115 120 125

Pro Tyr Ile Ile Val Phe Leu Asn Lys Cys Asp Met Val Asp Asp Glu
130 135 140

Glu Leu Leu Glu Leu Val Glu Met Glu Val Arg Glu Leu Leu Ser Gln
145 150 155 160

Tyr Asp Phe Pro Gly Asp Asp Thr Pro Ile Val Arg Gly Ser Ala Leu
165 170 175

Lys Ala Leu Glu Gly Asp Ala Glu Trp Glu Ala Lys Ile Leu Glu Leu
180 185 190

Ala Gly Phe Leu Asp Ser Tyr Ile Pro Glu Pro Glu Arg Ala Ile Asp
195 200 205

Lys Pro Phe Leu Leu Pro Ile Glu Asp Val Phe Ser Ile Ser Gly Arg
210 215 220

Gly Thr Val Val Thr Gly Arg Val Glu Arg Gly Ile Ile Lys Val Gly
225 230 235 240

Glu Glu Val Glu Ile Val Gly Ile Lys Glu Thr Gln Lys Ser Thr Cys
245 250 255

Thr Gly Val Glu Met Phe Arg Lys Leu Leu Asp Glu Gly Arg Ala Gly
260 265 270

Glu Asn Val Gly Val Leu Leu Arg Gly Ile Lys Arg Glu Glu Ile Glu
275 280 285

Arg Gly Gln Val Leu Ala Lys Pro Gly Thr Ile Lys Pro His Thr Lys
290 295 300

Phe Glu Ser Glu Val Tyr Ile Leu Ser Lys Asp Glu Gly Gly Arg His
305 310 315 320

Thr Pro Phe Phe Lys Gly Tyr Arg Pro Gln Phe Tyr Phe Arg Thr Thr
325 330 335

Asp Val Thr Gly Thr Ile Glu Leu Pro Glu Gly Val Glu Met Val Met
340 345 350

Pro Gly Asp Asn Ile Lys Met Val Val Thr Leu Ile His Pro Ile Ala
355 360 365

Met Asp Asp Gly Leu Arg Phe Ala Ile Arg Glu Gly Gly Arg Thr Val
370 375 380

Gly Ala Gly Val Val Ala Lys Val Leu Gly
385 390

<210> 105
<211> 747
<212> PRT
<213> Escherichia coli

<400> 105
Met Ala Pro Ser Lys Thr Ala Gln Pro Lys His Ser Leu Arg Lys Ile
1 5 10 15

Ala Val Val Val Ala Thr Ala Val Ser Gly Met Ser Val Tyr Ala Gln
20 25 30

Ala Ala Val Glu Pro Lys Glu Asp Thr Ile Thr Val Thr Ala Ala Pro
35 40 45

Ala Pro Gln Glu Ser Ala Trp Gly Pro Ala Ala Thr Ile Ala Ala Arg
50 55 60

Gln Ser Ala Thr Gly Thr Lys Thr Asp Thr Pro Ile Gln Lys Val Pro
65 70 75 80

Gln Ser Ile Ser Val Val Thr Ala Glu Glu Met Ala Leu His Gln Pro
85 90 95

Lys Ser Val Lys Glu Ala Leu Ser Tyr Thr Pro Gly Val Ser Val Gly
100 105 110

Thr Arg Gly Ala Ser Asn Thr Tyr Asp His Leu Ile Ile Arg Gly Phe
115 120 125

Ala Ala Glu Gly Gln Ser Gln Asn Asn Tyr Leu Asn Gly Leu Lys Leu
130 135 140

Gln Gly Asn Phe Tyr Asn Asp Ala Val Ile Asp Pro Tyr Met Leu Glu
145 150 155 160

Arg Ala Glu Ile Met Arg Gly Pro Val Ser Val Leu Tyr Gly Lys Ser
165 170 175

Ser Pro Gly Gly Leu Leu Asn Met Val Ser Lys Arg Pro Thr Thr Glu
180 185 190

Pro Leu Lys Glu Val Gln Phe Lys Ala Gly Thr Asp Ser Leu Phe Gln
195 200 205

Thr Gly Phe Asp Phe Ser Asp Ala Leu Asp Asp Asp Gly Val Tyr Ser
210 215 220

```
Tyr Arg Leu Thr Gly Leu Ala Arg Ser Ala Asn Ala Gln Gln Lys Gly
225                 230                 235                 240

Ser Glu Glu Gln Arg Tyr Ala Ile Ala Pro Ala Phe Thr Trp Arg Pro
                245                 250                 255

Asp Asp Lys Thr Asn Phe Thr Phe Leu Ser Tyr Phe Gln Asn Glu Pro
            260                 265                 270

Glu Thr Gly Tyr Tyr Gly Trp Leu Pro Lys Glu Gly Thr Val Glu Pro
        275                 280                 285

Leu Pro Asn Gly Lys Arg Leu Pro Thr Asp Phe Asn Glu Gly Ala Lys
    290                 295                 300

Asn Asn Thr Tyr Ser Arg Asn Glu Lys Met Val Gly Tyr Ser Phe Asp
305                 310                 315                 320

His Glu Phe Asn Asp Thr Phe Thr Val Arg Gln Asn Leu Arg Phe Ala
                325                 330                 335

Glu Asn Lys Thr Ser Gln Asn Ser Val Tyr Gly Tyr Gly Val Cys Ser
            340                 345                 350

Asp Pro Ala Asn Ala Tyr Ser Lys Gln Cys Ala Ala Leu Ala Pro Ala
        355                 360                 365

Asp Lys Gly His Tyr Leu Ala Arg Lys Tyr Val Val Asp Asp Glu Lys
    370                 375                 380

Leu Gln Asn Phe Ser Val Asp Thr Gln Leu Gln Ser Lys Phe Ala Thr
385                 390                 395                 400

Gly Asp Ile Asp His Thr Leu Leu Thr Gly Val Asp Phe Met Arg Met
                405                 410                 415

Arg Asn Asp Ile Asn Ala Trp Phe Gly Tyr Asp Asp Ser Val Pro Leu
            420                 425                 430

Leu Asp Leu Tyr Asn Pro Val Asn Thr Asp Phe Asp Phe Asn Ala Lys
        435                 440                 445

Asp Pro Asp Asn Ser Gly Pro Tyr Arg Ile Leu Asn Lys Gln Lys Gln
    450                 455                 460

Thr Gly Val Tyr Val Gln Asp Gln Ala Gln Trp Asp Lys Val Leu Val
465                 470                 475                 480

Thr Leu Gly Gly Arg Tyr Asp Trp Ala Asp Gln Glu Ser Leu Asn Arg
                485                 490                 495

Val Ala Gly Thr Thr Asp Lys Arg Asp Asp Lys Gln Phe Thr Trp Arg
            500                 505                 510

Gly Gly Val Asn Tyr Leu Phe Asp Asn Gly Val Thr Pro Tyr Phe Ser
        515                 520                 525

Tyr Ser Glu Ser Phe Glu Pro Ser Ser Gln Val Gly Lys Asp Gly Asn
    530                 535                 540

Ile Phe Ala Pro Ser Lys Gly Lys Gln Tyr Glu Val Gly Val Lys Tyr
545                 550                 555                 560

Val Pro Glu Asp Arg Pro Ile Val Val Thr Gly Ala Val Tyr Asn Leu
```

565 570 575

Thr Lys Thr Asn Asn Leu Met Ala Asp Pro Glu Gly Ser Phe Phe Ser
580 585 590

Val Glu Gly Gly Glu Ile Arg Ala Arg Gly Val Glu Ile Glu Ala Lys
595 600 605

Ala Ala Leu Ser Ala Ser Val Asn Val Val Gly Ser Tyr Thr Tyr Thr
610 615 620

Asp Ala Glu Tyr Thr Thr Asp Thr Thr Tyr Lys Gly Asn Thr Pro Ala
625 630 635 640

Gln Val Pro Lys His Met Ala Ser Leu Trp Ala Asp Tyr Thr Phe Phe
645 650 655

Asp Gly Pro Leu Ser Gly Leu Thr Leu Gly Thr Gly Gly Arg Tyr Thr
660 665 670

Gly Ser Ser Tyr Gly Asp Pro Ala Asn Ser Phe Lys Val Gly Ser Tyr
675 680 685

Thr Val Val Asp Ala Leu Val Arg Tyr Asp Leu Ala Arg Val Gly Met
690 695 700

Ala Gly Ser Asn Val Ala Leu His Val Asn Asn Leu Phe Asp Arg Glu
705 710 715 720

Tyr Val Ala Ser Cys Phe Asn Thr Tyr Gly Cys Phe Trp Gly Ala Glu
725 730 735

Arg Gln Val Val Ala Thr Ala Thr Phe Arg Phe
740 745

<210> 106
<211> 171
<212> PRT
<213> Escherichia coli

<400> 106
Met Lys Lys Ile Ala Cys Leu Ser Ala Leu Ala Ala Val Leu Ala Phe
1 5 10 15

Thr Ala Gly Thr Ser Val Ala Ala Thr Ser Thr Val Thr Gly Gly Tyr
20 25 30

Ala Gln Ser Asp Ala Gln Gly Gln Met Asn Lys Met Gly Gly Phe Asn
35 40 45

Leu Lys Tyr Arg Tyr Glu Glu Asp Asn Ser Pro Leu Gly Val Ile Gly
50 55 60

Ser Phe Thr Tyr Thr Glu Lys Ser Arg Thr Ala Ser Ser Gly Asp Tyr
65 70 75 80

Asn Lys Asn Gln Tyr Tyr Gly Ile Thr Ala Gly Pro Ala Tyr Arg Ile
85 90 95

Asn Asp Trp Ala Ser Ile Tyr Gly Val Val Gly Val Gly Tyr Gly Lys
100 105 110

Phe Gln Thr Thr Glu Tyr Pro Thr Tyr Lys His Asp Thr Ser Asp Tyr
115 120 125

```
Gly Phe Ser Tyr Gly Ala Gly Leu Gln Phe Asn Pro Met Glu Asn Val
    130                 135                 140

Ala Leu Asp Phe Ser Tyr Glu Gln Ser Arg Ile Arg Ser Val Asp Val
145                 150                 155                 160

Gly Thr Trp Ile Ala Gly Val Gly Tyr Arg Phe
                165                 170

<210>   107
<211>   360
<212>   PRT
<213>   Escherichia coli

<400>   107
Met Lys Lys Leu Thr Val Ala Ile Ser Ala Val Ala Ala Ser Val Leu
1               5                   10                  15

Met Ala Met Ser Ala Gln Ala Ala Glu Ile Tyr Asn Lys Asp Ser Asn
            20                  25                  30

Lys Leu Asp Leu Tyr Gly Lys Val Asn Ala Lys His Tyr Phe Ser Ser
        35                  40                  45

Asn Asp Ala Asp Asp Gly Asp Thr Thr Tyr Ala Arg Leu Gly Phe Lys
    50                  55                  60

Gly Glu Thr Gln Ile Asn Asp Gln Leu Thr Gly Phe Gly Gln Trp Glu
65                  70                  75                  80

Tyr Glu Phe Lys Gly Asn Arg Ala Glu Ser Gln Gly Ser Ser Lys Asp
                85                  90                  95

Lys Thr Arg Leu Ala Phe Ala Gly Leu Lys Phe Gly Asp Tyr Gly Ser
            100                 105                 110

Ile Asp Tyr Gly Arg Asn Tyr Gly Val Ala Tyr Asp Ile Gly Ala Trp
        115                 120                 125

Thr Asp Val Leu Pro Glu Phe Gly Gly Asp Thr Trp Thr Gln Thr Asp
    130                 135                 140

Val Phe Met Thr Gly Arg Thr Thr Gly Val Ala Thr Tyr Arg Asn Asn
145                 150                 155                 160

Asp Phe Phe Gly Leu Val Asp Gly Leu Asn Phe Ala Ala Gln Tyr Gln
                165                 170                 175

Gly Lys Asn Asp Arg Thr Asp Val Thr Glu Ala Asn Gly Asp Gly Phe
            180                 185                 190

Gly Phe Ser Thr Thr Tyr Glu Tyr Glu Gly Phe Gly Val Gly Ala Thr
        195                 200                 205

Tyr Ala Lys Ser Asp Arg Thr Asp Gly Gln Val Ala Tyr Gly Lys Ser
    210                 215                 220

Lys Phe Asn Ala Ser Gly Lys Asn Ala Glu Val Trp Ala Ala Gly Leu
225                 230                 235                 240

Lys Tyr Asp Ala Asn Asn Ile Tyr Leu Ala Thr Thr Tyr Ser Glu Thr
                245                 250                 255

Gln Asn Met Thr Val Phe Gly Asn Asn His Ile Ala Asn Lys Ala Gln
            260                 265                 270
```

Asn Phe Glu Ala Val Ala Gln Tyr Gln Phe Asp Phe Gly Leu Arg Pro
        275                 280                 285

Ser Val Ala Tyr Leu Gln Ser Lys Gly Lys Asp Leu Gly Val His Gly
    290                 295                 300

Asp Arg Asp Leu Val Lys Tyr Val Asp Val Gly Ala Thr Tyr Tyr Phe
305                 310                 315                 320

Asn Lys Asn Met Ser Thr Phe Val Asp Tyr Lys Ile Asn Leu Ile Asp
                325                 330                 335

Asp Ser Lys Phe Thr Lys Thr Ala Gly Ile Asp Thr Asp Asp Ile Val
            340                 345                 350

Ala Val Gly Leu Val Tyr Gln Phe
        355                 360

<210> 108
<211> 245
<212> PRT
<213> Escherichia coli

<400> 108
Met Thr Arg Met Lys Tyr Leu Val Ala Ala Ala Thr Leu Ser Leu Phe
1               5                   10                  15

Leu Ala Gly Cys Ser Gly Ser Lys Glu Glu Val Pro Asp Asn Pro Pro
            20                  25                  30

Asn Glu Ile Tyr Ala Thr Ala Gln Gln Lys Leu Gln Asp Gly Asn Trp
        35                  40                  45

Arg Gln Ala Ile Thr Gln Leu Glu Ala Leu Asp Asn Arg Tyr Pro Phe
    50                  55                  60

Gly Pro Tyr Ser Gln Gln Val Gln Leu Asp Leu Ile Tyr Ala Tyr Tyr
65                  70                  75                  80

Lys Asn Ala Asp Leu Pro Leu Ala Gln Ala Ala Ile Asp Arg Phe Ile
                85                  90                  95

Arg Leu Asn Pro Thr His Pro Asn Ile Asp Tyr Val Met Tyr Met Arg
            100                 105                 110

Gly Leu Thr Asn Met Ala Leu Asp Asp Ser Ala Leu Gln Arg Phe Phe
        115                 120                 125

Gly Val Asp Arg Ser Asp Arg Asp Pro Gln His Ala Arg Ala Ala Phe
    130                 135                 140

Ser Asp Phe Ser Lys Leu Val Arg Gly Tyr Pro Asn Ser Gln Tyr Thr
145                 150                 155                 160

Thr Asp Ala Thr Lys Arg Leu Val Phe Leu Lys Asp Arg Leu Ala Lys
                165                 170                 175

Tyr Glu Tyr Ser Val Ala Glu Tyr Tyr Thr Glu Arg Gly Ala Trp Val
            180                 185                 190

Ala Val Val Asn Arg Val Glu Gly Met Leu Arg Asp Tyr Pro Asp Thr
        195                 200                 205

Gln Ala Thr Arg Asp Ala Leu Pro Leu Met Glu Asn Ala Tyr Arg Gln

210 215 220

Met Gln Met Asn Ala Gln Ala Glu Lys Val Ala Lys Ile Ile Ala Ala
225 230 235 240

Asn Ser Ser Asn Thr
245

<210> 109
<211> 493
<212> PRT
<213> Escherichia coli

<400> 109
Met Lys Lys Leu Leu Pro Ile Leu Ile Gly Leu Ser Leu Ser Gly Phe
1 5 10 15

Ser Ser Leu Ser Gln Ala Glu Asn Leu Met Gln Val Tyr Gln Gln Ala
20 25 30

Arg Leu Ser Asn Pro Glu Leu Arg Lys Ser Ala Ala Asp Arg Asp Ala
35 40 45

Ala Phe Glu Lys Ile Asn Glu Ala Arg Ser Pro Leu Leu Pro Gln Leu
50 55 60

Gly Leu Gly Ala Asp Tyr Thr Tyr Ser Asn Gly Tyr Arg Asp Ala Asn
65 70 75 80

Gly Ile Asn Ser Asn Ala Thr Ser Ala Ser Leu Gln Leu Thr Gln Ser
85 90 95

Ile Phe Asp Met Ser Lys Trp Arg Ala Leu Thr Leu Gln Glu Lys Ala
100 105 110

Ala Gly Ile Gln Asp Val Thr Tyr Gln Thr Asp Gln Gln Thr Leu Ile
115 120 125

Leu Asn Thr Ala Thr Ala Tyr Phe Asn Val Leu Asn Ala Ile Asp Val
130 135 140

Leu Ser Tyr Thr Gln Ala Gln Lys Glu Ala Ile Tyr Arg Gln Leu Asp
145 150 155 160

Gln Thr Thr Gln Arg Phe Asn Val Gly Leu Val Ala Ile Thr Asp Val
165 170 175

Gln Asn Ala Arg Ala Gln Tyr Asp Thr Val Leu Ala Asn Glu Val Thr
180 185 190

Ala Arg Asn Asn Leu Asp Asn Ala Val Glu Gln Leu Arg Gln Ile Thr
195 200 205

Gly Asn Tyr Tyr Pro Glu Leu Ala Ala Leu Asn Val Glu Asn Phe Lys
210 215 220

Thr Asp Lys Pro Gln Pro Val Asn Thr Leu Leu Lys Glu Ala Glu Lys
225 230 235 240

Arg Asn Leu Ser Leu Leu Gln Ala Arg Leu Ser Gln Asp Leu Ala Arg
245 250 255

Glu Gln Ile Arg Gln Ala Gln Asp Gly His Leu Pro Thr Leu Asp Leu
260 265 270

Thr Ala Ser Thr Gly Ile Ser Asp Thr Ser Tyr Ser Gly Ser Lys Thr
275 280 285

Arg Gly Ala Ala Gly Thr Gln Tyr Asp Asp Ser Asn Met Gly Gln Asn
290 295 300

Lys Val Gly Leu Ser Phe Ser Leu Pro Ile Tyr Gln Gly Gly Met Val
305 310 315 320

Asn Ser Gln Val Lys Gln Ala Gln Tyr Asn Phe Val Gly Ala Ser Glu
325 330 335

Gln Leu Glu Ser Ala His Arg Ser Val Val Gln Thr Val Arg Ser Ser
340 345 350

Phe Asn Asn Ile Asn Ala Ser Ile Ser Ser Ile Asn Ala Tyr Lys Gln
355 360 365

Ala Val Val Ser Ala Gln Ser Ser Leu Asp Ala Met Glu Ala Gly Tyr
370 375 380

Ser Val Gly Thr Arg Thr Ile Val Asp Val Leu Asp Ala Thr Thr Thr
385 390 395 400

Leu Tyr Asn Ala Lys Gln Glu Leu Ala Asn Ala Arg Tyr Asn Tyr Leu
405 410 415

Ile Asn Gln Leu Asn Ile Lys Ser Ala Leu Gly Thr Leu Asn Glu Gln
420 425 430

Asp Leu Leu Ala Leu Asn Asn Ala Leu Ser Lys Pro Val Ser Thr Asn
435 440 445

Pro Glu Asn Val Ala Pro Gln Thr Pro Glu Gln Asn Ala Ile Ala Asp
450 455 460

Gly Tyr Ala Pro Asp Ser Pro Ala Pro Val Val Gln Gln Thr Ser Ala
465 470 475 480

Arg Thr Thr Thr Ser Asn Gly His Asn Pro Phe Arg Asn
485 490

<210> 110
<211> 236
<212> PRT
<213> Escherichia coli

<400> 110
Met Val Lys Lys Ala Ile Val Thr Ala Met Ala Val Ile Ser Leu Phe
1 5 10 15

Thr Leu Met Gly Cys Asn Asn Arg Ala Glu Val Asp Thr Leu Ser Pro
20 25 30

Ala Gln Ala Ala Glu Leu Lys Pro Met Pro Gln Ser Trp Arg Gly Val
35 40 45

Leu Pro Cys Ala Asp Cys Glu Gly Ile Glu Thr Ser Leu Phe Leu Glu
50 55 60

Lys Asp Gly Thr Trp Val Met Asn Glu Arg Tyr Leu Gly Ala Arg Glu
65 70 75 80

Glu Pro Ser Ser Phe Ala Ser Tyr Gly Thr Trp Ala Arg Thr Ala Asp
85 90 95

Lys Leu Val Leu Thr Asp Ser Lys Gly Glu Lys Ser Tyr Tyr Arg Ala
100 105 110

Lys Gly Asp Ala Leu Glu Met Leu Asp Arg Glu Gly Asn Pro Ile Glu
115 120 125

Ser Gln Phe Asn Tyr Thr Leu Glu Pro Ala Gln Ser Ser Leu Pro Met
130 135 140

Thr Pro Met Thr Leu Arg Gly Met Tyr Phe Tyr Met Ala Asp Ala Ala
145 150 155 160

Thr Phe Thr Asp Cys Ala Thr Gly Lys Arg Phe Met Val Ala Asn Asn
165 170 175

Ala Glu Leu Glu Arg Gly Tyr Leu Ala Ala Arg Gly Asn Ser Glu Lys
180 185 190

Pro Val Leu Leu Ser Val Glu Gly His Phe Thr Leu Glu Ala Asn Pro
195 200 205

Asp Thr Gly Ala Pro Thr Lys Val Leu Ala Pro Asp Thr Ala Gly Lys
210 215 220

Phe Tyr Pro Asn Gln Asp Cys Ser Ser Leu Gly Leu
225 230 235

<210> 111
<211> 173
<212> PRT
<213> Escherichia coli

<400> 111
Met Gln Leu Asn Lys Val Leu Lys Gly Leu Met Ile Ala Leu Pro Val
1 5 10 15

Met Ala Ile Ala Ala Cys Ser Ser Asn Lys Asn Ala Ser Asn Asp Gly
20 25 30

Ser Glu Gly Met Leu Gly Ala Gly Thr Gly Met Asp Ala Asn Gly Gly
35 40 45

Asn Gly Asn Met Ser Ser Glu Glu Gln Ala Arg Leu Gln Met Gln Gln
50 55 60

Leu Gln Gln Asn Asn Ile Val Tyr Phe Asp Leu Asp Lys Tyr Asp Ile
65 70 75 80

Arg Ser Asp Phe Ala Gln Met Leu Asp Ala His Ala Asn Phe Leu Arg
85 90 95

Ser Asn Pro Ser Tyr Lys Val Thr Val Glu Gly His Ala Asp Glu Arg
100 105 110

Gly Thr Pro Glu Tyr Asn Ile Ser Leu Gly Glu Arg Arg Ala Asn Ala
115 120 125

Val Lys Met Tyr Leu Gln Gly Lys Gly Val Ser Ala Asp Gln Ile Ser
130 135 140

Ile Val Ser Tyr Gly Lys Glu Lys Pro Ala Val Leu Gly His Asp Glu
145 150 155 160

Ala Ala Tyr Ala Lys Asn Arg Arg Ala Val Leu Val Tyr

165 170

<210> 112
<211> 332
<212> PRT
<213> Escherichia coli

<400> 112
Met Asn Lys Lys Val Leu Thr Leu Ser Ala Val Met Ala Ser Met Leu
1 5 10 15

Phe Gly Ala Ala Ala His Ala Ala Asp Thr Arg Ile Gly Val Thr Ile
20 25 30

Tyr Lys Tyr Asp Asp Asn Phe Met Ser Val Val Arg Lys Ala Ile Glu
35 40 45

Gln Asp Ala Lys Ala Ala Pro Asp Val Gln Leu Leu Met Asn Asp Ser
50 55 60

Gln Asn Asp Gln Ser Lys Gln Asn Asp Gln Ile Asp Val Leu Leu Ala
65 70 75 80

Lys Gly Val Lys Ala Leu Ala Ile Asn Leu Val Asp Pro Ala Ala Ala
85 90 95

Gly Thr Val Ile Glu Lys Ala Arg Gly Gln Asn Val Pro Val Val Phe
100 105 110

Phe Asn Lys Glu Pro Ser Arg Lys Ala Leu Asp Ser Tyr Asp Lys Ala
115 120 125

Tyr Tyr Val Gly Thr Asp Ser Lys Glu Ser Gly Ile Ile Gln Gly Asp
130 135 140

Leu Ile Ala Lys His Trp Ala Ala Asn Gln Gly Trp Asp Leu Asn Lys
145 150 155 160

Asp Gly Gln Ile Gln Phe Val Leu Leu Lys Gly Glu Pro Gly His Pro
165 170 175

Asp Ala Glu Ala Arg Thr Thr Tyr Val Ile Lys Glu Leu Asn Asp Lys
180 185 190

Gly Ile Lys Thr Glu Gln Leu Gln Leu Asp Thr Ala Met Trp Asp Thr
195 200 205

Ala Gln Ala Lys Asp Lys Met Asp Ala Trp Leu Ser Gly Pro Asn Ala
210 215 220

Asn Lys Ile Glu Val Val Ile Ala Asn Asn Asp Ala Met Ala Met Gly
225 230 235 240

Ala Val Glu Ala Leu Lys Ala His Asn Lys Ser Ser Ile Pro Val Phe
245 250 255

Gly Val Asp Ala Leu Pro Glu Ala Leu Ala Leu Val Lys Ser Gly Ala
260 265 270

Leu Ala Gly Thr Val Leu Asn Asp Ala Asn Asn Gln Ala Lys Ala Thr
275 280 285

Phe Asp Leu Ala Lys Asn Leu Ala Asp Gly Lys Gly Ala Ala Asp Gly
290 295 300

```
Thr Asn Trp Lys Ile Asp Asn Lys Val Val Arg Val Pro Tyr Val Gly
305                 310                 315                 320

Val Asp Lys Asp Asn Leu Ala Glu Phe Ser Lys Lys
                325                 330

<210>   113
<211>   375
<212>   PRT
<213>   Escherichia coli

<400>   113
Met Lys Val Lys Val Leu Ser Leu Leu Val Pro Ala Leu Leu Val Ala
1               5                   10                  15

Gly Ala Ala Asn Ala Ala Glu Val Tyr Asn Lys Asp Gly Asn Lys Leu
            20                  25                  30

Asp Leu Tyr Gly Lys Val Asp Gly Leu His Tyr Phe Ser Asp Asp Lys
        35                  40                  45

Ser Val Asp Gly Asp Gln Thr Tyr Met Arg Leu Gly Phe Lys Gly Glu
    50                  55                  60

Thr Gln Val Thr Asp Gln Leu Thr Gly Tyr Gly Gln Trp Glu Tyr Gln
65                  70                  75                  80

Ile Gln Gly Asn Ala Pro Glu Ser Glu Asn Asn Ser Trp Thr Arg Val
                85                  90                  95

Ala Phe Ala Gly Leu Lys Phe Gln Asp Ile Gly Ser Phe Asp Tyr Gly
            100                 105                 110

Arg Asn Tyr Gly Val Val Tyr Asp Val Thr Ser Trp Thr Asp Val Leu
        115                 120                 125

Pro Glu Phe Gly Gly Asp Thr Tyr Gly Ser Asp Asn Phe Met Gln Gln
    130                 135                 140

Arg Gly Asn Gly Phe Ala Thr Tyr Arg Asn Thr Asp Phe Phe Gly Leu
145                 150                 155                 160

Val Asp Gly Leu Asn Phe Ala Val Gln Tyr Gln Gly Gln Asn Gly Ser
                165                 170                 175

Val Ser Gly Glu Asn Asp Pro Asp Phe Thr Gly His Gly Ile Thr Asn
            180                 185                 190

Asn Gly Arg Lys Ala Leu Arg Gln Asn Gly Asp Gly Val Gly Gly Ser
        195                 200                 205

Ile Thr Tyr Asp Tyr Glu Gly Phe Gly Val Gly Ala Ala Val Ser Ser
    210                 215                 220

Ser Lys Arg Thr Asp Ala Gln Asn Thr Ala Ala Tyr Ile Gly Asn Gly
225                 230                 235                 240

Asp Arg Ala Glu Thr Tyr Thr Gly Gly Leu Lys Tyr Asp Ala Asn Asn
                245                 250                 255

Ile Tyr Leu Ala Ala Gln Tyr Thr Gln Thr Tyr Asn Ala Thr Arg Val
            260                 265                 270

Gly Ser Leu Gly Trp Ala Asn Lys Ala Gln Asn Phe Glu Ala Val Ala
        275                 280                 285
```

Gln Tyr Gln Phe Asp Phe Gly Leu Arg Pro Ser Val Ala Tyr Leu Gln
290 295 300

Ser Lys Gly Lys Asn Leu Gly Thr Ile Gly Thr Arg Asn Tyr Asp Asp
305 310 315 320

Glu Asp Ile Leu Lys Tyr Val Asp Val Gly Ala Thr Tyr Tyr Phe Asn
325 330 335

Lys Asn Met Ser Thr Tyr Val Asp Tyr Lys Ile Asn Leu Leu Asp Asp
340 345 350

Asn Gln Phe Thr Arg Asp Ala Gly Ile Asn Thr Asp Asn Ile Val Ala
355 360 365

Leu Gly Leu Val Tyr Gln Phe
370 375

<210> 114
<211> 1157
<212> PRT
<213> Escherichia coli

<400> 114
Met Arg Lys Phe Thr Leu Asn Ile Phe Thr Leu Ser Leu Gly Leu Ala
1 5 10 15

Val Met Pro Met Val Glu Ala Ala Pro Thr Ala Gln Gln Gln Leu Leu
20 25 30

Glu Gln Val Arg Leu Gly Glu Ala Thr His Arg Glu Asp Leu Val Gln
35 40 45

Gln Ser Leu Tyr Arg Leu Glu Leu Ile Asp Pro Asn Asn Pro Asp Val
50 55 60

Ile Ala Ala Arg Phe Arg Ser Leu Leu Arg Gln Gly Asp Ile Asp Gly
65 70 75 80

Ala Gln Lys Gln Leu Asp Arg Leu Ser Gln Leu Ala Pro Ser Ser Asn
85 90 95

Ala Tyr Lys Ser Ser Arg Thr Thr Met Leu Leu Ser Thr Ser Asp Gly
100 105 110

Arg Gln Ala Leu Gln Gln Ala Arg Leu Gln Ala Thr Thr Gly His Ala
115 120 125

Glu Glu Ala Val Ala Ser Tyr Asn Lys Leu Phe Asn Gly Ala Pro Pro
130 135 140

Glu Gly Asp Ile Ala Val Glu Tyr Trp Ser Thr Val Ala Lys Ile Pro
145 150 155 160

Ala Arg Arg Gly Glu Ala Ile Asn Gln Leu Lys Arg Ile Asn Ala Asp
165 170 175

Thr Pro Gly Asn Thr Gly Leu Gln Asn Asn Leu Ala Leu Leu Leu Phe
180 185 190

Ser Ser Asp Arg Arg Asp Glu Gly Phe Ala Val Leu Glu Gln Met Ala
195 200 205

Lys Ser Asn Ala Gly Arg Glu Gly Ala Ser Lys Ile Trp Tyr Gly Gln

210 215 220

Ile Lys Asp Met Pro Val Ser Asp Ala Ser Val Gln Ala Leu Lys Lys
225 230 235 240

Tyr Leu Ser Ile Phe Ser Asp Gly Asp Ser Val Ala Ala Ala Gln Ser
245 250 255

Gln Leu Ala Glu Gln Gln Lys Gln Leu Ala Asp Pro Ala Phe Arg Ala
260 265 270

Arg Ala Gln Gly Leu Ala Ala Val Asp Ser Gly Met Ala Gly Lys Ala
275 280 285

Ile Pro Glu Leu Gln Gln Ala Val Arg Ala Asn Pro Lys Asp Ser Glu
290 295 300

Ala Leu Gly Ala Leu Gly Gln Ala Tyr Ser Gln Lys Gly Asp Arg Ala
305 310 315 320

Asn Ala Val Ala Asn Leu Glu Lys Ala Leu Ala Leu Asp Pro His Asn
325 330 335

Ser Asn Asn Asp Lys Trp Asn Ser Leu Leu Lys Val Asn Arg Tyr Trp
340 345 350

Leu Ala Ile Gln Gln Gly Asp Ala Ala Leu Lys Ala Asn Asn Pro Asp
355 360 365

Arg Ala Glu Arg Leu Phe Gln Gln Ala Arg Asn Val Asp Asn Thr Asp
370 375 380

Ser Tyr Ala Val Leu Gly Leu Gly Asp Val Ala Met Ala Arg Lys Asp
385 390 395 400

Tyr Pro Ala Ala Glu Arg Tyr Tyr Gln Gln Thr Leu Arg Met Asp Ser
405 410 415

Gly Asn Thr Asn Ala Val Arg Gly Leu Ala Asn Ile Tyr Arg Gln Gln
420 425 430

Ser Pro Glu Lys Ala Glu Ala Tyr Ile Ala Ser Leu Ser Ala Ser Gln
435 440 445

Arg Arg Ser Ile Asp Asp Ile Glu Arg Ser Leu Gln Asn Asp Arg Leu
450 455 460

Ala Gln Gln Ala Glu Ala Leu Glu Asn Gln Gly Lys Trp Ala Gln Thr
465 470 475 480

Ala Ala Leu Gln Arg Gln Arg Leu Ala Leu Asp Pro Gly Ser Val Trp
485 490 495

Ile Thr Tyr Arg Leu Ser Gln Asp Leu Trp Gln Ala Gly Gln Arg Ser
500 505 510

Gln Ala Asp Thr Leu Met Arg Asn Leu Ala Gln Gln Lys Pro Asn Asp
515 520 525

Pro Glu Gln Val Tyr Ala Tyr Gly Leu Tyr Leu Ser Gly His Asp Gln
530 535 540

Asp Arg Ala Ala Leu Ala His Ile Asn Ser Leu Pro His Ala Gln Trp
545 550 555 560

Asn Ser Asn Ile Gln Glu Leu Val Asn Arg Leu Gln Asn Asp Gln Val
565 570 575

Leu Glu Thr Ala Asn Arg Leu Arg Glu Asn Gly Lys Glu Ala Glu Ala
580 585 590

Glu Ala Met Leu Arg Gln Gln Pro Pro Ser Ser Arg Ile Asp Leu Thr
595 600 605

Leu Ala Asp Trp Ala Gln Gln Arg Arg Asp Tyr Thr Ala Ala Arg Ala
610 615 620

Ala Tyr Gln Asn Val Leu Thr Arg Glu Pro Thr Asn Ala Asp Ala Ile
625 630 635 640

Leu Gly Leu Thr Glu Val Asp Ile Ala Ala Gly Asp Lys Ala Ala Ala
645 650 655

Arg Ser Gln Leu Ala Lys Leu Pro Ala Thr Asp Asn Ala Ser Leu Asn
660 665 670

Thr Gln Arg Arg Val Ala Leu Ala Gln Ala Gln Leu Gly Asp Thr Ala
675 680 685

Ala Ala Gln Gln Thr Phe Asn Lys Leu Ile Pro Gln Ala Lys Ser Gln
690 695 700

Pro Pro Ser Met Glu Ser Ala Met Val Leu Arg Asp Gly Ala Lys Phe
705 710 715 720

Glu Ala Gln Ala Gly Asp Pro Thr Gln Ala Leu Glu Thr Tyr Lys Asp
725 730 735

Ala Met Val Ala Ser Gly Val Thr Thr Thr Arg Pro Gln Asp Asn Asp
740 745 750

Thr Phe Thr Arg Leu Thr Arg Asn Asp Asp Lys Asp Asp Trp Leu Lys
755 760 765

Arg Gly Val Arg Ser Asp Ala Ala Asp Leu Tyr Arg Gln Gln Asp Leu
770 775 780

Asn Val Thr Leu Glu His Asp Tyr Trp Gly Ser Ser Gly Thr Gly Gly
785 790 795 800

Tyr Ser Asp Leu Lys Ala His Thr Thr Met Leu Gln Val Asp Ala Pro
805 810 815

Tyr Ser Asp Gly Arg Met Phe Phe Arg Ser Asp Phe Val Asn Met Asn
820 825 830

Val Gly Ser Phe Ser Thr Asn Ala Asp Gly Lys Trp Asp Asp Asn Trp
835 840 845

Gly Thr Cys Thr Leu Gln Asp Cys Ser Gly Asn Arg Ser Gln Ser Asp
850 855 860

Ser Gly Ala Ser Val Ala Val Gly Trp Arg Asn Asp Val Trp Ser Trp
865 870 875 880

Asp Ile Gly Thr Thr Pro Met Gly Phe Asn Val Val Asp Val Val Gly
885 890 895

Gly Ile Ser Tyr Ser Asp Asp Ile Gly Pro Leu Gly Tyr Thr Val Asn
900 905 910

Ala His Arg Arg Pro Ile Ser Ser Ser Leu Leu Ala Phe Gly Gly Gln
915 920 925

Lys Asp Ser Pro Ser Asn Thr Gly Lys Lys Trp Gly Gly Val Arg Ala
930 935 940

Asp Gly Val Gly Leu Ser Leu Ser Tyr Asp Lys Gly Glu Ala Asn Gly
945 950 955 960

Val Trp Ala Ser Leu Ser Gly Asp Gln Leu Thr Gly Lys Asn Val Glu
965 970 975

Asp Asn Trp Arg Val Arg Trp Met Thr Gly Tyr Tyr Tyr Lys Val Ile
980 985 990

Asn Gln Asn Asn Arg Arg Val Thr Ile Gly Leu Asn Asn Met Ile Trp
995 1000 1005

His Tyr Asp Lys Asp Leu Ser Gly Tyr Ser Leu Gly Gln Gly Gly Tyr
1010 1015 1020

Tyr Ser Pro Gln Glu Tyr Leu Ser Phe Ala Ile Pro Val Met Trp Arg
1025 1030 1035 1040

Glu Arg Thr Glu Asn Trp Ser Trp Glu Leu Gly Ala Ser Gly Ser Trp
1045 1050 1055

Ser His Ser Arg Thr Lys Thr Met Pro Arg Tyr Pro Leu Met Asn Leu
1060 1065 1070

Ile Pro Thr Asp Trp Gln Glu Glu Ala Ala Arg Gln Ser Asn Asp Gly
1075 1080 1085

Gly Ser Ser Gln Gly Phe Gly Tyr Thr Ala Arg Ala Leu Leu Glu Arg
1090 1095 1100

Arg Val Thr Ser Asn Trp Phe Val Gly Met Ala Ile Asp Ile Gln Gln
1105 1110 1115 1120

Ala Lys Asp Tyr Ala Pro Ser His Phe Leu Leu Tyr Val Arg Tyr Ser
1125 1130 1135

Ala Ala Gly Trp Gln Gly Asp Met Asp Leu Pro Pro Gln Pro Leu Ile
1140 1145 1150

Pro Tyr Ala Asp Trp
1155

<210> 115
<211> 562
<212> PRT
<213> Escherichia coli

<400> 115
Met Ser Gly Glu Lys Lys Ala Lys Gly Trp Arg Phe Tyr Gly Leu Val
1 5 10 15

Gly Leu Gly Ala Ile Val Leu Leu Ser Thr Gly Val Trp Ala Leu Gln
20 25 30

Tyr Ala Gly Ser Gly Pro Glu Lys Thr Leu Ser Pro Leu Val Val His
35 40 45

Asn Asn Leu Gln Ile Asp Leu Asn Glu Pro Asp Leu Phe Leu Asp Ser

50 55 60

Asp Ser Leu Ser Gln Leu Pro Lys Asp Leu Leu Thr Ile Pro Phe Leu
65 70 75 80

His Asp Val Leu Ser Glu Asp Phe Val Phe Tyr Tyr Gln Asn His Ala
85 90 95

Asp Arg Leu Gly Ile Glu Gly Ser Ile Arg Arg Ile Val Tyr Glu His
100 105 110

Asp Leu Thr Leu Lys Asp Lys Leu Phe Ser Ser Leu Leu Asp Gln Pro
115 120 125

Ala Gln Ala Ala Leu Trp His Asp Lys Gln Gly His Leu Ser His Tyr
130 135 140

Met Val Leu Ile Gln Arg Ser Gly Leu Ser Lys Leu Leu Glu Pro Leu
145 150 155 160

Leu Phe Ala Ala Thr Ser Asp Ser Gln Leu Ser Lys Thr Glu Ile Ser
165 170 175

Ser Ile Lys Leu Asn Ser Glu Thr Ile Pro Val Tyr Gln Leu Arg Tyr
180 185 190

Asn Gly Asn Asn Ala Leu Met Phe Ala Thr Tyr Gln Asp Lys Met Leu
195 200 205

Val Phe Ser Ser Thr Asp Met Leu Phe Lys Asp Asp Gln Gln Asp Thr
210 215 220

Glu Ala Ala Ala Ile Ala Ser Asp Leu Leu Ser Gly Lys Lys Arg Trp
225 230 235 240

Glu Thr Ser Phe Gly Leu Glu Glu Arg Thr Ala Glu Lys Thr Pro Val
245 250 255

Arg Gln Arg Ile Val Val Ser Ala Arg Leu Leu Gly Phe Gly Tyr Gln
260 265 270

Arg Leu Val Pro Ser Phe Ala Gly Met Arg Phe Glu Met Gly Asn Asp
275 280 285

Gly Trp His Ser Phe Leu Ala Leu Asn Asp Glu Ser Ala Ser Val Asp
290 295 300

Ala Ser Phe Asp Phe Thr Pro Val Trp Asn Ser Met Pro Ala Gly Ala
305 310 315 320

Ser Phe Cys Val Ala Val Pro Tyr Ser His Gly Ile Ala Glu Glu Met
325 330 335

Leu Ser His Ile Ser Gln Glu Asn Asp Lys Leu Asn Gly Ala Leu Asp
340 345 350

Gly Gly Ala Gly Leu Cys Trp Tyr Glu Asp Ser Lys Leu Gln Thr Pro
355 360 365

Leu Phe Val Gly Gln Phe Asp Gly Thr Ala Glu Gln Ala Gln Leu Pro
370 375 380

Gly Lys Leu Phe Thr Gln Asn Ile Gly Ala His Glu Ser Lys Ala Pro
385 390 395 400

```
Glu Gly Val Leu Pro Val Ser Gln Thr Gln Gln Gly Glu Ala Gln Ile
                405                 410                 415

Trp Arg Arg Glu Val Ser Ser Arg Tyr Gly Gln Tyr Pro Lys Ala Gln
            420                 425                 430

Ala Ala Gln Pro Asp Gln Leu Met Ser Asp Tyr Phe Phe Arg Val Ser
        435                 440                 445

Leu Ala Met Gln Asn Lys Thr Leu Leu Phe Ser Leu Asp Asp Thr Leu
    450                 455                 460

Val Asn Asn Ala Leu Gln Ala Leu Asn Lys Thr Arg Pro Ala Met Val
465                 470                 475                 480

Asp Val Ile Pro Thr Asp Gly Ile Val Pro Leu Tyr Ile Asn Pro Gln
                485                 490                 495

Gly Met Ala Lys Leu Leu Arg Asn Glu Thr Leu Thr Ser Leu Pro Lys
            500                 505                 510

Asn Leu Glu Pro Val Phe Tyr Asn Ala Ala Gln Thr Leu Leu Met Pro
        515                 520                 525

Lys Leu Asp Ala Leu Ser Gln Gln Pro Arg Tyr Val Met Lys Leu Ala
    530                 535                 540

Gln Met Glu Pro Gly Ala Ala Trp Gln Trp Leu Pro Ile Thr Trp Gln
545                 550                 555                 560

Pro Leu


<210>   116
<211>   474
<212>   PRT
<213>   Escherichia coli

<400>   116
Met Lys Lys Thr Thr Leu Ala Leu Ser Ala Leu Ala Leu Ser Leu Gly
1               5                   10                  15

Leu Ala Leu Ser Pro Leu Ser Ala Thr Ala Ala Glu Thr Ser Ser Ala
            20                  25                  30

Thr Thr Ala Gln Gln Met Pro Ser Leu Ala Pro Met Leu Glu Lys Val
        35                  40                  45

Met Pro Ser Val Val Ser Ile Asn Val Glu Gly Ser Thr Thr Val Asn
    50                  55                  60

Thr Pro Arg Met Pro Arg Asn Phe Gln Gln Phe Phe Gly Asp Asp Ser
65                  70                  75                  80

Pro Phe Cys Gln Glu Gly Ser Pro Phe Gln Ser Ser Pro Phe Cys Gln
                85                  90                  95

Gly Gly Gln Gly Gly Asn Gly Gly Gly Gln Gln Gln Lys Phe Met Ala
            100                 105                 110

Leu Gly Ser Gly Val Ile Ile Asp Ala Asp Lys Gly Tyr Val Val Thr
        115                 120                 125

Asn Asn His Val Val Asp Asn Ala Thr Val Ile Lys Val Gln Leu Ser
    130                 135                 140
```

```
Asp Gly Arg Lys Phe Asp Ala Lys Met Val Gly Lys Asp Pro Arg Ser
145                 150                 155                 160

Asp Ile Ala Leu Ile Gln Ile Gln Asn Pro Lys Asn Leu Thr Ala Ile
                165                 170                 175

Lys Met Ala Asp Ser Asp Ala Leu Arg Val Gly Asp Tyr Thr Val Ala
            180                 185                 190

Ile Gly Asn Pro Phe Gly Leu Gly Glu Thr Val Thr Ser Gly Ile Val
        195                 200                 205

Ser Ala Leu Gly Arg Ser Gly Leu Asn Ala Glu Asn Tyr Glu Asn Phe
    210                 215                 220

Ile Gln Thr Asp Ala Ala Ile Asn Arg Gly Asn Ser Gly Gly Ala Leu
225                 230                 235                 240

Val Asn Leu Asn Gly Glu Leu Ile Gly Ile Asn Thr Ala Ile Leu Ala
                245                 250                 255

Pro Asp Gly Gly Asn Ile Gly Ile Gly Phe Ala Ile Pro Ser Asn Met
            260                 265                 270

Val Lys Asn Leu Thr Ser Gln Met Val Glu Tyr Gly Gln Val Lys Arg
        275                 280                 285

Gly Glu Leu Gly Ile Met Gly Thr Glu Leu Asn Ser Asp Leu Ala Lys
    290                 295                 300

Ala Met Lys Val Asp Ala Gln Arg Gly Ala Phe Val Ser Gln Val Leu
305                 310                 315                 320

Pro Asn Ser Ser Ala Ala Lys Ala Gly Ile Lys Ala Gly Asp Val Ile
                325                 330                 335

Thr Ser Leu Asn Gly Lys Pro Ile Ser Ser Phe Ala Ala Leu Arg Ala
            340                 345                 350

Gln Val Gly Thr Met Pro Val Gly Ser Lys Leu Thr Leu Gly Leu Leu
        355                 360                 365

Arg Asp Gly Lys Gln Val Asn Val Asn Leu Glu Leu Gln Gln Ser Ser
    370                 375                 380

Gln Asn Gln Val Asp Ser Ser Thr Ile Phe Asn Gly Ile Glu Gly Ala
385                 390                 395                 400

Glu Met Ser Asn Lys Gly Lys Asp Gln Gly Val Val Val Asn Asn Val
                405                 410                 415

Lys Thr Gly Thr Pro Ala Ala Gln Ile Gly Leu Lys Lys Gly Asp Val
            420                 425                 430

Ile Ile Gly Ala Asn Gln Gln Ala Val Lys Asn Ile Ala Glu Leu Arg
        435                 440                 445

Lys Val Leu Asp Ser Lys Pro Ser Val Leu Ala Leu Asn Ile Gln Arg
    450                 455                 460

Gly Asp Ser Thr Ile Tyr Leu Leu Met Gln
465                 470
```

<210> 117

<211> 344
<212> PRT
<213> Escherichia coli

<400> 117

```
Met Ala Tyr Ser Val Gln Lys Ser Arg Leu Ala Lys Val Ala Gly Val
1               5                   10                  15

Ser Leu Val Leu Leu Leu Ala Ala Cys Ser Ser Asp Ser Arg Tyr Lys
            20                  25                  30

Arg Gln Val Ser Gly Asp Glu Ala Tyr Leu Glu Ala Ala Pro Leu Ala
        35                  40                  45

Glu Leu His Ala Pro Ala Gly Met Ile Leu Pro Val Thr Ser Gly Asp
    50                  55                  60

Tyr Ala Ile Pro Val Thr Asn Gly Ser Gly Ala Val Gly Lys Ala Leu
65                  70                  75                  80

Asp Ile Arg Pro Pro Ala Gln Pro Leu Ala Leu Val Ser Gly Ala Arg
                85                  90                  95

Thr Gln Phe Thr Gly Asp Thr Ala Ser Leu Leu Val Glu Asn Gly Arg
            100                 105                 110

Gly Asn Thr Leu Trp Pro Gln Val Val Ser Val Leu Gln Ala Lys Asn
        115                 120                 125

Tyr Thr Ile Thr Gln Arg Asp Asp Ala Gly Gln Thr Leu Thr Thr Asp
    130                 135                 140

Trp Val Gln Trp Asn Arg Leu Asp Glu Asp Glu Gln Tyr Arg Gly Arg
145                 150                 155                 160

Tyr Gln Ile Ser Val Lys Pro Gln Gly Tyr Gln Gln Ala Val Thr Val
                165                 170                 175

Lys Leu Leu Asn Leu Glu Gln Ala Gly Lys Pro Val Ala Asp Ala Ala
            180                 185                 190

Ser Met Gln Arg Tyr Ser Thr Glu Met Met Asn Val Ile Ser Ala Gly
        195                 200                 205

Leu Asp Lys Ser Ala Thr Asp Ala Ala Asn Ala Ala Gln Asn Arg Ala
    210                 215                 220

Ser Thr Thr Met Asp Val Gln Ser Ala Ala Asp Asp Thr Gly Leu Pro
225                 230                 235                 240

Met Leu Val Val Arg Gly Pro Phe Asn Val Val Trp Gln Arg Leu Pro
                245                 250                 255

Ala Ala Leu Glu Lys Val Gly Met Lys Val Thr Asp Ser Thr Arg Ser
            260                 265                 270

Gln Gly Asn Met Ala Val Thr Tyr Lys Pro Leu Ser Asp Ser Glu Trp
        275                 280                 285

His Glu Leu Gly Ala Ser Asp Pro Gly Leu Ala Ser Gly Asp Tyr Lys
    290                 295                 300

Leu Gln Val Gly Asp Leu Asp Asn Arg Ser Ser Leu Gln Phe Ile Asp
305                 310                 315                 320
```

```
Pro Lys Gly His Thr Leu Thr Gln Ser Gln Asn Asp Ala Leu Val Ala
                325                 330                 335

Val Phe Gln Ala Ala Phe Ser Lys
            340

<210>   118
<211>   420
<212>   PRT
<213>   Escherichia coli

<400>   118
Met Ile Lys Ala Thr Asp Arg Lys Leu Val Val Gly Leu Glu Ile Gly
1               5                   10                  15

Thr Ala Lys Val Ala Ala Leu Val Gly Glu Val Leu Pro Asp Gly Met
            20                  25                  30

Val Asn Ile Ile Gly Val Gly Ser Cys Pro Ser Arg Gly Met Asp Lys
        35                  40                  45

Gly Gly Val Asn Asp Leu Glu Ser Val Val Lys Cys Val Gln Arg Ala
    50                  55                  60

Ile Asp Gln Ala Glu Leu Met Ala Asp Cys Gln Ile Ser Ser Val Tyr
65                  70                  75                  80

Leu Ala Leu Ser Gly Lys His Ile Ser Cys Gln Asn Glu Ile Gly Met
                85                  90                  95

Val Pro Ile Ser Glu Glu Glu Val Thr Gln Glu Asp Val Glu Asn Val
            100                 105                 110

Val His Thr Ala Lys Ser Val Arg Val Arg Asp Glu His Arg Val Leu
        115                 120                 125

His Val Ile Pro Gln Glu Tyr Ala Ile Asp Tyr Gln Glu Gly Ile Lys
    130                 135                 140

Asn Pro Val Gly Leu Ser Gly Val Arg Met Gln Ala Lys Val His Leu
145                 150                 155                 160

Ile Thr Cys His Asn Asp Met Ala Lys Asn Ile Val Lys Ala Val Glu
                165                 170                 175

Arg Cys Gly Leu Lys Val Asp Gln Leu Ile Phe Ala Gly Leu Ala Ser
            180                 185                 190

Ser Tyr Ser Val Leu Thr Glu Asp Glu Arg Glu Leu Gly Val Cys Val
        195                 200                 205

Val Asp Ile Gly Gly Gly Thr Met Asp Ile Ala Val Tyr Thr Gly Gly
    210                 215                 220

Ala Leu Arg His Thr Lys Val Ile Pro Tyr Ala Gly Asn Val Val Thr
225                 230                 235                 240

Ser Asp Ile Ala Tyr Ala Phe Gly Thr Pro Pro Ser Asp Ala Glu Ala
                245                 250                 255

Ile Lys Val Arg His Gly Cys Ala Leu Gly Ser Ile Val Gly Lys Asp
            260                 265                 270

Glu Ser Val Glu Val Pro Ser Val Gly Gly Arg Pro Pro Arg Ser Leu
        275                 280                 285
```

Gln Arg Gln Thr Leu Ala Glu Val Ile Glu Pro Arg Tyr Thr Glu Leu
290 295 300

Leu Asn Leu Val Asn Glu Glu Ile Leu Gln Leu Gln Glu Lys Leu Arg
305 310 315 320

Gln Gln Gly Val Lys His His Leu Ala Ala Gly Ile Val Leu Thr Gly
325 330 335

Gly Ala Ala Gln Ile Glu Gly Leu Ala Ala Cys Ala Gln Arg Val Phe
340 345 350

His Thr Gln Val Arg Ile Gly Ala Pro Leu Asn Ile Thr Gly Leu Thr
355 360 365

Asp Tyr Ala Gln Glu Pro Tyr Tyr Ser Thr Ala Val Gly Leu Leu His
370 375 380

Tyr Gly Lys Glu Ser His Leu Asn Gly Glu Ala Glu Val Glu Lys Arg
385 390 395 400

Val Thr Ala Ser Val Gly Ser Trp Ile Lys Arg Leu Asn Ser Trp Leu
405 410 415

Arg Lys Glu Phe
420

<210> 119
<211> 161
<212> PRT
<213> Escherichia coli

<400> 119
Val Lys Lys Trp Leu Leu Ala Ala Gly Leu Gly Leu Ala Leu Ala Thr
1 5 10 15

Ser Ala Gln Ala Ala Asp Lys Ile Ala Ile Val Asn Met Gly Ser Leu
20 25 30

Phe Gln Gln Val Ala Gln Lys Thr Gly Val Ser Asn Thr Leu Glu Asn
35 40 45

Glu Phe Lys Gly Arg Ala Ser Glu Leu Gln Arg Met Glu Thr Asp Leu
50 55 60

Gln Ala Lys Met Lys Lys Leu Gln Ser Met Lys Ala Gly Ser Asp Arg
65 70 75 80

Thr Lys Leu Glu Lys Asp Val Met Ala Gln Arg Gln Thr Phe Ala Gln
85 90 95

Lys Ala Gln Ala Phe Glu Gln Asp Arg Ala Arg Arg Ser Asn Glu Glu
100 105 110

Arg Gly Lys Leu Val Thr Arg Ile Gln Thr Ala Val Lys Ser Val Ala
115 120 125

Asn Ser Gln Asp Ile Asp Leu Val Val Asp Ala Asn Ala Val Ala Tyr
130 135 140

Asn Ser Ser Asp Val Lys Asp Ile Thr Ala Asp Val Leu Lys Gln Val
145 150 155 160

Lys

<210> 120
<211> 213
<212> PRT
<213> Escherichia coli

<400> 120
Met Thr Lys Met Ser Arg Tyr Ala Leu Ile Thr Ala Leu Ala Met Phe
1 5 10 15

Leu Ala Gly Cys Val Gly Gln Arg Glu Pro Ala Pro Val Glu Glu Val
20 25 30

Lys Pro Ala Pro Glu Gln Pro Ala Glu Pro Gln Gln Pro Val Pro Thr
35 40 45

Val Pro Ser Val Pro Thr Ile Pro Gln Gln Pro Gly Pro Ile Glu His
50 55 60

Glu Asp Gln Thr Ala Pro Pro Ala Pro His Ile Arg His Tyr Asp Trp
65 70 75 80

Asn Gly Ala Met Gln Pro Met Val Ser Lys Met Leu Gly Ala Asp Gly
85 90 95

Val Thr Ala Gly Ser Val Leu Leu Val Asp Ser Val Asn Asn Arg Thr
100 105 110

Asn Gly Ser Leu Asn Ala Ala Glu Ala Thr Glu Thr Leu Arg Asn Ala
115 120 125

Leu Ala Asn Asn Gly Lys Phe Thr Leu Val Ser Ala Gln Gln Leu Ser
130 135 140

Met Ala Lys Gln Gln Leu Gly Leu Ser Pro Gln Asp Ser Leu Gly Thr
145 150 155 160

Arg Ser Lys Ala Ile Gly Ile Ala Arg Asn Val Gly Ala His Tyr Val
165 170 175

Leu Tyr Ser Ser Ala Ser Gly Asn Val Asn Ala Pro Thr Leu Gln Met
180 185 190

Gln Leu Met Leu Val Gln Thr Gly Glu Ile Ile Trp Ser Gly Lys Gly
195 200 205

Ala Val Ser Gln Gln
210

<210> 121
<211> 392
<212> PRT
<213> Escherichia coli

<400> 121
Met Gln Leu Arg Lys Leu Leu Leu Pro Gly Leu Leu Ser Val Thr Leu
1 5 10 15

Leu Ser Gly Cys Ser Leu Phe Asn Ser Glu Glu Asp Val Val Lys Met
20 25 30

Ser Pro Leu Pro Thr Val Glu Asn Gln Phe Thr Pro Thr Thr Val Trp
35 40 45

```
Ser Thr Ser Val Gly Ser Gly Ile Gly Asn Phe Tyr Ser Asn Leu His
    50                  55                  60

Pro Ala Leu Ala Asp Asn Val Val Tyr Ala Ala Asp Arg Ala Gly Leu
65                  70                  75                  80

Val Lys Ala Leu Asn Ala Asp Asp Gly Lys Glu Ile Trp Ser Val Asn
                85                  90                  95

Leu Ala Glu Lys Asp Gly Trp Phe Ser Lys Asp Pro Ala Leu Leu Ser
            100                 105                 110

Gly Gly Val Thr Val Ser Gly Gly His Val Tyr Ile Gly Thr Glu Lys
        115                 120                 125

Ala Gln Val Tyr Ala Leu Asn Thr Ser Asp Gly Thr Val Ala Trp Gln
    130                 135                 140

Thr Lys Val Ala Gly Glu Ala Leu Ser Arg Pro Val Val Ser Asp Gly
145                 150                 155                 160

Leu Val Leu Ile His Thr Ser Asn Gly Gln Leu Gln Ala Leu Asn Glu
                165                 170                 175

Ala Asp Gly Ala Val Lys Trp Thr Val Asn Leu Asp Met Pro Ser Leu
            180                 185                 190

Ser Leu Arg Gly Glu Ser Ala Pro Ala Thr Ala Phe Gly Ala Ala Val
        195                 200                 205

Val Gly Gly Asp Asn Gly Arg Val Ser Ala Val Leu Met Glu Gln Gly
    210                 215                 220

Gln Met Ile Trp Gln Gln Arg Ile Ser Gln Ala Thr Gly Ser Thr Glu
225                 230                 235                 240

Ile Asp Arg Leu Ser Asp Val Asp Thr Thr Pro Val Val Val Asn Gly
                245                 250                 255

Val Val Phe Ala Leu Ala Tyr Asn Gly Asn Leu Thr Ala Leu Asp Leu
            260                 265                 270

Arg Ser Gly Gln Ile Met Trp Lys Arg Glu Leu Gly Ser Val Asn Asp
        275                 280                 285

Phe Ile Val Asp Gly Asn Arg Ile Tyr Leu Val Asp Gln Asn Asp Arg
    290                 295                 300

Val Met Ala Leu Thr Ile Asp Gly Gly Val Thr Leu Trp Thr Gln Ser
305                 310                 315                 320

Asp Leu Leu His Arg Leu Leu Thr Ser Pro Val Leu Tyr Asn Gly Asn
                325                 330                 335

Leu Val Val Gly Asp Ser Glu Gly Tyr Leu His Trp Ile Asn Val Glu
            340                 345                 350

Asp Gly Arg Phe Val Ala Gln Gln Lys Val Asp Ser Ser Gly Phe Gln
        355                 360                 365

Thr Glu Pro Val Ala Ala Asp Gly Lys Leu Leu Ile Gln Ala Lys Asp
    370                 375                 380

Gly Thr Val Tyr Ser Ile Thr Arg
385                 390
```

<210> 122
<211> 678
<212> PRT
<213> Escherichia coli

<400> 122

```
Met Val Pro Ser Thr Phe Ser Arg Leu Lys Ala Ala Arg Cys Leu Pro
1               5                   10                  15

Val Val Leu Ala Ala Leu Ile Phe Ala Gly Cys Gly Thr His Thr Pro
            20                  25                  30

Asp Gln Ser Thr Ala Tyr Met Gln Gly Thr Ala Gln Ala Asp Ser Ala
        35                  40                  45

Phe Tyr Leu Gln Gln Met Gln Gln Ser Ser Asp Asp Thr Arg Ile Asn
    50                  55                  60

Trp Gln Leu Leu Ala Ile Arg Ala Leu Val Lys Glu Gly Lys Thr Gly
65                  70                  75                  80

Gln Ala Val Glu Leu Phe Asn Gln Leu Pro Gln Glu Leu Asn Asp Ser
                85                  90                  95

Gln Arg Arg Glu Lys Thr Leu Leu Ala Ala Glu Ile Lys Leu Ala Gln
            100                 105                 110

Lys Asp Phe Ala Gly Ala Gln Asn Leu Leu Ala Lys Ile Thr Pro Ala
        115                 120                 125

Asp Leu Glu Gln Asn Gln Gln Ala Arg Tyr Trp Gln Ala Lys Ile Asp
    130                 135                 140

Ala Ser Gln Gly Arg Pro Ser Ile Asp Leu Leu Arg Ala Leu Ile Ala
145                 150                 155                 160

Gln Glu Pro Leu Leu Gly Ala Lys Glu Lys Gln Gln Asn Ile Asp Ala
                165                 170                 175

Thr Trp Gln Ala Leu Ser Ser Met Thr Gln Glu Gln Ala Asn Thr Leu
            180                 185                 190

Val Ile Asn Ala Asp Glu Asn Ile Leu Gln Gly Trp Leu Asp Leu Gln
        195                 200                 205

Arg Val Trp Phe Asp Asn Arg Asn Asp Pro Asp Met Met Lys Ala Gly
    210                 215                 220

Ile Ala Asp Trp Gln Lys Arg Tyr Pro Asn Asn Pro Gly Ala Lys Met
225                 230                 235                 240

Leu Pro Thr Gln Leu Val Asn Val Lys Ala Phe Lys Pro Ala Ser Thr
                245                 250                 255

Asn Lys Ile Ala Leu Leu Leu Pro Leu Asn Gly Gln Ala Ala Val Phe
            260                 265                 270

Gly Arg Thr Ile Gln Gln Gly Phe Glu Ala Ala Lys Asn Ile Gly Thr
        275                 280                 285

Gln Pro Val Ala Ala Gln Val Ala Ala Ala Pro Ala Ala Asp Val Ala
    290                 295                 300

Glu Gln Pro Gln Pro Gln Thr Val Asp Gly Val Ala Ser Pro Ala Gln
```

305 310 315 320

Ala Ser Val Ser Asp Leu Thr Gly Glu Gln Pro Ala Ala Gln Ser Val
325 330 335

Pro Val Ser Ala Pro Ala Thr Ser Thr Ala Ala Val Ser Ala Pro Ala
340 345 350

Asn Pro Ser Ala Glu Leu Lys Ile Tyr Asp Thr Ser Ser Gln Pro Leu
355 360 365

Ser Gln Ile Leu Ser Gln Val Gln Gln Asp Gly Ala Ser Ile Val Val
370 375 380

Gly Pro Leu Leu Lys Asn Asn Val Glu Glu Leu Leu Lys Ser Asn Thr
385 390 395 400

Pro Leu Asn Val Leu Ala Leu Asn Gln Pro Glu Asn Ile Glu Asn Arg
405 410 415

Val Asn Ile Cys Tyr Phe Ala Leu Ser Pro Glu Asp Glu Ala Arg Asp
420 425 430

Ala Ala Arg His Ile Arg Asp Gln Gly Lys Gln Ala Pro Leu Val Leu
435 440 445

Ile Pro Arg Ser Ser Leu Gly Asp Arg Val Ala Asn Ala Phe Ala Gln
450 455 460

Glu Trp Gln Lys Leu Gly Gly Gly Pro Val Leu Gln Gln Lys Phe Gly
465 470 475 480

Ser Thr Ser Glu Leu Arg Ala Gly Val Asn Gly Gly Ser Gly Ile Ala
485 490 495

Leu Thr Gly Ser Pro Ile Thr Pro Arg Glu Thr Thr Asp Ser Gly Met
500 505 510

Thr Thr Asn Asn Pro Thr Leu Gln Thr Thr Pro Thr Asp Asp Gln Phe
515 520 525

Thr Asn Asn Gly Gly Arg Val Asp Ala Val Tyr Ile Val Ala Thr Pro
530 535 540

Gly Glu Ile Ala Phe Ile Lys Pro Met Ile Ala Met Arg Asn Gly Ser
545 550 555 560

Gln Ser Gly Ala Thr Leu Tyr Ala Ser Ser Arg Ser Ala Gln Gly Thr
565 570 575

Ala Gly Pro Asp Phe Arg Leu Glu Met Glu Gly Leu Gln Tyr Ser Glu
580 585 590

Ile Pro Met Leu Ala Gly Gly Asn Leu Pro Leu Met Gln Gln Ala Leu
595 600 605

Ser Ala Val Asn Asn Asp Tyr Ser Leu Ala Arg Met Tyr Ala Met Gly
610 615 620

Val Asp Ala Trp Ser Leu Ala Asn His Phe Ser Gln Met Arg Gln Val
625 630 635 640

Gln Gly Phe Glu Ile Asn Gly Asn Thr Gly Ser Leu Thr Ala Asn Pro
645 650 655

Asp Cys Val Ile Asn Arg Lys Leu Ser Trp Leu Gln Tyr Gln Gln Gly
660 665 670

Gln Val Val Pro Ala Ser
675

<210> 123
<211> 191
<212> PRT
<213> Escherichia coli

<400> 123
Met Lys Ala Leu Ser Pro Ile Ala Val Leu Ile Ser Ala Leu Leu Leu
1 5 10 15

Gln Gly Cys Val Ala Ala Ala Val Val Gly Thr Ala Ala Val Gly Thr
20 25 30

Lys Ala Ala Thr Asp Pro Arg Ser Val Gly Thr Gln Val Asp Asp Gly
35 40 45

Thr Leu Glu Val Arg Val Asn Ser Ala Leu Ser Lys Asp Glu Gln Ile
50 55 60

Lys Lys Glu Ala Arg Ile Asn Val Thr Ala Tyr Gln Gly Lys Val Leu
65 70 75 80

Leu Val Gly Gln Ser Pro Asn Ala Glu Leu Ser Ala Arg Ala Lys Gln
85 90 95

Ile Ala Met Gly Val Asp Gly Ala Asn Glu Val Tyr Asn Glu Ile Arg
100 105 110

Gln Gly Gln Pro Ile Gly Leu Gly Glu Ala Ser Asn Asp Thr Trp Ile
115 120 125

Thr Thr Lys Val Arg Ser Gln Leu Leu Thr Ser Gly Leu Val Lys Ser
130 135 140

Ser Asn Val Lys Val Thr Thr Glu Asn Gly Glu Val Phe Leu Met Gly
145 150 155 160

Leu Val Thr Glu Arg Glu Ala Lys Ala Ala Ala Asp Ile Ala Ser Arg
165 170 175

Val Ser Gly Val Lys Arg Val Thr Thr Ala Phe Thr Phe Ile Lys
180 185 190

<210> 124
<211> 270
<212> PRT
<213> Escherichia coli

<400> 124
Met Lys Ser Leu Phe Lys Val Thr Leu Leu Pro Thr Thr Met Ala Val
1 5 10 15

Val Leu His Ala Pro Ile Thr Phe Ala Ala Glu Ala Ala Lys Pro Ala
20 25 30

Thr Thr Ala Asp Ser Lys Ala Ala Phe Lys Asn Asp Asp Gln Lys Ser
35 40 45

Ala Tyr Ala Leu Gly Ala Ser Leu Gly Arg Tyr Met Glu Asn Ser Leu
50 55 60

Lys Glu Gln Glu Lys Leu Gly Ile Lys Leu Asp Lys Asp Gln Leu Ile
65 70 75 80

Ala Gly Val Gln Asp Ala Phe Ala Asp Lys Ser Lys Leu Ser Asp Gln
85 90 95

Glu Ile Glu Gln Thr Leu Gln Ala Phe Glu Ala Arg Val Lys Ser Ser
100 105 110

Ala Gln Ala Lys Met Glu Lys Asp Ala Ala Asp Asn Glu Ala Lys Gly
115 120 125

Lys Glu Tyr Arg Glu Lys Phe Ala Lys Glu Lys Gly Val Lys Thr Ser
130 135 140

Ser Thr Gly Leu Val Tyr Gln Val Val Glu Ala Gly Lys Gly Glu Ala
145 150 155 160

Pro Lys Asp Ser Asp Thr Val Val Val Asn Tyr Lys Gly Thr Leu Ile
165 170 175

Asp Gly Lys Glu Phe Asp Asn Ser Tyr Thr Arg Gly Glu Pro Leu Ser
180 185 190

Phe Arg Leu Asp Gly Val Ile Pro Gly Trp Thr Glu Gly Leu Lys Asn
195 200 205

Ile Lys Lys Gly Gly Lys Ile Lys Leu Val Ile Pro Pro Glu Leu Ala
210 215 220

Tyr Gly Lys Ala Gly Val Pro Gly Ile Pro Pro Asn Ser Thr Leu Val
225 230 235 240

Phe Asp Val Glu Leu Leu Asp Val Lys Pro Ala Pro Lys Ala Asp Ala
245 250 255

Lys Pro Glu Ala Asp Ala Lys Ala Ala Asp Ser Ala Lys Lys
260 265 270

<210> 125
<211> 427
<212> PRT
<213> Escherichia coli

<400> 125
Val Asn Thr Phe Ser Val Ser Arg Leu Ala Leu Thr Leu Ala Phe Gly
1 5 10 15

Val Thr Leu Thr Ala Cys Ser Ser Thr Pro Pro Asp Gln Arg Pro Ser
20 25 30

Asp Gln Thr Ala Pro Gly Thr Ser Ser Arg Pro Ile Leu Ser Ala Lys
35 40 45

Glu Ala Gln Asn Phe Asp Ala Gln His Tyr Phe Ala Ser Leu Thr Pro
50 55 60

Gly Ala Ala Ala Trp Asn Pro Ser Pro Ile Thr Leu Pro Ala Gln Pro
65 70 75 80

Asp Phe Val Val Gly Pro Ala Gly Thr Gln Gly Val Thr His Thr Thr
85 90 95

Ile Gln Ala Ala Val Asp Ala Ala Ile Ile Lys Arg Thr Asn Lys Arg

100 105 110

Gln Tyr Ile Ala Val Met Pro Gly Glu Tyr Gln Gly Thr Val Tyr Val
115 120 125

Pro Ala Ala Pro Gly Gly Ile Thr Leu Tyr Gly Thr Gly Glu Lys Pro
130 135 140

Ile Asp Val Lys Ile Gly Leu Ser Leu Asp Gly Gly Met Ser Pro Ala
145 150 155 160

Asp Trp Arg His Asp Val Asn Pro Arg Gly Lys Tyr Met Pro Gly Lys
165 170 175

Pro Ala Trp Tyr Met Tyr Asp Ser Cys Gln Ser Lys Arg Ser Asp Ser
180 185 190

Ile Gly Val Leu Cys Ser Ala Val Phe Trp Ser Gln Asn Asn Gly Leu
195 200 205

Gln Leu Gln Asn Leu Thr Ile Glu Asn Thr Leu Gly Asp Ser Val Asp
210 215 220

Ala Gly Asn His Pro Ala Val Ala Leu Arg Thr Asp Gly Asp Gln Val
225 230 235 240

Gln Ile Asn Asn Val Asn Ile Leu Gly Arg Gln Asn Thr Phe Phe Val
245 250 255

Thr Asn Ser Gly Val Gln Asn Arg Leu Glu Thr Asn Arg Gln Pro Arg
260 265 270

Thr Leu Val Thr Asn Ser Tyr Ile Glu Gly Asp Val Asp Ile Val Ser
275 280 285

Gly Arg Gly Ala Val Val Phe Asp Asn Thr Glu Phe Arg Val Val Asn
290 295 300

Ser Arg Thr Gln Gln Glu Ala Tyr Val Phe Ala Pro Ala Thr Leu Ser
305 310 315 320

Asn Ile Tyr Tyr Gly Phe Leu Ala Val Asn Ser Arg Phe Asn Ala Ser
325 330 335

Gly Asp Gly Val Ala Gln Leu Gly Arg Ser Leu Asp Val Asp Ala Asn
340 345 350

Thr Asn Gly Gln Val Val Ile Arg Asp Ser Ala Ile Asn Glu Gly Phe
355 360 365

Asn Thr Ala Lys Pro Trp Ala Asp Ala Val Ile Ser Asn Arg Pro Phe
370 375 380

Ala Gly Asn Thr Gly Ser Val Asp Asp Asn Asp Glu Val Gln Arg Asn
385 390 395 400

Leu Asn Asp Thr Asn Tyr Asn Arg Met Trp Glu Tyr Asn Asn Arg Gly
405 410 415

Val Gly Ser Lys Val Val Ala Glu Ala Lys Lys
420 425

<210> 126
<211> 700
<212> PRT

<213> Escherichia coli

<400> 126

```
Met Ala Asn Gly Trp Thr Gly Asn Ile Leu Arg Val Asn Leu Thr Thr
1               5                   10                  15

Gly Asn Ile Thr Leu Glu Asp Ser Ser Lys Phe Lys Ser Phe Val Gly
            20                  25                  30

Gly Met Gly Phe Gly Tyr Lys Ile Met Tyr Asp Glu Val Pro Pro Gly
        35                  40                  45

Thr Lys Pro Phe Asp Glu Ala Asn Lys Leu Val Phe Ala Thr Gly Pro
    50                  55                  60

Leu Thr Gly Ser Gly Ala Pro Cys Ser Ser Arg Val Asn Ile Thr Ser
65                  70                  75                  80

Leu Ser Thr Phe Thr Lys Gly Asn Leu Val Val Asp Ala His Met Gly
                85                  90                  95

Gly Phe Phe Ala Ala Gln Met Lys Phe Ala Gly Tyr Asp Val Ile Ile
            100                 105                 110

Ile Glu Gly Lys Ala Lys Ser Pro Val Trp Leu Lys Ile Lys Asp Asp
        115                 120                 125

Lys Val Ser Leu Glu Lys Ala Asp Phe Leu Trp Gly Lys Gly Thr Arg
    130                 135                 140

Ala Thr Thr Glu Glu Ile Cys Arg Leu Thr Ser Pro Glu Thr Cys Val
145                 150                 155                 160

Ala Ala Ile Gly Gln Ala Gly Glu Asn Leu Val Pro Leu Ser Gly Met
                165                 170                 175

Leu Asn Ser Arg Asn His Ser Gly Gly Ala Gly Thr Gly Ala Ile Met
            180                 185                 190

Gly Ser Lys Asn Leu Lys Ala Ile Ala Val Glu Gly Thr Lys Gly Val
        195                 200                 205

Asn Ile Ala Asp Arg Gln Glu Met Lys Arg Leu Asn Asp Tyr Met Met
    210                 215                 220

Thr Glu Leu Ile Gly Ala Asn Asn Asn His Val Val Pro Ser Thr Pro
225                 230                 235                 240

Gln Ser Trp Ala Glu Tyr Ser Asp Pro Lys Ser Arg Trp Thr Ala Arg
                245                 250                 255

Lys Gly Leu Phe Trp Gly Ala Ala Glu Gly Gly Pro Ile Glu Thr Gly
            260                 265                 270

Glu Ile Pro Pro Gly Asn Gln Asn Thr Val Gly Phe Arg Thr Tyr Lys
        275                 280                 285

Ser Val Phe Asp Leu Gly Pro Ala Ala Glu Lys Tyr Thr Val Lys Met
    290                 295                 300

Ser Gly Cys His Ser Cys Pro Ile Arg Cys Met Thr Gln Met Asn Ile
305                 310                 315                 320

Pro Arg Val Lys Glu Phe Gly Val Pro Ser Thr Gly Gly Asn Thr Cys
                325                 330                 335
```

Val Ala Asn Phe Val His Thr Thr Ile Phe Pro Asn Gly Pro Lys Asp
340 345 350

Phe Glu Asp Lys Asp Asp Gly Arg Val Ile Gly Asn Leu Val Gly Leu
355 360 365

Asn Leu Phe Asp Asp Tyr Gly Leu Trp Cys Asn Tyr Gly Gln Leu His
370 375 380

Arg Asp Phe Thr Tyr Cys Tyr Ser Lys Gly Val Phe Lys Arg Val Leu
385 390 395 400

Pro Ala Glu Glu Tyr Ala Glu Ile Arg Trp Asp Gln Leu Glu Ala Gly
405 410 415

Asp Val Asn Phe Ile Lys Asp Phe Tyr Tyr Arg Leu Ala His Arg Val
420 425 430

Gly Glu Leu Ser His Leu Ala Asp Gly Ser Tyr Ala Ile Ala Glu Arg
435 440 445

Trp Asn Leu Gly Glu Glu Tyr Trp Gly Tyr Ala Lys Asn Lys Leu Trp
450 455 460

Ser Pro Phe Gly Tyr Pro Val His His Ala Asn Glu Ala Ser Ala Gln
465 470 475 480

Val Gly Ser Ile Val Asn Cys Met Phe Asn Arg Asp Cys Met Thr His
485 490 495

Thr His Ile Asn Phe Ile Gly Ser Gly Leu Pro Leu Lys Leu Gln Arg
500 505 510

Glu Val Ala Lys Glu Leu Phe Gly Ser Glu Asp Ala Tyr Asp Glu Thr
515 520 525

Lys Asn Tyr Thr Pro Ile Asn Asp Ala Lys Ile Lys Tyr Ala Lys Trp
530 535 540

Ser Leu Leu Arg Val Cys Leu His Asn Ala Val Thr Leu Cys Asn Trp
545 550 555 560

Val Trp Pro Met Thr Val Ser Pro Leu Lys Ser Arg Asn Tyr Arg Gly
565 570 575

Asp Leu Ala Leu Glu Ala Lys Phe Phe Lys Ala Ile Thr Gly Glu Asp
580 585 590

Met Thr Gln Glu Lys Leu Asp Leu Ala Ala Glu Arg Ile Phe Thr Leu
595 600 605

His Arg Ala Tyr Thr Val Lys Leu Met Gln Thr Lys Asp Met Arg Asn
610 615 620

Glu His Asp Leu Ile Cys Ser Trp Val Phe Asp Lys Asp Pro Gln Ile
625 630 635 640

Pro Val Phe Thr Glu Gly Thr Asp Lys Met Asp Arg Asp Asp Met His
645 650 655

Ala Ser Leu Thr Met Phe Tyr Lys Glu Met Gly Trp Asp Pro Gln Leu
660 665 670

Gly Cys Pro Thr Arg Glu Thr Leu Gln Arg Leu Gly Leu Glu Asp Ile

675 680 685

Ala Ala Asp Leu Ala Ala His Asn Leu Leu Pro Ala
690 695 700

<210> 127
<211> 270
<212> PRT
<213> Escherichia coli

<400> 127
Met Ala Arg Ile Ile Val Val Thr Ser Gly Lys Gly Gly Val Gly Lys
1 5 10 15

Thr Thr Ser Ser Ala Ala Ile Ala Thr Gly Leu Ala Gln Lys Gly Lys
20 25 30

Lys Thr Val Val Ile Asp Phe Asp Ile Gly Leu Arg Asn Leu Asp Leu
35 40 45

Ile Met Gly Cys Glu Arg Arg Val Val Tyr Asp Phe Val Asn Val Ile
50 55 60

Gln Gly Asp Ala Thr Leu Asn Gln Ala Leu Ile Lys Asp Lys Arg Thr
65 70 75 80

Glu Asn Leu Tyr Ile Leu Pro Ala Ser Gln Thr Arg Asp Lys Asp Ala
85 90 95

Leu Thr Arg Glu Gly Val Ala Lys Val Leu Asp Asp Leu Lys Ala Met
100 105 110

Asp Phe Glu Phe Ile Val Cys Asp Ser Pro Ala Gly Ile Glu Thr Gly
115 120 125

Ala Leu Met Ala Leu Tyr Phe Ala Asp Glu Ala Ile Ile Thr Thr Asn
130 135 140

Pro Glu Val Ser Ser Val Arg Asp Ser Asp Arg Ile Leu Gly Ile Leu
145 150 155 160

Ala Ser Lys Ser Arg Arg Ala Glu Asn Gly Glu Glu Pro Ile Lys Glu
165 170 175

His Leu Leu Leu Thr Arg Tyr Asn Pro Gly Arg Val Ser Arg Gly Asp
180 185 190

Met Leu Ser Met Glu Asp Val Leu Glu Ile Leu Arg Ile Lys Leu Val
195 200 205

Gly Val Ile Pro Glu Asp Gln Ser Val Leu Arg Ala Ser Asn Gln Gly
210 215 220

Glu Pro Val Ile Leu Asp Ile Asn Ala Asp Ala Gly Lys Ala Tyr Ala
225 230 235 240

Asp Thr Val Glu Arg Leu Leu Gly Glu Glu Arg Pro Phe Arg Phe Ile
245 250 255

Glu Glu Glu Lys Lys Gly Phe Leu Lys Arg Leu Phe Gly Gly
260 265 270

<210> 128
<211> 155
<212> PRT

<213> Escherichia coli

<400> 128
Met Ile Lys Arg Val Leu Val Val Ser Met Val Gly Leu Ser Leu Val
1 5 10 15

Gly Cys Val Asn Asn Asp Thr Leu Ser Gly Asp Val Tyr Thr Ala Ser
20 25 30

Glu Ala Lys Gln Val Gln Asn Val Ser Tyr Gly Thr Ile Val Asn Val
35 40 45

Arg Gln Val Gln Ile Gln Gly Gly Asp Asp Ser Asn Val Ile Gly Ala
50 55 60

Ile Gly Gly Ala Val Leu Gly Gly Phe Leu Gly Asn Thr Ile Gly Gly
65 70 75 80

Gly Thr Gly Arg Ser Leu Ala Thr Ala Ala Gly Ala Val Ala Gly Gly
85 90 95

Val Ala Gly Gln Gly Val Gln Ser Ala Met Asn Lys Thr Gln Gly Val
100 105 110

Glu Leu Glu Ile Arg Lys Asp Asp Gly Asn Thr Ile Met Val Val Gln
115 120 125

Lys Gln Gly Asn Thr His Phe Ser Pro Gly Gln Arg Val Val Leu Ala
130 135 140

Ser Asn Gly Ser Gln Val Thr Val Ser Pro Arg
145 150 155

<210> 129
<211> 1042
<212> PRT
<213> Escherichia coli

<400> 129
Met Leu Met Lys Arg His Leu Asn Thr Cys Tyr Arg Leu Val Trp Asn
1 5 10 15

His Ile Thr Gly Ala Phe Val Val Ala Ser Glu Leu Ala Arg Ala Arg
20 25 30

Gly Lys Arg Gly Gly Val Ala Val Ala Leu Ser Leu Ala Ala Val Thr
35 40 45

Ser Leu Pro Val Leu Ala Ala Asp Ile Val Val His Pro Gly Glu Thr
50 55 60

Val Asn Gly Gly Thr Leu Val Asn His Asp Asn Gln Phe Val Ser Gly
65 70 75 80

Thr Ala Asp Gly Val Thr Val Ser Thr Gly Leu Glu Leu Gly Pro Asp
85 90 95

Ser Asp Glu Asn Thr Gly Gly Gln Trp Ile Lys Ala Gly Gly Thr Gly
100 105 110

Arg Asn Thr Thr Val Thr Ala Asn Gly Arg Gln Ile Val Gln Ala Gly
115 120 125

Gly Thr Ala Ser Asp Thr Val Ile Arg Asp Gly Gly Gly Gln Ser Leu
130 135 140

Asn Gly Leu Ala Val Asn Thr Thr Leu Asp Asn Arg Gly Glu Gln Trp
145 150 155 160

Val His Gly Gly Gly Lys Ala Ala Gly Thr Ile Ile Asn Gln Asp Gly
165 170 175

Tyr Gln Thr Ile Lys His Gly Gly Leu Ala Thr Gly Thr Ile Val Asn
180 185 190

Thr Gly Ala Glu Gly Gly Pro Glu Ser Glu Asn Val Ser Thr Gly Gln
195 200 205

Met Val Gly Gly Thr Ala Glu Ser Thr Thr Ile Asn Asn Asn Gly Arg
210 215 220

Gln Val Ile Trp Ser Ser Gly Val Ser Arg Asp Thr Leu Ile Tyr Thr
225 230 235 240

Gly Gly Asp Gln Thr Val His Gly Glu Ala His Asn Thr Arg Leu Glu
245 250 255

Gly Gly Asn Gln Tyr Val His Lys Tyr Gly Leu Ala Leu Asn Thr Val
260 265 270

Ile Asn Glu Gly Gly Trp Gln Val Val Lys Ala Gly Gly Thr Ala Gly
275 280 285

Asn Thr Thr Ile Asn Gln Asn Gly Glu Leu Lys Val His Ala Gly Gly
290 295 300

Glu Ala Ser Asp Val Thr Gln Asn Thr Gly Gly Ala Leu Val Thr Ser
305 310 315 320

Thr Ala Ala Thr Val Thr Gly Thr Asn Arg Leu Gly Ala Phe Ser Val
325 330 335

Val Glu Gly Lys Ala Asp Asn Val Val Leu Glu Asn Gly Gly Arg Leu
340 345 350

Asp Val Leu Ser Gly His Thr Ala Thr Asn Thr Arg Val Asp Asp Gly
355 360 365

Gly Thr Leu Asp Val Arg Asn Gly Gly Thr Ala Thr Thr Val Ser Met
370 375 380

Gly Asn Gly Gly Val Leu Leu Ala Asp Ser Gly Ala Ala Val Ser Gly
385 390 395 400

Thr Arg Ser Asp Gly Thr Ala Phe Arg Ile Gly Gly Gly Gln Ala Asp
405 410 415

Ala Leu Met Leu Glu Lys Gly Ser Ser Phe Thr Leu Asn Ala Gly Asp
420 425 430

Thr Ala Thr Asp Thr Thr Val Asn Gly Gly Leu Phe Thr Ala Arg Gly
435 440 445

Gly Ser Leu Ala Gly Thr Thr Thr Leu Asn Asn Gly Ala Ile Leu Thr
450 455 460

Leu Ser Gly Lys Thr Val Asn Asn Asp Thr Leu Thr Ile Arg Glu Gly
465 470 475 480

Asp Ala Leu Leu Gln Gly Gly Ser Leu Thr Gly Asn Gly Ser Val Glu

```
                485                 490                 495

Lys Ser Gly Ser Gly Thr Leu Thr Val Ser Asn Thr Thr Leu Thr Gln
            500                 505                 510

Lys Ala Val Asn Leu Asn Glu Gly Thr Leu Thr Leu Asn Asp Ser Thr
        515                 520                 525

Val Thr Thr Asp Val Ile Ala Gln Arg Gly Thr Ala Leu Lys Leu Thr
    530                 535                 540

Gly Ser Thr Val Leu Asn Gly Ala Ile Asp Pro Thr Asn Val Thr Leu
545                 550                 555                 560

Ala Ser Gly Ala Thr Trp Asn Ile Pro Asp Asn Ala Thr Val Gln Ser
                565                 570                 575

Val Val Asp Asp Leu Ser His Ala Gly Gln Ile His Phe Thr Ser Thr
            580                 585                 590

Arg Thr Gly Lys Phe Val Pro Ala Thr Leu Lys Val Lys Asn Leu Asn
        595                 600                 605

Gly Gln Asn Gly Thr Ile Ser Leu Arg Val Arg Pro Asp Met Ala Gln
    610                 615                 620

Asn Asn Ala Asp Arg Leu Val Ile Asp Gly Gly Arg Ala Thr Gly Lys
625                 630                 635                 640

Thr Ile Leu Asn Leu Val Asn Ala Gly Asn Ser Ala Ser Gly Leu Ala
                645                 650                 655

Thr Ser Gly Lys Gly Ile Gln Val Val Glu Ala Ile Asn Gly Ala Thr
            660                 665                 670

Thr Glu Glu Gly Ala Phe Val Gln Gly Asn Arg Leu Gln Ala Gly Ala
        675                 680                 685

Phe Asn Tyr Ser Leu Asn Arg Asp Ser Asp Glu Ser Trp Tyr Leu Arg
    690                 695                 700

Ser Glu Asn Ala Tyr Arg Ala Glu Val Pro Leu Tyr Ala Ser Met Leu
705                 710                 715                 720

Thr Gln Ala Met Asp Tyr Asp Arg Ile Leu Ala Gly Ser Arg Ser His
                725                 730                 735

Gln Thr Gly Val Asn Val Lys Asn Asn Ser Val Arg Leu Ser Ile Gln
            740                 745                 750

Gly Gly His Leu Gly His Asp Asn Asn Gly Gly Ile Ala Arg Gly Ala
        755                 760                 765

Thr Pro Glu Ser Ser Gly Ser Tyr Gly Phe Val Arg Leu Glu Gly Asp
    770                 775                 780

Leu Leu Arg Thr Glu Val Ala Gly Met Ser Val Thr Ala Gly Val Tyr
785                 790                 795                 800

Gly Ala Ala Gly His Ser Ser Val Asp Val Lys Asp Asp Asp Gly Ser
                805                 810                 815

Arg Ala Gly Thr Val Arg Asp Asp Ala Gly Ser Leu Gly Gly Tyr Leu
            820                 825                 830
```

Asn Leu Thr His Thr Ser Ser Gly Leu Trp Ala Asp Ile Val Ala Gln
835 840 845

Gly Thr Arg His Ser Met Lys Ala Ser Ser Asp Asn Asn Asp Phe Arg
850 855 860

Ala Arg Gly Trp Gly Trp Leu Gly Ser Leu Glu Thr Gly Leu Pro Phe
865 870 875 880

Ser Ile Thr Asp Asn Leu Met Leu Glu Pro Gln Leu Gln Tyr Thr Trp
885 890 895

Gln Gly Leu Ser Leu Asp Asp Gly Gln Asp Asn Ala Gly Tyr Val Lys
900 905 910

Phe Gly His Gly Ser Ala Gln His Val Arg Ala Gly Phe Arg Leu Gly
915 920 925

Ser His Ser Asp Met Thr Phe Gly Glu Gly Thr Ser Ser Arg Asp Thr
930 935 940

Leu Arg Asp Ser Ala Lys His Ser Val Arg Glu Leu Pro Val Asn Trp
945 950 955 960

Trp Val Gln Pro Ser Val Ile Arg Thr Phe Ser Ser Arg Gly Asp Met
965 970 975

Ser Met Gly Thr Ala Ala Ala Gly Ser Asn Met Thr Phe Ser Pro Ser
980 985 990

Gln Asn Gly Thr Ser Leu Asp Leu Gln Ala Gly Leu Glu Ala Arg Val
995 1000 1005

Arg Glu Asn Ile Thr Leu Gly Val Gln Ala Gly Tyr Ala His Ser Val
1010 1015 1020

Ser Gly Ser Ser Ala Glu Gly Tyr Asn Gly Gln Ala Thr Leu Asn Val
1025 1030 1035 1040

Thr Phe

<210> 130
<211> 263
<212> PRT
<213> Escherichia coli

<400> 130
Met Ser Ser Asn Phe Arg His Gln Leu Leu Ser Leu Ser Leu Leu Val
1 5 10 15

Gly Ile Ala Ala Pro Trp Ala Ala Phe Ala Gln Ala Pro Ile Ser Ser
20 25 30

Val Gly Ser Gly Ser Val Glu Asp Arg Val Thr Gln Leu Glu Arg Ile
35 40 45

Ser Asn Ala His Ser Gln Leu Leu Thr Gln Leu Gln Gln Gln Leu Ser
50 55 60

Asp Asn Gln Ser Asp Ile Asp Ser Leu Arg Gly Gln Ile Gln Glu Asn
65 70 75 80

Gln Tyr Gln Leu Asn Gln Val Val Glu Arg Gln Lys Gln Ile Leu Leu
85 90 95

Gln Ile Asp Ser Leu Ser Ser Gly Gly Ala Ala Ala Gln Ser Thr Ser
100 105 110

Gly Asp Gln Ser Gly Val Ala Ala Ser Thr Thr Pro Thr Ala Asp Ala
115 120 125

Gly Thr Ala Asn Ala Gly Ala Pro Val Lys Ser Gly Asp Ala Asn Thr
130 135 140

Asp Tyr Asn Ala Ala Ile Ala Leu Val Gln Asp Lys Ser Arg Gln Asp
145 150 155 160

Asp Ala Met Val Ala Phe Gln Asn Phe Ile Lys Asn Tyr Pro Asp Ser
165 170 175

Thr Tyr Leu Pro Asn Ala Asn Tyr Trp Leu Gly Gln Leu Asn Tyr Asn
180 185 190

Lys Gly Lys Lys Asp Asp Ala Ala Tyr Tyr Phe Ala Ser Val Val Lys
195 200 205

Asn Tyr Pro Lys Ser Pro Lys Ala Ala Asp Ala Met Phe Lys Val Gly
210 215 220

Val Ile Met Gln Asp Lys Gly Asp Thr Ala Lys Ala Lys Ala Val Tyr
225 230 235 240

Gln Gln Val Ile Ser Lys Tyr Pro Gly Thr Asp Gly Ala Lys Gln Ala
245 250 255

Gln Lys Arg Leu Asn Ala Met
260

<210> 131
<211> 362
<212> PRT
<213> Escherichia coli

<400> 131
Met Met Lys Arg Asn Ile Leu Ala Val Ile Val Pro Ala Leu Leu Val
1 5 10 15

Ala Gly Thr Ala Asn Ala Ala Glu Ile Tyr Asn Lys Asp Gly Asn Lys
20 25 30

Val Asp Leu Tyr Gly Lys Ala Val Gly Leu His Tyr Phe Ser Lys Gly
35 40 45

Asn Gly Glu Asn Ser Tyr Gly Gly Asn Gly Asp Met Thr Tyr Ala Arg
50 55 60

Leu Gly Phe Lys Gly Glu Thr Gln Ile Asn Ser Asp Leu Thr Gly Tyr
65 70 75 80

Gly Gln Trp Glu Tyr Asn Phe Gln Gly Asn Asn Ser Glu Gly Ala Asp
85 90 95

Ala Gln Thr Gly Asn Lys Thr Arg Leu Ala Phe Ala Gly Leu Lys Tyr
100 105 110

Ala Asp Val Gly Ser Phe Asp Tyr Gly Arg Asn Tyr Gly Val Val Tyr
115 120 125

Asp Ala Leu Gly Tyr Thr Asp Met Leu Pro Glu Phe Gly Gly Asp Thr

130 135 140

Ala Tyr Ser Asp Asp Phe Phe Val Gly Arg Val Gly Gly Val Ala Thr
145 150 155 160

Tyr Arg Asn Ser Asn Phe Phe Gly Leu Val Asp Gly Leu Asn Phe Ala
165 170 175

Val Gln Tyr Leu Gly Lys Asn Glu Arg Asp Thr Ala Arg Arg Ser Asn
180 185 190

Gly Asp Gly Val Gly Gly Ser Ile Ser Tyr Glu Tyr Glu Gly Phe Gly
195 200 205

Ile Val Gly Ala Tyr Gly Ala Ala Asp Arg Thr Asn Leu Gln Glu Glu
210 215 220

Ser Ser Leu Gly Lys Gly Lys Lys Ala Glu Gln Trp Ala Thr Gly Leu
225 230 235 240

Lys Tyr Asp Ala Asn Asn Ile Tyr Leu Ala Ala Asn Tyr Gly Glu Thr
245 250 255

Arg Asn Ala Thr Pro Ile Thr Asn Lys Phe Thr Asn Thr Ser Gly Phe
260 265 270

Ala Asn Lys Thr Gln Asp Val Leu Leu Val Ala Gln Tyr Gln Phe Asp
275 280 285

Phe Gly Leu Arg Pro Ser Ile Ala Tyr Thr Lys Ser Lys Ala Lys Asp
290 295 300

Val Glu Gly Ile Gly Asp Val Asp Leu Val Asn Tyr Phe Glu Val Gly
305 310 315 320

Ala Thr Tyr Tyr Phe Asn Lys Asn Met Ser Thr Tyr Val Asp Tyr Ile
325 330 335

Ile Asn Gln Ile Asp Ser Asp Asn Lys Leu Gly Val Gly Ser Asp Asp
340 345 350

Thr Val Ala Val Gly Ile Val Tyr Gln Phe
355 360

<210> 132
<211> 891
<212> PRT
<213> Escherichia coli

<400> 132
Met Ala Val Thr Asn Val Ala Glu Leu Asn Ala Leu Val Glu Arg Val
1 5 10 15

Lys Lys Ala Gln Arg Glu Tyr Ala Ser Phe Thr Gln Glu Gln Val Asp
20 25 30

Lys Ile Phe Arg Ala Ala Ala Leu Ala Ala Ala Asp Ala Arg Ile Pro
35 40 45

Leu Ala Lys Met Ala Val Ala Glu Ser Gly Met Gly Ile Val Glu Asp
50 55 60

Lys Val Ile Lys Asn His Phe Ala Ser Glu Tyr Ile Tyr Asn Ala Tyr
65 70 75 80

Lys Asp Glu Lys Thr Cys Gly Val Leu Ser Glu Asp Asp Thr Phe Gly
85 90 95

Thr Ile Thr Ile Ala Glu Pro Ile Gly Ile Ile Cys Gly Ile Val Pro
100 105 110

Thr Thr Asn Pro Thr Ser Thr Ala Ile Phe Lys Ser Leu Ile Ser Leu
115 120 125

Lys Thr Arg Asn Ala Ile Ile Phe Ser Pro His Pro Arg Ala Lys Asp
130 135 140

Ala Thr Asn Lys Ala Ala Asp Ile Val Leu Gln Ala Ala Ile Ala Ala
145 150 155 160

Gly Ala Pro Lys Asp Leu Ile Gly Trp Ile Asp Gln Pro Ser Val Glu
165 170 175

Leu Ser Asn Ala Leu Met His His Pro Asp Ile Asn Leu Ile Leu Ala
180 185 190

Thr Gly Gly Pro Gly Met Val Lys Ala Ala Tyr Ser Ser Gly Lys Pro
195 200 205

Ala Ile Gly Val Gly Ala Gly Asn Thr Pro Val Val Ile Asp Glu Thr
210 215 220

Ala Asp Ile Lys Arg Ala Val Ala Ser Val Leu Met Ser Lys Thr Phe
225 230 235 240

Asp Asn Gly Val Ile Cys Ala Ser Glu Gln Ser Val Val Val Val Asp
245 250 255

Ser Val Tyr Asp Ala Val Arg Glu Arg Phe Ala Thr His Gly Gly Tyr
260 265 270

Leu Leu Gln Gly Lys Glu Leu Lys Ala Val Gln Asp Val Ile Leu Lys
275 280 285

Asn Gly Ala Leu Asn Ala Ala Ile Val Gly Gln Pro Ala Tyr Lys Ile
290 295 300

Ala Glu Leu Ala Gly Phe Ser Val Pro Glu Asn Thr Lys Ile Leu Ile
305 310 315 320

Gly Glu Val Thr Val Val Asp Glu Ser Glu Pro Phe Ala His Glu Lys
325 330 335

Leu Ser Pro Thr Leu Ala Met Tyr Arg Ala Lys Asp Phe Glu Asp Ala
340 345 350

Val Glu Lys Ala Glu Lys Leu Val Ala Met Gly Gly Ile Gly His Thr
355 360 365

Ser Cys Leu Tyr Thr Asp Gln Asp Asn Gln Pro Ala Arg Val Ser Tyr
370 375 380

Phe Gly Gln Lys Met Lys Thr Ala Arg Ile Leu Ile Asn Thr Pro Ala
385 390 395 400

Ser Gln Gly Gly Ile Gly Asp Leu Tyr Asn Phe Lys Leu Ala Pro Ser
405 410 415

Leu Thr Leu Gly Cys Gly Ser Trp Gly Gly Asn Ser Ile Ser Glu Asn
420 425 430

Val Gly Pro Lys His Leu Ile Asn Lys Lys Thr Val Ala Lys Arg Ala
435 440 445

Glu Asn Met Leu Trp His Lys Leu Pro Lys Ser Ile Tyr Phe Arg Arg
450 455 460

Gly Ser Leu Pro Ile Ala Leu Asp Glu Val Ile Thr Asp Gly His Lys
465 470 475 480

Arg Ala Leu Ile Val Thr Asp Arg Phe Leu Phe Asn Asn Gly Tyr Ala
485 490 495

Asp Gln Ile Thr Ser Val Leu Lys Ala Ala Gly Val Glu Thr Glu Val
500 505 510

Phe Phe Glu Val Glu Ala Asp Pro Thr Leu Ser Ile Val Arg Lys Gly
515 520 525

Ala Glu Leu Ala Asn Ser Phe Lys Pro Asp Val Ile Ile Ala Leu Gly
530 535 540

Gly Gly Ser Pro Met Asp Ala Ala Lys Ile Met Trp Val Met Tyr Glu
545 550 555 560

His Pro Glu Thr His Phe Glu Glu Leu Ala Leu Arg Phe Met Asp Ile
565 570 575

Arg Lys Arg Ile Tyr Lys Phe Pro Lys Met Gly Val Lys Ala Lys Met
580 585 590

Ile Ala Val Thr Thr Thr Ser Gly Thr Gly Ser Glu Val Thr Pro Phe
595 600 605

Ala Val Val Thr Asp Asp Thr Thr Gly Gln Lys Tyr Pro Leu Ala Asp
610 615 620

Tyr Ala Leu Thr Pro Asp Met Ala Ile Val Asp Ala Asn Leu Val Met
625 630 635 640

Asp Met Pro Lys Ser Leu Cys Ala Phe Gly Gly Leu Asp Ala Val Thr
645 650 655

His Ala Met Glu Ala Tyr Val Ser Val Leu Ala Ser Glu Phe Ser Asp
660 665 670

Gly Gln Ala Leu Gln Ala Leu Lys Leu Leu Lys Glu Tyr Leu Pro Ala
675 680 685

Ser Tyr His Glu Gly Ser Lys Asn Pro Val Ala Arg Glu Arg Val His
690 695 700

Ser Ala Ala Thr Ile Ala Gly Ile Ala Phe Ala Asn Ala Phe Leu Gly
705 710 715 720

Val Cys His Ser Met Ala His Lys Leu Gly Ser Gln Phe His Ile Pro
725 730 735

His Gly Leu Ala Asn Ala Leu Leu Ile Cys Asn Val Ile Arg Tyr Asn
740 745 750

Ala Asn Asp Asn Pro Thr Lys Gln Thr Ala Phe Ser Gln Tyr Asp Arg
755 760 765

Pro Gln Ala Arg Arg Arg Tyr Ala Glu Ile Ala Asp His Leu Gly Leu

770 775 780

Ser Ala Pro Gly Asp Arg Thr Ala Ala Lys Ile Glu Lys Leu Leu Ala
785 790 795 800

Trp Leu Glu Thr Leu Lys Ala Glu Leu Gly Ile Pro Lys Ser Ile Arg
805 810 815

Glu Ala Gly Val Gln Glu Ala Asp Phe Leu Ala Asn Val Asp Lys Leu
820 825 830

Ser Glu Asp Ala Phe Asp Asp Gln Cys Thr Gly Ala Asn Pro Arg Tyr
835 840 845

Pro Leu Ile Ser Glu Leu Lys Gln Ile Leu Leu Asp Thr Tyr Tyr Gly
850 855 860

Arg Asp Tyr Val Glu Gly Glu Thr Ala Ala Lys Lys Glu Ala Ala Pro
865 870 875 880

Ala Lys Ala Glu Lys Lys Ala Lys Lys Ser Ala
885 890

<210> 133
<211> 539
<212> PRT
<213> Escherichia coli

<400> 133
Met Ala Ala Val Cys Gly Thr Ser Gly Ile Ala Ser Leu Phe Ser Gln
1 5 10 15

Ala Ala Phe Ala Ala Asp Ser Asp Ile Ala Asp Gly Gln Thr Gln Arg
20 25 30

Phe Asp Phe Ser Ile Leu Gln Ser Met Ala His Asp Leu Ala Gln Thr
35 40 45

Ala Trp Arg Gly Ala Pro Arg Pro Leu Pro Asp Thr Leu Ala Thr Met
50 55 60

Thr Pro Gln Ala Tyr Asn Ser Ile Gln Tyr Asp Ala Glu Lys Ser Leu
65 70 75 80

Trp His Asn Val Glu Asn Arg Gln Leu Asp Ala Gln Phe Phe His Met
85 90 95

Gly Met Gly Phe Arg Arg Arg Val Arg Met Phe Ser Val Asp Pro Ala
100 105 110

Thr His Leu Ala Arg Glu Ile His Phe Arg Pro Glu Leu Phe Lys Tyr
115 120 125

Asn Asp Ala Gly Val Asp Thr Lys Gln Leu Glu Gly Gln Ser Asp Leu
130 135 140

Gly Phe Ala Gly Phe Arg Val Phe Lys Ala Pro Glu Leu Ala Arg Arg
145 150 155 160

Asp Val Val Ser Phe Leu Gly Ala Ser Tyr Phe Arg Ala Val Asp Asp
165 170 175

Thr Tyr Gln Tyr Gly Leu Ser Ala Arg Gly Leu Ala Ile Asp Thr Tyr
180 185 190

```
Thr Asp Ser Lys Glu Glu Phe Pro Asp Phe Thr Ala Phe Trp Phe Asp
        195                 200                 205

Thr Val Lys Pro Gly Ala Thr Thr Phe Thr Val Tyr Ala Leu Leu Asp
    210                 215                 220

Ser Ala Ser Ile Thr Gly Ala Tyr Lys Phe Thr Ile His Cys Glu Lys
225                 230                 235                 240

Asn Gln Val Ile Met Asp Val Glu Asn His Leu Tyr Ala Arg Lys Asp
                245                 250                 255

Ile Lys Gln Leu Gly Ile Ala Pro Met Thr Ser Met Phe Ser Cys Gly
            260                 265                 270

Thr Asn Glu Arg Arg Met Cys Asp Thr Ile His Pro Gln Ile His Asp
        275                 280                 285

Ser Asp Arg Leu Ser Met Trp Arg Gly Asn Gly Glu Trp Ile Cys Arg
    290                 295                 300

Pro Leu Asn Asn Pro Gln Lys Leu Gln Phe Asn Ala Tyr Thr Asp Asn
305                 310                 315                 320

Asn Pro Lys Gly Phe Gly Leu Leu Gln Leu Asp Arg Asp Phe Ser His
                325                 330                 335

Tyr Gln Asp Ile Met Gly Trp Tyr Asn Lys Arg Pro Ser Leu Trp Val
            340                 345                 350

Glu Pro Arg Asn Lys Trp Gly Lys Gly Thr Ile Gly Leu Met Glu Ile
        355                 360                 365

Pro Thr Thr Gly Glu Thr Leu Asp Asn Ile Val Cys Phe Trp Gln Pro
    370                 375                 380

Glu Lys Ala Val Lys Ala Gly Asp Glu Phe Ala Phe Gln Tyr Arg Leu
385                 390                 395                 400

Tyr Trp Ser Ala Gln Pro Pro Val His Cys Pro Leu Ala Arg Val Met
                405                 410                 415

Ala Thr Arg Thr Gly Met Gly Gly Phe Pro Glu Gly Trp Ala Pro Gly
            420                 425                 430

Glu His Tyr Pro Glu Lys Trp Ala Arg Arg Phe Ala Val Asp Phe Val
        435                 440                 445

Gly Gly Asp Leu Lys Ala Ala Ala Pro Lys Gly Ile Glu Pro Val Ile
    450                 455                 460

Thr Leu Ser Ser Gly Glu Ala Lys Gln Ile Glu Ile Leu Tyr Ile Glu
465                 470                 475                 480

Pro Ile Asp Gly Tyr Arg Ile Gln Phe Asp Trp Tyr Pro Thr Ser Asp
                485                 490                 495

Ser Thr Asp Pro Val Asp Met Arg Met Tyr Leu Arg Cys Gln Gly Asp
            500                 505                 510

Ala Ile Ser Glu Thr Trp Leu Tyr Gln Tyr Phe Pro Pro Ala Pro Asp
        515                 520                 525

Lys Arg Gln Tyr Val Asp Asp Arg Val Met Ser
    530                 535
```

<210> 134
<211> 167
<212> PRT
<213> Escherichia coli

<400> 134
Met Ala Thr Ala Gly Met Leu Leu Lys Leu Asn Ser Gln Met Asn Arg
1 5 10 15

Glu Phe Tyr Ala Ser Asn Leu Tyr Leu His Leu Ser Asn Trp Cys Ser
20 25 30

Glu Gln Ser Leu Asn Gly Thr Ala Thr Phe Leu Arg Ala Gln Ala Gln
35 40 45

Ser Asn Val Thr Gln Met Met Arg Met Phe Asn Phe Met Lys Ser Val
50 55 60

Gly Ala Thr Pro Ile Val Lys Ala Ile Asp Val Pro Gly Glu Lys Leu
65 70 75 80

Asn Ser Leu Glu Glu Leu Phe Gln Lys Thr Met Glu Glu Tyr Glu Gln
85 90 95

Arg Ser Ser Thr Leu Ala Gln Leu Ala Asp Glu Ala Lys Glu Leu Asn
100 105 110

Asp Asp Ser Thr Val Asn Phe Leu Arg Asp Leu Glu Lys Glu Gln Gln
115 120 125

His Asp Gly Leu Leu Leu Gln Thr Ile Leu Asp Glu Val Arg Ser Ala
130 135 140

Lys Leu Ala Gly Met Cys Pro Val Gln Thr Asp Gln His Val Leu Asn
145 150 155 160

Val Val Ser His Gln Leu His
165

<210> 135
<211> 487
<212> PRT
<213> Escherichia coli

<400> 135
Met Phe Arg Gln Leu Lys Lys Asn Leu Val Ala Thr Leu Ile Ala Ala
1 5 10 15

Met Thr Ile Gly Gln Val Ala Pro Ala Phe Ala Asp Ser Ala Asp Thr
20 25 30

Leu Pro Asp Met Gly Thr Ser Ala Gly Ser Thr Leu Ser Ile Gly Gln
35 40 45

Glu Met Gln Met Gly Asp Tyr Tyr Val Arg Gln Leu Arg Gly Ser Ala
50 55 60

Pro Leu Ile Asn Asp Pro Leu Leu Thr Gln Tyr Ile Asn Ser Leu Gly
65 70 75 80

Met Arg Leu Val Ser His Ala Asn Ser Val Lys Thr Pro Phe His Phe
85 90 95

Phe Leu Ile Asn Asn Asp Glu Ile Asn Ala Phe Ala Phe Phe Gly Gly

100 105 110

Asn Val Val Leu His Ser Ala Leu Phe Arg Tyr Ser Asp Asn Glu Ser
115 120 125

Gln Leu Ala Ser Val Met Ala His Glu Ile Ser His Val Thr Gln Arg
130 135 140

His Leu Ala Arg Ala Met Glu Asp Gln Gln Arg Ser Ala Pro Leu Thr
145 150 155 160

Trp Val Gly Ala Leu Gly Ser Ile Leu Leu Ala Met Ala Ser Pro Gln
165 170 175

Ala Gly Met Ala Ala Leu Thr Gly Thr Leu Ala Gly Thr Arg Gln Gly
180 185 190

Met Ile Ser Phe Thr Gln Gln Asn Glu Gln Glu Ala Asp Arg Ile Gly
195 200 205

Ile Gln Val Leu Gln Arg Ser Gly Phe Asp Pro Gln Ala Met Pro Thr
210 215 220

Phe Leu Glu Lys Leu Leu Asp Gln Ala Arg Tyr Ser Ser Arg Pro Pro
225 230 235 240

Glu Ile Leu Leu Thr His Pro Leu Pro Glu Ser Arg Leu Ala Asp Ala
245 250 255

Arg Asn Arg Ala Asn Gln Met Arg Pro Ile Val Val Gln Ser Ser Glu
260 265 270

Asp Phe Tyr Leu Ala Lys Val Arg Thr Leu Gly Met Tyr Asn Ser Gly
275 280 285

Arg Asn Gln Leu Thr Ser Asp Leu Leu Asp Glu Trp Ala Lys Gly Asn
290 295 300

Val Arg Gln Gln Arg Ala Ala Gln Tyr Gly Arg Ala Leu Gln Ala Met
305 310 315 320

Glu Ala Asn Lys Tyr Asp Glu Ala Arg Lys Thr Leu Gln Pro Leu Leu
325 330 335

Ala Ala Glu Pro Gly Asn Ala Trp Tyr Leu Asp Leu Ala Thr Asp Ile
340 345 350

Asp Leu Gly Gln Asn Lys Ala Asn Asp Ala Ile Asn Arg Leu Lys Asn
355 360 365

Ala Arg Asp Leu Arg Thr Asp Pro Val Leu Gln Leu Asn Leu Ala Asn
370 375 380

Ala Tyr Leu Gln Gly Gly Gln Pro Gln Glu Ala Ala Asn Ile Leu Asn
385 390 395 400

Arg Tyr Thr Phe Asn Asn Lys Asp Asp Ser Asn Gly Trp Asp Leu Leu
405 410 415

Ala Gln Ala Glu Ala Ala Leu Asn Asn Arg Asp Gln Glu Leu Ala Ala
420 425 430

Arg Ala Glu Gly Tyr Ala Leu Ala Gly Arg Leu Asp Gln Ala Ile Ser
435 440 445

Leu Leu Ser Ser Ala Ser Ser Gln Val Lys Leu Gly Ser Leu Gln Gln
    450                 455                 460

Ala Arg Tyr Asp Ala Arg Ile Asp Gln Leu Arg Gln Leu Gln Glu Arg
465                 470                 475                 480

Phe Lys Pro Tyr Thr Lys Met
                485

<210> 136
<211> 524
<212> PRT
<213> Escherichia coli

<400> 136
Met Phe Ser Thr Leu Arg Arg Thr Leu Phe Ala Leu Leu Ala Cys Ala
1               5                   10                  15

Ser Phe Ile Val His Ala Ala Ala Pro Asp Glu Ile Thr Thr Ala Trp
            20                  25                  30

Pro Val Asn Val Gly Pro Leu Asn Pro His Leu Tyr Thr Pro Asn Gln
        35                  40                  45

Met Phe Ala Gln Ser Met Val Tyr Glu Pro Leu Val Lys Tyr Gln Ala
    50                  55                  60

Asp Gly Ser Val Ile Pro Trp Leu Ala Lys Ser Trp Thr His Ser Glu
65                  70                  75                  80

Asp Gly Lys Thr Trp Thr Phe Thr Leu Arg Asp Asp Val Lys Phe Ser
                85                  90                  95

Asn Gly Glu Pro Phe Asp Ala Glu Ala Ala Ala Glu Asn Phe Arg Ala
            100                 105                 110

Val Leu Asp Asn Arg Gln Arg His Ala Trp Leu Glu Leu Ala Asn Gln
        115                 120                 125

Ile Val Asp Val Lys Ala Leu Ser Lys Asn Glu Leu Gln Ile Thr Leu
    130                 135                 140

Lys Ser Ala Tyr Tyr Pro Phe Leu Gln Glu Leu Ala Leu Pro Arg Pro
145                 150                 155                 160

Phe Arg Phe Ile Ala Pro Ser Gln Phe Lys Asn His Glu Thr Met Asn
                165                 170                 175

Gly Ile Lys Ala Pro Ile Gly Thr Gly Pro Trp Val Leu Gln Glu Ser
            180                 185                 190

Lys Leu Asn Gln Tyr Asp Val Phe Val Arg Asn Glu Asn Tyr Trp Gly
        195                 200                 205

Glu Lys Pro Ala Ile Lys Lys Ile Thr Phe Asn Val Ile Pro Asp Pro
    210                 215                 220

Thr Thr Arg Ala Val Ala Phe Glu Thr Gly Asp Ile Asp Leu Leu Tyr
225                 230                 235                 240

Gly Asn Glu Gly Leu Leu Pro Leu Asp Thr Phe Ala Arg Phe Ser Gln
                245                 250                 255

Asn Pro Ala Tyr His Thr Gln Leu Ser Gln Pro Ile Glu Thr Val Met
            260                 265                 270

Leu Ala Leu Asn Thr Ala Lys Ala Pro Thr Asn Glu Leu Ala Val Arg
275 280 285

Glu Ala Leu Asn Tyr Ala Val Asn Lys Lys Ser Leu Ile Asp Asn Ala
290 295 300

Leu Tyr Gly Thr Gln Gln Val Ala Asp Thr Leu Phe Ala Pro Ser Val
305 310 315 320

Pro Tyr Ala Asn Leu Gly Leu Lys Pro Arg Gln Tyr Asp Pro Gln Lys
325 330 335

Ala Lys Ala Leu Leu Glu Lys Ala Gly Trp Thr Leu Pro Ala Gly Lys
340 345 350

Asp Ile Arg Glu Lys Asn Gly Gln Pro Leu Arg Ile Glu Leu Ser Phe
355 360 365

Ile Gly Thr Asp Ala Leu Ser Lys Ser Met Ala Glu Ile Ile Gln Ala
370 375 380

Asp Met Arg Gln Ile Gly Ala Asp Val Ser Leu Ile Gly Glu Glu Glu
385 390 395 400

Ser Ser Ile Tyr Ala Arg Gln Arg Asp Gly Arg Phe Gly Met Ile Phe
405 410 415

His Arg Thr Trp Gly Ala Pro Tyr Asp Pro His Ala Phe Leu Ser Ser
420 425 430

Met Arg Val Pro Ser His Ala Asp Phe Gln Ala Gln Gln Gly Leu Ala
435 440 445

Asp Lys Pro Leu Ile Asp Lys Glu Ile Gly Glu Val Leu Ala Thr His
450 455 460

Asp Glu Thr Gln Arg Gln Ala Leu Tyr Arg Asp Ile Leu Thr Arg Leu
465 470 475 480

His Asp Glu Ala Val Tyr Leu Pro Ile Ser Tyr Ile Ser Met Met Val
485 490 495

Val Ser Lys Pro Glu Leu Gly Asn Ile Pro Tyr Ala Pro Ile Ala Thr
500 505 510

Glu Ile Pro Phe Glu Gln Ile Lys Pro Val Lys Pro
515 520

<210> 137
<211> 471
<212> PRT
<213> Escherichia coli

<400> 137
Met Glu Asn Phe Lys His Leu Pro Glu Pro Phe Arg Ile Arg Val Ile
1 5 10 15

Glu Pro Val Lys Arg Thr Thr Arg Ala Tyr Arg Glu Glu Ala Ile Ile
20 25 30

Lys Ser Gly Met Asn Pro Phe Leu Leu Asp Ser Glu Asp Val Phe Ile
35 40 45

Asp Leu Leu Thr Asp Ser Gly Thr Gly Ala Val Thr Gln Ser Met Gln

50 55 60

Ala Ala Met Met Arg Gly Asp Glu Ala Tyr Ser Gly Ser Arg Ser Tyr
65 70 75 80

Tyr Ala Leu Ala Glu Ser Val Lys Asn Ile Phe Gly Tyr Gln Tyr Thr
85 90 95

Ile Pro Thr His Gln Gly Arg Gly Ala Glu Gln Ile Tyr Ile Pro Val
100 105 110

Leu Ile Lys Lys Arg Glu Gln Glu Lys Gly Leu Asp Arg Ser Lys Met
115 120 125

Val Ala Phe Ser Asn Tyr Phe Phe Asp Thr Thr Gln Gly His Ser Gln
130 135 140

Ile Asn Gly Cys Thr Val Arg Asn Val Tyr Ile Lys Glu Ala Phe Asp
145 150 155 160

Thr Gly Val Arg Tyr Asp Phe Lys Gly Asn Phe Asp Leu Glu Gly Leu
165 170 175

Glu Arg Gly Ile Glu Glu Val Gly Pro Asn Asn Val Pro Tyr Ile Val
180 185 190

Ala Thr Ile Thr Ser Asn Ser Ala Gly Gly Gln Pro Val Ser Leu Ala
195 200 205

Asn Leu Lys Ala Met Tyr Ser Ile Ala Lys Lys Tyr Asp Ile Pro Val
210 215 220

Val Met Asp Ser Ala Arg Phe Ala Glu Asn Ala Tyr Phe Ile Lys Gln
225 230 235 240

Arg Glu Ala Glu Tyr Lys Asp Trp Thr Ile Glu Gln Ile Thr Arg Glu
245 250 255

Thr Tyr Lys Tyr Ala Asp Met Leu Ala Met Ser Ala Lys Lys Asp Ala
260 265 270

Met Val Pro Met Gly Gly Leu Leu Cys Met Lys Asp Asp Ser Phe Phe
275 280 285

Asp Val Tyr Thr Glu Cys Arg Thr Leu Cys Val Val Gln Glu Gly Phe
290 295 300

Pro Thr Tyr Gly Gly Leu Glu Gly Gly Ala Met Glu Arg Leu Ala Val
305 310 315 320

Gly Leu Tyr Asp Gly Met Asn Leu Asp Trp Leu Ala Tyr Arg Ile Ala
325 330 335

Gln Val Gln Tyr Leu Val Asp Gly Leu Glu Glu Ile Gly Val Val Cys
340 345 350

Gln Gln Ala Gly Gly His Ala Ala Phe Val Asp Ala Gly Lys Leu Leu
355 360 365

Pro His Ile Pro Ala Asp Gln Phe Pro Ala Gln Ala Leu Ala Cys Glu
370 375 380

Leu Tyr Lys Val Ala Gly Ile Arg Ala Val Glu Ile Gly Ser Phe Leu
385 390 395 400

Leu Gly Arg Asp Pro Lys Thr Gly Lys Gln Leu Pro Cys Pro Ala Glu
405 410 415

Leu Leu Arg Leu Thr Ile Pro Arg Ala Thr Tyr Thr Gln Thr His Met
420 425 430

Asp Phe Ile Ile Glu Ala Phe Lys His Val Lys Glu Asn Ala Ala Asn
435 440 445

Ile Lys Gly Leu Thr Phe Thr Tyr Glu Pro Lys Val Leu Arg His Phe
450 455 460

Thr Ala Lys Leu Lys Glu Val
465 470

<210> 138
<211> 628
<212> PRT
<213> Escherichia coli

<400> 138
Met Lys Pro Ala Ala Ser Phe Phe Tyr Cys Gly Cys Phe Thr Met Ile
1 5 10 15

Lys Lys Ala Ser Leu Leu Thr Ala Cys Ser Val Thr Ala Phe Ser Ala
20 25 30

Trp Ala Gln Asp Thr Ser Pro Asp Thr Leu Val Val Thr Ala Asn Arg
35 40 45

Phe Glu Gln Pro Arg Ser Thr Val Leu Ala Pro Thr Thr Val Val Thr
50 55 60

Arg Gln Asp Ile Asp Arg Trp Gln Ser Thr Ser Val Asn Asp Val Leu
65 70 75 80

Arg Arg Leu Pro Gly Val Asp Ile Thr Gln Asn Gly Gly Ser Gly Gln
85 90 95

Leu Ser Ser Ile Phe Ile Arg Gly Thr Asn Ala Ser His Val Leu Val
100 105 110

Leu Ile Asp Gly Val Arg Leu Asn Leu Ala Gly Val Ser Gly Ser Ala
115 120 125

Asp Leu Ser Gln Phe Pro Ile Ala Leu Val Gln Arg Val Glu Tyr Ile
130 135 140

Arg Gly Pro Arg Ser Ala Val Tyr Gly Ser Asp Ala Ile Gly Gly Val
145 150 155 160

Val Asn Ile Ile Thr Thr Arg Asp His Pro Gly Thr Glu Ile Ser Ala
165 170 175

Gly Trp Gly Ser Asn Ser Tyr Gln Asn Tyr Asp Val Ser Thr Gln Gln
180 185 190

Gln Leu Gly Asp Lys Thr Arg Val Thr Leu Leu Gly Asp Tyr Ala His
195 200 205

Thr His Gly Tyr Asp Val Val Ala Tyr Gly Asn Thr Gly Thr Gln Ala
210 215 220

Gln Pro Asp Asn Asp Gly Phe Leu Ser Lys Thr Leu Tyr Gly Ala Leu
225 230 235 240

```
Glu His Asn Phe Thr Asp Val Trp Ser Gly Phe Val Arg Gly Tyr Gly
                245                 250                 255

Tyr Asp Asn Arg Thr Asn Tyr Asp Ala Tyr Tyr Ser Pro Gly Leu Pro
            260                 265                 270

Leu Val Asp Thr Arg Lys Leu Tyr Ser Gln Ser Trp Asp Ala Gly Leu
        275                 280                 285

Arg Tyr Asn Gly Glu Leu Ile Lys Ser Gln Leu Ile Thr Ser Tyr Ser
    290                 295                 300

His Ser Lys Asp Tyr Asn Tyr Asp Pro His Tyr Gly Arg Tyr Asp Ser
305                 310                 315                 320

Ser Ala Thr Leu Asp Glu Met Lys Gln Tyr Thr Val Gln Trp Ala Asn
                325                 330                 335

Asn Ile Ile Ile Gly His Gly Asn Ile Gly Ala Gly Val Asp Trp Gln
            340                 345                 350

Lys Gln Ser Thr Ala Pro Gly Thr Ala Tyr Val Glu Asp Gly Tyr Asp
        355                 360                 365

Gln Arg Asn Thr Gly Ile Tyr Leu Thr Gly Leu Gln Gln Val Gly Asp
    370                 375                 380

Phe Thr Phe Glu Gly Ala Gly Arg Ser Asp Asp Asn Ser Gln Phe Gly
385                 390                 395                 400

Arg His Gly Thr Trp Gln Thr Ser Ala Gly Trp Glu Phe Ile Glu Gly
                405                 410                 415

Tyr Arg Phe Ile Ala Ser Tyr Gly Thr Ser Tyr Lys Ala Pro Asn Leu
            420                 425                 430

Gly Gln Leu Tyr Gly Ser Tyr Gly Asn Pro Asn Leu Asn Pro Glu Lys
        435                 440                 445

Ser Lys Gln Trp Glu Gly Ala Phe Glu Gly Leu Thr Ala Gly Val Asn
    450                 455                 460

Trp Arg Ile Ser Gly Tyr Arg Asn Asp Val Ser Asp Leu Ile Asp Tyr
465                 470                 475                 480

Asp Asp His Thr Leu Lys Tyr Tyr Asn Glu Gly Lys Ala Arg Ile Lys
                485                 490                 495

Gly Val Glu Ala Thr Ala Asn Phe Asp Thr Gly Pro Leu Thr His Thr
            500                 505                 510

Val Ser Tyr Asp Tyr Val Asp Ala Arg Asn Ala Ile Thr Asp Thr Pro
        515                 520                 525

Leu Leu Arg Arg Ala Lys Gln Gln Val Lys Tyr Gln Leu Asp Trp Gln
    530                 535                 540

Leu Tyr Asp Phe Asp Trp Gly Ile Thr Tyr Gln Tyr Leu Gly Thr Arg
545                 550                 555                 560

Tyr Asp Lys Asp Tyr Ser Ser Tyr Pro Tyr Gln Thr Val Lys Met Gly
                565                 570                 575

Gly Val Ser Leu Trp Asp Leu Ala Val Ala Tyr Pro Val Thr Ser His
```

580 585 590

Leu Thr Val Arg Gly Lys Ile Ala Asn Leu Phe Asp Lys Asp Tyr Glu
595 600 605

Thr Val Tyr Gly Tyr Gln Thr Ala Gly Arg Glu Tyr Thr Leu Ser Gly
610 615 620

Ser Tyr Thr Phe
625

<210> 139
<211> 878
<212> PRT
<213> Escherichia coli

<400> 139
Met Ser Tyr Leu Asn Leu Arg Leu Tyr Gln Arg Asn Thr Gln Cys Leu
1 5 10 15

His Ile Arg Lys His Arg Leu Ala Gly Phe Phe Val Arg Leu Phe Val
20 25 30

Ala Cys Ala Phe Ala Ala Gln Ala Pro Leu Ser Ser Ala Glu Leu Tyr
35 40 45

Phe Asn Pro Arg Phe Leu Ala Asp Asp Pro Gln Ala Val Ala Asp Leu
50 55 60

Ser Arg Phe Glu Asn Gly Gln Glu Leu Pro Pro Gly Thr Tyr Arg Val
65 70 75 80

Asp Ile Tyr Leu Asn Asn Gly Tyr Met Ala Thr Arg Asp Val Thr Phe
85 90 95

Asn Thr Gly Asp Ser Glu Gln Gly Ile Val Pro Cys Leu Thr Arg Ala
100 105 110

Gln Leu Ala Ser Met Gly Leu Asn Thr Ala Ser Val Ser Gly Met Asn
115 120 125

Leu Leu Ala Asp Asp Ala Cys Val Pro Leu Thr Ser Met Ile His Asp
130 135 140

Ala Thr Ala Gln Leu Asp Val Gly Gln Gln Arg Leu Asn Leu Thr Ile
145 150 155 160

Pro Gln Ala Phe Met Ser Asn Arg Ala Arg Gly Tyr Ile Pro Pro Glu
165 170 175

Leu Trp Asp Pro Gly Ile Asn Ala Gly Leu Leu Asn Tyr Asn Phe Ser
180 185 190

Gly Asn Ser Val Gln Asn Arg Ile Gly Gly Asn Ser His Tyr Ala Tyr
195 200 205

Leu Asn Leu Gln Ser Gly Leu Asn Ile Gly Ala Trp Arg Leu Arg Asp
210 215 220

Asn Thr Ser Trp Ser Tyr Asn Ser Ser Asp Ser Ser Ser Gly Ser Lys
225 230 235 240

Asn Lys Trp Gln His Ile Asn Thr Trp Leu Glu Arg Asp Ile Ile Pro
245 250 255

Leu Arg Ser Arg Leu Thr Leu Gly Asp Gly Tyr Thr Gln Gly Asp Ile
260 265 270

Phe Asp Gly Ile Asn Phe Arg Gly Ala Gln Leu Ala Ser Asp Asp Asn
275 280 285

Met Leu Pro Asp Ser Gln Arg Gly Phe Ala Pro Val Ile His Gly Ile
290 295 300

Ala Arg Gly Thr Ala Gln Val Thr Ile Lys Gln Asn Gly Tyr Asp Ile
305 310 315 320

Tyr Asn Ser Thr Val Pro Pro Gly Pro Phe Thr Ile Asn Asp Ile Tyr
325 330 335

Ala Ala Gly Asn Ser Gly Asp Leu Gln Val Thr Ile Lys Glu Ala Asp
340 345 350

Gly Ser Thr Gln Ile Phe Thr Val Pro Tyr Ser Ser Val Pro Leu Leu
355 360 365

Gln Arg Glu Gly His Thr Arg Tyr Ser Ile Thr Ala Gly Glu Tyr Arg
370 375 380

Ser Gly Asn Ala Gln Gln Glu Lys Pro Arg Phe Phe Gln Ser Thr Leu
385 390 395 400

Leu His Gly Leu Pro Ala Gly Trp Thr Ile Tyr Gly Gly Thr Gln Leu
405 410 415

Ala Asp Arg Tyr Arg Ala Phe Asn Phe Gly Ile Gly Lys Asn Met Gly
420 425 430

Ala Leu Gly Ala Leu Ser Val Asp Met Thr Gln Ala Asn Ser Thr Leu
435 440 445

Pro Asp Asp Ser Gln His Asp Gly Gln Ser Val Arg Phe Leu Tyr Asn
450 455 460

Lys Ser Leu Asn Glu Ser Gly Thr Asn Ile Gln Leu Val Gly Tyr Arg
465 470 475 480

Tyr Ser Thr Ser Gly Tyr Phe Asn Phe Ala Asp Thr Thr Tyr Ser Arg
485 490 495

Met Asn Gly Tyr Asn Ile Glu Thr Gln Asp Gly Val Ile Gln Val Lys
500 505 510

Pro Lys Phe Thr Asp Tyr Tyr Asn Leu Ala Tyr Asn Lys Arg Gly Lys
515 520 525

Leu Gln Leu Thr Val Thr Gln Gln Leu Gly Arg Thr Ser Thr Leu Tyr
530 535 540

Leu Ser Gly Ser His Gln Thr Tyr Trp Gly Thr Ser Asn Val Asp Glu
545 550 555 560

Gln Phe Gln Ala Gly Leu Asn Thr Ala Phe Glu Asp Ile Asn Trp Thr
565 570 575

Leu Ser Tyr Ser Leu Thr Lys Asn Ala Trp Gln Lys Gly Arg Asp Gln
580 585 590

Met Leu Ala Leu Asn Val Asn Ile Pro Phe Ser His Trp Leu Arg Ser
595 600 605

Asp Ser Lys Ser Gln Trp Arg His Ala Ser Ala Ser Tyr Ser Met Ser
610 615 620

His Asp Leu Asn Gly Arg Met Thr Asn Leu Ala Gly Val Tyr Gly Thr
625 630 635 640

Leu Leu Glu Asp Asn Asn Leu Ser Tyr Ser Val Gln Thr Gly Tyr Ala
645 650 655

Gly Gly Gly Asp Gly Asn Ser Gly Ser Thr Gly Tyr Ala Thr Leu Asn
660 665 670

Tyr Arg Gly Gly Tyr Gly Asn Ala Asn Ile Gly Tyr Ser His Ser Asp
675 680 685

Asp Ile Lys Gln Leu Tyr Tyr Gly Val Ser Gly Gly Val Leu Ala His
690 695 700

Ala Asn Gly Val Thr Leu Gly Gln Pro Leu Asn Asp Thr Val Val Leu
705 710 715 720

Val Lys Ala Pro Gly Ala Lys Asp Ala Lys Val Glu Asn Gln Thr Gly
725 730 735

Val Arg Thr Asp Trp Arg Gly Tyr Ala Val Leu Pro Tyr Ala Thr Glu
740 745 750

Tyr Arg Glu Asn Arg Val Ala Leu Asp Thr Asn Thr Leu Ala Asp Asn
755 760 765

Val Asp Leu Asp Asn Ala Val Ala Asn Val Val Pro Thr Arg Gly Ala
770 775 780

Ile Val Arg Ala Glu Phe Lys Ala Arg Val Gly Ile Lys Leu Leu Met
785 790 795 800

Thr Leu Thr His Asn Asn Lys Pro Leu Pro Phe Gly Ala Met Val Thr
805 810 815

Ser Glu Ser Ser Gln Ser Ser Gly Ile Val Ala Asp Asn Gly Gln Val
820 825 830

Tyr Leu Ser Gly Met Pro Leu Ala Gly Lys Val Gln Val Lys Trp Gly
835 840 845

Glu Glu Glu Asn Ala His Cys Val Ala Asn Tyr Gln Leu Pro Pro Glu
850 855 860

Ser Gln Gln Gln Leu Leu Thr Gln Leu Ser Ala Glu Cys Arg
865 870 875

<210> 140
<211> 645
<212> PRT
<213> Escherichia coli

<400> 140
Val Glu Lys Ala Lys His Val Thr Trp Arg Leu Leu Ala Val Gly Val
1 5 10 15

Cys Leu Leu Thr Val Ser Ser Val Ala Arg Ala Asp Ser Leu Asp Glu
20 25 30

Gln Arg Ser Arg Tyr Ala Gln Ile Lys Gln Ala Trp Asp Asn Arg Gln

35 40 45

Met Asp Val Val Glu Gln Met Met Pro Gly Leu Lys Asp Tyr Pro Leu
50 55 60

Tyr Pro Tyr Leu Glu Tyr Arg Gln Ile Thr Asp Asp Leu Met Asn Gln
65 70 75 80

Pro Ala Val Thr Val Thr Asn Phe Val Arg Ala Asn Pro Thr Leu Pro
85 90 95

Pro Ala Arg Thr Leu Gln Ser Arg Phe Val Asn Glu Leu Ala Arg Arg
100 105 110

Glu Asp Trp Arg Gly Leu Leu Ala Phe Ser Pro Glu Lys Pro Gly Thr
115 120 125

Thr Glu Ala Gln Cys Asn Tyr Tyr Tyr Ala Lys Trp Asn Thr Gly Gln
130 135 140

Ser Glu Glu Ala Trp Gln Gly Ala Lys Glu Leu Trp Leu Thr Gly Lys
145 150 155 160

Ser Gln Pro Asn Ala Cys Asp Lys Leu Phe Ser Val Trp Arg Ala Ser
165 170 175

Gly Lys Gln Asp Pro Leu Ala Tyr Leu Glu Arg Ile Arg Leu Ala Met
180 185 190

Lys Ala Gly Asn Thr Gly Leu Val Thr Val Leu Ala Gly Gln Met Pro
195 200 205

Ala Asp Tyr Gln Thr Ile Ala Ser Ala Ile Ile Ser Leu Ala Asn Asn
210 215 220

Pro Asn Thr Val Leu Thr Phe Ala Arg Thr Thr Gly Ala Thr Asp Phe
225 230 235 240

Thr Arg Gln Met Ala Ala Val Ala Phe Ala Ser Val Ala Arg Gln Asp
245 250 255

Ala Glu Asn Ala Arg Leu Met Ile Pro Ser Leu Ala Gln Ala Gln Gln
260 265 270

Leu Asn Glu Asp Gln Ile Gln Glu Leu Arg Asp Ile Val Ala Trp Arg
275 280 285

Leu Met Gly Asn Asp Val Thr Asp Glu Gln Ala Lys Trp Arg Asp Asp
290 295 300

Ala Ile Met Arg Ser Gln Ser Thr Ser Leu Ile Glu Arg Arg Val Arg
305 310 315 320

Met Ala Leu Gly Thr Gly Asp Arg Arg Gly Leu Asn Thr Trp Leu Ala
325 330 335

Arg Leu Pro Met Glu Ala Lys Glu Lys Asp Glu Trp Arg Tyr Trp Gln
340 345 350

Ala Asp Leu Leu Leu Glu Arg Gly Arg Glu Ala Glu Ala Lys Glu Ile
355 360 365

Leu His Gln Leu Met Gln Gln Arg Gly Phe Tyr Pro Met Val Ala Ala
370 375 380

```
Gln Arg Ile Gly Glu Glu Tyr Glu Leu Lys Ile Asp Lys Ala Pro Gln
385                 390                 395                 400

Asn Val Asp Ser Ala Leu Thr Gln Gly Pro Glu Met Ala Arg Val Arg
                405                 410                 415

Glu Leu Met Tyr Trp Asn Leu Asp Asn Thr Ala Arg Ser Glu Trp Ala
            420                 425                 430

Asn Leu Val Lys Ser Lys Ser Lys Thr Glu Gln Ala Gln Leu Ala Arg
        435                 440                 445

Tyr Ala Phe Asn Asn Gln Trp Trp Asp Leu Ser Val Gln Ala Thr Ile
    450                 455                 460

Ala Gly Lys Leu Trp Asp His Leu Glu Glu Arg Phe Pro Leu Ala Tyr
465                 470                 475                 480

Asn Asp Leu Phe Lys Arg Tyr Thr Ser Gly Lys Glu Ile Pro Gln Ser
                485                 490                 495

Tyr Ala Met Ala Ile Ala Arg Gln Glu Ser Ala Trp Asn Pro Lys Val
            500                 505                 510

Lys Ser Pro Val Gly Ala Ser Gly Leu Met Gln Ile Met Pro Gly Thr
        515                 520                 525

Ala Thr His Thr Val Lys Met Phe Ser Ile Pro Gly Tyr Ser Ser Pro
    530                 535                 540

Gly Gln Leu Leu Asp Pro Glu Thr Asn Ile Asn Ile Gly Thr Ser Tyr
545                 550                 555                 560

Leu Gln Tyr Val Tyr Gln Gln Phe Gly Asn Asn Arg Ile Phe Ser Ser
                565                 570                 575

Ala Ala Tyr Asn Ala Gly Pro Gly Arg Val Arg Thr Trp Leu Gly Asn
            580                 585                 590

Ser Ala Gly Arg Ile Asp Ala Val Ala Phe Val Glu Ser Ile Pro Phe
        595                 600                 605

Ser Glu Thr Arg Gly Tyr Val Lys Asn Val Leu Ala Tyr Asp Ala Tyr
    610                 615                 620

Tyr Arg Tyr Phe Met Gly Asp Lys Pro Thr Leu Met Ser Ala Thr Glu
625                 630                 635                 640

Trp Gly Arg Arg Tyr
                645

<210>   141
<211>   314
<212>   PRT
<213>   Escherichia coli

<400>   141
Met Lys Leu Lys Asn Thr Leu Leu Ala Ser Ala Leu Leu Ser Ala Thr
1               5                   10                  15

Ala Phe Ser Val Asn Ala Ala Thr Glu Leu Thr Pro Glu Gln Ala Ala
            20                  25                  30

Ala Val Lys Pro Phe Asp Arg Val Val Ile Thr Gly Arg Phe Asn Ala
        35                  40                  45
```

Ile Gly Glu Ala Val Lys Ala Val Ser Arg Arg Ala Asp Lys Glu Gly
    50                  55                  60

Ala Ala Ser Phe Tyr Val Val Asp Thr Ser Asp Phe Gly Asn Ser Gly
65                  70                  75                  80

Asn Trp Arg Val Val Ala Asp Leu Tyr Lys Ala Asp Ala Glu Lys Ala
                85                  90                  95

Glu Glu Thr Ser Asn Arg Val Ile Asn Ser Val Val Glu Leu Pro Lys
            100                 105                 110

Asp Gln Ala Val Leu Ile Glu Pro Phe Asp Thr Val Thr Val Gln Gly
        115                 120                 125

Phe Tyr Arg Ser Gln Pro Glu Val Asn Asp Ala Ile Thr Lys Ala Ala
    130                 135                 140

Lys Ala Lys Gly Ala Tyr Ser Phe Tyr Ile Val Arg Gln Ile Asp Ala
145                 150                 155                 160

Asn Gln Gly Gly Asn Gln Arg Ile Thr Ala Phe Ile Tyr Lys Lys Asp
                165                 170                 175

Ala Lys Lys Arg Ile Val Gln Ser Pro Asp Val Ile Pro Ala Asp Ser
            180                 185                 190

Glu Ala Gly Arg Ala Ala Leu Ala Ala Gly Gly Glu Ala Ala Lys Lys
        195                 200                 205

Val Glu Ile Pro Gly Val Ala Thr Thr Ala Ser Pro Ser Ser Glu Val
    210                 215                 220

Gly Arg Phe Phe Glu Thr Gln Ser Ser Lys Gly Gly Arg Tyr Thr Val
225                 230                 235                 240

Thr Leu Pro Asp Gly Thr Lys Val Glu Glu Leu Asn Lys Ala Thr Ala
                245                 250                 255

Ala Met Met Val Pro Phe Asp Ser Ile Lys Phe Ser Gly Asn Tyr Gly
            260                 265                 270

Asn Met Thr Glu Val Ser Tyr Gln Val Ala Lys Arg Ala Ala Lys Lys
        275                 280                 285

Gly Ala Lys Tyr Tyr His Ile Thr Arg Gln Trp Gln Glu Arg Gly Asn
    290                 295                 300

Asn Leu Thr Val Ser Ala Asp Leu Tyr Lys
305                 310

<210> 142
<211> 438
<212> PRT
<213> Escherichia coli

<400> 142
Met Ala Asp Tyr Gln Gly Lys Asn Val Val Ile Ile Gly Leu Gly Leu
1               5                   10                  15

Thr Gly Leu Ser Cys Val Asp Phe Phe Leu Ala Arg Gly Val Thr Pro
            20                  25                  30

Arg Val Met Asp Thr Arg Met Thr Pro Pro Gly Leu Asp Lys Leu Pro

35 40 45

Glu Ala Val Glu Arg His Thr Gly Gly Leu Asn Asp Glu Trp Leu Met
50 55 60

Ala Ala Asp Leu Ile Val Ala Ser Pro Gly Ile Ala Leu Ala His Pro
65 70 75 80

Ser Leu Ser Ala Ala Ala Asp Ala Gly Ile Glu Ile Val Gly Asp Ile
85 90 95

Glu Leu Phe Cys Arg Glu Ala Gln Ala Pro Ile Val Ala Ile Thr Gly
100 105 110

Ser Asn Gly Lys Ser Thr Val Thr Thr Leu Val Gly Glu Met Ala Lys
115 120 125

Ala Ala Gly Val Asn Val Gly Val Gly Gly Asn Ile Gly Leu Pro Ala
130 135 140

Leu Met Leu Leu Asp Asp Glu Cys Glu Leu Tyr Val Leu Glu Leu Ser
145 150 155 160

Ser Phe Gln Leu Glu Thr Thr Ser Ser Leu Gln Ala Val Ala Ala Thr
165 170 175

Ile Leu Asn Val Thr Glu Asp His Met Asp Arg Tyr Pro Phe Gly Leu
180 185 190

Gln Gln Tyr Arg Ala Ala Lys Leu Arg Ile Tyr Glu Asn Ala Lys Val
195 200 205

Cys Val Val Asn Ala Asp Asp Ala Leu Thr Met Pro Ile Arg Gly Ala
210 215 220

Asp Glu Arg Cys Val Ser Phe Gly Val Asn Met Gly Asp Tyr His Leu
225 230 235 240

Asn His Gln Gln Gly Glu Thr Trp Leu Arg Val Lys Gly Glu Lys Val
245 250 255

Leu Asn Val Lys Glu Met Lys Leu Ser Gly Gln His Asn Tyr Thr Asn
260 265 270

Ala Leu Ala Ala Leu Ala Leu Ala Asp Ala Ala Gly Leu Pro Arg Ala
275 280 285

Ser Ser Leu Lys Ala Leu Thr Thr Phe Thr Gly Leu Pro His Arg Phe
290 295 300

Glu Val Val Leu Glu His Asn Gly Val Arg Trp Val Asn Asp Ser Lys
305 310 315 320

Ala Thr Asn Val Gly Ser Thr Glu Ala Ala Leu Asn Gly Leu His Val
325 330 335

Asp Gly Thr Leu His Leu Leu Leu Gly Gly Asp Gly Lys Ser Ala Asp
340 345 350

Phe Ser Pro Leu Ala Arg Tyr Leu Asn Gly Asp Asn Val Arg Leu Tyr
355 360 365

Cys Phe Gly Arg Asp Gly Ala Gln Leu Ala Ala Leu Arg Pro Glu Val
370 375 380

Ala Glu Gln Thr Glu Thr Met Glu Gln Ala Met Arg Leu Leu Ala Pro
385 390 395 400

Arg Val Gln Pro Gly Asp Met Val Leu Leu Ser Pro Ala Cys Ala Ser
405 410 415

Leu Asp Gln Phe Lys Asn Phe Glu Gln Arg Gly Asn Glu Phe Ala Arg
420 425 430

Leu Ala Lys Glu Leu Gly
435

<210> 143
<211> 229
<212> PRT
<213> Escherichia coli

<400> 143
Met Ile Leu Thr Asp Pro Glu Trp Gln Ala Val Leu Leu Ser Leu Lys
1 5 10 15

Val Ser Ser Leu Ala Val Leu Phe Ser Leu Pro Phe Gly Ile Phe Phe
20 25 30

Ala Trp Leu Leu Val Arg Cys Thr Phe Pro Gly Lys Ala Leu Leu Asp
35 40 45

Ser Val Leu His Leu Pro Leu Val Leu Pro Pro Val Val Val Gly Tyr
50 55 60

Leu Leu Leu Val Ser Met Gly Arg Arg Gly Phe Ile Gly Glu Arg Leu
65 70 75 80

Tyr Asp Trp Phe Gly Leu Ser Phe Ala Phe Ser Trp Arg Gly Ala Val
85 90 95

Leu Ala Ala Ala Val Met Ser Phe Pro Leu Met Val Arg Ala Ile Arg
100 105 110

Leu Ala Leu Glu Gly Val Asp Ile Lys Leu Glu Gln Ala Ala Arg Thr
115 120 125

Leu Gly Ala Gly Arg Trp Arg Val Phe Phe Thr Ile Thr Leu Pro Leu
130 135 140

Thr Leu Pro Gly Ile Ile Val Gly Thr Val Leu Ala Phe Ala Arg Ser
145 150 155 160

Leu Gly Glu Phe Gly Ala Thr Ile Thr Phe Val Ser Asn Ile Pro Gly
165 170 175

Glu Thr Arg Thr Ile Pro Ser Ala Met Tyr Thr Leu Ile Gln Thr Pro
180 185 190

Gly Gly Glu Ser Gly Ala Ala Arg Leu Cys Ile Ile Ser Ile Ala Leu
195 200 205

Ala Met Ile Ser Leu Leu Ile Ser Glu Trp Leu Ala Arg Ile Ser Arg
210 215 220

Glu Arg Ala Gly Arg
225

<210> 144
<211> 363

<212> PRT
<213> Escherichia coli

<400> 144
Met Lys Ile Gly Val Phe Val Pro Ile Gly Asn Asn Gly Trp Leu Ile
1 5 10 15

Ser Thr His Ala Pro Gln Tyr Met Pro Thr Phe Glu Leu Asn Lys Ala
20 25 30

Ile Val Gln Lys Ala Glu His Tyr His Phe Asp Phe Ala Leu Ser Met
35 40 45

Ile Lys Leu Arg Gly Phe Gly Gly Lys Thr Glu Phe Trp Asp His Asn
50 55 60

Leu Glu Ser Phe Thr Leu Met Ala Gly Leu Ala Ala Val Thr Ser Arg
65 70 75 80

Ile Gln Ile Tyr Ala Thr Ala Ala Thr Leu Thr Leu Pro Pro Ala Ile
85 90 95

Val Ala Arg Met Ala Ala Thr Ile Asp Ser Ile Ser Gly Gly Arg Phe
100 105 110

Gly Val Asn Leu Val Thr Gly Trp Gln Lys Pro Glu Tyr Glu Gln Met
115 120 125

Gly Ile Trp Pro Gly Asp Asp Tyr Phe Ser Arg Arg Tyr Asp Tyr Leu
130 135 140

Thr Glu Tyr Val Gln Val Leu Arg Asp Leu Trp Gly Ser Gly Lys Ser
145 150 155 160

Asp Phe Lys Gly Asp Phe Phe Thr Met Asp Asp Cys Arg Val Ser Pro
165 170 175

Gln Pro Ser Val Pro Met Lys Val Ile Cys Ala Gly Gln Ser Asp Ala
180 185 190

Gly Met Ala Phe Ser Ala Arg Tyr Ala Asp Phe Asn Phe Cys Phe Gly
195 200 205

Lys Gly Val Asn Thr Pro Thr Ala Phe Ala Pro Thr Ala Ala Arg Met
210 215 220

Lys Gln Ala Ala Glu Gln Thr Gly Arg Asp Val Gly Ser Tyr Val Leu
225 230 235 240

Phe Met Val Ile Ala Asp Glu Thr Asp Asp Ala Ala Arg Ala Lys Trp
245 250 255

Glu His Tyr Lys Ala Gly Ala Asp Glu Glu Ala Leu Ser Trp Leu Thr
260 265 270

Glu Gln Ser Gln Lys Asp Thr Arg Ser Gly Thr Asp Thr Asn Val Arg
275 280 285

Gln Met Ala Asp Pro Thr Ser Ala Val Asn Ile Asn Met Gly Thr Leu
290 295 300

Val Gly Ser Tyr Ala Ser Val Ala Arg Met Leu Asp Glu Val Ala Ser
305 310 315 320

Val Pro Gly Ala Glu Gly Val Leu Leu Thr Phe Asp Asp Phe Leu Ser

                325                 330                 335

Gly Ile Glu Thr Phe Gly Glu Arg Ile Gln Pro Leu Met Gln Cys Arg
            340                 345                 350

Ala His Leu Pro Ala Leu Thr Gln Glu Val Ala
        355                 360

<210> 145
<211> 78
<212> PRT
<213> Escherichia coli

<400> 145
Met Lys Ala Thr Lys Leu Val Leu Gly Ala Val Ile Leu Gly Ser Thr
1               5                   10                  15

Leu Leu Ala Gly Cys Ser Ser Asn Ala Lys Ile Asp Gln Leu Ser Ser
            20                  25                  30

Asp Val Gln Thr Leu Asn Ala Lys Val Asp Gln Leu Ser Asn Asp Val
        35                  40                  45

Asn Ala Met Arg Ser Asp Val Gln Ala Ala Lys Asp Asp Ala Ala Arg
    50                  55                  60

Ala Asn Gln Arg Leu Asp Asn Met Ala Thr Lys Tyr Arg Lys
65                  70                  75

<210> 146
<211> 518
<212> PRT
<213> Escherichia coli

<400> 146
Met Glu Phe Leu Met Asp Pro Ser Ile Trp Ala Gly Leu Leu Thr Leu
1               5                   10                  15

Val Val Leu Glu Ile Val Leu Gly Ile Asp Asn Leu Val Phe Ile Ala
            20                  25                  30

Ile Leu Ala Asp Lys Leu Pro Pro Lys Gln Arg Asp Lys Ala Arg Leu
        35                  40                  45

Leu Gly Leu Ser Leu Ala Leu Ile Met Arg Leu Gly Leu Leu Ser Leu
    50                  55                  60

Ile Ser Trp Met Val Thr Leu Thr Lys Pro Leu Phe Thr Val Met Asp
65                  70                  75                  80

Phe Ser Phe Ser Gly Arg Asp Leu Ile Met Leu Phe Gly Gly Ile Phe
                85                  90                  95

Leu Leu Phe Lys Ala Thr Thr Glu Leu His Glu Arg Leu Glu Asn Arg
            100                 105                 110

Asp His Asp Ser Gly His Gly Lys Gly Tyr Ala Ser Phe Trp Val Val
        115                 120                 125

Val Thr Gln Ile Val Ile Leu Asp Ala Val Phe Ser Leu Asp Ala Val
    130                 135                 140

Ile Thr Ala Val Gly Met Val Asn His Leu Pro Val Met Met Ala Ala
145                 150                 155                 160

Val Val Ile Ala Met Ala Val Met Leu Leu Ala Ser Lys Pro Leu Thr
165 170 175

Arg Phe Val Asn Gln His Pro Thr Val Val Val Leu Cys Leu Ser Phe
180 185 190

Leu Leu Met Ile Gly Leu Ser Leu Val Ala Glu Gly Phe Gly Phe His
195 200 205

Ile Pro Lys Gly Tyr Leu Tyr Ala Ala Ile Gly Phe Ser Ile Ile Ile
210 215 220

Glu Val Phe Asn Gln Ile Ala Arg Arg Asn Phe Ile Arg His Gln Ser
225 230 235 240

Thr Leu Pro Leu Arg Ala Arg Thr Ala Asp Ala Ile Leu Arg Leu Met
245 250 255

Gly Gly Lys Arg Gln Ala Asn Val Gln His Asp Ala Asp Asn Pro Met
260 265 270

Pro Met Pro Ile Pro Glu Gly Ala Phe Ala Glu Glu Glu Arg Tyr Met
275 280 285

Ile Asn Gly Val Leu Thr Leu Ala Ser Arg Ser Leu Arg Gly Ile Met
290 295 300

Thr Pro Arg Gly Glu Ile Ser Trp Val Asp Ala Asn Leu Gly Val Asp
305 310 315 320

Glu Ile Arg Glu Gln Leu Leu Ser Ser Pro His Ser Leu Phe Pro Val
325 330 335

Cys Arg Gly Glu Leu Asp Glu Ile Ile Gly Ile Val Arg Ala Lys Glu
340 345 350

Leu Leu Val Ala Leu Glu Glu Gly Val Asp Val Ala Ala Ile Ala Ser
355 360 365

Ala Ser Pro Ala Ile Ile Val Pro Glu Thr Leu Asp Pro Ile Asn Leu
370 375 380

Leu Gly Val Leu Arg Arg Ala Arg Gly Ser Phe Val Ile Val Thr Asn
385 390 395 400

Glu Phe Gly Val Val Gln Gly Leu Val Thr Pro Leu Asp Val Leu Glu
405 410 415

Ala Ile Ala Gly Glu Phe Pro Asp Ala Asp Glu Thr Pro Glu Ile Ile
420 425 430

Thr Asp Gly Asp Gly Trp Leu Val Lys Gly Gly Thr Asp Leu His Ala
435 440 445

Leu Gln Gln Ala Leu Asp Val Glu His Leu Ala Asp Asp Asp Asp Ile
450 455 460

Ala Thr Val Ala Gly Leu Val Ile Ser Ala Asn Gly His Ile Pro Arg
465 470 475 480

Val Gly Asp Val Ile Asp Val Gly Pro Leu His Ile Thr Ile Ile Glu
485 490 495

Ala Asn Asp Tyr Arg Val Asp Leu Val Arg Ile Val Lys Glu Gln Pro
500 505 510

Ala His Asp Glu Asp Glu
        515

<210>   147
<211>   446
<212>   PRT
<213>   Escherichia coli

<400>   147
Met Ser Gln Lys Thr Leu Phe Thr Lys Ser Ala Leu Ala Val Ala Val
1               5                   10                  15

Ala Leu Ile Ser Thr Gln Ala Trp Ser Ala Gly Phe Gln Leu Asn Glu
            20                  25                  30

Phe Ser Ser Ser Ser Leu Gly Arg Ala Tyr Ser Gly Glu Gly Ala Ile
        35                  40                  45

Ala Asp Asp Ala Gly Asn Val Ser Arg Asn Pro Ala Leu Ile Thr Met
    50                  55                  60

Phe Asp Arg Pro Thr Phe Ser Ala Gly Ala Val Tyr Ile Asp Pro Asp
65                  70                  75                  80

Val Asn Ile Ser Gly Thr Ser Pro Ser Gly Arg Ser Leu Lys Ala Asp
                85                  90                  95

Asn Ile Ala Pro Thr Ala Trp Val Pro Asn Met His Phe Val Ala Pro
            100                 105                 110

Ile Asn Asp Gln Phe Gly Trp Gly Ala Ser Ile Thr Ser Asn Tyr Gly
        115                 120                 125

Leu Ala Thr Glu Phe Asn Asp Thr Tyr Ala Gly Gly Ser Val Gly Gly
    130                 135                 140

Thr Thr Asp Leu Glu Thr Met Asn Leu Asn Leu Ser Gly Ala Tyr Arg
145                 150                 155                 160

Leu Asn Asn Ala Trp Ser Phe Gly Leu Gly Phe Asn Ala Val Tyr Ala
                165                 170                 175

Arg Ala Lys Ile Glu Arg Phe Ala Gly Asp Leu Gly Gln Leu Val Ala
            180                 185                 190

Gly Gln Ile Met Gln Ser Pro Ala Gly Gln Thr Gln Gln Gly Gln Ala
        195                 200                 205

Leu Ala Ala Thr Ala Asn Gly Ile Asp Ser Asn Thr Lys Ile Ala His
    210                 215                 220

Leu Asn Gly Asn Gln Trp Gly Phe Gly Trp Asn Ala Gly Ile Leu Tyr
225                 230                 235                 240

Glu Leu Asp Lys Asn Asn Arg Tyr Ala Leu Thr Tyr Arg Ser Glu Val
                245                 250                 255

Lys Ile Asp Phe Lys Gly Asn Tyr Ser Ser Asp Leu Asn Arg Ala Phe
            260                 265                 270

Asn Asn Tyr Gly Leu Pro Ile Pro Thr Ala Thr Gly Gly Ala Thr Gln
        275                 280                 285

Ser Gly Tyr Leu Thr Leu Asn Leu Pro Glu Met Trp Glu Val Ser Gly

290 295 300

Tyr Asn Arg Val Ala Pro Gln Trp Ala Ile His Tyr Ser Leu Ala Tyr
305 310 315 320

Thr Ser Trp Ser Gln Phe Gln Gln Leu Lys Ala Thr Ser Thr Ser Gly
325 330 335

Asp Thr Leu Phe Gln Lys His Glu Gly Phe Lys Asp Ala Tyr Arg Ile
340 345 350

Ala Leu Gly Thr Thr Tyr Tyr Tyr Asp Asp Asn Trp Thr Phe Arg Thr
355 360 365

Gly Ile Ala Phe Asp Asp Ser Pro Val Pro Ala Gln Asn Arg Ser Ile
370 375 380

Ser Ile Pro Asp Gln Asp Arg Phe Trp Leu Ser Ala Gly Thr Thr Tyr
385 390 395 400

Ala Phe Asn Lys Asp Ala Ser Val Asp Val Gly Val Ser Tyr Met His
405 410 415

Gly Gln Ser Val Lys Ile Asn Glu Gly Pro Tyr Gln Phe Glu Ser Glu
420 425 430

Gly Lys Ala Trp Leu Phe Gly Thr Asn Phe Asn Tyr Ala Phe
435 440 445

<210> 148
<211> 446
<212> PRT
<213> Escherichia coli

<400> 148
Met Met Ile Thr Leu Arg Lys Leu Pro Leu Ala Val Ala Val Ala Ala
1 5 10 15

Gly Val Met Ser Ala Gln Ala Met Ala Val Asp Phe His Gly Tyr Ala
20 25 30

Arg Ser Gly Ile Gly Trp Thr Gly Ser Gly Gly Glu Gln Gln Cys Phe
35 40 45

Gln Thr Thr Gly Ala Gln Ser Lys Tyr Arg Leu Gly Asn Glu Cys Glu
50 55 60

Thr Tyr Ala Glu Leu Lys Leu Gly Gln Glu Val Trp Lys Glu Gly Asp
65 70 75 80

Lys Ser Phe Tyr Phe Asp Thr Asn Val Ala Tyr Ser Val Ala Gln Lys
85 90 95

Asn Asp Trp Glu Ala Thr Asp Pro Ala Phe Arg Glu Ala Asn Val Gln
100 105 110

Gly Lys Asn Leu Ile Glu Trp Leu Pro Gly Ser Thr Ile Trp Ala Gly
115 120 125

Lys Arg Phe Tyr Gln Arg His Asp Val His Met Ile Asp Phe Tyr Tyr
130 135 140

Trp Asp Ile Ser Gly Pro Gly Ala Gly Leu Glu Asn Ile Asp Val Gly
145 150 155 160

Phe Gly Lys Leu Ser Leu Ala Ala Thr Arg Ser Ser Glu Ala Gly Gly
165 170 175

Ser Ser Ser Phe Ala Ser Asn Asn Ile Tyr Asp Tyr Thr Asn Glu Thr
180 185 190

Ala Asn Asp Val Phe Asp Val Arg Leu Ala Gln Met Glu Ile Asn Pro
195 200 205

Gly Gly Thr Leu Glu Leu Gly Val Asp Tyr Gly Arg Ala Asn Leu Arg
210 215 220

Asp Asn Tyr Arg Leu Val Asp Gly Ala Ser Lys Asp Gly Trp Leu Phe
225 230 235 240

Thr Ala Glu His Thr Gln Ser Val Leu Lys Gly Phe Asn Lys Phe Val
245 250 255

Val Gln Tyr Ala Thr Asp Ser Met Thr Ser Gln Gly Lys Gly Leu Ser
260 265 270

Gln Gly Ser Gly Val Ala Phe Asp Asn Glu Lys Phe Ala Tyr Asn Ile
275 280 285

Asn Asn Asn Gly His Met Leu Arg Ile Leu Asp His Gly Ala Ile Ser
290 295 300

Met Gly Asp Asn Trp Asp Met Met Tyr Val Gly Met Tyr Gln Asp Ile
305 310 315 320

Asn Trp Asp Asn Asp Asn Gly Thr Lys Trp Trp Thr Val Gly Ile Arg
325 330 335

Pro Met Tyr Lys Trp Thr Pro Ile Met Ser Thr Val Met Glu Ile Gly
340 345 350

Tyr Asp Asn Val Glu Ser Gln Arg Thr Gly Asp Lys Asn Asn Gln Tyr
355 360 365

Lys Ile Thr Leu Ala Gln Gln Trp Gln Ala Gly Asp Ser Ile Trp Ser
370 375 380

Arg Pro Ala Ile Arg Val Phe Ala Thr Tyr Ala Lys Trp Asp Glu Lys
385 390 395 400

Trp Gly Tyr Asp Tyr Thr Gly Ser Ser Ser Thr Asn Pro Tyr Tyr Gly
405 410 415

Lys Ala Val Ser Ala Asp Phe Asn Gly Gly Ser Phe Gly Arg Gly Asp
420 425 430

Ser Asp Glu Trp Thr Phe Gly Ala Gln Met Glu Ile Trp Trp
435 440 445

<210> 149
<211> 237
<212> PRT
<213> Escherichia coli

<400> 149
Val Asn Glu Ala Glu Ser Gln Ala Arg Ala Ile Val Asp Gln Ala Arg
1 5 10 15

Asn Gly Ala Asp Phe Gly Lys Leu Ala Ile Ala His Ser Ala Asp Gln
20 25 30

Gln Ala Leu Asn Gly Gly Gln Met Gly Trp Gly Arg Ile Gln Glu Leu
35 40 45

Pro Gly Ile Phe Ala Gln Ala Leu Ser Thr Ala Lys Lys Gly Asp Ile
50 55 60

Val Gly Pro Ile Arg Ser Gly Val Gly Phe His Ile Leu Lys Val Asn
65 70 75 80

Asp Leu Arg Gly Glu Ser Lys Asn Ile Ser Val Thr Glu Val His Ala
85 90 95

Arg His Ile Leu Leu Lys Pro Ser Pro Ile Met Thr Asp Glu Gln Ala
100 105 110

Arg Val Lys Leu Glu Gln Ile Ala Ala Asp Ile Lys Ser Gly Lys Thr
115 120 125

Thr Phe Ala Ala Ala Ala Lys Glu Phe Ser Gln Asp Pro Gly Ser Ala
130 135 140

Asn Gln Gly Gly Asp Leu Gly Trp Ala Thr Pro Asp Ile Phe Asp Pro
145 150 155 160

Ala Phe Arg Asp Ala Leu Thr Arg Leu Asn Lys Gly Gln Met Ser Ala
165 170 175

Pro Val His Ser Ser Phe Gly Trp His Leu Ile Glu Leu Leu Asp Thr
180 185 190

Arg Asn Val Asp Lys Thr Asp Ala Ala Gln Lys Asp Arg Ala Tyr Arg
195 200 205

Met Leu Met Asn Arg Lys Phe Ser Glu Glu Ala Ala Ser Trp Met Gln
210 215 220

Glu Gln Arg Ala Ser Ala Tyr Val Lys Ile Leu Ser Asn
225 230 235

<210> 150
<211> 191
<212> PRT
<213> Escherichia coli

<400> 150
Met Lys Asn Trp Lys Thr Leu Leu Leu Gly Ile Ala Met Ile Ala Asn
1 5 10 15

Thr Ser Phe Ala Ala Pro Gln Val Val Asp Lys Val Ala Ala Val Val
20 25 30

Asn Asn Gly Val Val Leu Glu Ser Asp Val Asp Gly Leu Met Gln Ser
35 40 45

Val Lys Leu Asn Ala Ala Gln Ala Arg Gln Gln Leu Pro Asp Asp Ala
50 55 60

Thr Leu Arg His Gln Ile Met Glu Arg Leu Ile Met Asp Gln Ile Ile
65 70 75 80

Leu Gln Met Gly Gln Lys Met Gly Val Lys Ile Ser Asp Glu Gln Leu
85 90 95

Asp Gln Ala Ile Ala Asn Ile Ala Lys Gln Asn Asn Met Thr Leu Asp

100 105 110

Gln Met Arg Ser Arg Leu Ala Tyr Asp Gly Leu Asn Tyr Asn Thr Tyr
115 120 125

Arg Asn Gln Ile Arg Lys Glu Met Ile Ile Ser Glu Val Arg Asn Asn
130 135 140

Glu Val Arg Arg Arg Ile Thr Ile Leu Pro Gln Glu Val Glu Ile Leu
145 150 155 160

Ala Gln Gln Val Gly Asn Gln Asn Asp Ala Ser Thr Glu Leu Asn Leu
165 170 175

Ser His Ile Leu Asn Pro Leu Ala Glu Lys Pro Thr Leu Ile Arg
180 185 190

<210> 151
<211> 630
<212> PRT
<213> Escherichia coli

<400> 151
Met Ala Ile Glu Ile Lys Val Pro Asp Ile Gly Ala Asp Glu Val Glu
1 5 10 15

Ile Thr Glu Ile Leu Val Lys Val Gly Asp Lys Val Glu Ala Glu Gln
20 25 30

Ser Leu Ile Thr Val Glu Gly Asp Lys Ala Ser Met Glu Val Pro Ser
35 40 45

Pro Gln Ala Gly Ile Val Lys Glu Ile Lys Val Ser Val Gly Asp Lys
50 55 60

Thr Gln Thr Gly Ala Leu Ile Met Ile Phe Asp Ser Ala Asp Gly Ala
65 70 75 80

Ala Asp Ala Ala Pro Ala Gln Ala Glu Glu Lys Lys Glu Ala Ala Pro
85 90 95

Ala Ala Ala Pro Ala Ala Ala Ala Ala Lys Asp Val Asn Val Pro Asp
100 105 110

Ile Gly Ser Asp Glu Val Glu Val Thr Glu Ile Leu Val Lys Val Gly
115 120 125

Asp Lys Val Glu Ala Glu Gln Ser Leu Ile Thr Val Glu Gly Asp Lys
130 135 140

Ala Ser Met Glu Val Pro Ala Pro Phe Ala Gly Thr Val Lys Glu Ile
145 150 155 160

Lys Val Asn Val Gly Asp Lys Val Ser Thr Gly Ser Leu Ile Met Ile
165 170 175

Phe Glu Val Ala Gly Glu Ala Gly Ala Ala Ala Pro Ala Ala Lys Gln
180 185 190

Glu Ala Ala Pro Ala Ala Ala Pro Ala Pro Ala Ala Gly Val Lys Glu
195 200 205

Val Asn Val Pro Asp Ile Gly Gly Asp Glu Val Glu Val Thr Glu Val
210 215 220

Met Val Lys Val Gly Asp Lys Val Ala Ala Glu Gln Ser Leu Ile Thr
225 230 235 240

Val Glu Gly Asp Lys Ala Ser Met Glu Val Pro Ala Pro Phe Ala Gly
245 250 255

Val Val Lys Glu Leu Lys Val Asn Val Gly Asp Lys Val Lys Thr Gly
260 265 270

Ser Leu Ile Met Ile Phe Glu Val Glu Gly Ala Ala Pro Ala Ala Ala
275 280 285

Pro Ala Lys Gln Glu Ala Ala Ala Pro Ala Pro Ala Ala Lys Ala Glu
290 295 300

Ala Pro Ala Ala Ala Pro Ala Ala Lys Ala Glu Gly Lys Ser Glu Phe
305 310 315 320

Ala Glu Asn Asp Ala Tyr Val His Ala Thr Pro Leu Ile Arg Arg Leu
325 330 335

Ala Arg Glu Phe Gly Val Asn Leu Ala Lys Val Lys Gly Thr Gly Arg
340 345 350

Lys Gly Arg Ile Leu Arg Glu Asp Val Gln Ala Tyr Val Lys Glu Ala
355 360 365

Ile Lys Arg Ala Glu Ala Ala Pro Ala Ala Thr Gly Gly Gly Ile Pro
370 375 380

Gly Met Leu Pro Trp Pro Lys Val Asp Phe Ser Lys Phe Gly Glu Ile
385 390 395 400

Glu Glu Val Glu Leu Gly Arg Ile Gln Lys Ile Ser Gly Ala Asn Leu
405 410 415

Ser Arg Asn Trp Val Met Ile Pro His Val Thr His Phe Asp Lys Thr
420 425 430

Asp Ile Thr Glu Leu Glu Ala Phe Arg Lys Gln Gln Asn Glu Glu Ala
435 440 445

Ala Lys Arg Lys Leu Asp Val Lys Ile Thr Pro Val Val Phe Ile Met
450 455 460

Lys Ala Val Ala Ala Ala Leu Glu Gln Met Pro Arg Phe Asn Ser Ser
465 470 475 480

Leu Ser Glu Asp Gly Gln Arg Leu Thr Leu Lys Lys Tyr Ile Asn Ile
485 490 495

Gly Val Ala Val Asp Thr Pro Asn Gly Leu Val Val Pro Val Phe Lys
500 505 510

Asp Val Asn Lys Lys Gly Ile Ile Glu Leu Ser Arg Glu Leu Met Thr
515 520 525

Ile Ser Lys Lys Ala Arg Asp Gly Lys Leu Thr Ala Gly Glu Met Gln
530 535 540

Gly Gly Cys Phe Thr Ile Ser Ser Ile Gly Gly Leu Gly Thr Thr His
545 550 555 560

Phe Ala Pro Ile Val Asn Ala Pro Glu Val Ala Ile Leu Gly Val Ser
565 570 575

Lys Ser Ala Met Glu Pro Val Trp Asn Gly Lys Glu Phe Val Pro Arg
580 585 590

Leu Met Leu Pro Ile Ser Leu Ser Phe Asp His Arg Val Ile Asp Gly
595 600 605

Ala Asp Gly Ala Arg Phe Ile Thr Ile Ile Asn Asn Thr Leu Ser Asp
610 615 620

Ile Arg Arg Leu Val Met
625 630

<210> 152
<211> 550
<212> PRT
<213> Escherichia coli

<400> 152
Met Lys Leu Leu Gln Arg Gly Val Ala Leu Ala Leu Leu Thr Thr Phe
1 5 10 15

Thr Leu Ala Ser Glu Thr Ala Leu Ala Tyr Glu Gln Asp Lys Thr Tyr
20 25 30

Lys Ile Thr Val Leu His Thr Asn Asp His His Gly His Phe Trp Arg
35 40 45

Asn Glu Tyr Gly Glu Tyr Gly Leu Ala Ala Gln Lys Thr Leu Val Asp
50 55 60

Gly Ile Arg Lys Glu Val Ala Ala Glu Gly Gly Ser Val Leu Leu Leu
65 70 75 80

Ser Gly Gly Asp Ile Asn Thr Gly Val Pro Glu Ser Asp Leu Gln Asp
85 90 95

Ala Glu Pro Asp Phe Arg Gly Met Asn Leu Val Gly Tyr Asp Ala Met
100 105 110

Ala Ile Gly Asn His Glu Phe Asp Asn Pro Leu Thr Val Leu Arg Gln
115 120 125

Gln Glu Lys Trp Ala Lys Phe Pro Leu Leu Ser Ala Asn Ile Tyr Gln
130 135 140

Lys Ser Thr Gly Glu Arg Leu Phe Lys Pro Trp Ala Leu Phe Lys Arg
145 150 155 160

Gln Asp Leu Lys Ile Ala Val Ile Gly Leu Thr Thr Asp Asp Thr Ala
165 170 175

Lys Ile Gly Asn Pro Glu Tyr Phe Thr Asp Ile Glu Phe Arg Lys Pro
180 185 190

Ala Asp Glu Ala Lys Leu Val Ile Gln Glu Leu Gln Gln Thr Glu Lys
195 200 205

Pro Asp Ile Ile Ile Ala Ala Thr His Met Gly His Tyr Asp Asn Gly
210 215 220

Glu His Gly Ser Asn Ala Pro Gly Asp Val Glu Met Ala Arg Ala Leu
225 230 235 240

Pro Ala Arg Ser Leu Ala Met Ile Val Gly Gly His Ser Gln Asp Pro

245 250 255

Val Cys Met Ala Ala Glu Asn Lys Lys Gln Val Asp Tyr Val Pro Gly
260 265 270

Thr Pro Cys Lys Pro Asp Gln Gln Asn Gly Ile Trp Ile Val Gln Ala
275 280 285

His Glu Trp Gly Lys Tyr Val Gly Arg Ala Asp Phe Glu Phe Arg Asn
290 295 300

Gly Glu Met Lys Met Val Asn Tyr Gln Leu Ile Pro Val Asn Leu Lys
305 310 315 320

Lys Lys Val Thr Trp Glu Asp Gly Lys Ser Glu Arg Val Leu Tyr Thr
325 330 335

Pro Glu Ile Ala Glu Asn Gln Gln Met Ile Ser Leu Leu Ser Pro Phe
340 345 350

Gln Asn Lys Gly Lys Ala Gln Leu Glu Val Lys Ile Gly Glu Thr Asn
355 360 365

Gly Arg Leu Glu Gly Asp Arg Asp Lys Val Arg Phe Val Gln Thr Asn
370 375 380

Met Gly Arg Leu Ile Leu Ala Ala Gln Met Asp Arg Thr Gly Ala Asp
385 390 395 400

Phe Ala Val Met Ser Gly Gly Gly Ile Arg Asp Ser Ile Glu Ala Gly
405 410 415

Asp Ile Ser Tyr Lys Asn Val Leu Lys Val Gln Pro Phe Gly Asn Val
420 425 430

Val Val Tyr Ala Asp Met Thr Gly Lys Glu Val Ile Asp Tyr Leu Thr
435 440 445

Ala Val Ala Gln Met Lys Pro Asp Ser Gly Ala Tyr Pro Gln Phe Ala
450 455 460

Asn Val Ser Phe Val Ala Lys Asp Gly Lys Leu Asn Asp Leu Lys Ile
465 470 475 480

Lys Gly Glu Pro Val Asp Pro Ala Lys Thr Tyr Arg Met Ala Thr Leu
485 490 495

Asn Phe Asn Ala Thr Gly Gly Asp Gly Tyr Pro Arg Leu Asp Asn Lys
500 505 510

Pro Gly Tyr Val Asn Thr Gly Phe Ile Asp Ala Glu Val Leu Lys Ala
515 520 525

Tyr Ile Gln Lys Ser Ser Pro Leu Asp Val Ser Val Tyr Glu Pro Lys
530 535 540

Gly Glu Val Ser Trp Gln
545 550

<210> 153
<211> 430
<212> PRT
<213> Escherichia coli

<400> 153

```
Met Lys Gln Ala Leu Arg Val Ala Phe Gly Phe Leu Ile Leu Trp Ala
1               5                   10                  15

Ser Val Leu His Ala Glu Val Arg Ile Val Ile Asp Ser Gly Val Asp
            20                  25                  30

Ser Gly Arg Pro Ile Gly Val Val Pro Phe Gln Trp Ala Gly Pro Gly
        35                  40                  45

Ala Ala Pro Glu Asp Ile Gly Gly Ile Val Ala Ala Asp Leu Arg Asn
    50                  55                  60

Ser Gly Lys Phe Asn Pro Leu Asp Arg Ala Arg Leu Pro Gln Gln Pro
65                  70                  75                  80

Gly Ser Ala Gln Glu Val Gln Pro Ala Ala Trp Ser Ala Leu Gly Ile
                85                  90                  95

Asp Ala Val Val Val Gly Gln Val Thr Pro Asn Pro Asp Gly Ser Tyr
            100                 105                 110

Asn Val Ala Tyr Gln Leu Val Asp Thr Gly Gly Ala Pro Gly Thr Val
        115                 120                 125

Leu Ala Gln Asn Ser Tyr Lys Val Asn Lys Gln Trp Leu Arg Tyr Ala
    130                 135                 140

Gly His Thr Ala Ser Asp Glu Val Phe Glu Lys Leu Thr Gly Ile Lys
145                 150                 155                 160

Gly Ala Phe Arg Thr Arg Ile Ala Tyr Val Val Gln Thr Asn Gly Gly
                165                 170                 175

Gln Phe Pro Tyr Glu Leu Arg Val Ser Asp Tyr Asp Gly Tyr Asn Gln
            180                 185                 190

Phe Val Val His Arg Ser Pro Gln Pro Leu Met Ser Pro Ala Trp Ser
        195                 200                 205

Pro Asp Gly Ser Lys Leu Ala Tyr Val Thr Phe Glu Ser Gly Arg Ser
    210                 215                 220

Ala Leu Val Ile Gln Thr Leu Ala Asn Gly Ala Val Arg Gln Val Ala
225                 230                 235                 240

Ser Phe Pro Arg His Asn Gly Ala Pro Ala Phe Ser Pro Asp Gly Ser
                245                 250                 255

Lys Leu Ala Phe Ala Leu Ser Lys Thr Gly Ser Leu Asn Leu Tyr Val
            260                 265                 270

Met Asp Leu Ala Ser Gly Gln Ile Arg Gln Val Thr Asp Gly Arg Ser
        275                 280                 285

Asn Asn Thr Glu Pro Thr Trp Phe Pro Asp Ser Gln Asn Leu Ala Phe
    290                 295                 300

Thr Ser Asp Gln Ala Gly Arg Pro Gln Val Tyr Lys Val Asn Ile Asn
305                 310                 315                 320

Gly Gly Ala Pro Gln Arg Ile Thr Trp Glu Gly Ser Gln Asn Gln Asp
                325                 330                 335

Ala Asp Val Ser Ser Asp Gly Lys Phe Met Val Met Val Ser Ser Asn
            340                 345                 350
```

Gly Gly Gln Gln His Ile Ala Lys Gln Asp Leu Ala Thr Gly Gly Val
355 360 365

Gln Val Leu Ser Ser Thr Phe Leu Asp Glu Thr Pro Ser Leu Ala Pro
370 375 380

Asn Gly Thr Met Val Ile Tyr Ser Ser Ser Gln Gly Met Gly Ser Val
385 390 395 400

Leu Asn Leu Val Ser Thr Asp Gly Arg Phe Lys Ala Arg Leu Pro Ala
405 410 415

Thr Asp Gly Gln Val Lys Phe Pro Ala Trp Ser Pro Tyr Leu
420 425 430

<210> 154
<211> 112
<212> PRT
<213> Escherichia coli

<400> 154
Met Asn Lys Asn Met Ala Gly Ile Leu Ser Ala Ala Ala Val Leu Thr
1 5 10 15

Met Leu Ala Gly Cys Thr Ala Tyr Asp Arg Thr Lys Asp Gln Phe Val
20 25 30

Gln Pro Val Val Lys Asp Val Lys Lys Gly Met Ser Arg Ala Gln Val
35 40 45

Ala Gln Ile Ala Gly Lys Pro Ser Ser Glu Val Ser Met Ile His Ala
50 55 60

Arg Gly Thr Cys Gln Thr Tyr Ile Leu Gly Gln Arg Asp Gly Lys Ala
65 70 75 80

Glu Thr Tyr Phe Val Ala Leu Asp Asp Thr Gly His Val Ile Asn Ser
85 90 95

Gly Tyr Gln Thr Cys Ala Glu Tyr Asp Thr Asp Pro Gln Ala Thr Lys
100 105 110

<210> 155
<211> 191
<212> PRT
<213> Escherichia coli

<400> 155
Met Lys Ser Leu Arg Leu Met Leu Cys Ala Met Pro Leu Met Leu Thr
1 5 10 15

Gly Cys Ser Thr Met Ser Ser Val Asn Trp Ser Ala Ala Asn Pro Trp
20 25 30

Asn Trp Phe Gly Ser Ser Thr Lys Val Ser Glu Gln Gly Val Gly Glu
35 40 45

Leu Thr Ala Ser Thr Pro Leu Gln Glu Gln Ala Ile Ala Asp Ala Leu
50 55 60

Asp Gly Asp Tyr Arg Leu Arg Ser Gly Met Lys Thr Ala Asn Gly Asn
65 70 75 80

```
Val Val Arg Phe Phe Glu Val Met Lys Gly Asp Asn Val Ala Met Val
                85                  90                  95

Ile Asn Gly Asp Gln Gly Thr Ile Ser Arg Ile Asp Val Leu Asp Ser
            100                 105                 110

Asp Ile Pro Ala Asp Thr Gly Val Lys Ile Gly Thr Pro Phe Ser Asp
        115                 120                 125

Leu Tyr Ser Lys Ala Phe Gly Asn Cys Gln Lys Ala Asp Ser Asp Asp
    130                 135                 140

Asn Arg Ala Val Glu Cys Lys Ala Glu Gly Ser Gln His Ile Ser Tyr
145                 150                 155                 160

Gln Phe Ser Gly Glu Trp Ser Gly Pro Glu Gly Leu Met Pro Ser Asp
                165                 170                 175

Asp Thr Leu Lys Asn Trp Lys Val Ser Lys Ile Ile Trp Arg Arg
            180                 185                 190

<210>   156
<211>   230
<212>   PRT
<213>   Escherichia coli

<400>   156
Met Ala Ala Gln Ala Asn Glu Leu Pro Asp Gly Pro His Ile Val Thr
1               5                   10                  15

Ser Gly Thr Ala Ser Val Asp Ala Val Pro Asp Ile Ala Thr Leu Ala
            20                  25                  30

Ile Glu Val Asn Val Ala Ala Lys Asp Ala Ala Thr Ala Lys Lys Gln
        35                  40                  45

Ala Asp Glu Arg Val Ala Gln Tyr Ile Ser Phe Leu Glu Leu Asn Gln
    50                  55                  60

Ile Ala Lys Lys Asp Ile Ser Ser Ala Asn Leu Arg Thr Gln Pro Asp
65                  70                  75                  80

Tyr Asp Tyr Gln Asp Gly Lys Ser Ile Leu Lys Gly Tyr Arg Ala Val
                85                  90                  95

Arg Thr Val Glu Val Thr Leu Arg Gln Leu Asp Lys Leu Asn Ser Leu
            100                 105                 110

Leu Asp Gly Ala Leu Lys Ala Gly Leu Asn Glu Ile Arg Ser Val Ser
        115                 120                 125

Leu Gly Val Ala Gln Pro Asp Ala Tyr Lys Asp Lys Ala Arg Lys Ala
    130                 135                 140

Ala Ile Asp Asn Ala Ile His Gln Ala Gln Glu Leu Ala Asn Gly Phe
145                 150                 155                 160

His Arg Lys Leu Gly Pro Val Tyr Ser Val Arg Tyr His Val Ser Asn
                165                 170                 175

Tyr Gln Pro Ser Pro Met Val Arg Met Met Lys Ala Asp Ala Ala Pro
            180                 185                 190

Val Ser Ala Gln Glu Thr Tyr Glu Gln Ala Ala Ile Gln Phe Asp Asp
        195                 200                 205
```

Gln Val Asp Val Val Phe Gln Leu Glu Pro Val Asp Gln Pro Pro Ala
    210                 215                 220

Lys Thr Pro Ala Ala His
225                 230

<210> 157
<211> 130
<212> PRT
<213> Escherichia coli

<400> 157
Met Lys Lys Phe Ala Ala Val Ile Ala Val Met Ala Leu Cys Ser Ala
1               5                   10                  15

Pro Val Met Ala Ala Glu Gln Gly Gly Phe Ser Gly Pro Ser Ala Thr
            20                  25                  30

Gln Ser Gln Ala Gly Gly Phe Gln Gly Pro Asn Gly Ser Val Thr Thr
        35                  40                  45

Val Glu Ser Ala Lys Ser Leu Arg Asp Asp Thr Trp Val Thr Leu Arg
    50                  55                  60

Gly Asn Ile Val Glu Arg Ile Ser Asp Asp Leu Tyr Val Phe Lys Asp
65                  70                  75                  80

Ala Ser Gly Thr Ile Asn Val Asp Ile Asp His Lys Arg Trp Asn Gly
                85                  90                  95

Val Thr Val Thr Pro Lys Asp Thr Val Glu Ile Gln Gly Glu Val Asp
            100                 105                 110

Lys Asp Trp Asn Ser Val Glu Ile Asp Val Lys Gln Ile Arg Lys Val
        115                 120                 125

Asn Pro
    130

<210> 158
<211> 127
<212> PRT
<213> Escherichia coli

<400> 158
Met Arg His Arg Lys Ser Gly Arg Gln Leu Asn Arg Asn Ser Ser His
1               5                   10                  15

Arg Gln Ala Met Phe Arg Asn Met Ala Gly Ser Leu Val Arg His Glu
            20                  25                  30

Ile Ile Lys Thr Thr Leu Pro Lys Ala Lys Glu Leu Arg Arg Val Val
        35                  40                  45

Glu Pro Leu Ile Thr Leu Ala Lys Thr Asp Ser Val Ala Asn Arg Arg
    50                  55                  60

Leu Ala Phe Ala Arg Thr Arg Asp Asn Glu Ile Val Ala Lys Leu Phe
65                  70                  75                  80

Asn Glu Leu Gly Pro Arg Phe Ala Ser Arg Ala Gly Gly Tyr Thr Arg
                85                  90                  95

Ile Leu Lys Cys Gly Phe Arg Ala Gly Asp Asn Ala Pro Met Ala Tyr

100 105 110

Ile Glu Leu Val Asp Arg Ser Glu Lys Ala Glu Ala Ala Ala Glu
115 120 125

<210> 159
<211> 206
<212> PRT
<213> Escherichia coli

<400> 159
Met Ala Arg Tyr Leu Gly Pro Lys Leu Lys Leu Ser Arg Arg Glu Gly
1 5 10 15

Thr Asp Leu Phe Leu Lys Ser Gly Val Arg Ala Ile Asp Thr Lys Cys
20 25 30

Lys Ile Glu Gln Ala Pro Gly Gln His Gly Ala Arg Lys Pro Arg Leu
35 40 45

Ser Asp Tyr Gly Val Gln Leu Arg Glu Lys Gln Lys Val Arg Arg Ile
50 55 60

Tyr Gly Val Leu Glu Arg Gln Phe Arg Asn Tyr Tyr Lys Glu Ala Ala
65 70 75 80

Arg Leu Lys Gly Asn Thr Gly Glu Asn Leu Leu Ala Leu Leu Glu Gly
85 90 95

Arg Leu Asp Asn Val Val Tyr Arg Met Gly Phe Gly Ala Thr Arg Ala
100 105 110

Glu Ala Arg Gln Leu Val Ser His Lys Ala Ile Met Val Asn Gly Arg
115 120 125

Val Val Asn Ile Ala Ser Tyr Gln Val Ser Pro Asn Asp Val Val Ser
130 135 140

Ile Arg Glu Lys Ala Lys Lys Gln Ser Arg Val Lys Ala Ala Leu Glu
145 150 155 160

Leu Ala Glu Gln Arg Glu Lys Pro Thr Trp Leu Glu Val Asp Ala Gly
165 170 175

Lys Met Glu Gly Thr Phe Lys Arg Lys Pro Glu Arg Ser Asp Leu Ser
180 185 190

Ala Asp Ile Asn Glu His Leu Ile Val Glu Leu Tyr Ser Lys
195 200 205

<210> 160
<211> 188
<212> PRT
<213> Escherichia coli

<400> 160
Met Asn Met Thr Lys Gly Ala Leu Ile Leu Ser Leu Ser Phe Leu Leu
1 5 10 15

Ala Ala Cys Ser Ser Ile Pro Gln Asn Ile Lys Gly Asn Asn Gln Pro
20 25 30

Asp Ile Gln Lys Ser Phe Val Ala Val His Asn Gln Pro Gly Leu Tyr
35 40 45

Val Gly Gln Gln Ala Arg Phe Gly Gly Lys Val Ile Asn Val Ile Asn
    50                  55                  60

Gly Lys Thr Asp Thr Leu Leu Glu Ile Ala Val Leu Pro Leu Asp Ser
65                  70                  75                  80

Tyr Ala Lys Pro Asp Ile Glu Ala Asn Tyr Gln Gly Arg Leu Leu Ala
                85                  90                  95

Arg Gln Ser Gly Phe Leu Asp Pro Val Asn Tyr Arg Asn His Phe Val
            100                 105                 110

Thr Ile Leu Gly Thr Ile Gln Gly Glu Gln Pro Gly Phe Ile Asn Lys
        115                 120                 125

Val Pro Tyr Asn Phe Leu Glu Val Asn Met Gln Gly Ile Gln Val Trp
    130                 135                 140

His Leu Arg Glu Val Val Asn Thr Thr Tyr Asn Leu Trp Asp Tyr Gly
145                 150                 155                 160

Tyr Gly Ala Phe Trp Pro Glu Pro Gly Trp Gly Ala Pro Tyr Tyr Thr
                165                 170                 175

Asn Ala Val Ser Gln Val Thr Pro Glu Leu Val Lys
            180                 185

<210> 161
<211> 548
<212> PRT
<213> Escherichia coli

<400> 161
Met Ala Ala Lys Asp Val Lys Phe Gly Asn Asp Ala Arg Val Lys Met
1               5                   10                  15

Leu Arg Gly Val Asn Val Leu Ala Asp Ala Val Lys Val Thr Leu Gly
            20                  25                  30

Pro Lys Gly Arg Asn Val Val Leu Asp Lys Ser Phe Gly Ala Pro Thr
        35                  40                  45

Ile Thr Lys Asp Gly Val Ser Val Ala Arg Glu Ile Glu Leu Glu Asp
    50                  55                  60

Lys Phe Glu Asn Met Gly Ala Gln Met Val Lys Glu Val Ala Ser Lys
65                  70                  75                  80

Ala Asn Asp Ala Ala Gly Asp Gly Thr Thr Thr Ala Thr Val Leu Ala
                85                  90                  95

Gln Ala Ile Ile Thr Glu Gly Leu Lys Ala Val Ala Ala Gly Met Asn
            100                 105                 110

Pro Met Asp Leu Lys Arg Gly Ile Asp Lys Ala Val Ala Ala Ala Val
        115                 120                 125

Glu Glu Leu Lys Thr Leu Ser Val Pro Cys Ser Asp Ser Lys Ala Ile
    130                 135                 140

Ala Gln Val Gly Thr Ile Ser Ala Asn Ser Asp Glu Thr Val Gly Lys
145                 150                 155                 160

Leu Ile Ala Glu Ala Met Asp Lys Val Gly Lys Glu Gly Val Ile Thr
                165                 170                 175

```
Val Glu Asp Gly Thr Gly Leu Gln Asp Glu Leu Asp Val Val Glu Gly
            180                 185                 190

Met Gln Phe Asp Arg Gly Tyr Leu Ser Pro Tyr Phe Ile Asn Lys Pro
        195                 200                 205

Glu Thr Gly Ala Val Glu Leu Glu Ser Pro Phe Ile Leu Leu Ala Asp
    210                 215                 220

Lys Lys Ile Ser Asn Ile Arg Glu Met Leu Pro Val Leu Glu Ala Val
225                 230                 235                 240

Ala Lys Ala Gly Lys Pro Leu Leu Ile Ile Ala Glu Asp Val Glu Gly
                245                 250                 255

Glu Ala Leu Ala Thr Leu Val Val Asn Thr Met Arg Gly Ile Val Lys
            260                 265                 270

Val Ala Ala Val Lys Ala Pro Gly Phe Gly Asp Arg Arg Lys Ala Met
        275                 280                 285

Leu Gln Asp Ile Ala Thr Leu Thr Gly Gly Thr Val Ile Ser Glu Glu
    290                 295                 300

Ile Gly Met Glu Leu Glu Lys Ala Thr Leu Glu Asp Leu Gly Gln Ala
305                 310                 315                 320

Lys Arg Val Val Ile Asn Lys Asp Thr Thr Thr Ile Ile Asp Gly Val
                325                 330                 335

Gly Glu Glu Ala Ala Ile Gln Gly Arg Val Ala Gln Ile Arg Gln Gln
            340                 345                 350

Ile Glu Glu Ala Thr Ser Asp Tyr Asp Arg Glu Lys Leu Gln Glu Arg
        355                 360                 365

Val Ala Lys Leu Ala Gly Gly Val Ala Val Ile Lys Val Gly Ala Ala
    370                 375                 380

Thr Glu Val Glu Met Lys Glu Lys Lys Ala Arg Val Glu Asp Ala Leu
385                 390                 395                 400

His Ala Thr Arg Ala Ala Val Glu Glu Gly Val Val Ala Gly Gly Gly
                405                 410                 415

Val Ala Leu Ile Arg Val Ala Ser Lys Leu Ala Asp Leu Arg Gly Gln
            420                 425                 430

Asn Glu Asp Gln Asn Val Gly Ile Lys Val Ala Leu Arg Ala Met Glu
        435                 440                 445

Ala Pro Leu Arg Gln Ile Val Leu Asn Cys Gly Glu Glu Pro Ser Val
    450                 455                 460

Val Ala Asn Thr Val Lys Gly Gly Asp Gly Asn Tyr Gly Tyr Asn Ala
465                 470                 475                 480

Ala Thr Glu Glu Tyr Gly Asn Met Ile Asp Met Gly Ile Leu Asp Pro
                485                 490                 495

Thr Lys Val Thr Arg Ser Ala Leu Gln Tyr Ala Ala Ser Val Ala Gly
            500                 505                 510

Leu Met Ile Thr Thr Glu Cys Met Val Thr Asp Leu Pro Lys Asn Asp
```

515 520 525

Ala Ala Asp Leu Gly Ala Ala Gly Gly Met Gly Gly Met Gly Gly Met
530 535 540

Gly Gly Met Met
545

<210> 162
<211> 201
<212> PRT
<213> Escherichia coli

<400> 162
Met Thr Met Thr Arg Leu Lys Ile Ser Lys Thr Leu Leu Ala Val Met
1 5 10 15

Leu Thr Ser Ala Val Ala Thr Gly Ser Ala Tyr Ala Glu Asn Asn Ala
20 25 30

Gln Thr Thr Asn Glu Ser Ala Gly Gln Lys Val Asp Ser Ser Met Asn
35 40 45

Lys Val Gly Asn Phe Met Asp Asp Ser Ala Ile Thr Ala Lys Val Lys
50 55 60

Ala Ala Leu Val Asp His Asp Asn Ile Lys Ser Thr Asp Ile Ser Val
65 70 75 80

Lys Thr Asp Gln Lys Val Val Thr Leu Ser Gly Phe Val Glu Ser Gln
85 90 95

Ala Gln Ala Glu Glu Ala Val Lys Val Ala Lys Gly Val Glu Gly Val
100 105 110

Thr Ser Val Ser Asp Lys Leu His Val Arg Asp Ala Lys Glu Gly Ser
115 120 125

Val Lys Gly Tyr Ala Gly Asp Thr Ala Thr Thr Ser Glu Ile Lys Ala
130 135 140

Lys Leu Leu Ala Asp Asp Ile Val Pro Ser Arg His Val Lys Val Glu
145 150 155 160

Thr Thr Asp Gly Val Val Gln Leu Ser Gly Thr Val Asp Ser Gln Ala
165 170 175

Gln Ser Asp Arg Ala Glu Ser Ile Ala Lys Ala Val Asp Gly Val Lys
180 185 190

Ser Val Lys Asn Asp Leu Lys Thr Lys
195 200

<210> 163
<211> 749
<212> PRT
<213> Escherichia coli

<400> 163
Met Asn Lys Lys Ile His Ser Leu Ala Leu Leu Val Asn Leu Gly Ile
1 5 10 15

Tyr Gly Val Ala Leu Pro Val Leu Ala Glu Glu Thr Ser Asp Asn Ala
20 25 30

```
Val Ser His Asp Asp Thr Ile Val Val Thr Ala Ala Glu Gln Asn Leu
        35                  40                  45

Gln Ala Pro Gly Val Ser Thr Ile Thr Ala Asp Glu Ile Arg Lys Asn
    50                  55                  60

Pro Val Ala Arg Asp Val Ser Glu Ile Ile Arg Thr Met Pro Gly Val
65                  70                  75                  80

Asn Leu Thr Gly Asn Ser Thr Ser Gly Gln Arg Gly Asn Asn Arg Gln
                85                  90                  95

Ile Asp Ile Arg Gly Met Gly Pro Glu Asn Thr Leu Ile Leu Ile Asp
            100                 105                 110

Gly Lys Pro Val Ser Ser Arg Asn Ser Val Arg Gln Gly Trp Arg Gly
        115                 120                 125

Glu Arg Asp Thr Arg Gly Asp Thr Ser Trp Val Pro Pro Glu Met Ile
    130                 135                 140

Glu Arg Ile Glu Val Leu Arg Gly Pro Ala Ala Ala Arg Tyr Gly Asn
145                 150                 155                 160

Gly Ala Ala Gly Gly Val Val Asn Ile Ile Thr Lys Lys Gly Ser Gly
                165                 170                 175

Glu Trp His Gly Ser Trp Asp Ala Tyr Phe Asn Ala Pro Glu His Lys
            180                 185                 190

Glu Glu Gly Ala Thr Lys Arg Thr Asn Phe Ser Leu Thr Gly Pro Leu
        195                 200                 205

Gly Asp Glu Phe Ser Phe Arg Leu Tyr Gly Asn Leu Asp Lys Thr Gln
    210                 215                 220

Ala Asp Ala Trp Asp Ile Asn Gln Gly His Gln Ser Ala Arg Ala Gly
225                 230                 235                 240

Thr Tyr Ala Thr Thr Leu Pro Ala Gly Arg Glu Gly Val Ile Asn Lys
                245                 250                 255

Asp Ile Asn Gly Val Val Arg Trp Asp Phe Ala Pro Leu Gln Ser Leu
            260                 265                 270

Glu Leu Glu Ala Gly Tyr Ser Arg Gln Gly Asn Leu Tyr Ala Gly Asp
        275                 280                 285

Thr Gln Asn Thr Asn Ser Asp Ala Tyr Thr Arg Ser Lys Tyr Gly Asp
    290                 295                 300

Glu Thr Asn Arg Leu Tyr Arg Gln Asn Tyr Ser Leu Thr Trp Asn Gly
305                 310                 315                 320

Gly Trp Asp Asn Gly Val Thr Thr Ser Asn Trp Val Gln Tyr Glu His
                325                 330                 335

Thr Arg Asn Ser Arg Ile Pro Glu Gly Leu Ala Gly Gly Thr Glu Gly
            340                 345                 350

Lys Phe Asn Glu Lys Ala Ala Gln Asp Phe Val Asp Ile Asp Leu Asp
        355                 360                 365

Asp Val Met Leu His Ser Glu Val Asn Leu Pro Ile Asp Phe Leu Val
    370                 375                 380
```

Asn Gln Thr Leu Thr Leu Gly Thr Glu Trp Asn Gln Gln Arg Met Lys
385 390 395 400

Asp Leu Ser Ser Asn Thr Gln Ala Leu Thr Gly Thr Asn Thr Gly Gly
405 410 415

Ala Ile Asp Gly Val Ser Ala Thr Asp Arg Ser Pro Tyr Ser Lys Ala
420 425 430

Glu Ile Phe Ser Leu Phe Ala Glu Asn Asn Met Glu Leu Thr Asp Ser
435 440 445

Thr Ile Val Thr Pro Gly Leu Arg Phe Asp His His Ser Ile Val Gly
450 455 460

Asn Asn Trp Ser Pro Ala Leu Asn Ile Ser Gln Ser Leu Gly Asp Asp
465 470 475 480

Phe Thr Leu Lys Met Gly Ile Ala Arg Ala Tyr Lys Ala Pro Ser Leu
485 490 495

Tyr Gln Thr Asn Pro Asn Tyr Ile Leu Tyr Ser Lys Gly Gln Gly Cys
500 505 510

Tyr Ala Ser Ala Gly Gly Cys Tyr Leu Gln Gly Asn Asp Asp Leu Lys
515 520 525

Ala Glu Thr Ser Ile Asn Lys Glu Ile Gly Leu Glu Phe Lys Arg Asp
530 535 540

Gly Trp Leu Ala Gly Val Thr Trp Phe Arg Asn Asp Tyr Arg Asn Lys
545 550 555 560

Ile Glu Ala Gly Tyr Val Ala Val Gly Gln Asn Ala Val Gly Thr Asp
565 570 575

Leu Tyr Gln Trp Asp Asn Val Pro Lys Ala Val Val Glu Gly Leu Glu
580 585 590

Gly Ser Leu Asn Val Pro Val Ser Glu Thr Val Met Trp Thr Asn Asn
595 600 605

Ile Thr Tyr Met Leu Lys Ser Glu Asn Lys Thr Thr Gly Asp Arg Leu
610 615 620

Ser Ile Ile Pro Glu Tyr Thr Leu Asn Ser Thr Leu Ser Trp Gln Ala
625 630 635 640

Arg Glu Asp Leu Ser Met Gln Thr Thr Phe Thr Trp Tyr Gly Lys Gln
645 650 655

Gln Pro Lys Lys Tyr Asn Tyr Lys Gly Gln Pro Ala Val Gly Pro Glu
660 665 670

Thr Lys Glu Ile Ser Pro Tyr Ser Ile Val Gly Leu Ser Ala Thr Trp
675 680 685

Asp Val Thr Lys Asn Val Ser Leu Thr Gly Gly Val Asp Asn Leu Phe
690 695 700

Asp Lys Arg Leu Trp Arg Ala Gly Asn Ala Gln Thr Thr Gly Asp Leu
705 710 715 720

Ala Gly Ala Asn Tyr Ile Ala Gly Ala Gly Ala Tyr Thr Tyr Asn Glu

725 730 735

Pro Gly Arg Thr Trp Tyr Met Ser Ile Asn Thr His Phe
740 745

<210> 164
<211> 350
<212> PRT
<213> Escherichia coli

<400> 164
Met Thr Asp Ile Ala Gln Leu Leu Gly Lys Asp Ala Asp Asn Leu Leu
1 5 10 15

Gln His Arg Cys Met Thr Ile Pro Ser Asp Gln Leu Tyr Leu Pro Gly
20 25 30

His Asp Tyr Val Asp Arg Val Met Ile Asp Asn Asn Arg Pro Pro Ala
35 40 45

Val Leu Arg Asn Met Gln Thr Leu Tyr Asn Thr Gly Arg Leu Ala Gly
50 55 60

Thr Gly Tyr Leu Ser Ile Leu Pro Val Asp Gln Gly Val Glu His Ser
65 70 75 80

Ala Gly Ala Ser Phe Ala Ala Asn Pro Leu Tyr Phe Asp Pro Lys Asn
85 90 95

Ile Val Glu Leu Ala Ile Glu Ala Gly Cys Asn Cys Val Ala Ser Thr
100 105 110

Tyr Gly Val Leu Ala Ser Val Ser Arg Arg Tyr Ala His Arg Ile Pro
115 120 125

Phe Leu Val Lys Leu Asn His Asn Glu Thr Leu Ser Tyr Pro Asn Thr
130 135 140

Tyr Asp Gln Thr Leu Tyr Ala Ser Val Glu Gln Ala Phe Asn Met Gly
145 150 155 160

Ala Val Ala Val Gly Ala Thr Ile Tyr Phe Gly Ser Glu Glu Ser Arg
165 170 175

Arg Gln Ile Glu Glu Ile Ser Ala Ala Phe Glu Arg Ala His Glu Leu
180 185 190

Gly Met Val Thr Val Leu Trp Ala Tyr Leu Arg Asn Ser Ala Phe Lys
195 200 205

Lys Asp Gly Val Asp Tyr His Val Ser Ala Asp Leu Thr Gly Gln Ala
210 215 220

Asn His Leu Ala Ala Thr Ile Gly Ala Asp Ile Val Lys Gln Lys Met
225 230 235 240

Ala Glu Asn Asn Gly Gly Tyr Lys Ala Ile Asn Tyr Gly Tyr Thr Asp
245 250 255

Asp Arg Val Tyr Ser Lys Leu Thr Ser Glu Asn Pro Ile Asp Leu Val
260 265 270

Arg Tyr Gln Leu Ala Asn Cys Tyr Met Gly Arg Ala Gly Leu Ile Asn
275 280 285

Ser Gly Gly Ala Ala Gly Gly Glu Thr Asp Leu Ser Asp Ala Val Arg
290 295 300

Thr Ala Val Ile Asn Lys Arg Ala Gly Gly Met Gly Leu Ile Leu Gly
305 310 315 320

Arg Lys Ala Phe Lys Lys Ser Met Ala Asp Gly Val Lys Leu Ile Asn
325 330 335

Ala Val Gln Asp Val Tyr Leu Asp Ser Lys Ile Thr Ile Ala
340 345 350

<210> 165
<211> 714
<212> PRT
<213> Escherichia coli

<400> 165
Val Ser Arg Ile Ile Met Leu Ile Pro Thr Gly Thr Ser Val Gly Leu
1 5 10 15

Thr Ser Val Ser Leu Gly Val Ile Arg Ala Met Glu Arg Lys Gly Val
20 25 30

Arg Leu Ser Val Phe Lys Pro Ile Ala Gln Pro Arg Thr Gly Gly Asp
35 40 45

Ala Pro Asp Gln Thr Thr Thr Ile Val Arg Ala Asn Ser Ser Thr Thr
50 55 60

Thr Ala Ala Glu Pro Leu Lys Met Ser Tyr Val Glu Gly Leu Leu Ser
65 70 75 80

Ser Asn Gln Lys Asp Val Leu Met Glu Glu Ile Ile Ala Asn Tyr His
85 90 95

Ala Asn Thr Lys Asp Ala Glu Val Val Leu Val Glu Gly Leu Val Pro
100 105 110

Thr Arg Lys His Gln Phe Ala Gln Ser Leu Asn Tyr Glu Ile Ala Lys
115 120 125

Thr Leu Asn Ala Glu Ile Val Phe Val Met Ser Gln Gly Thr Asp Thr
130 135 140

Pro Glu Gln Leu Lys Glu Arg Ile Glu Leu Thr Arg Asn Ser Phe Gly
145 150 155 160

Gly Ala Lys Asn Thr Asn Ile Thr Gly Val Ile Val Asn Lys Leu Asn
165 170 175

Ala Pro Val Asp Glu Gln Gly Arg Thr Arg Pro Asp Leu Ser Glu Ile
180 185 190

Phe Asp Asp Ser Thr Lys Ala Lys Val Asn Asn Val Asp Pro Ala Lys
195 200 205

Leu Gln Glu Ser Ser Pro Leu Pro Val Leu Gly Ala Val Pro Trp Ser
210 215 220

Phe Asp Leu Ile Ala Thr Arg Ala Ile Asp Met Ala Arg His Leu Asn
225 230 235 240

Ala Thr Ile Ile Asn Glu Gly Asp Ile Asn Thr Arg Arg Val Lys Ser
245 250 255

Val Thr Phe Cys Ala Arg Ser Ile Pro His Met Leu Glu His Phe Arg
260 265 270

Ala Gly Ser Leu Leu Val Thr Ser Ala Asp Arg Pro Asp Val Leu Val
275 280 285

Ala Ala Cys Leu Ala Ala Met Asn Gly Val Glu Ile Gly Ala Leu Leu
290 295 300

Leu Thr Gly Gly Tyr Glu Met Asp Ala Arg Ile Ser Lys Leu Cys Glu
305 310 315 320

Arg Ala Phe Ala Thr Gly Leu Pro Val Phe Met Val Asn Thr Asn Thr
325 330 335

Trp Gln Thr Ser Leu Ser Leu Gln Ser Phe Asn Leu Glu Val Pro Val
340 345 350

Asp Asp His Glu Arg Ile Glu Lys Val Gln Glu Tyr Val Ala Asn Tyr
355 360 365

Ile Asn Ala Asp Trp Ile Asp Ser Leu Thr Ala Thr Ser Glu Arg Ser
370 375 380

Arg Arg Leu Ser Pro Pro Ala Phe Arg Tyr Gln Leu Thr Glu Leu Ala
385 390 395 400

Arg Lys Ala Gly Lys Arg Ile Val Leu Pro Glu Gly Asp Glu Pro Arg
405 410 415

Thr Val Lys Ala Ala Ala Ile Cys Ala Glu Arg Gly Ile Ala Thr Cys
420 425 430

Val Leu Leu Gly Asn Pro Ala Glu Ile Asn Arg Val Ala Ala Ser Gln
435 440 445

Gly Val Glu Leu Gly Ala Gly Ile Glu Ile Val Asp Pro Glu Val Val
450 455 460

Arg Glu Asn Tyr Val Gly Arg Leu Val Glu Leu Arg Lys Asn Lys Gly
465 470 475 480

Met Thr Glu Thr Val Ala Arg Glu Gln Leu Glu Asp Asn Val Val Leu
485 490 495

Gly Thr Leu Met Leu Glu Gln Asp Glu Val Asp Gly Leu Val Ser Gly
500 505 510

Ala Val His Thr Thr Ala Asn Thr Ile Arg Pro Pro Leu Gln Leu Ile
515 520 525

Lys Thr Ala Pro Gly Ser Ser Leu Val Ser Ser Val Phe Phe Met Leu
530 535 540

Leu Pro Glu Gln Val Tyr Val Tyr Gly Asp Cys Ala Ile Asn Pro Asp
545 550 555 560

Pro Thr Ala Glu Gln Leu Ala Glu Ile Ala Ile Gln Ser Ala Asp Ser
565 570 575

Ala Ala Ala Phe Gly Ile Glu Pro Arg Val Ala Met Leu Ser Tyr Ser
580 585 590

Thr Gly Thr Ser Gly Ala Gly Ser Asp Val Glu Lys Val Arg Glu Ala

595 600 605

Thr Arg Leu Ala Gln Glu Lys Arg Pro Asp Leu Met Ile Asp Gly Pro
610 615 620

Leu Gln Tyr Asp Ala Ala Val Ile Ala Asp Val Ala Lys Ser Lys Ala
625 630 635 640

Pro Asn Ser Pro Val Ala Gly Arg Ala Thr Val Phe Ile Phe Pro Asp
645 650 655

Leu Asn Thr Gly Asn Thr Thr Tyr Lys Ala Val Gln Arg Ser Ala Asp
660 665 670

Leu Ile Ser Ile Gly Pro Met Leu Gln Gly Met Arg Lys Pro Val Asn
675 680 685

Asp Leu Ser Arg Gly Ala Leu Val Asp Asp Ile Val Tyr Thr Ile Ala
690 695 700

Leu Thr Ala Ile Gln Ser Ala Gln Gln Gln
705 710

<210> 166
<211> 195
<212> PRT
<213> Escherichia coli

<400> 166
Met Arg Leu Gln Phe Ser Ala Leu Phe Phe Phe Leu Tyr Cys Val Phe
1 5 10 15

Ile Arg Gly Val Phe Ala Asp Pro Phe Pro Pro Pro Gly Met Ser Leu
20 25 30

Pro Glu Tyr Trp Gly Glu Glu His Val Trp Trp Asp Gly Arg Ala Ala
35 40 45

Phe His Gly Glu Val Val Arg Pro Ala Cys Thr Leu Ala Met Glu Asp
50 55 60

Ala Trp Gln Ile Ile Asp Met Gly Glu Thr Pro Val Arg Asp Leu Gln
65 70 75 80

Asn Gly Phe Ser Gly Pro Glu Arg Lys Phe Ser Leu Arg Leu Arg Asn
85 90 95

Cys Glu Phe Asn Ser Gln Gly Gly Asn Leu Phe Ser Asp Ser Arg Ile
100 105 110

Arg Val Thr Phe Asp Gly Val Arg Gly Glu Thr Pro Asp Lys Phe Asn
115 120 125

Leu Ser Gly Gln Ala Lys Gly Ile Asn Leu Gln Ile Ala Asp Ala Arg
130 135 140

Gly Asn Ile Ala Arg Ala Gly Lys Val Met Pro Ala Ile Pro Leu Thr
145 150 155 160

Gly Asn Glu Glu Ala Leu Asp Tyr Thr Leu Arg Ile Val Arg Asn Gly
165 170 175

Lys Lys Leu Glu Ala Gly Asn Tyr Phe Ala Val Leu Gly Phe Arg Val
180 185 190

Asp Tyr Glu
        195

<210> 167
<211> 170
<212> PRT
<213> Escherichia coli

<400> 167
Met Asn Met Asn Asn Pro Leu Glu Val Leu Gly His Val Ser Trp Leu
1               5                   10                  15

Trp Ala Ser Ser Pro Leu His Arg Asn Trp Pro Val Ser Leu Phe Ala
            20                  25                  30

Ile Asn Val Leu Pro Ala Ile Arg Ala Asn Gln Tyr Ala Leu Leu Thr
        35                  40                  45

Arg Asp Asn Tyr Pro Val Ala Tyr Cys Ser Trp Ala Asn Leu Ser Leu
    50                  55                  60

Glu Asn Glu Ile Lys Tyr Leu Asn Asp Val Thr Ser Leu Val Ala Glu
65                  70                  75                  80

Asp Trp Thr Ser Gly Asp Arg Lys Trp Phe Ile Asp Trp Ile Ala Pro
                85                  90                  95

Phe Gly Asp Asn Gly Ala Leu Tyr Lys Tyr Met Arg Lys Lys Phe Pro
            100                 105                 110

Asp Glu Leu Phe Arg Ala Ile Arg Val Asp Pro Lys Thr His Val Gly
        115                 120                 125

Lys Val Ser Glu Phe His Gly Gly Lys Ile Asp Lys Gln Leu Ala Asn
    130                 135                 140

Lys Ile Phe Lys Gln Tyr His His Glu Leu Ile Thr Glu Val Lys Asn
145                 150                 155                 160

Lys Ser Asp Phe Asn Phe Ser Leu Thr Gly
                165                 170

<210> 168
<211> 449
<212> PRT
<213> Escherichia coli

<400> 168
Ala Asp Ile Thr Glu Ile Glu Thr Thr Thr Gly Glu Lys Lys Asn Thr
1               5                   10                  15

Asn Val Thr Cys Pro Ala Asp Pro Gly Lys Leu Ser Pro Glu Glu Leu
            20                  25                  30

Lys Arg Leu Pro Ser Glu Cys Ser Ser Val Val Glu Gln Asn Leu Met
        35                  40                  45

Pro Trp Leu Val Thr Gly Ala Ala Thr Ala Leu Ile Thr Thr Leu Ala
    50                  55                  60

Ile Val Glu Leu Asn Asp Asp Asp Asp His His Arg Asn Asn Ser Pro
65                  70                  75                  80

Leu Pro Pro Thr Pro Pro Asp Asp Asp Ser Asp Asp Thr Pro Val Pro
                85                  90                  95

```
Pro Thr Pro Gly Gly Asp Glu Ile Ile Pro Asp Asp Gly Pro Asp Asp
            100                 105                 110

Thr Pro Ala Pro Pro Lys Pro Ile Ala Phe Asn Asn Asp Val Thr Leu
        115                 120                 125

Asp Lys Thr Ala Lys Thr Leu Thr Ile Arg Asp Ser Val Phe Ser Tyr
    130                 135                 140

Thr Glu Asn Ala Asp Gly Thr Ile Ser Leu Gln Asp Ser Asn Gly Arg
145                 150                 155                 160

Lys Ala Thr Ile Asn Leu Trp Gln Ile Asp Glu Thr Asn Asn Thr Val
                165                 170                 175

Ala Leu Glu Gly Met Ser Ala Asp Gly Ala Thr Lys Trp Gln Tyr Asn
            180                 185                 190

His Asn Gly Glu Leu Val Ile Thr Gly Asp Asn Thr Thr Val Asn Asn
        195                 200                 205

Thr Gly Lys Thr Ile Val Asp Gly Lys Gly Ala Thr Gly Thr Glu Ile
    210                 215                 220

Ala Gly Asn Asn Ala Val Val Asn Gln Asp Gly Glu Leu Asp Val Ser
225                 230                 235                 240

Gly Gly Gly His Gly Ile Asp Ile Thr Gly Asp Ser Ala Thr Val Asp
                245                 250                 255

Asn Lys Gly Gly Met Thr Val Thr Asp Pro Asp Ser Ile Gly Ile Gln
            260                 265                 270

Ile Asp Gly Asp Lys Ala Val Val Asn Asn Asp Gly Asp Asn Ala Ile
        275                 280                 285

Ser Asn Gly Gly Thr Gly Thr Gln Val Asn Gly Asp Glu Ala Thr Val
    290                 295                 300

Asn Asn Asn Gly Ser Thr Thr Val Asp Gly Lys Asp Ser Thr Gly Thr
305                 310                 315                 320

Glu Ile Asn Gly Asp Lys Ala Ile Val Asn Asn Asp Gly Asp Ser Thr
                325                 330                 335

Ile Leu Asp Gly Gly Thr Glu Thr Arg Ile Thr Gly Asp Asp Ala Thr
            340                 345                 350

Ala Asn Asn Ser Gly Asn Thr Thr Val Asp Gly Gln Gly Ser Thr Gly
        355                 360                 365

Thr Glu Ile Ala Gly Asn Asn Ala Val Val Asn Gln Asp Gly Glu Leu
    370                 375                 380

Asp Val Ser Gly Gly Gly His Gly Ile Asp Ile Thr Gly Asp Ser Ala
385                 390                 395                 400

Thr Val Asp Asn Lys Gly Gly Met Thr Val Ile Asp Pro Asp Ser Ile
                405                 410                 415

Gly Ile Gln Ile Asp Gly Asp Lys Ala Val Val Asn Asn Asp Gly Asp
            420                 425                 430

Asn Ala Ile Ser Asn Gly Gly Thr Gly Thr Gln Ile Asn Gly Asp Glu
```

435 440 445

Ala

## Description

All documents cited herein are incorporated by reference in their entirety.

## TECHNICAL FIELD

This invention is in the field of *Escherichia coli* biology, and in particular relates to immunogens for use in immunising against extraintestinal pathogenic *E. coli* (ExPEC) strains.

## BACKGROUND OF THE INVENTION

Few microorganisms are as versatile as *E. coli.* As well as being an important member of the normal intestinal microflora of mammals, it has been widely exploited as a host in recombinant DNA technology. In addition, however, *E. coli* can also be a deadly pathogen.

*E. coli* strains have traditionally been classified as either commensal or pathogenic, and pathogenic strains are then sub-classified as intestinal or extraintestinal strains. More recent taxonomic techniques such as multilocus enzyme electrophoresis (MLEE) classify *E. coli* into five phylogenetic groups (A, B1, B2, D & E), and these groupings do not match the traditional ones. For instance, MLEE group B1 includes both commensal and pathogenic strains, and group D includes both intestinal and extraintestinal strains.

The extraintestinal pathogenic strains (or 'ExPEC' strains [1]) of *E. coli* fall into MLEE groups B2 and D, and include both uropathogenic (UPEC) strains and meningitis/sepsis-associated (MNEC) strains. UPEC strains cause urinary tract infections (UTIs), and are the most common form of cystitis. They also cause pyelonephritis (and its complications such as sepsis) and catheter-associated infections. MNEC strains cause neonatal meningitis (0.1 cases per 1000 live births) with case fatality rates ranging from 25 to 40%, and are also responsible for around 1/6 of sepsis cases.

Most previous ExPEC vaccines have been based on cell lysates or on cellular structures. SOLCOUROVAC™ includes ten different heat-killed bacteria including six ExPEC strains, and a successful phase II clinical trial was reported in reference 2. URO-VAXOM™ is an oral tablet vaccine containing lyophilised bacterial lysates of 18 selected *E. coli* strains [3]. Baxter Vaccines developed a UTI vaccine based on pili from 6 to 10 different strains, but this product has been abandoned. MedImmune developed a product called MEDI 516 based on the FimH adhesin complex [4], but phase II clinical trials shows inadequate efficacy. Moreover, there was a risk with this vaccine that it would also affect non-pathogenic FimH$^{+ve}$ strains in the normal intestinal flora, and it was expected that this vaccine would be effective against UPEC strains only, because of its bladder-specific adherence mechanism, leaving other ExPEC strains uncontrolled.

There is thus a need for improved ExPEC vaccines, including a need to move away from crude cell lysates and towards better-defined molecules, and a need to identify further antigens that are suitable for inclusion in vaccines, particularly antigens that are prevalent among clinical ExPEC strains without also being found in commensal strains.

One way of addressing these needs was reported in reference 5, where the inventors looked for genes present in genomes of MLEE types B2 and D but absent from MLEE types A and B 1. Further comparative approaches, based on subtractive hybridisation, were reported in references 6 and 7. Virulence genes in ExPEC strains have also been identified in reference 8. Reference 9 discloses an analysis of four pathogenicity islands in UPEC *E. coli* strain 536.

Reference 10 used the genome sequence of UPEC (O6:K2:H1) strain CFT073 [11,12] to identify sequences not present in non-pathogenic *E. coli* strains. Reference 13 discloses a comparison of the genome sequence of *E. coli* human pyelonephritis isolate 536 (O6:K15:H31), an UPEC, with sequence data for strains CFT073 (UPEC), EDL933 (enterohemorrhagic) and MG1655 (non-pathogenic laboratory strain). Genome sequences of pathogenic strains are available in the databases under accession numbers AE005174 (gi:56384585), BA000007 (gi:47118301) and NC-004431 (gi:26245917). A sequence from a non-pathogenic strain is available under accession number U00096 (gi:48994873).

It is an object of the invention to provide further antigens for use in immunisation against pathogenic *E. coli* strains, particularly ExPEC strains, and more particularly UPEC strains.

## SUMMARY OF THE INVENTION

The inventors have identified various genes that can be included in immunogenic compositions specific for pathogenic *E. coli* strains. The genes are from uropathogenic strains (UPEC) but are absent from non-pathogenic strains, and their encoded proteins have cellular locations which render them accessible to the immune system.

In one aspect, the invention relates to a polypeptide comprising: (a) an amino acid sequence selected from the group consisting of SEQ ID NOs 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114,

115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166 and 167; (b) an amino acid sequence having at least 80% sequence identity to an amino acid sequence of (a); (c) an amino acid sequence which is a fragment of at least 10 consecutive amino acids from an amino acid sequence of (a); or (d) an amino acid sequence having at least 80% sequence identity to an amino acid sequence of (a) and including a fragment of at least 10 consecutive amino acids from an amino acid sequence of (a). In a particular embodiment, polypeptides of this aspect of the invention comprise a fragment which comprises at least one B-cell epitope of (a).

In another aspect, the invention relates to a polypeptide comprising: (a) an amino acid sequence selected from the group consisting of SEQ ID NOs 1, 2, 3, 4, 5, 6, and 7; (b) an amino acid sequence having at least 80% sequence identity to an amino acid sequence of (a); (c) an amino acid sequence which is a fragment of at least 10 consecutive amino acids from an amino acid sequence of (a); or (d) an amino acid sequence having at least 80% sequence identity to an amino acid sequence of (a) and including a fragment of at least 10 consecutive amino acids from an amino acid sequence of (a). In a particular embodiment, polypeptides of this aspect of the invention comprise a fragment which comprises at least one B-cell epitope of (a).

The present invention further relates to immunogenic compositions comprising one or more outer membrane vesicles (OMVs) expressing one or more polypeptides comprising: (a) an amino acid sequence selected from the group consisting of SEQ ID NOs 1, 2, 3, 4, 5, 6, and 7; (b) an amino acid sequence having at least 80% sequence identity to an amino acid sequence of (a); (c) an amino acid sequence which is a fragment of at least 10 consecutive amino acids from an amino acid sequence of (a); or (d) an amino acid sequence having at least 80% sequence identity to an amino acid sequence of (a) and including a fragment of at least 10 consecutive amino acids from an amino acid sequence of (a). In a particular embodiment, the immunogenic composition of this aspect of the invention comprises one or more polypeptides comprising a fragment which comprises at least one B-cell epitope of (a).

The polypeptides of the invention can be used in medicine and in the manufacture of a medicament for raising an immune response in a patient.

The present invention also relates to a pharmaceutical composition comprising a polypeptide of the invention in admixture with a pharmaceutically acceptable carrier. The invention further relates to a pharmaceutical composition comprising two or more polypeptides of the invention in admixture with a pharmaceutically acceptable carrier. In a particular embodiment, the pharmaceutical compositions of the invention further comprise a vaccine adjuvant.

The present invention also relates to methods for raising an immune response in a patient, comprising administering to the patient a pharmaceutical composition or immunogenic composition of the invention. In a particular embodiment, the immune response is protective against ExPEC infection.

Further aspects of the invention are described below.

## DETAILED DESCRIPTION OF THE INVENTION

The inventors have identified various and polypeptides that can be included in immunogenic compositions specific for pathogenic *E. coli* strains. The polypeptides have cellular locations which render them accessible to the immune system. The genes encoding the polypeptides were initially identified as being present in uropathogenic strain 536 but absent from non-pathogenic strains.

### *Polypeptides*

The invention provides polypeptides comprising the amino acid sequences disclosed in the examples. These amino acid sequences are given in the sequence listing as SEQ ID NOs 1 to 167. A preferred subset of SEQ ID NOs 1 to 167 is given in Table 2.

The invention also provides polypeptides comprising amino acid sequences that have sequence identity to the amino acid sequences disclosed in the examples (*i.e.* to SEQ ID NOs 1 to 168). Depending on the particular sequence, the degree of sequence identity is preferably greater than 50% (e.g. 60%, 70%, 75%, 80%, 85%, 90%, 91%, 92%, 93%, 94%, 95%, 96%, 97%, 98%, 99% or more). These polypeptides include homologs, orthologs, allelic variants and mutants. Typically, 50% identity or more between two polypeptide sequences is considered to be an indication of functional equivalence. Identity between polypeptides is preferably determined by the Smith-Waterman homology search algorithm as implemented in the MPSRCH program (Oxford Molecular), using an affine gap search with parameters *gap open penalty=12* and *gap extension penalty=1.*

These polypeptide may, compared to the sequences of the examples, include one or more (*e.g.* 1, 2, 3, 4, 5, 6, 7, 8, 9, 10, *etc.)* conservative amino acid replacements *i.e.* replacements of one amino acid with another which has a related side chain. Genetically-encoded amino acids are generally divided into four families: (1) acidic *i.e.* aspartate, glutamate; (2) basic *i.e.* lysine, arginine, histidine; (3) non-polar *i.e.* alanine, valine, leucine, isoleucine, proline, phenylalanine, methionine, tryptophan; and (4) uncharged polar *i.e.* glycine, asparagine, glutamine, cysteine, serine, threonine, tyrosine. Phenylalanine, tryptophan, and tyrosine are sometimes classified jointly as aromatic amino acids. In general, substitution of single amino acids within these families does not have a major effect on the biological activity. The polypeptides may have one or more (*e.g.* 1, 2, 3, 4, 5, 6, 7, 8, 9, 10, *etc.)* single amino acid deletions relative to a reference sequence. The polypeptides may also include one or more (*e.g.* 1, 2, 3, 4, 5, 6, 7, 8, 9, 10, *etc.*) insertions (*e.g.* each of 1, 2, 3, 4 or 5 amino acids) relative to a reference sequence.

Preferred polypeptides include polypeptides that are lipidated, that are located in the outer membrane, that are located in the inner membrane, or that are located in the periplasm. Particularly preferred polypeptides are those that fall into more than one of these categories *e.g.* lipidated polypeptides that are located in the outer membrane. Lipoproteins may have a N-terminal cysteine to which lipid is covalently attached, following post-translational processing of the signal peptide.

The invention further provides polypeptides comprising fragments of the amino acid sequences disclosed in the examples. The fragments should comprise at least *n* consecutive amino acids from the sequences and, depending on the particular sequence, *n* is 7 or more (*e.g.* 8, 10, 12, 14, 16, 18, 20, 22, 24, 26, 28, 30, 35, 40, 45, 50, 60, 70, 80, 90, 100 or more). The fragment may comprise at least one T-cell or, preferably, a B-cell epitope of the sequence. T- and B-cell epitopes can be identified empirically (*e.g.* using PEPSCAN [14,15] or similar methods), or they can be predicted (*e.g.* using the Jameson-Wolf antigenic index [16], matrix-based approaches [17], TEPITOPE [18], neural networks [19], OptiMer & EpiMer [20,21], ADEPT [22], Tsites [23], hydrophilicity [24], antigenic index [25] or the methods disclosed in reference 26, *etc.*).

Other preferred fragments are (a) the N-terminal signal peptides of the polypeptides of the invention, (b) the polypeptides, but without their N-terminal signal peptides, (c) the polypeptides, but without their N-terminal amino acid residue. Thus the invention further provides truncated sequences of the polypeptides of the invention. The sequences may be truncated at the N-terminus and/or the C-terminus. Truncation may involve a single amino acid or a longer sequence. A truncated sequence preferably retains at least one epitope of the pre-truncation sequence. For example, the invention provides truncated sequence, SEQ ID NO: 168 which is amino acids 21-470 of SEQ ID NO:56.

Other preferred fragments are those that are common to a polypeptide of the invention and to a polypeptide identified in any of references 5, 6, 8, 10 and 11.

Other preferred fragments are those that are common to a polypeptide of the invention and to a polypeptide identified in reference 27, reference 28, U.S. Provisional Application No. 60/654,632 (filed February 18, 2005; priority application for refs 27 & 28) or U.S. Provisional Application No. 60/712,720 (filed August 29, 2005; priority application for ref. 28).

The invention also provides polypeptides comprising amino acid sequences that have sequence identity to, and comprise fragments of, the amino acid sequences disclosed in the examples. Depending on the particular sequence, the degree of sequence identity is preferably greater than 50% (*e.g.* 60%, 70%, 75%, 80%, 85%, 90%, 91%, 92%, 93%, 94%, 95%, 96%, 97%, 98%, 99% or more), and the fragments should comprise at least *n* consecutive amino acids from the sequences and, depending on the particular sequence, *n* is 7 or more (*e.g.* 8, 10, 12, 14, 16, 18, 20, 22, 24, 26, 28, 30, 35, 40, 45, 50, 60, 70, 80, 90, 100 or more).

Polypeptides of the invention can be prepared in many ways *e.g.* by chemical synthesis (in whole or in part), by digesting longer polypeptides using proteases, by translation from RNA, by purification from cell culture (*e.g.* from recombinant expression), from the organism itself (*e.g.* after bacterial culture, or direct from patients), *etc.* A preferred method for production of peptides <40 amino acids long involves *in vitro* chemical synthesis [29,30]. Solid-phase peptide synthesis is particularly preferred, such as methods based on tBoc or Fmoc [31] chemistry. Enzymatic synthesis [32] may also be used in part or in full. As an alternative to chemical synthesis, biological synthesis may be used *e.g.* the polypeptides may be produced by translation. This may be carried out *in vitro* or *in vivo*. Biological methods are in general restricted to the production of polypeptides based on L-amino acids, but manipulation of translation machinery (*e.g.* of aminoacyl tRNA molecules) can be used to allow the introduction of D-amino acids (or of other non natural amino acids, such as iodotyrosine or methylphenylalanine, azidohomoalanine, etc.) [33]. Where D-amino acids are included, however, it is preferred to use chemical synthesis. Polypeptides of the invention may have covalent modifications at the C-terminus and/or N-terminus.

Polypeptides of the invention can take various forms (*e.g.* native, fusions, glycosylated, non-glycosylated, lipidated, non-lipidated, phosphorylated, non-phosphorylated, myristoylated, non-myristoylated, monomeric, multimeric, particulate, denatured, *etc.*).

Polypeptides of the invention are preferably provided in purified or substantially purified form *i.e.* substantially free from other polypeptides (*e.g.* free from naturally-occurring polypeptides), particularly from other ExPEC or host cell polypeptides, and are generally at least about 50% pure (by weight), and usually at least about 90% pure *i.e.* less than about 50%, and more preferably less than about 10% (*e.g.* 5% or less) of a composition is made up of other expressed polypeptides. Polypeptides of the invention are preferably ExPEC polypeptides.

Polypeptides of the invention may be attached to a solid support. Polypeptides of the invention may comprise

a detectable label (*e.g.* a radioactive or fluorescent label, or a biotin label).

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.*), as well as other modifications known in the art. Polypeptides can occur as single chains or associated chains. Polypeptides of the invention can be naturally or non-naturally glycosylated (*i.e.* the polypeptide has a glycosylation pattern that differs from the glycosylation pattern found in the corresponding naturally occurring polypeptide).

Polypeptides of the invention may be at least 40 amino acids long (*e.g.* at least 40, 50, 60, 70, 80, 90, 100, 120, 140, 160, 180, 200, 220, 240, 260, 280, 300, 350, 400, 450, 500 or more). Polypeptides of the invention may be shorter than 500 amino acids (*e.g.* no longer than 40, 50, 60, 70, 80, 90, 100, 120, 140, 160, 180, 200, 220, 240, 260, 280, 300, 350, 400 or 450 amino acids).

The invention provides polypeptides comprising a sequence -X-Y- or -Y-X-, wherein: -X- is an amino acid sequence as defined above and -Y- is not a sequence as defined above *i.e.* the invention provides fusion proteins. Where the N-terminus codon of a polypeptide-coding sequence is not ATG then that codon will be translated as the standard amino acid for that codon rather than as a Met, which occurs when the codon is translated as a start codon.

The invention provides a process for producing polypeptides of the invention, comprising culturing a host cell of to the invention under conditions which induce polypeptide expression.

The invention provides a process for producing a polypeptide of the invention, wherein the polypeptide is synthesised in part or in whole using chemical means.

The invention provides a composition comprising two or more polypeptides of the invention.

The invention also provides a hybrid polypeptide represented by the formula $NH_2$-A-[-X-L-]$_n$-B-COOH, wherein X is a polypeptide of the invention as defined above, L is an optional linker amino acid sequence, A is an optional N-terminal amino acid sequence, B is an optional C-terminal amino acid sequence, and *n* is an integer greater than 1. The value of *n* is between 2 and x, and the value of *x* is typically 3, 4, 5, 6, 7, 8, 9 or 10. Preferably *n* is 2, 3 or 4; it is more preferably 2 or 3; most preferably, $n$ = 2. For each *n* instances, -X- may be the same or different. For each *n* instances of [-X-L-], linker amino acid sequence -L- may be present or absent. For instance, when $n$=2 the hybrid may be $NH_2$-$X_1$-$L_1$-$X_2$-$L_2$-COOH, $NH_2$-$X_1$-$X_2$-COOH, $NH_2$-$X_1$-$L_1$-$X_2$-COOH, $NH_2$-$X_1$-$X_2$-$L_2$-COOH, *etc.* Linker amino acid sequence(s) -L- will typically be short (*e.g.* 20 or fewer amino acids *i.e.* 19, 18, 17, 16, 15, 14, 13, 12, 11, 10, 9, 8, 7, 6, 5, 4, 3, 2, 1). Examples include short peptide sequences which facilitate cloning, poly-glycine linkers (*i.e.* $Gly_n$ where $n$ = 2, 3, 4, 5, 6, 7, 8, 9, 10 or more), and histidine tags (*i.e.* $His_n$ where $n$ = 3, 4, 5, 6, 7, 8, 9, 10 or more). Other suitable linker amino acid sequences will be apparent to those skilled in the art. -A- and -B- are optional sequences which will typically be short (*e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct polypeptide trafficking, or short peptide sequences which facilitate cloning or purification (*e.g.* histidine tags *i.e.* $His_n$ where $n$ = 3, 4, 5, 6, 7, 8, 9, 10 or more). Other suitable N-terminal and C-terminal amino acid sequences will be apparent to those skilled in the art.

Various tests can be used to assess the *in vivo* immunogenicity of polypeptides of the invention. For example, polypeptides can be expressed recombinantly and used to screen patient sera by immunoblot. A positive reaction between the polypeptide and patient serum indicates that the patient has previously mounted an immune response to the protein in question *i.e.* the protein is an immunogen. This method can also be used to identify immunodominant proteins.

### *Antibodies*

The invention provides antibodies that bind to polypeptides of the invention. These may be polyclonal or monoclonal and may be produced by any suitable means (*e.g.* by recombinant expression). To increase compatibility with the human immune system, the antibodies may be chimeric or humanised [*e.g.* refs. 34 & 35], or fully human antibodies may be used. The antibodies may include a detectable label (*e.g.* for diagnostic assays). Antibodies of the invention may be attached to a solid support. Antibodies of the invention are preferably neutralising antibodies.

Monoclonal antibodies are particularly useful in identification and purification of the individual polypeptides against which they are directed. Monoclonal antibodies of the invention may also be employed as reagents in immunoassays, radioimmunoassays (RIA) or enzyme-linked immunosorbent assays (ELISA), *etc.* In these applications, the antibodies can be labelled with an analytically-detectable reagent such as a radioisotope, a fluorescent molecule or an enzyme. The monoclonal antibodies produced by the above method may also be used for the molecular identification and characterization (epitope mapping) of polypeptides of the invention.

Antibodies of the invention are preferably specific to ExPEC strains of *E. coli, i.e.* they bind preferentially to ExPEC *E. coli* relative to other bacteria (*e.g.* relative to non-ExPEC *E. coli* and relative to non-*E.coli* bacteria). More preferably, the antibodies are specific to UPEC strains *i.e.* they bind preferentially to UPEC bacteria relative to other bacteria, including other ExPEC *E. coli.*

Antibodies of the invention are preferably provided in purified or substantially purified form. Typically, the antibody will be present in a composition that is substantially free of other polypeptides *e.g.* where less than 90% (by weight), usually less than 60% and more usually less than 50% of the composition is made up of other polypeptides.

Antibodies of the invention can be of any isotype (*e.g.* IgA, IgG, IgM *i.e.* an $\alpha$, $\gamma$ or $\mu$ heavy chain), but will generally be IgG. Within the IgG isotype, antibodies may be IgG1, IgG2, IgG3 or IgG4 subclass. Antibodies of the invention may have a $\kappa$ or a $\lambda$ light chain.

Antibodies of the invention can take various forms, including whole antibodies, antibody fragments such as $F(ab')_2$ and F(ab) fragments, Fv fragments (non-covalent heterodimers), single-chain antibodies such as single chain Fv molecules (scFv), minibodies, oligobodies, *etc.* The term "antibody" does not imply any particular origin, and includes antibodies obtained through non-conventional processes, such as phage display.

The invention provides a process for detecting polypeptides of the invention, comprising the steps of: (a) contacting an antibody of the invention with a biological sample under conditions suitable for the formation of an antibody-antigen complexes; and (b) detecting said complexes.

The invention provides a process for detecting antibodies of the invention, comprising the steps of: (a) contacting a polypeptide of the invention with a biological sample (*e.g.* a blood or serum sample) under conditions suitable for the formation of an antibody-antigen complexes; and (b) detecting said complexes.

Preferred antibodies bind to a polypeptide of the invention with substantially greater affinity than antibodies known in the art. Preferably, the affinity is at least 1.5-fold, 2-fold, 5-fold 10-fold, 100-fold, $10^3$-fold, $10^4$-fold, $10^5$-fold, $10^6$-fold *etc.* stronger than antibodies known in the art.

### *Nucleic acids*

The invention also provides nucleic acid comprising a nucleotide sequence encoding the polypeptides of the invention. The invention also provides nucleic acid comprising nucleotide sequences having sequence identity to such nucleotide sequences. Identity between sequences is preferably determined by the Smith-Waterman homology search algorithm as described above. Such nucleic acids include those using alternative codons to encode the same amino acid.

The invention also provides nucleic acid which can hybridize to these nucleic acids. Hybridization reactions can be performed under conditions of different "stringency". Conditions that increase stringency of a hybridization reaction of widely known and published in the art [*e.g.* page 7.52 of reference 297]. Examples of relevant conditions include (in order of increasing stringency): incubation temperatures of 25°C, 37°C, 50°C, 55°C and 68°C; buffer concentrations of 10 x SSC, 6 x SSC, 1 x SSC, 0.1 x SSC (where SSC is 0.15 M NaCl and 15 mM citrate buffer) and their equivalents using other buffer systems; formamide concentrations of 0%, 25%, 50%, and 75%; incubation times from 5 minutes to 24 hours; 1, 2, or more washing steps; wash incubation times of 1, 2, or 15 minutes; and wash solutions of 6 x SSC, 1 x SSC, 0.1 x SSC, or de-ionized water. Hybridization techniques and their optimization are well known in the art [*e.g.* see refs 36, 37, 297, 299, *etc.*].

In some embodiments, nucleic acid of the invention hybridizes to a target under low stringency conditions; in other embodiments it hybridizes under intermediate stringency conditions; in preferred embodiments, it hybridizes under high stringency conditions. An exemplary set of low stringency hybridization conditions is 50°C and 10 x SSC. An exemplary set of intermediate stringency hybridization conditions is 55°C and 1 x SSC. An exemplary set of high stringency hybridization conditions is 68°C and 0.1 x SSC.

Nucleic acid comprising fragments of these sequences are also provided. These should comprise at least *n* consecutive nucleotides from the sequences and, depending on the particular sequence, *n* is 10 or more (*e.g.* 12, 14, 15, 18, 20, 25, 30, 35, 40, 50, 60, 70, 80, 90, 100, 150, 200 or more). Preferred fragments are those that are common to a nucleic acid sequence of the invention and to a nucleic acid sequence identified in any of references 5, 6, 8, 10 and 11.

The invention provides nucleic acid of formula 5'-X-Y-Z-3', wherein: -X- is a nucleotide sequence consisting of x nucleotides; -Z- is a nucleotide sequence consisting of *z* nucleotides; -Y- is a nucleotide sequence consisting of either (a) a fragment of a nucleic acid sequence encoding one of SEQ ID NOS: 1 to 168 or (b) the complement of (a); and said nucleic acid 5'-X-Y-Z-3' is neither (i) a fragment of either a nucleic acid sequence encoding one of SEQ ID NOS: 1 to 168 nor (ii) the complement of (i). The -X- and/or -Z- moieties may comprise a promoter sequence (or its complement).

The invention includes nucleic acid comprising sequences complementary to these sequences (*e.g.* for antisense or probing, or for use as primers).

Nucleic acids of the invention can be used in hybridisation reactions (*e.g.* Northern or Southern blots, or in nucleic acid microarrays or 'gene chips') and amplification reactions (*e.g.* PCR, SDA, SSSR, LCR, TMA, NASBA, *etc.)* and other nucleic acid techniques.

Nucleic acid according to the invention can take various forms (*e.g.* single-stranded, double-stranded, vectors, primers, probes, labelled *etc.*). Nucleic acids of the invention may be circular or branched, but will generally be linear. Unless otherwise specified or required, any embodiment of the invention that utilizes a nucleic acid may utilize both the double-stranded form and each of two complementary single-stranded forms which make up the double-stranded form. Primers and probes are generally single-stranded, as are antisense nucleic acids.

Nucleic acids of the invention are preferably provided in purified or substantially purified form *i.e.* substantially free from other nucleic acids (*e.g.* free from naturally-occurring nucleic acids), particularly from other ExPEC or host cell nucleic acids, generally being at least about 50% pure (by weight), and usually at least about 90% pure. Nucleic acids of the invention are preferably ExPEC nucleic acids.

Nucleic acids of the invention may be prepared in many ways *e.g.* by chemical synthesis *(e.g.* phosphoramidite synthesis of DNA) in whole or in part, by digesting longer nucleic acids using nucleases (*e.g.* restriction enzymes), by joining shorter nucleic acids or nucleotides (*e.g.* using ligases or polymerases), from genomic or cDNA libraries, etc.

Nucleic acid of the invention may be attached to a solid support (*e.g.* a bead, plate, filter, film, slide, microarray support, resin, *etc.*). Nucleic acid of the invention may be labelled *e.g.* with a radioactive or fluorescent label, or a biotin label. This is particularly useful where the nucleic acid is to be used in detection techniques e.g. where the nucleic acid is a primer or as a probe.

The term "nucleic acid" includes in general means a polymeric form of nucleotides of any length, which contain deoxyribonucleotides, ribonucleotides, and/or their analogs. It includes DNA, RNA, DNA/RNA hybrids. It also includes DNA or RNA analogs, such as those containing modified backbones (*e.g.* peptide nucleic acids (PNAs) or phosphorothioates) or modified bases. Thus the invention includes mRNA, tRNA, rRNA, ribozymes, DNA, cDNA, recombinant nucleic acids, branched nucleic acids, plasmids, vectors, probes, primers, *etc.*. Where nucleic acid of the invention takes the form of RNA, it may or may not have a 5' cap.

Nucleic acids of the invention comprise sequences, but they may also comprise non-ExPEC sequences (*e.g.* in nucleic acids of formula 5'-X-Y-Z-3', as defined above). This is particularly useful for primers, which may thus comprise a first sequence complementary to a nucleic acid target and a second sequence which is not complementary to the nucleic acid target. Any such non-complementary sequences in the primer are preferably 5' to the complementary sequences. Typical non-complementary sequences comprise restriction sites or promoter sequences.

Nucleic acids of the invention may be part of a vector *i.e.* part of a nucleic acid construct designed for transduction/transfection of one or more cell types. Vectors may be, for example, "cloning vectors" which are designed for isolation, propagation and replication of inserted nucleotides, "expression vectors" which are designed for expression of a nucleotide sequence in a host cell, "viral vectors" which is designed to result in the production of a recombinant virus or virus-like particle, or "shuttle vectors", which comprise the attributes of more than one type of vector. Preferred vectors are plasmids. A "host cell" includes an individual cell or cell culture which can be or has been a recipient of exogenous nucleic acid. Host cells include progeny of a single host cell, and the progeny may not necessarily be completely identical (in morphology or in total DNA complement) to the original parent cell due to natural, accidental, or deliberate mutation and/or change. Host cells include cells transfected or infected *in vivo* or *in vitro* with nucleic acid of the invention.

Where a nucleic acid is DNA, it will be appreciated that "U" in a RNA sequence will be replaced by "T" in the DNA. Similarly, where a nucleic acid is RNA, it will be appreciated that "T" in a DNA sequence will be replaced by "U" in the RNA.

The term "complement" or "complementary" when used in relation to nucleic acids refers to Watson-Crick base pairing. Thus the complement of C is G, the complement of G is C, the complement of A is T (or U), and the complement of T (or U) is A. It is also possible to use bases such as I (the purine inosine) *e.g.* to complement pyrimidines (C or T). The terms also imply a direction - the complement of 5'-ACAGT-3' is 5'-ACTGT-3' rather than 5'-TGTCA-3'.

Nucleic acids of the invention can be used, for example: to produce polypeptides; as hybridization probes for the detection of nucleic acid in biological samples; to generate additional copies of the nucleic acids; to generate ribozymes or antisense oligonucleotides; as single-stranded DNA primers or probes; or as triple-strand forming oligonucleotides.

The invention provides a process for producing nucleic acid of the invention, wherein the nucleic acid is synthesised in part or in whole using chemical means.

The invention provides vectors comprising nucleotide sequences of the invention (*e.g.* cloning or expression vectors) and host cells transformed with such vectors.

The invention also provides a kit comprising primers (*e.g.* PCR primers) for amplifying a template sequence contained within an ExPEC nucleic acid sequence, the kit comprising a first primer and a second primer, wherein the first primer is substantially complementary to said template sequence and the second primer is substantially complementary to a complement of said template sequence, wherein the parts of said primers which have substantial complementarity define the termini of the template sequence to be amplified. The first primer and/or the second primer may include a detectable label (*e.g.* a fluorescent label).

The invention also provides a kit comprising first and second single-stranded oligonucleotides which allow

amplification of a ExPEC template nucleic acid sequence contained in a single- or double-stranded nucleic acid (or mixture thereof), wherein: (a) the first oligonucleotide comprises a primer sequence which is substantially complementary to said template nucleic acid sequence; (b) the second oligonucleotide comprises a primer sequence which is substantially complementary to the complement of said template nucleic acid sequence; (c) the first oligonucleotide and/or the second oligonucleotide comprise(s) sequence which is not complementary to said template nucleic acid; and (d) said primer sequences define the termini of the template sequence to be amplified. The non-complementary sequence(s) of feature (c) are preferably upstream of *(i.e.* 5' to) the primer sequences. One or both of these (c) sequences may comprise a restriction site [e.g. ref. 38] or a promoter sequence [*e.g.* 39]. The first oligonucleotide and/or the second oligonucleotide may include a detectable label (*e.g.* a fluorescent label).

The invention provides a process for detecting nucleic acid of the invention, comprising: (a) contacting a nucleic probe according to the invention with a biological sample under hybridising conditions to form duplexes; and (b) detecting said duplexes.

The invention provides a process for detecting in a biological sample (*e.g.* blood), comprising contacting nucleic acid according to the invention with the biological sample under hybridising conditions. The process may involve nucleic acid amplification (*e.g.* PCR, SDA, SSSR, LCR, TMA, NASBA, *etc.*) or hybridisation (*e.g.* microarrays, blots, hybridisation with a probe in solution *etc.).* PCR detection of ExPEC in clinical samples has been reported [*e.g.* see ref. 40]. Clinical assays based on nucleic acid are described in general in ref. 41.

The invention provides a process for preparing a fragment of a target sequence, wherein the fragment is prepared by extension of a nucleic acid primer. The target sequence and/or the primer are nucleic acids of the invention. The primer extension reaction may involve nucleic acid amplification (e.g. PCR, SDA, SSSR, LCR, TMA, NASBA, *etc.*).

Nucleic acid amplification according to the invention may be quantitative and/or real-time.

For certain embodiments of the invention, nucleic acids are preferably at least 7 nucleotides in length (e.g. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 90, 100, 110, 120, 130, 140, 150, 160, 170, 180, 190, 200, 225, 250, 275, 300 nucleotides or longer).

For certain embodiments of the invention, nucleic acids are preferably at most 500 nucleotides in length (e.g. 450, 400, 350, 300, 250, 200, 150, 140, 130, 120, 110, 100, 90, 80, 75, 70, 65, 60, 55, 50, 45, 40, 39, 38, 37, 36, 35, 34, 33, 32, 31, 30, 29, 28, 27, 26, 25, 24, 23, 22, 21, 20, 19, 18, 17, 16, 15 nucleotides or shorter).

Primers and probes of the invention, and other nucleic acids used for hybridization, are preferably between 10 and 30 nucleotides in length (*e.g.* 10, 11, 12, 13, 14, 15, 16, 17, 18, 19, 20, 21, 22, 23, 24, 25, 26, 27, 28, 29, or 30 nucleotides).

***Vesicles***

Reference 42 describes the preparation of vesicles from a uropathogenic (UPEC) strain by the knockout of *mltA* (a murein lytic transglycosylase) or one or more of the components of the *E.coli* Tol-Pal complex [43], such as *tolA, tolQ, tolB, pal* and/or *tolR.* These vesicles can be improved by making one or more further genetic changes to the chromosome of the bacterium or through insertion of episomal elements (*e.g.* expression vectors) in order to increase the amount of and/or immunoaccessibility of protective antigens on the surface the vesicles.

One way of obtaining such improvements is to up-regulate the expression of the polypeptides of the invention. Many different genetic strategies for increasing the expression of a target protein are well-known in the art and can be distinguished into two broad categories: one relying on modifications of the chromosome (*e.g.* replacement of the wild-type promoter with a stronger promoter, inactivation of natural repressor genes, *etc.)* to increase expression of an endogenous gene, and the other based on recombinant expression by episomal elements (*e.g.* high-copy number plasmids, vectors harboring an engineered target gene, *etc.*) or integration of a exogenous gene in the chromosome. Practical examples for each of these approaches can be found in references 44 to 50.

Another way of increasing vesicle immunogenicity and selectivity is to down-regulate the expression of immunodominant non-protective antigens or to down-regulate proteins that are homologous to proteins found in commensal strains. Further improvements can be achieved by detoxification of the Lipid A moiety of LPS. Similar changes have been previously described to produce improved vesicles from other Gram-negative pathogens (see for example references 51 & 52).

All the above strategies can be used either alone or in combination to obtain improved vesicles for use in immunogenic compositions. The invention provides a pathogenic *Escherichia coli* bacterium (particularly a UPEC) having a knockout of *mltA* and/or of a component of its *Tol-Pal* complex, and one or more of: (i) a chromosomal gene encoding a polypeptide of the invention under the control of a promoter that provides higher expression levels of the polypeptide than the promoter that is naturally associated with the gene encoding the polypeptide; or (ii) an autonomously-replicating extrachromosomal element encoding a polypeptide of the invention, and optionally also (iii) a genetic modification to reduce the toxicity of the Lipid A moiety *of E. coli* LPS relative to wild-type LPS.

The invention also provides vesicles obtainable by culturing such a bacterium, such as the vesicles that, during culture of the bacterium, are released into the culture medium.

In a particular aspect, the invention provides immunogenic compositions comprising one or more outer membrane vesicles (OMVs) expressing one or more polypeptides of the invention. In a particular embodiment, the invention provides an immunogenic composition comprising one or more OMVs expressing one or more polypeptides comprising: (a) an amino acid sequence selected from the group consisting of SEQ ID NOs 1 to 168; (b) an amino acid sequence having at least 80% sequence identity to an amino acid sequence of (a); (c) an amino acid sequence which is a fragment of at least 10 consecutive amino acids from an amino acid sequence of (a); or (d) an amino acid sequence having at least 80% sequence identity to an amino acid sequence of (a) and including a fragment of at least 10 consecutive amino acids from an amino acid sequence of (a). In a further embodiment, the immunogenic composition comprises a polypeptide comprising a fragment which comprises at least one B-cell epitope of an amino acid sequence selected from the group consisting of SEQ ID NOs 1-168.

***Pharmaceutical compositions***

The invention provides compositions comprising: (a) polypeptide, antibody, vesicles and/or nucleic acid of the invention; and (b) a pharmaceutically acceptable carrier. These compositions may be suitable as immunogenic compositions, for instance, or as diagnostic reagents, or as vaccines. Vaccines according to the invention may either be prophylactic (*i.e.* to prevent infection) or therapeutic (*i.e.* to treat infection), but will typically be prophylactic.

A 'pharmaceutically acceptable carrier' includes any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, sucrose, trehalose, lactose, and lipid aggregates (such as oil droplets or liposomes). Such carriers are well known to those of ordinary skill in the art. The vaccines may also contain diluents, such as water, saline, glycerol, *etc.* Additionally, auxiliary substances, such as wetting or emulsifying agents, pH buffering substances, and the like, may be present. Sterile pyrogen-free, phosphate-buffered physiologic saline is a typical carrier. A thorough discussion of pharmaceutically acceptable excipients is available in ref. 294.

Compositions of the invention may include an antimicrobial, particularly if packaged in a multiple dose format.

Compositions of the invention may comprise detergent *e.g.* a Tween (polysorbate), such as Tween 80. Detergents are generally present at low levels e.g. $<0.01$ %.

Compositions of the invention may include sodium salts (*e.g.* sodium chloride) to give tonicity. A concentration of $10\pm2$mg/ml NaCl is typical.

Compositions of the invention will generally include a buffer. A phosphate buffer is typical.

Compositions of the invention may comprise a sugar alcohol (*e.g.* mannitol) or a disaccharide (*e.g.* sucrose or trehalose) *e.g.* at around 15-30mg/ml *(e.g.* 25 mg/ml), particularly if they are to be lyophilised or if they include material which has been reconstituted from lyophilised material. The pH of a composition for lyophilisation may be adjusted to around 6.1 prior to lyophilisation.

Polypeptides of the invention may be administered in conjunction with other immunoregulatory agents. In particular, compositions will usually include a vaccine adjuvant. The adjuvant may be selected from one or more of the group consisting of a TH1 adjuvant and TH2 adjuvant, further discussed below. Adjuvants which may be used in compositions of the invention include, but are not limited to:

*A. Mineral-containing compositions*

Mineral containing compositions suitable for use as adjuvants in the invention include mineral salts, such as aluminium salts and calcium salts. The invention includes mineral salts such as hydroxides (*e.g.* oxyhydroxides), phosphates (*e.g.* hydroxyphosphates, orthophosphates), sulphates, *etc.* [*e.g.* see chapters 8 & 9 of ref. 53], or mixtures of different mineral compounds (*e.g.* a mixture of a phosphate and a hydroxide adjuvant, optionally with an excess of the phosphate), with the compounds taking any suitable form (*e.g.* gel, crystalline, amorphous, *etc.),* and with adsorption to the salt(s) being preferred. Mineral containing compositions may also be formulated as a particle of metal salt [54].

A typical aluminium phosphate adjuvant is amorphous aluminium hydroxyphosphate with $PO_4$/Al molar ratio between 0.84 and 0.92, included at 0.6mg $Al^{3+}$/ml. Adsorption with a low dose of aluminium phosphate may be used *e.g.* between 50 and 100$\mu$g $Al^{3+}$ per conjugate per dose. Where an aluminium phosphate it used and it is desired not to adsorb an antigen to the adjuvant, this is favoured by including free phosphate ions in solution (*e.g.* by the use of a phosphate buffer).

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate

ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g.* about 5.7.

Suspensions of aluminium salts used to prepare compositions of the invention may contain a buffer (*e.g.* a phosphate or a histidine or a Tris buffer), but this is not always necessary. The suspensions are preferably sterile and pyrogen-free. A suspension may include free aqueous phosphate ions *e.g.* present at a concentration between 1.0 and 20 mM, preferably between 5 and 15 mM, and more preferably about 10 mM. The suspensions may also comprise sodium chloride.

The invention can use a mixture of both an aluminium hydroxide and an aluminium phosphate. In this case there may be more aluminium phosphate than hydroxide *e.g.* a weight ratio of at least 2:1 *e.g.* ≥5:1, ≥6:1, ≥7:1, ≥8:1, ≥9:1, *etc.*

Aluminum salts may be included in vaccines of the invention such that the dose of $Al^{3+}$ is between 0.2 and 1.0 mg per dose.

*B. Oil Emulsions*

Oil emulsion compositions suitable for use as adjuvants in the invention include squalene-water emulsions, such as MF59 (5% Squalene, 0.5% Tween 80, and 0.5% Span 85, formulated into submicron particles using a microfluidizer) [Chapter 10 of ref. 53; see also refs. 55-57, chapter 12 of ref. 58]. MF59 is used as the adjuvant in the FLUAD™ influenza virus trivalent subunit vaccine. The emulsion advantageously includes citrate ions e.g. 10mM sodium citrate buffer.

Particularly preferred adjuvants for use in the compositions are submicron oil-in-water emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v Tween 80 (polyoxyelthylenesorbitan monooleate), and/or 0.25-1.0% Span 85 (sorbitan trioleate), and, optionally, N-acetylmuramyl-L-alanyl-D-isogluatminyl-L-alanine-2-(1'-2'-dipalmitoyl-sn-glycero-3-hydroxyphosphophoryloxy)-ethylamine (MTP-PE). Submicron oil-in-water emulsions, methods of making the same and immunostimulating agents, such as muramyl peptides, for use in the compositions, are described in detail in references 55 & 59-60.

An emulsion of squalene, a tocopherol, and Tween 80 can be used. The emulsion may include phosphate buffered saline. It may also include Span 85 *(e.g.* at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. One such emulsion can be made by dissolving Tween 80 in PBS to give a 2% solution, then mixing 90ml of this solution with a mixture of (5 g of DL-$\alpha$-tocopherol and 5 ml squalene), then microfluidising the mixture. The resulting emulsion may have submicron oil droplets e.g. with an average diameter of between 100 and 250 nm, preferably about 180nm.

An emulsion of squalene, a tocopherol, and a Triton detergent (*e.g.* Triton X-100) can be used.

An emulsion of squalane, polysorbate 80 and poloxamer 401 ("Pluronic™ L121") can be used. The emulsion can be formulated in phosphate buffered saline, pH 7.4. This emulsion is a useful delivery vehicle for muramyl dipeptides, and has been used with threonyl-MDP in the "SAF-1" adjuvant [61] (0.05-1% Thr-MDP, 5% squalane, 2.5% Pluronic L121 and 0.2% polysorbate 80). It can also be used without the Thr-MDP, as in the "AF" adjuvant [62] (5% squalane, 1.25% Pluronic L121 and 0.2% polysorbate 80). Microfluidisation is preferred.

Complete Freund's adjuvant (CFA) and incomplete Freund's adjuvant (IFA) may also be used as adjuvants in the invention.

*C. Saponin formulations [chapter 22 of ref. 53]*

Saponin formulations may also be used as adjuvants in the invention. Saponins are a heterologous group of sterol glycosides and triterpenoid glycosides that are found in the bark, leaves, stems, roots and even flowers of a wide range of plant species. Saponins isolated from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs.

Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 63. Saponin formulations may also comprise a sterol, such as cholesterol [64].

Combinations of saponins and cholesterols can be used to form unique particles called immunostimulating complexs (ISCOMs) [chapter 23 of ref. 53]. ISCOMs typically also include a phospholipid such as phosphatidylethanolamine or phosphatidylcholine. Any known saponin can be used in ISCOMs. Preferably, the ISCOM includes one or more of QuilA, QHA and QHC. ISCOMs are further described in refs. 64-66. Optionally, the ISCOMS may be devoid of additional detergent(s) [67].

A review of the development of saponin based adjuvants can be found in refs. 68 & 69.

*D. Virosomes and virus-like particles*

Virosomes and virus-like particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in refs. 70-75. Virosomes are discussed further in, for example, ref. 76

*E. Bacterial or microbial derivatives*

Adjuvants suitable for use in the invention include bacterial or microbial derivatives such as non-toxic derivatives of enterobacterial lipopolysaccharide (LPS), Lipid A derivatives, immunostimulatory oligonucleotides and ADP-ribosylating toxins and detoxified derivatives thereof.

Non-toxic derivatives of LPS include monophosphoryl lipid A (MPL) and 3-O-deacylated MPL (3dMPL). 3dMPL is a mixture of 3 de-O-acylated monophosphoryl lipid A with 4, 5 or 6 acylated chains. A preferred "small particle" form of 3 De-O-acylated monophosphoryl lipid A is disclosed in ref. 77. Such "small particles" of 3dMPL are small enough to be sterile filtered through a 0.22$\mu$m membrane [77]. Other non-toxic LPS derivatives include monophosphoryl lipid A mimics, such as aminoalkyl glucosaminide phosphate derivatives e.g. RC-529 [78,79].

Lipid A derivatives include derivatives of lipid A from *Escherichia coli* such as OM-174. OM-174 is described for example in refs. 80 & 81.

Immunostimulatory oligonucleotides suitable for use as adjuvants in the invention include nucleotide sequences containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

The CpG's can include nucleotide modifications/analogs such as phosphorothioate modifications and can be double-stranded or single-stranded. References 82, 83 and 84 disclose possible analog substitutions *e.g.* replacement of guanosine with 2'-deoxy-7-deazaguanosine. The adjuvant effect of CpG oligonucleotides is further discussed in refs. 85-90.

The CpG sequence may be directed to TLR9, such as the motif GTCGTT or TTCGTT [91]. The CpG sequence may be specific for inducing a Th1 immune response, such as a CpG-A ODN, or it may be more specific for inducing a B cell response, such a CpG-B ODN. CpG-A and CpG-B ODNs are discussed in refs. 92-94. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, refs. 91 & 95-97.

Other immunostimulatory oligonucleotides include a double-stranded RNA, or an oligonucleotide containing a palindromic sequence, or an oligonucleotide containing a poly(dG) sequence.

Bacterial ADP-ribosylating toxins and detoxified derivatives thereof may be used as adjuvants in the invention. Preferably, the protein is derived from *E.coli* (*E.coli* heat labile enterotoxin "LT"), cholera ("CT"), or pertussis ("PT"). The use of detoxified ADP-ribosylating toxins as mucosal adjuvants is described in ref. 98 and as parenteral adjuvants in ref. 99. The toxin or toxoid is preferably in the form of a holotoxin, comprising both A and B subunits. Preferably, the A subunit contains a detoxifying mutation; preferably the B subunit is not mutated. Preferably, the adjuvant is a detoxified LT mutant such as LT-K63, LT-R72, and LT-G192. The use of ADP-ribosylating toxins and detoxified derivaties thereof, particularly LT-K63 and LT-R72, as adjuvants can be found in refs. 100-107. Numerical reference for amino acid substitutions is preferably based on the alignments of the A and B subunits of ADP-ribosylating toxins set forth in ref. 108.

Compounds of formula I, II or III, or salts thereof, can also be used as adjuvants:

I

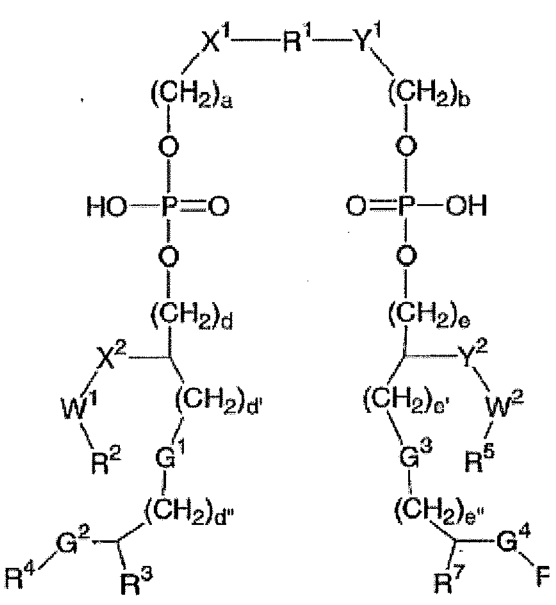

II

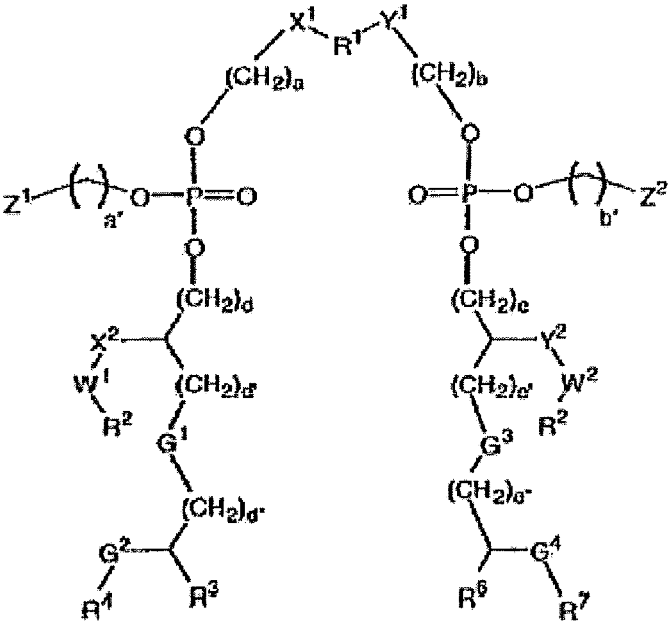

III

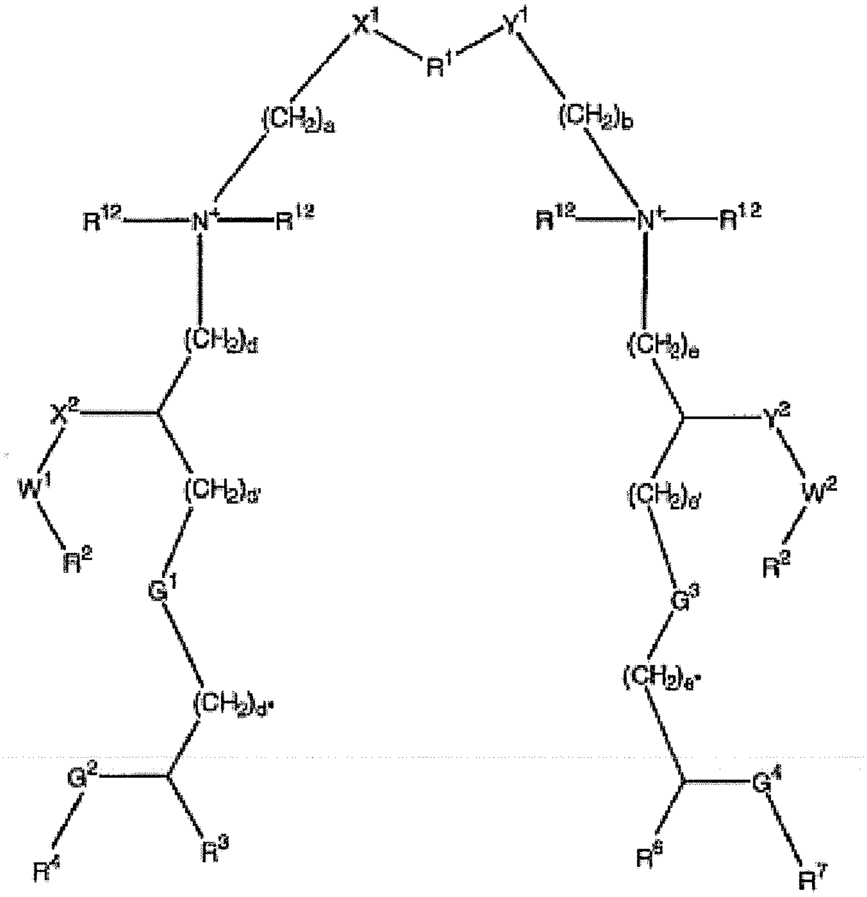

as defined in reference 109, such as 'ER 803058', 'ER 803732', 'ER 804053', ER 804058', 'ER 804059', 'ER 804442', 'ER 804680', 'ER 804764', ER 803022 or 'ER 804057' e.g.:

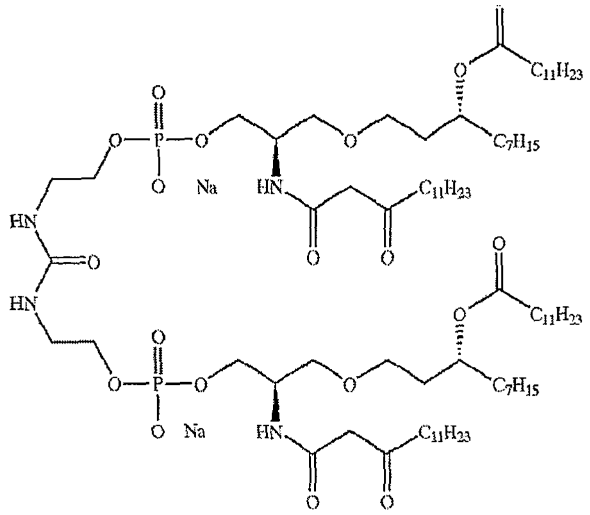

ER804057

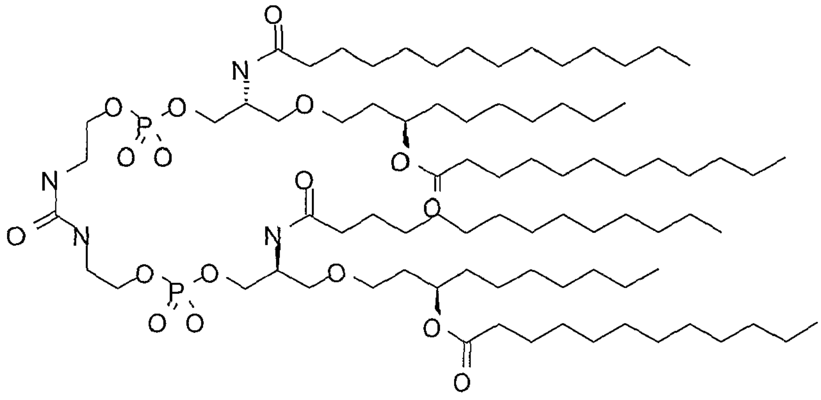

ER-803022:

*F. Human immunomodulators*

Human immunomodulators suitable for use as adjuvants in the invention include cytokines, such as interleukins (*e.g.* IL-1, IL-2, IL-4, IL-5, IL-6, IL-7, IL-12 [110], *etc.*) [111], interferons (*e.g.* interferon-$\gamma$), macrophage colony stimulating factor, tumor necrosis factor and macrophage inflammatory protein-1alpha (MIP-1alpha) and MIP-1beta [112].

*G. Bioadhesives and Mucoadhesives*

Bioadhesives and mucoadhesives may also be used as adjuvants in the invention. Suitable bioadhesives include esterified hyaluronic acid microspheres [113] or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention [114].

*H. Microparticles*

Microparticles may also be used as adjuvants in the invention. Microparticles (*i.e.* a particle of ~100nm to ~150$\mu$m in diameter, more preferably ~200nm to ~30$\mu$m in diameter, and most preferably ~500nm to ~10$\mu$m in diameter) formed from materials that are biodegradable and non-toxic (*e.g.* a poly($\alpha$-hydroxy acid), a polyhydroxybutyric acid, a polyorthoester, a polyanhydride, a polycaprolactone, *etc.*), with poly(lactide-co-glycolide) are preferred, optionally treated to have a negatively-charged surface (*e.g.* with SDS) or a positively-charged surface (*e.g.* with a cationic detergent, such as CTAB).

*I. Liposomes (Chapters 13 & 14 of ref. 53)*

Examples of liposome formulations suitable for use as adjuvants are described in refs. 115-117.

*J. Polyoxyethylene ether and polyoxyethylene ester formulations*

Adjuvants suitable for use in the invention include polyoxyethylene ethers and polyoxyethylene esters [118]. Such formulations further include polyoxyethylene sorbitan ester surfactants in combination with an octoxynol [119] as well as polyoxyethylene alkyl ethers or ester surfactants in combination with at least one additional non-ionic surfactant such as an octoxynol [120]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

*K Phosphazenes e.g. PCPP.*

Phosphazene adjuvants include poly[di(carboxylatophenoxy)phosphazene] ("PCPP") as described, for example, in refs. 121 and 122.

*L. Muramyl peptides*

Examples of muramyl peptides suitable for use as adjuvants in the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-sn-glycero-3-hydroxyphosphoryloxy)-ethylamine MTP-PE).

*M Imidazoquinoline Compounds.*

Imidazoquinoline adjuvants include Imiquimod ("R-837") [123,124], Resiquimod ("R-848") [125], and their analogs; and salts thereof (*e.g.* the hydrochloride salts). Further details about immunostimulatory imidazoquinolines can be found in references 126 to 130.

*N. Thiosemicarbazone Compounds.*

Examples of thiosemicarbazone compounds, as well as methods of formulating, manufacturing, and screening for compounds all suitable for use as adjuvants in the invention include those described in ref. 131. The thiosemicarbazones are particularly effective in the stimulation of human peripheral blood mononuclear cells for the production of cytokines, such as TNF-α.

*O. Tryptanthrin Compounds.*

Examples of tryptanthrin compounds, as well as methods of formulating, manufacturing, and screening for compounds all suitable for use as adjuvants in the invention include those described in ref. 132. The tryptanthrin compounds are particularly effective in the stimulation of human peripheral blood mononuclear cells for the production of cytokines, such as TNF-α.

*P. Nucleoside analogs*

Various nucleoside analogs can be used as adjuvants, such as (a) Isatorabine (ANA-245; 7-thia-8-oxoguanosine):

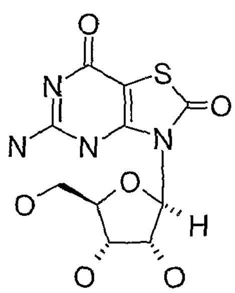

and prodrugs thereof; (b) ANA975; (c) ANA-025-1; (d) ANA380; (e) the compounds disclosed in references 133 to 135; (f) a compound having the formula:

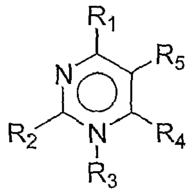

wherein:

$R_1$ and $R_2$ are each independently H, halo, $NR_aR_b$, -OH, $C_{1-6}$ alkoxy, substituted $C_{1-6}$ alkoxy, heterocyclyl, substituted heterocyclyl, $C_{6-10}$ aryl, substituted $C_{6-10}$ aryl, $C_{1-6}$ alkyl, or substituted $C_{1-6}$ alkyl;
$R_3$ is absent, H, $C_{1-6}$ alkyl, substituted $C_{1-6}$ alkyl, $C_{6-10}$ aryl, substituted $C_{6-10}$ aryl, heterocyclyl, or substituted heterocyclyl;
$R_4$ and $R_5$ are each independently H, halo, heterocyclyl, substituted heterocyclyl, -C(O)-$R_d$, $C_{1-6}$ alkyl, substituted $C_{1-6}$ alkyl, or bound together to form a 5 membered ring as in $R_{4-5}$:

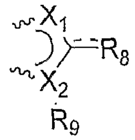

$R_{4-5}$

the binding being achieved at the bonds indicated by a



$X_1$ and $X_2$ are each independently N, C, O, or S;
$R_8$ is H, halo, -OH, $C_{1-6}$ alkyl, $C_{2-6}$ alkenyl, $C_{2-6}$ alkynyl, -OH, -$NR_aR_b$, -$(CH_2)_n$-O-$R_c$, -O-($C_{1-6}$ alkyl), -$S(O)_pR_e$, or -C(O)-$R_d$;
$R_9$ is H, $C_{1-6}$ alkyl, substituted $C_{1-6}$ alkyl, heterocyclyl, substituted heterocyclyl or $R_{9a}$, wherein $R_{9a}$ is:

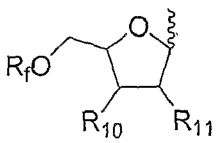

$R_{9a}$

the binding being achieved at the bond indicated by a 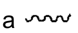
$R_{10}$ and $R_{11}$ are each independently H, halo, $C_{1-6}$ alkoxy, substituted $C_{1-6}$ alkoxy, $NR_aR_b$, or -OH;
each $R_a$ and $R_b$ is independently H, $C_{1-6}$ alkyl, substituted $C_{1-6}$ alkyl, -C(O)$R_d$, $C_{6-10}$ aryl;
each $R_c$ is independently H, phosphate, diphosphate, triphosphate, $C_{1-6}$ alkyl, or substituted $C_{1-6}$ alkyl;
each $R_d$ is independently H, halo, $C_{1-6}$ alkyl, substituted $C_{1-6}$ alkyl, $C_{1-6}$ alkoxy, substituted $C_{1-6}$ alkoxy, -$NH_2$, -NH($C_{1-6}$ alkyl), -NH(substituted $C_{1-6}$ alkyl), -N($C_{1-6}$ alkyl)$_2$, -N(substituted $C_{1-6}$ alkyl)$_2$, $C_{6-10}$ aryl, or heterocyclyl;
each $R_e$ is independently H, $C_{1-6}$ alkyl, substituted $C_{1-6}$ alkyl, $C_{6-10}$ aryl, substituted $C_{6-10}$ aryl, heterocyclyl, or substituted heterocyclyl;
each $R_f$ is independently H, $C_{1-6}$ alkyl, substituted $C_{1-6}$ alkyl, -C(O)$R_d$, phosphate, diphosphate, or triphosphate;
each n is independently 0, 1, 2, or 3;
each p is independently 0, 1, or 2; or

or (g) a pharmaceutically acceptable salt of any of (a) to (f), a tautomer of any of (a) to (f), or a pharmaceutically acceptable salt of the tautomer.

*Q. Lipids linked to a phosphate-containing acyclic backbone*

Adjuvants containing lipids linked to a phosphate-containing acyclic backbone include the TLR4 antagonist E5564 [136,137]:

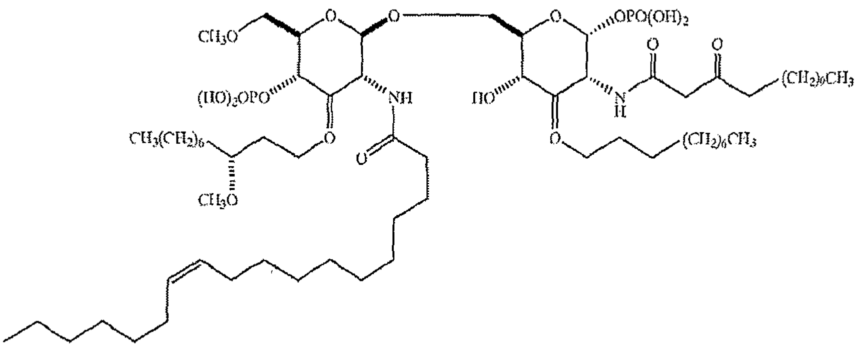

*R. Small molecule immunopotentiators (SMIPs)*

SMIPs include:

- N2-methyl-1-(2-methylpropyl)-1H-imidazo[4,5-c]quinoline-2,4-diamine;
- N2,N2-dimethyl-1-(2-methylpropyl)-1H-imidazo[4,5-c]quinoline-2,4-diamine;
- N2-ethyl N2-methyl-1-(2-methylpropyl)-1H-imidazo[4,5-c]quinoline-2,4-diamine;
- N2-methyl-1-(2-methylpropyl)-N2-propyl-1H-imidazo[4,5-c]quinoline-2,4-diamine;
- 1-(2-methylpropyl)-N2-propyl-1H-imidazo[4,5-c]quinoline-2,4-diamine;
- N2-butyl-1-(2-methylpropyl)-1H-imidazo[4,5-c]quinoline-2,4-diamine;
- N2-butyl-N2-methyl-1-(2-methylpropyl)-1H-imidazo[4,5-c]quinoline-2,4-diamine;
- N2-methyl-1-(2-methylpropyl)-N2-pentyl-1H-imidazo[4,5-c]quinoline-2,4-diamine;
- N2-methyl-1-(2-methylpropyl)-N2-prop-2-enyl-1H-imidazo[4,5-c]quinoline-2,4-diamine;
- 1-(2-methylpropyl)-2-[(phenylmethyl)thio]-1H-imidazo[4,5-c]quinolin-4-amine;
- 1-(2-methylpropyl)-2-(propylthio)-1H-imidazo[4,5-c]quinolin-4-amine ;
- 2-[[4-amino-1-(2-methylpropyl)-1H-imidazo[4,5-c]quinolin-2-yl](methyl)amino]ethanol;
- 2-[[4-amino-1-(2-methylpropyl)-1H-imidazo[4,5-c]quinolin-2-yl](methyl)amino]ethyl acetate;
- 4-amino-1-(2-methylpropyl)-1,3-dihydro-2H-imidazo[4,5-c]quinolin-2-one;
- N2-butyl-1-(2-methylpropyl)-N4,N4-bis(phenylmethyl)-1H-imidazo[4,5-c]quinoline-2,4-diamine;
- N2-butyl-N2-methyl-1-(2-methylpropyl)-N4,N4-bis(phenylmethyl)-1H-imidazo[4,5-c]quinoline-2,4-diamine;
- N2-methyl-1-(2-methylpropyl)-N4,N4-bis(phenylmethyl)-1H-imidazo[4,5-c]quinoline-2,4-diamine;
- N2,N2-dimethyl-1-(2-methylpropyl)-N4,N4-bis(phenylmethyl)-1H-imidazo[4,5-c]quinoline-2,4-diamine;
- 1-{4-amino-2-[methyl(propyl)amino]-1H-imidazo[4,5-c]quinolin-1-yl}-2-methylpropan-2-ol;
- 1-[4-amino-2-(propylamino)-1H-imidazo[4,5-c]quinalin-1-yl]-2-methylpropan-2-ol;
- N4,N4-dibenzyl-1-(2-methoxy-2-methylpropyl)-N2-propyl-1H-imidazo[4,5-c]quinoline-2,4-diamine.

*S. Proteosomes*

One adjuvant is an outer membrane protein proteosome preparation prepared from a first Gram-negative bacterium in combination with a liposaccharide preparation derived from a second Gram-negative bacterium, wherein the outer membrane protein proteosome and liposaccharide preparations form a stable non-covalent adjuvant complex. Such complexes include "IVX-908", a complex comprised of *Neisseria meningitidis* outer membrane and lipopolysaccharides. They have been used as adjuvants for influenza vaccines [138].

*T. Other adjuvants*

Other substances that act as immunostimulating agents are disclosed in references 53 and 58. Further useful adjuvant substances include:

- Methyl inosine 5'-monophosphate ("MIMP") [139].
- A polyhydroxlated pyrrolizidine compound [140], such as one having formula:

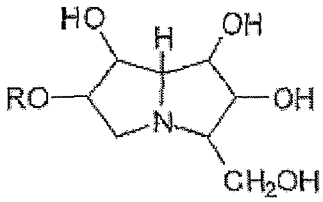

where R is selected from the group comprising hydrogen, straight or branched, unsubstituted or substituted, saturated or unsaturated acyl, alkyl (*e.g.* cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-*epi*-casuarine, 3,7-di*epi*-casuarine, *etc*.

- A gamma inulin [141] or derivative thereof, such as algammulin.
- Compounds disclosed in reference 142.
- Compounds disclosed in reference 143, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [144,145], Hydrapthalamide compounds, Benzophenone compounds, Isoxazole compounds, Sterol compounds, Quinazilinone compounds, Pyrrole compounds [146], Anthraquinone compounds, Quinoxaline compounds, Triazine compounds, Pyrazalopyrimidine compounds, and Benzazole compounds [147].
- Loxoribine (7-allyl-8-oxoguanosine) [148].
- A formulation of a cationic lipid and a (usually neutral) co-lipid, such as aminopropyl-dimethyl-myristoleyloxy-propanaminium bromide-diphytanoylphosphatidyl-ethanolamine ("Vaxfectin™") or aminopropyl-dimethyl-bis-dodecyloxy-propanaminium bromide-dioleoylphosphatidyl-ethanolamine ("GAP-DLRIE:DOPE"). Formulations containing (±)-N-(3-aminopropyl)-N,N-dimethyl-2,3-bis(syn-9-tetradeceneyloxy)-1-propanaminium salts are preferred [149].

The invention may also comprise combinations of one or more of the adjuvants identified above. For example, the following combinations may be used as adjuvant compositions in the invention: (1) a saponin and an oil-in-water emulsion [150]; (2) a saponin (*e.g.* QS21) + a non-toxic LPS derivative (*e.g.* 3dMPL) [151]; (3) a saponin (*e.g.* QS21) + a non-toxic LPS derivative (*e.g.* 3dMPL) + a cholesterol; (4) a saponin (*e.g.* QS21) + 3dMPL + IL-12 (optionally + a sterol) [152]; (5) combinations of 3dMPL with, for example, QS21 and/or oil-in-water emulsions [153]; (6) SAF, containing 10% squalane, 0.4% Tween 80™, 5% pluronic-block polymer L121, and thr-MDP, either microfluidized into a submicron emulsion or vortexed to generate a larger particle size emulsion. (7) Ribi™ adjuvant system (RAS), (Ribi Immunochem) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (8) one or more mineral salts (such as an aluminum salt) + a non-toxic derivative of LPS (such as 3dMPL); and (9) one or more mineral salts (such as an aluminum salt) + an immunostimulatory oligonucleotide (such as a nucleotide sequence including a CpG motif).

The compositions of the invention will preferably elicit both a cell mediated immune response as well as a humoral immune response in order to effectively address a uropathogenic infection. This immune response will preferably induce long lasting (*e.g.* neutralising) antibodies and a cell mediated immunity that can quickly respond upon exposure to UPEC-associated antigens.

Two types of T cells, CD4 and CD8 cells, are generally thought necessary to initiate and/or enhance cell mediated immunity and humoral immunity. CD8 T cells can express a CD8 co-receptor and are commonly referred to as cytotoxic T lymphocytes (CTLs). CD8 T cells are able to recognized or interact with antigens displayed on MHC Class I molecules. CD4 T cells can express a CD4 co-receptor and are commonly referred to as T helper cells. CD4 T cells are able to recognize antigenic peptides bound to MHC class II molecules. Upon interaction with a MHC class II molecule, the CD4 cells can secrete factors such as cytokines. These secreted cytokines can activate B cells, cytotoxic T cells, macrophages, and other cells that participate in an immune response. Helper T cells or $CD4^+$ cells can be further divided into two functionally distinct subsets: TH1 phenotype and TH2 phenotypes which differ in their cytokine and effector function.

Activated THI cells enhance cellular immunity (including an increase in antigen-specific CTL production) and are therefore of particular value in responding to intracellular infections. Activated TH1 cells may secrete one or more of IL-2, IFN-γ, and TNF-β. A TH1 immune response may result in local inflammatory reactions by activating macrophages, NK (natural killer) cells, and CD8 cytotoxic T cells (CTLs). A TH1 immune response may also act to expand the immune response by stimulating growth of B and T cells with IL-12. TH1 stimulated B cells may secrete IgG2a.

Activated TH2 cells enhance antibody production and are therefore of particular value in responding to extracellular infections. Activated TH2 cells may secrete one or more of IL-4, IL-5, IL-6, and IL-10. A TH2 immune response may result in the production of IgG1, IgE, IgA and memory B cells for future protection.

An enhanced immune response may include one or more of an enhanced TH1 immune response and a TH2 immune response. An enhanced TH1 immune response may include one or more of an increase in CTLs, an increase in one or more of the cytokines associated with a TH1 immune response (such as IL-2, IFN-$\gamma$, and TNF-$\beta$), an increase in activated macrophages, an increase in NK activity, or an increase in the production of IgG2a. Preferably, the enhanced TH1 immune response will include an increase in IgG2a production. An enhanced TH2 immune response may include one or more of an increase in one or more of the cytokines associated with a TH2 immune response (such as IL-4, IL-5, IL-6 and IL-10), or an increase in the production of IgG1, IgE, IgA and memory B cells. Preferably, the enhanced TH2 immune resonse will include an increase in IgG 1 production.

A TH1 immune response may be elicited using a TH1 adjuvant. A TH1 adjuvant will generally elicit increased levels of IgG2a production relative to immunization of the antigen without adjuvant. TH1 adjuvants suitable for use in the invention may include for example saponin formulations, virosomes and virus like particles, non-toxic derivatives of enterobacterial lipopolysaccharide (LPS), immunostimulatory oligonucleotides. Immunostimulatory oligonucleotides, such as oligonucleotides containing a CpG motif, are preferred TH1 adjuvants for use in the invention.

A TH2 immune response may be elicited using a TH2 adjuvant. A TH2 adjuvant will generally elicit increased levels of IgG1 production relative to immunization of the antigen without adjuvant. TH2 adjuvants suitable for use in the invention include, for example, mineral containing compositions, oil-emulsions, and ADP-ribosylating toxins and detoxified derivatives hereof. Mineral containing compositions, such as aluminium salts are preferred TH2 adjuvants for use in the invention.

Preferably, the invention includes a composition comprising a combination of a TH1 adjuvant and a TH2 adjuvant. Preferably, such a composition elicits an enhanced TH1 and an enhanced TH2 response *i.e.* an increase in the production of both IgG1 and IgG2a production relative to immunization without an adjuvant. Still more preferably, the composition comprising a combination of a TH1 and a TH2 adjuvant elicits an increased TH1 and/or an increased TH2 immune response relative to immunization with a single adjuvant (i.e. relative to immunization with a TH1 adjuvant alone or immunization with a TH2 adjuvant alone).

The immune response may be one or both of a TH1 immune response and a TH2 response. Preferably, immune response provides for one or both of an enhanced TH1 response and an enhanced TH2 response.

The enhanced immune response may be one or both of a systemic and a mucosal immune response. Preferably, the immune response provides for one or both of an enhanced systemic and an enhanced mucosal immune response. Preferably the mucosal immune response is a TH2 immune response. Preferably, the mucosal immune response includes an increase in the production of IgA.

The use of an aluminium hydroxide or aluminium phosphate adjuvant is particularly preferred, and antigens are generally adsorbed to these salts.

The pH of compositions of the invention is preferably between 6 and 8, preferably about 7. Stable pH may be maintained by the use of a buffer. Where a composition comprises an aluminium hydroxide salt, it is preferred to use a histidine buffer [154]. The composition may be sterile and/or pyrogen-free. Compositions of the invention may be isotonic with respect to humans.

Compositions may be presented in vials, or they may be presented in ready-filled syringes. The syringes may be supplied with or without needles. A syringe will include a single dose of the composition, whereas a vial may include a single dose or multiple doses. Injectable compositions will usually be liquid solutions or suspensions. Alternatively, they may be presented in solid form (e.g. freeze-dried) for solution or suspension in liquid vehicles prior to injection.

Compositions of the invention may be packaged in unit dose form or in multiple dose form. For multiple dose forms, vials are preferred to pre-filled syringes. Effective dosage volumes can be routinely established, but a typical human dose of the composition for injection has a volume of 0.5ml.

Where a composition of the invention is to be prepared extemporaneously prior to use (*e.g.* where a component is presented in lyophilised form) and is presented as a kit, the kit may comprise two vials, or it may comprise one ready-filled syringe and one vial, with the contents of the syringe being used to reactivate the contents of the vial prior to injection.

Thus the invention provides for a kit comprising a first component and a second component, wherein: the first component comprises one or more polypeptide, antibody, vesicle and/or nucleic acid of the invention; and the second component comprises one or more of the following: instructions for administering a composition to a patient, a syringe or other delivery device, an adjuvant, and/or a pharmaceutically acceptable formulating solution.

The invention also provides a delivery device (*e.g.* a syringe) pre-filled with the immunogenic compositions of the invention.

Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc.*), the capacity of the individual's immune system

to synthesise antibodies, the degree of protection desired, the formulation of the vaccine, the treating doctor's assessment of the medical situation, and other relevant factors. It is expected that the amount will fall in a relatively broad range that can be determined through routine trials, and a typical quantity of each antigen per dose is between 0.1 μg and 1mg per antigen.

*Nucleic acid immunisation*

The immunogenic compositions described above include polypeptide antigens from UPEC. As an alternative to using proteins antigens in the immunogenic compositions of the invention, nucleic acid (preferably DNA e.*g.* in the form of a plasmid) encoding the antigen may be used, to give compositions, methods and uses based on nucleic acid immunisation. Nucleic acid immunisation is now a developed field (*e.g.* see references 155 to 162 *etc.*), and has been applied to many vaccines.

The nucleic acid encoding the immunogen is expressed *in vivo* after delivery to a patient and the expressed immunogen then stimulates the immune system. The active ingredient will typically take the form of a nucleic acid vector comprising: (i) a promoter; (ii) a sequence encoding the immunogen, operably linked to the promoter; and optionally (iii) a selectable marker. Preferred vectors may further comprise (iv) an origin of replication; and (v) a transcription terminator downstream of and operably linked to (ii). In general, (i) and (v) will be eukaryotic and (iii) and (iv) will be prokaryotic.

Preferred promoters are viral promoters *e.g.* from cytomegalovirus (CMV). The vector may also include transcriptional regulatory sequences (*e.g.* enhancers) in addition to the promoter and which interact functionally with the promoter. Preferred vectors include the immediate-early CMV enhancer/promoter, and more preferred vectors also include CMV intron A. The promoter is operably linked to a downstream sequence encoding an immunogen, such that expression of the immunogen-encoding sequence is under the promoter's control.

Where a marker is used, it preferably functions in a microbial host (*e.g.* in a prokaryote, in a bacteria, in a yeast). The marker is preferably a prokaryotic selectable marker (*e.g.* transcribed under the control of a prokaryotic promoter). For convenience, typical markers are antibiotic resistance genes.

The vector of the invention is preferably an autonomously replicating episomal or extrachromosomal vector, such as a plasmid.

The vector of the invention preferably comprises an origin of replication. It is preferred that the origin of replication is active in prokaryotes but not in eukaryotes.

Preferred vectors thus include a prokaryotic marker for selection of the vector, a prokaryotic origin of replication, but a eukaryotic promoter for driving transcription of the immunogen-encoding sequence. The vectors will therefore (a) be amplified and selected in prokaryotic hosts without polypeptide expression, but (b) be expressed in eukaryotic hosts without being amplified. This arrangement is ideal for nucleic acid immunization vectors.

The vector of the invention may comprise a eukaryotic transcriptional terminator sequence downstream of the coding sequence. This can enhance transcription levels. Where the coding sequence does not have its own, the vector of the invention preferably comprises a polyadenylation sequence. A preferred polyadenylation sequence is from bovine growth hormone.

The vector of the invention may comprise a multiple cloning site.

In addition to sequences encoding the immunogen and a marker, the vector may comprise a second eukaryotic coding sequence. The vector may also comprise an IRES upstream of said second sequence in order to permit translation of a second eukaryotic polypeptide from the same transcript as the immunogen. Alternatively, the immunogen-coding sequence may be downstream of an IRES.

The vector of the invention may comprise unmethylated CpG motifs e.g. unmethylated DNA sequences which have in common a cytosine preceding a guanosine, flanked by two 5' purines and two 3' pyrimidines. In their unmethylated form these DNA motifs have been demonstrated to be potent stimulators of several types of immune cell.

Vectors may be delivered in a targeted way. Receptor-mediated DNA therapy techniques are described in, for example, references 163 to 168. Therapeutic compositions containing a nucleic acid are administered in a range of about 100ng to about 200mg of DNA for local administration in a gene therapy protocol. Concentration ranges of about 500 ng to about 50 mg, about 1μg to about 2 mg, about 5μg to about 500μg, and about 20μg to about 100μg of DNA can also be used during a gene therapy protocol. Factors such as method of action (*e.g.* for enhancing or inhibiting levels of the encoded gene product) and efficacy of transformation and expression are considerations which will affect the dosage required for ultimate efficacy. Where greater expression is desired over a larger area of tissue, larger amounts of vector or the same amounts re-administered in a successive protocol of administrations, or several administrations to different adjacent or close tissue portions may be required to effect a positive therapeutic outcome. In all cases, routine experimentation in clinical trials will determine specific ranges for optimal therapeutic effect.

Vectors can be delivered using gene delivery vehicles. The gene delivery vehicle can be of viral or non-viral origin (see generally references 169 to 172).

Viral-based vectors for delivery of a desired nucleic acid and expression in a desired cell are well known in the

art. Exemplary viral-based vehicles include, but are not limited to, recombinant retroviruses (*e.g.* references 173 to 183), alphavirus-based vectors (*e.g.* Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532); hybrids or chimeras of these viruses may also be used (*e.g.* U.S. Publication No. US20030148262; International Publication No. WO02/099035)), poxvirus vectors (*e.g.* vaccinia, fowlpox, canarypox, modified vaccinia Ankara, etc.), adenovirus vectors, and adeno-associated virus (AAV) vectors (*e.g.* see refs. 184 to 189). Administration of DNA linked to killed adenovirus [190] can also be employed.

Non-viral delivery vehicles and methods can also be employed, including, but not limited to, polycationic condensed DNA linked or unlinked to killed adenovirus alone [*e.g.* 190], ligand-linked DNA [191], eukaryotic cell delivery vehicles cells [e.g. refs. 192 to 196] and nucleic charge neutralization or fusion with cell membranes. Naked DNA can also be employed. Exemplary naked DNA introduction methods are described in refs. 197 and 198. Liposomes (*e.g.* immunoliposomes) that can act as gene delivery vehicles are described in refs. 199 to 203. Additional approaches are described in references 204 & 205.

Further non-viral delivery suitable for use includes mechanical delivery systems such as the approach described in ref. 205. Moreover, the coding sequence and the product of expression of such can be delivered through deposition of photopolymerized hydrogel materials or use of ionizing radiation [*e.g.* refs. 206 & 207]. Other conventional methods for gene delivery that can be used for delivery of the coding sequence include, for example, use of hand-held gene transfer particle gun [208] or use of ionizing radiation for activating transferred genes [206 & 207].

Delivery DNA using PLG {poly(lactide-co-glycolide)} microparticles is a particularly preferred method *e.g.* by adsorption to the microparticles, which are optionally treated to have a negatively-charged surface *(e.g.* treated with SDS) or a positively-charged surface (e.g. treated with a cationic detergent, such as CTAB).

*Pharmaceutical uses*

The invention also provides a method of treating a patient, comprising administering to the patient a therapeutically effective amount of a composition of the invention. The patient may either be at risk from the disease themselves or may be a pregnant woman ('maternal immunisation' [209]).

The invention provides nucleic acid, polypeptide, vesicle or antibody of the invention for use as medicaments (*e.g.* as immunogenic compositions or as vaccines, or in a method of treating a patient) or as diagnostic reagents. It also provides the use of nucleic acid, polypeptide, vesicle or antibody of the invention in the manufacture of: (i) a medicament for treating or preventing disease and/or infection caused by an ExPEC bacterium; (ii) a diagnostic reagent for detecting the presence of or of antibodies raised against an ExPEC bacterium; and/or (iii) a reagent which can raise antibodies against an ExPEC bacterium. Said ExPEC bacterium can be of any serotype or strain. Preferably the ExPEC bacterium is a UPEC strain.

The invention is useful for the prevention and/or treatment of diseases such as bacteremia, meningitis, a urinary tract infection, pyelonephritis and/or cystitis. The invention is particularly useful for the treatment of urinary tract infections.

The patient is preferably a human. The human is preferably an adult (e.g. aged between 20 and 55). A vaccine intended for children or adolescents may also be administered to adults e.g. to assess safety, dosage, immunogenicity, *etc.* Female patients are a preferred subset, with sexually-active females aged 20-55 being a particularly preferred patient group. Another groups of patients is females aged 12-20, particularly for prophylactic use.

Other possible patient animals include dogs, which may be carriers of ExPEC [210,211].

One way of checking efficacy of therapeutic treatment involves monitoring infection after administration of the composition of the invention. One way of checking efficacy of prophylactic treatment involves monitoring immune responses against an administered polypeptide after administration. Immunogenicity of compositions of the invention can be determined by administering them to test subjects (*e.g.* children 12-16 months age, or animal models *e.g.* a mouse model) and then determining standard parameters including ELISA titres (GMT) of IgG. These immune responses will generally be determined around 4 weeks after administration of the composition, and compared to values determined before administration of the composition. Where more than one dose of the composition is administered, more than one post-administration determination may be made. Various mouse models of UTI are available [*e.g.* refs. 212 & 213-214].

Administration of polypeptide antigens is a preferred method of treatment for inducing immunity. Administration of antibodies of the invention is another preferred method of treatment. This method of passive immunisation is particularly useful for newborn children or for pregnant women. This method will typically use monoclonal antibodies, which will be humanised or fully human.

Compositions of the invention will generally be administered directly to a patient. Direct delivery may be accomplished by parenteral injection (*e.g.* subcutaneously, intraperitoneally, intravenously, intramuscularly, or to the interstitial space of a tissue), or by rectal, oral (*e.g.* tablet, spray), vaginal, topical, transdermal, transcutaneous, intranasal, sublingual, ocular, aural, pulmonary or other mucosal administration. Intramuscular administration to the thigh or the upper arm is preferred. Injection may be via a needle (*e.g.* a hypodermic needle), but needle-free injection may alternatively be used. A typical intramuscular dose is 0.5 ml.

The invention may be used to elicit systemic and/or mucosal immunity. Preferably the enhanced systemic and/or mucosal immunity is reflected in an enhanced TH1 and/or TH2 immune response. Preferably, the enhanced immune response includes an increase in the production of IgG1 and/or IgG2a and/or IgA.

Dosage treatment can be a single dose schedule or a multiple dose schedule. Multiple doses may be used in a primary immunisation schedule and/or in a booster immunisation schedule. A primary dose schedule may be followed by a booster dose schedule. In a multiple dose schedule the various doses may be given by the same or different routes *e.g.* a parenteral prime and mucosal boost, a mucosal prime and parenteral boost, *etc.* Suitable timing between priming doses (*e.g.* between 4-16 weeks), and between priming and boosting, can be routinely determined. For example, a primary course of vaccination may include 1-10 separate doses, followed by other doses given at subsequent time intervals required to maintain and/or reinforce an immune response, for example, at 1-4 months for a second dose, and if needed, a subsequent dose or doses after several months. A single dose schedule may comprise one administration or multiple administrations (collectively a single dose schedule). A multiple dose schedule comprises multiple doses, wherein each dose may comprise one administration or multiple administrations.

Bacterial infections affect various areas of the body and so compositions may be prepared in various forms. For example, the compositions may be prepared as injectables, either as liquid solutions or suspensions. Solid forms suitable for solution in, or suspension in, liquid vehicles prior to injection can also be prepared (*e.g.* a lyophilised or a spray-freeze dried composition). The composition may be prepared for topical administration *e.g.* as an ointment, cream or powder. The composition be prepared for oral administration *e.g.* as a tablet or capsule, as a spray or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration *e.g.* as spray, drops, gel or powder [*e.g.* refs 215 & 216]. The composition may be in kit form, designed such that a combined composition is reconstituted just prior to administration to a patient. Such kits may comprise one or more antigens in liquid form and one or more lyophilised antigens.

Compositions of the invention may be administered to patients at substantially the same time as (*e.g.* during the same medical consultation or visit to a healthcare professional) other vaccines *e.g.* at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H. influenzae* type b vaccine, a human papillomavirus vaccine, an inactivated poliovirus vaccine, a hepatitis B virus vaccine, a pneumococcal conjugate vaccine, a meningococcal conjugate vaccine, *etc.* Similarly, they may be administered to patients at substantially the same time as (*e.g.* during the same medical consultation or visit to a healthcare professional) an antibiotic, and in particular an antibiotic compound active against UPEC.

***Further antigenic components of compositions of the invention***

The invention also provides a composition comprising a polypeptide or the invention and one or more of the following further antigens:

- a saccharide antigen from *N.meningitidis* serogroup A, C, W135 and/or Y (preferably all four), such as the oligosaccharide disclosed in ref. 217 from serogroup C [see also ref. 218] or the oligosaccharides of ref. 219.
- an antigen from *N.meningitidis* serogroup B such as those disclosed in refs. 220-228, *etc.*
- a saccharide antigen from *Streptococcus pneumoniae* [*e.g.* 229, 230, 231].
- an antigen from hepatitis A virus, such as inactivated virus [*e.g.* 232, 233].
- an antigen from hepatitis B virus, such as the surface and/or core antigens [*e.g.* 233, 234].
- an antigen from hepatitis C virus [*e.g.* 235].
- an antigen from HIV [236]
- a diphtheria antigen, such as a diphtheria toxoid [*e.g.* chapter 3 of ref. 237] *e.g.* the $CRM_{197}$ mutant [*e.g.* 238].
- a tetanus antigen, such as a tetanus toxoid [*e.g.* chapter 4 of ref. 237].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 [*e.g.* refs. 239 & 240].
- a saccharide antigen from *Haemophilus influenzae* B [*e.g.* 218].
- polio antigen(s) [*e.g.* 241, 242] such as IPV.
- measles, mumps and/or rubella antigens [*e.g.* chapters 9, 10 & 11 of ref. 237].
- varicella antigens.
- influenza antigen(s) [*e.g.* chapter 19 of ref. 237], such as the haemagglutinin and/or neuraminidase surface proteins. Influenza antigens may be derived from interpandemic (annual) flu strains. Influenza antigens may be derived from strains with the potential to cause a pandemic outbreak (*i.e.*, influenza strains with new haemagglutinin compared to the haemagglutinin in currently circulating strains, or influenza strains which are pathogenic in avian subjects and

have the potential to be transmitted horizontally in the human population, or influenza strains which are pathogenic to humans). Influenza antigens may be derived from viruses grown in eggs or cell culture.

- an antigen from *Moraxella catarrhalis [e.g.* 243].
- a saccharide antigen from *Streptococcus agalactiae* (group B streptococcus).
- an protein antigen from *Streptococcus agalactiae* (group B streptococcus) [e.g. 244-249]
- an antigen from *N.gonorrhoeae* [*e.g.* 220-222 and 250].
- an antigen from *Chlamydia pneumoniae* [e.g. refs. 251 to 257] or a combination of antigens from *C.pneumoniae* [*e.g.* 258].
- an antigen from *Chlamydia trachomatis,* or a combination of antigens from *C.trachomatis* [*e.g.* 259].
- an antigen from *Porphyromonas gingivalis* [*e.g.* 260].
- rabies antigen(s) [*e.g.* 261] such as lyophilised inactivated virus [*e.g.* 262, RabAvert™].
- antigen(s) from a paramyxovirus such as respiratory syncytial virus (RSV [263, 264]) and/or parainfluenza virus (PIV3 [265]).
- an antigen from *Bacillus anthracis* [*e.g.* 266, 267, 268].
- an antigen from *Streptococcus pyogenes* (group A streptococcus) [*e.g.* 245, 269, 270].
- an antigen from *Staphylococcus aureus* [*e.g.* 271].
- an antigen from a virus in the flaviviridae family (genus flavivirus), such as from yellow fever virus, Japanese encephalitis virus, four serotypes of Dengue viruses, tick-borne encephalitis virus, West Nile virus.
- a pestivirus antigen, such as from classical porcine fever virus, bovine viral diarrhoea virus, and/or border disease virus.
- a parvovirus antigen *e.g.* from parvovirus B 19.
- a human papilloma virus (HPV) antigen [272]

The composition may comprise one or more of these further antigens.

In another embodiment, antigens of the invention are combined with one or more additional, non*E.coli* antigens suitable for use in a vaccine designed to protect females against genitourinary and/or sexually transmitted diseases. For example, the antigens may be combined with an antigen derived from the group consisting of *Streptococcus agalactiae, Chlamydia trachomatis, Neisseria gonorrhoeae,* papillomavirus and herpes simplex virus. Where human papillomavirus antigens are used, they may be from one or more of the strains, HPV 16, HPV 18, HPV 6 and/or HPV 11.

Preferred gonococcal antigens include one or more of ngs13 (OmpA), OmpH, ngs576 (peptidyl-prolyl cis/trans isomerase (PPIase) protein), ngs41 and ngs 117.

Preferred HPV antigens include one or more from the strains HPV 16, HPV 18, HPV 6 and HPV 11.

Preferred *Chlamydia trachomatis* antigens include one or more of: CT045, CT089, CT242, CT316, CT381. CT396, CT398, CT444, CT467, CT547, CT587, CT823, CT761 and specific combinations of these antigens as disclosed in reference273.

Preferred *Chlamydia pneumoniae* antigens include one or more of: CPn0324, Cpn0301, Cpn0482, Cpn0503, Cpn0525, Cpn0558, Cpn0584, Cpn0800, Cpn0979, Cpn0498, Cpn0300, Cpn0042, Cpn0013, Cpn450, Cpn0661, Cpn0557, Cpn0904, Clpn0795, Cpn0186 and Cpn0604 and specific combinations of these antigens as disclosed in reference274.

Preferred GBS antigens include one or more of GBS80, GBS 104, GBS 59, GBS 67, GBS 322 and GBS 276.

In another embodiment, the antigen combinations of the invention are combined with one or more additional, non-ExPEC antigens suitable for use in a vaccine designed to protect elderly or immunocompromised individuals. For example, the antigen combinations may be combined with an antigen derived from the group consisting of *Enterococcus faecalis, Staphylococcus aureus, Staphylococcus epidermis, Pseudomonas aeruginosa, Legionella pneumophila, Listeria monocytogenes, Neisseria meningitidies,* influenza, and Parainfluenza virus ('PIV').

Toxic protein antigens may be detoxified where necessary (*e.g*. detoxification of pertussis toxin by chemical and/or genetic means [240]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

Saccharide antigens are preferably in the form of conjugates. Carrier proteins for the conjugates include bacterial toxins (such as diphtheria toxoid or tetanus toxoid), the *N.meningitidis* outer membrane protein [275], synthetic peptides [276,277], heat shock proteins [278,279], pertussis proteins [280,281], protein D from *H.influenzae* [282,283], cytokines [284], lymphokines [284], *H.influenzae* proteins, hormones [284], growth factors [284], toxin A or B from *C.difficile* [285], iron-uptake proteins [286], artificial proteins comprising multiple human CD4+ T cell epitopes from various pathogen-derived antigens [287] such as the N19 protein [288], pneumococcal surface protein PspA [289], pneumolysin [290], *etc.* A preferred carrier protein is CRM197 protein [291].

Antigens in the composition will typically be present at a concentration of at least 1μg/ml each. In general, the concentration of any given antigen will be sufficient to elicit an immune response against that antigen.

Antigens are preferably adsorbed to an aluminium salt.

*General*

The term "comprising" encompasses "including" as well as "consisting" *e.g.* a composition "comprising" X may consist exclusively of X or may include something additional *e.g.* X + Y.

The term "about" in relation to a numerical value x means, for example, $x \pm 10\%$.

The word "substantially" does not exclude "completely" e.g. a composition which is "substantially free" from Y may be completely free from Y. Where necessary, the word "substantially" may be omitted from the definition of the invention.

The N-terminus residues in the amino acid sequences in the sequence listing are given as the amino acid encoded by the first codon in the corresponding nucleotide sequence. Where the first codon is not ATG, it will be understood that it will be translated as methionine when the codon is a start codon, but will be translated as the indicated non-Met amino acid when the sequence is at the C-terminus of a fusion partner. The invention specifically discloses and encompasses each of the amino acid sequences of the sequence listing having a N-terminus methionine residue (*e.g.* a formyl-methionine residue) in place of any indicated non-Met residue. It also specifically discloses and encompasses each of the amino acid sequences of the sequence listing starting at any internal methionine residues in the sequences.

The sequences disclosed herein were originally identified in the 536 strain. Genome sequences of several other strains of *E. coli* are available. Standard search and alignment techniques can be used to identify in any of these (or other) further genome sequences the homolog of any particular sequence of the present invention. Moreover, the sequences can be used to design primers for amplification of homologous sequences from other strains. Thus the invention is not limited to strain 536 sequences, but rather encompasses such variants and homologs from other strains of *E. coli,* particularly ExPEC and UPEC strains. In general, suitable variants of a particular SEQ ID NO include its allelic variants, its polymorphic forms, its homologs, its orthologs, its paralogs, its mutants, *etc.*

Thus, for instance, polypeptides used with the invention may, compared to the 536 sequence, include one or more (*e.g.* 1, 2, 3, 4, 5, 6, 7, 8, 9, *etc.*) amino acid substitutions, insertions, deletions, *etc.* as disclosed above.

As indicated in the above text, nucleic acids and polypeptides of the invention may include sequences that:

(a) are identical (*i.e.* 100% identical) to the sequences disclosed in the sequence listing;
(b) share sequence identity with the sequences disclosed in the sequence listing;
(c) have 1, 2, 3, 4, 5, 6, 7, 8, 9 or 10 single nucleotide or amino acid alterations (deletions, insertions, substitutions), which may be at separate locations or may be contiguous, as compared to the sequences of (a) or (b); and
(d) when aligned with a particular sequence from the sequence listing using a pairwise alignment algorithm, a moving window of *x* monomers (amino acids or nucleotides) moving from start (N-terminus or 5') to end (C-terminus of 3'), such that for an alignment that extends to *p* monomers (where $p>x$) there are $p-x+1$ such windows, each window has at least $x{\cdot}y$ identical aligned monomers, where: *x* is selected from 20, 25, 30, 35, 40, 45, 50, 60, 70, 80, 90, 100, 150, 200; *y* is selected from 0.50, 0.60, 0.70, 0.75, 0.80, 0.85, 0.90, 0.91, 0.92, 0.93, 0.94, 0.95, 0.96, 0.97, 0.98, 0.99; and if $x{\cdot}y$ is not an integer then it is rounded up to the nearest integer. The preferred pairwise alignment algorithm is the Needleman-Wunsch global alignment algorithm [292], using default parameters (*e.g.* with Gap opening penalty = 10.0, and with Gap extension penalty = 0.5, using the EBLOSUM62 scoring matrix). This algorithm is conveniently implemented in the *needle* tool in the EMBOSS package [293].

The nucleic acids and polypeptides of the invention may additionally have further sequences to the N-terminus/ 5' and/or C-terminus/3' of these sequences (a) to (d).

The practice of the present invention will employ, unless otherwise indicated, conventional methods of chemistry, biochemistry, molecular biology, immunology and pharmacology, within the skill of the art. Such techniques are explained fully in the literature. See, *e.g.*, references 294-301, *etc.*

In some embodiments, the invention does not encompass the polypeptides disclosed in reference 27, reference 28, U.S. Provisional Application No. 60/654,632 (filed February 18, 2005; priority application for refs 27 & 28) or U.S. Provisional Application No. 60/712,720 (filed August 29, 2005; priority application for ref. 28).

## MODES FOR CARRYING OUT THE INVENTION

Below are examples of specific embodiments or modes for carrying out the present invention. The examples are offered for illustrative purposes only, and are not intended to limit the scope of the present invention in any way.

Efforts have been made to ensure accuracy with respect to numbers used (*e.g*., amounts, temperatures, etc.), but some experimental error and deviation should, of course, be allowed for.

Computer-based comparative and predictive tools were used to identify 93 polypeptides from the UPEC 536 strain, using criteria such as (a) less than 90% sequence identity with two commensal strains (mg1655, DH10B), (b) have a length greater than 100 amino acids, (c) have a non-cytoplasmic cellular localisation, and (d) are common to another UPEC strain (CFT073) but are not found in an MNEC strain (IHE3034). These sequences are listed in the sequence listing. Their amino acid sequences are SEQ ID NOS: 8 to 100.

In parallel studies, assays were carried out (see below) in order to select surface-exposed proteins, which are specific for UPEC strains and absent in non-pathogenic strains (commensal and laboratory strains). 76 polypeptides were identified, 11 of which had less than 80% homology with commensal strains (mg1655). The sequences of these 11 polypeptides are listed in the sequence listing and their amino acid sequences are SEQ ID NOS I to 10 and SEQ ID NO 85. The sequences of 7 of these polypeptides were identified by this method but not by the computer-based method. These sequences are listed in the sequence listing and their amino acid sequences are SEQ ID NOS 1 to 7. Table 1 shows the 76 polypeptides that were identified in this way (SEQ ID NOs 1 to 10, 85 and 101-165).

In further work, SEQ ID NOs 166 and 167 were identified.

(SEQ ID NOs 1-167 are listed in the sequence listing. They are also referred to by 'RECP' nomenclature, as shown in Table 4.

The invention provides a credible utility for these polypeptides, namely in the provision of immunogenic compositions as described herein.

These polypeptides may be cloned, expressed and purified. The purified antigens may then be used to immunise mice, whose sera can be analysed by Western blot, ELISA and FACS, and further tested in both *in vitro* and *in vivo* experiments. Suitable *in vitro* experiments include testing the ability of antibodies to induce complement-mediated bacterial killing and/or opsonophagocytosis activity, to block binding of ExPEC strains (or the purified antigen) to human epithelial cells (*e.g*. in bladder cells) or other cell lines, and/or to inhibit adhesion/invasion of *E.coli* bacteria (*e.g*. K1 strain) to brain microvascular endothelial cells (BMEC). Suitable *in vivo* experiments include active and/or passive systemic immunisations and challenge in mouse models of UTI (adult mice), protection by active or passive immunisations against bacteremia and meningitis in 5-day-old rats challenged with *E.coli* K1 strain, and immunisation and intraperitoneal infection of adult mice with an ExPEC strain.

The importance of the proteins to the bacterial life-cycle may be tested by creating isogenic knockout mutants. The mutants can also be used to ensure that sera raised by an antigen are specific for that antigen. Microarrays may be used to study expression patterns. Conservation and/or variability is assessed by sequencing the genes from multiple different ExPEC strains. The invention also provides an *E.coli* in which one or more of the polypeptides of the invention has/have been knocked out. A knockout mutation may be situated in the coding region of the gene or may lie within its transcriptional control regions (*e.g*. within its promoter). A knockout mutation will reduce the level of mRNA encoding the antigen to <1% of that produced by the wild-type bacterium, preferably <0.5%, more preferably <0.1%, and most preferably to 0%.

Proteomic assays were carried out in order to select predicted surface-exposed proteins, which are specific for UPEC strains and absent in non-pathogenic strains (commensal and laboratory strains). Once selected these proteins may be expressed and purified and used to immunize mice.

It is known from reference 43 that a mutation in any of the tol-pal genes of *E. coli* results in the formation of vesicles containing native outer membrane proteins. Proteins present in vesicles of UPEC strains and which had low homology to commensal strains such as mg1655 were selected as a small potential group of proteins that could be used as potential antigens.

***Lambda red-mediated gene manipulation in commensal and pathogenic E. coli***

This method is a rapid PCR-based method used to inactivate the tolR gene from the wild-type *E. coli* strains [302]. Briefly, the first step consists in amplifying independently the upstream and downstream regions of the target gene (tolR) and the resistance marker cassette. The two PCR products obtained in step 1 are mixed with the amplification producer of the AB cassette at equimolar concentrations and submitted to a second round of PCR (a three way PCR) to generate a resistance marker cassette flanked by upstream and downstream 500bp (or more) regions homologous to the target gene. In the third step, large amounts (1$\mu$g) of the desired linear DNA are electroporated into lamda-red competent cells.

***Vesicle Preparation***

*1. Vesicle preparation by precipitation with TCA*

LB media was inoculated with bacteria grown on plates and incubated overnight at 37°C under gentle shaking. The culture was used to inoculate 200ml of LB at OD600 0.1. Bacteria were grown to OD600 0.4 (or as specified). Culture was centrifuged for 10 minutes at 4000 x g and the supernatant was filtered through a 0.22mm filter to remove residual bacteria.

The same experiments were also performed under iron limiting conditions by adding Dipyridyl (0.25mM) to the LB media.

Precipitation was performed by adding to the culture supernatant 10% final of a solution at 100% (w/v) TCA, 0.4% (w/v) deoxycholate. The precipitation was allowed to proceed for 30 minutes at 4°C. Precipitate was recovered by 10 minutes centrifugation at 20000 x g at 4°C. The pellet was washed once with 10% TCA (w/v) and twice with absolute ethanol. The pellet was dried with speed vac, and stored at -20°C.

The wild type and mutated strains were subjected to SDS polyacrylamide gel electrophoresis from which it could be observed that there were many more bands in the supernatant of the mutated strains than the wildtype strains. Randomly picked bands demonstrated that most of the proteins in the supernatant were membrane proteins, indicating enrichment in membrane content.

*2. Vesicle preparation by ultracentrifugation*

Culture supernatant was ultracentrifuged at 200000 x g for 2 hours at 4°C. The pellet was washed with PBS, resuspended in PBS, and stored at -20°C.

*3. Guanidinium denaturation of the vesicles*

Prior to the guanidinium denaturation, Vesicles were precipitated with ethanol. 10μg of OMV in PBS were precipitate by adding cold absolute ethanol to 90% final. Precipitation was allowed to proceed for 20 minutes at -20°C. Precipitate was recovered by 10 minutes centrifugation at 13000 x g. Pellet was resuspended with 50ml, 6M guanidinium, 15mM DTT, 200mM Tris-HCl, pH 8.0. Denaturation was allowed to proceed for 60 minutes at 60°C. Prior to digestion, solution was diluted 1/8 with a solution of 1.5M Tris pH 8.0 and 5mg of trypsin were added to the diluted solution. Digestion was allowed to proceed overnight at 37°C. Reaction was stopped by adding 0.1% final of formic acid. Peptides were extracted using Oasis extraction cartridges. Peptides were analyzed by LC coupled MS-MS.

*4. Surface digestion*

5mg of trypsin were added to 10mg of vesicles in PBS and incubated at 37°C for 3 hours. Reaction was stopped by adding 0.1 % final of formic acid. Peptides were recovered and desalted with Oasis extraction cartridge. Peptides were analyzed with LC coupled MSMS.

***VESICLE ANALYSIS***

*Protein quantification*

Proteins were quantified with the Bradford method, using the BSA as standard.

*SDS-PAGE*

Samples were analyzed with a sodium dodecyl sulfate (SDS) 4-12% polyacrylamide gel, using a Mini-Protean II electrophoresis apparatus. Samples were suspended in SDS sample buffer (0.06 M Tris-HCl pH 6.8, 10% (v/v) glycerol, 2% (w/v) SDS, 5% (v/v) 2-mercaptoethanol, 10 mg/ml bromophenol blue) and heated to 100°C for 5 min before SDS-polyacrylamide gel electrophoreis. After the run, gels were stained with Coomassie Blue

*MALDI-TOF mass spectrometry.*

Protein bands or spots were excised from gels, washed with 50 mM ammonium bicarbonate/acetonitrile (50/50, v/v) twice, washed once with pure acetonitrile and air-dried. The dried spots were digested at 37°C for 2 h by adding 7 to 10 ml of a solution containing 5 mM ammonium bicarbonate, 0.012 mg of sequencing-grade trypsin. After digestion

0.6 ml were loaded on a matrix pre-spotted target and air-dried. Spots were washed with 0.6ml of a solution of 70% ethanol, 0.1% trifluoracetic acid. Mass spectra were acquired on an ultraflex MALDI TOF mass spectrometer. Spectra were externally calibrated by using a combination of standards pre-spotted on the target. Protein identification was carried out by both automatic and manual comparisons of experimentally generated monoisotopic peaks of peptides in the mass range of 700 to 3,000 Da with computer-generated fingerprints, using the Mascot program.

*Bi-dimensional electrophoresis*

200mg of vesicles were resuspended in an Immobiline re-swelling solution (7M urea, 2M thiourea, 2% (w/v) CHAPS (2% w/v) ASB 14, 2% (v/v) IPG buffer pH 3-10 NL, 2mM TBP, 65mM DTT), and adsorbed overnight on 7 cm Immobiline DryStrips (pH 3-10 NL). Proteins were then separated by 2D electrophoresis. The first dimension was run using a IPGphor Isoelectric Focusing Unit, applying sequentially 150 V for 35 minutes, 500 V for 35 minutes, 1,000 V for 30 minutes, 2,600 V for 10 minutes, 3,500 V for 15 minutes, 4,200 V for 15 minutes, and finally 5,000 V to reach 10kVh. For the second dimension, the strips were equilibrated by two 10 minute -incubations in 4 M urea, 2 M thiourea, 30% glycerol, 2% SDS, 5mM TBP, 50Mm Tris HCl pH 8.8, 2.5% acrylamide, Bromo phenol Blue 0.2%: Proteins were then separated on linear 4-12 % precasted polyacrylamide gels.

Gels were stained with colloidal Coomassie Blue and scanned with a Personal Densitometer SI. Images were analyzed with Image Master 2D Elite software.

*Nano-LC/MS/MS*

Peptides were separated by nano-LC on a CapLC HPLC system connected to a Q-ToF Micro ESI mass spectrometer equipped with a nanospray source. Samples were loaded onto an Atlantis C18 NanoEase column (100$\mu$m i.d. x 100mm), through a C18 trap column (300$\mu$m i.d. x 5 mm). Peptides were eluted with a 50-min gradient from 2% to 60% of 95% ACN, in a solution of 0.1% formic acid at a flow rate of 400 nl/minute. The eluted peptides were subjected to an automated data-dependent acquisition program, using the MassLynx software, version 4.0, where a MS survey scan was used to automatically select multi-charged peptides over the m/z range of 400-2,000 for further MS/MS fragmentation. Up to three different components where subjected to MS/MS fragmentation at the same time. After data acquisition, the individual MS/MS spectra were combined, smoothed and centroided by MassLynx. Search and identification of peptides were performed in batch mode with a licensed version of MASCOT. The MASCOT search parameters were: (1) species: ExPEC (2) allowed number of missed cleavages (only for trypsin digestion): 6; (3) variable post-translational modifications: methionine oxidation; (4) peptide tolerance: $\pm$500 ppm; (5) MS/MS tolerance: $\pm$0.3 Da and (6): peptide charge: from +1 to +4. As for the previous platform, only significant hits as defined by MASCOT probability analysis were considered. The score thresholds for acceptance of protein identifications from at least one peptide were set by MASCOT as 18 for trypsin digestion and 36 for proteinase K digestion.

*Results*

As a result of the above analyses, 76 antigens were identified from the 536 strain as shown in Table 1. 11 of these sequences had a very low homology with commensal strains (mg1655) (SEQ ID NOS 1-10 and SEQ ID NO 85). The sequences of 7 of these polypeptides were identified by this method but not by the computer-based method. These sequences are listed in the sequence listing and their amino acid sequences are SEQ ID NOS 1 to 7. Table 1 shows the 76 polypeptides that were identified in this way.

***ANTIGEN ANALYSIS***

*Mouse Model of Systemic Infection*

To screen a large number of antigens selected by comparative genome analysis between pathogenic and non pathogenic *E. coli* strains, a protection model based on a classical virulence assay has been established. Alternative experimental models that may also be used include those outlined in references 212-214.

The experimental model (immunization and infection) uses 5 week old - CD1 outbreed mice which are challenged with intravenous inoculation of virulent UPEC 536 *E. coli* strain. The challenge dose has been experimentally determined as the amount of bacteria able to kill 80% of adult mice within 4 days and corresponds to 4x$10^7$ cfu/mouse for the 536 strain.

*Immunization Protocol*

A large number of antigens from UPEC 536 strain were cloned, expressed and purified. In addition SEQ ID

NO 168, which corresponds to the N-terminal region (amino acids 21-470) of SEQ ID NO 56, was cloned, expressed and purified. The purified antigens were used to immunize mice in the experimental mouse model in the following way. Mice are immunized three times by subcutaneous injection of 150μl of protein solution using freund's adjuvants as shown in the table below:

| | Control mice: | Immunized mice: |
|---|---|---|
| Day 0 | 75 μl of saline solution<br>75 μl of complete freund's adjuvant | 75 μl of protein solution (**20**μg)<br>75 μl of complete freund's adjuvant |
| Day 21 | 75 μl of saline solution<br>75 μl of incomplete freund's adjuvant | 75 μl of protein solution (**20**μg)<br>75 μl of incomplete freund's adjuvant |
| Day 35 | 75 μl of saline solution<br>75 μl of incomplete freund's adjuvant | 75 μl of protein solution (**20**μg)<br>75 μl of incomplete freund's adjuvant |

Blood samples are collected the day before the first immunization (preimmune serum), at day 34 and 48 (day before challenge). Sera from immunized animals are tested by western blot and ELISA to determine the antibodies titer.

*Challenge*

At day 48 *E.coli* UPEC 536 strain is streaked on LB agar plate from frozen stock and incubated overnight (ON) at 37° C in incubator. At Day 49 the ON plate-culture is used to inoculate 50 ml of LB medium to have an $O.D._{600} = 0.1$, and grown for 1.5 hours at 37°C under agitation until the bacterial culture reaches an $O.D._{600} = 1.3$ which corresponds to 4 x $10^8$ cfu/ml for the UPEC 536 strain. The culture is centrifuged and the pellet resuspended in the same volume with physiological solution and used for challenge undiluted. The culture is plated using a standard plate count method to verify the inoculum. 100μl of the cell suspension containing $4x10^7$ UPEC 536 bacteria is injected intravenously, using a 1ml syringe, to control and immunized mice. The number of deaths in each animal group at 24, 48, 72 and 96 hours after infection are recorded.

The protection due to vaccination is evaluated by comparison of the survival in the vaccinated group and the survival in control group of mice at 96 hours from the challenge. Percentage of survival relative to controls is calculated using the formula:

$$\frac{\text{rate of deaths in vaccine group} - \text{rate of deaths in control group}}{\text{rate of deaths in control group}}$$

*RESULTS*

The results can be seen in Table 3 which demonstrates that the % survival of the mice after challenge with UPEC 536 is increased following immunization with antigens of the invention.

It will be understood that the invention has been described by way of example only and modifications may be made whilst remaining within the scope and spirit of the invention.

**Table 1**

| Polypeptide from strain 536 | Length | Annotation | Psort |
|---|---|---|---|
| recp00182 | 810 | Hypothetical Protein | outer membrane |
| recp00955 | 346 | Outer membrane protein A precursor | periplasmic space |
| recp03768 | 1024 | Hemolysin A | inner membrane |
| recp03614 | 219 | Outer membrane protein | outer membrane |
| recp04758 | 394 | Elongation factor Tu-A | cytoplasm |
| recp00156 | 747 | Ferrichrome-iron receptor | outer membrane |
| recp00821 | 171 | Outer membrane protein X precursor | outer membrane |

(continued)

| Polypeptide from strain 536 | Length | Annotation | Psort |
|---|---|---|---|
| recp01147 | 360 | Outer membrane porin protein LC precursor | outer membrane |
| recp02564 | 245 | LIPOPROTEIN, COML FAMILY | outer membrane |
| recp03085 | 493 | Outer membrane protein tolC | outer membrane |
| recp00198 | 236 | COPPER HOMEOSTASIS PROTEIN CUTF | outer membrane |
| recp00751 | 173 | Peptidoglycan-associated lipoprotein | outer membrane |
| recp02152 | 332 | D-galactose-binding protein | inner membrane |
| recp02221 | 375 | Outer membrane protein C precursor | outer membrane |
| recp03591 | 1157 | Cellulose synthase operon C protein | periplasmic space |
| Recp02237 | 562 | Hypothetical Protein | periplasmic space |
| Recp00167 | 474 | PROTEASE DO (EC 3.4.21.-) | outer membrane |
| Recp02453 | 344 | Lipoprotein-34 precursor | outer membrane |
| Recp00092 | 420 | Cell division protein ftsA | inner membrane |
| Recp00183 | 161 | Histone-like protein HLP-1 precursor | periplasmic space |
| Recp01092 | 213 | putative lipoprotein | outer membrane |
| Recp02481 | 392 | Hypothetical Protein | outer membrane |
| Recp03191 | 678 | GS60 ANTIGEN | outer membrane |
| Recp03194 | 191 | Hypothetical Protein | outer membrane |
| Recp03399 | 270 | Peptidyl-prolyl cis-trans isomerase (EC 5.2.1.8) | periplasmic space |
| Recp04801 | 71 | Hypothetical Protein | periplasmic space |
| Recp00778 | 427 | Putative lipoprotein ybHC precursor | outer membrane |
| Recp01607 | 700 | Acetyl-CoA:acetoacetyl-CoA transferase alpha subunit (EC 2.8.3.-) | cytoplasm |
| Recp01205 | 270 | Septum site-determining protein minD | cytoplasm |
| Recp01576 | 155 | Outer membrane lipoprotein slyB precursor | outer membrane |
| Recp02965 | 948 | Antigen 43 precursor | outer membrane |
| Recp00323 | 1042 | Antigen 43 precursor | inner membrane |
| recp00299 | 725 | Hypothetical Protein | outer membrane |
| recp00752 | 263 | Hypothetical Protein | periplasmic space |
| recp00933 | 362 | Outer membrane protein F precursor | outer membrane |
| recp01276 | 891 | Aldehyde-alcohol dehydrogenase | inner membrane |
| recp01416 | 539 | Protein ydcG precursor | inner membrane |
| recp01834 | 167 | Ferritin-like protein 2 | cytoplasm |
| recp01846 | 555 | Flagellin | cytoplasm |
| recp02458 | 487 | Hypothetical Protein | inner membrane |
| recp02979 | 558 | Polysialic acid transport protein kpsD | outer membrane |
| recp03533 | 524 | Dipeptide-binding protein | periplasmic space |
| recp03742 | 409 | Hypothetical Protein | inner membrane |
| recp03841 | 471 | Tryptophanase (EC 4.1.99.1) | cytoplasm |

(continued)

| Polypeptide from strain 536 | Length | Annotation | Psort |
|---|---|---|---|
| recp04121 | 628 | Vitamin B12 receptor precursor | inner membrane |
| recp04474 | 1024 | Hemolysin, chromosomal | inner membrane |
| recp04553 | 878 | Outer membrane usher protein fimD | inner membrane |
| recp04625 | 645 | Soluble lytic murein transglycosylase (EC 3.2.1.-) | periplasmic space |
| recp04667 | 274 | Putative ATP binding protein SugR | cytoplasm |
| recp01536 | 314 | Protein ydgH precursor | periplasmic space |
| recp00086 | 438 | UDP-N-acetylmuramoylalanine--D-glutamate ligase (EC 6.3.2.9) | inner membrane |
| Recp00769 | 229 | Molybdenum transport system permease protein modB | inner membrane |
| Recp01006 | 363 | Putative monooxygenase ycdM | inner membrane |
| Recp01611 | 78 | Major outer membrane lipoprotein precursor | outer membrane |
| Recp01750 | 518 | Hypothetical Protein | inner membrane |
| Recp02349 | 446 | Long-chain fatty acid transport protein precursor | outer membrane |
| Recp04188 | 446 | Maltoporin precursor | outer membrane |
| Recp00053 | 237 | Hypothetical Protein | cytoplasm |
| Recp00054 | 191 | Peptidyl-prolyl cis-trans isomerase (EC 5.2.1.8) | periplasmic space |
| Recp00118 | 630 | Dihydrolipoamide acetyltransferase component of pyruvate dehydrogenase | inner membrane |
| Recp00535 | 550 | Protein ushA precursor | periplasmic space |
| Recp00750 | 430 | TolB protein | outer membrane |
| Recp01673 | 112 | Osmotically inducible lipoprotein E precursor | outer membrane |
| Recp02416 | 191 | putative lipoprotein | outer membrane |
| Recp02876 | 230 | PERIPLASMIC IMMUNOGENIC PROTEIN | cytoplasm |
| Recp03068 | 130 | Protein ygiW precursor | periplasmic space |
| recp03340 | 127 | LSU ribosomal protein L17P | cytoplasm |
| recp03342 | 206 | SSU ribosomal protein S4P | cytoplasm |
| recp03556 | 188 | Outer membrane protein slp precursor | outer membrane |
| recp04322 | 548 | 60 kDa chaperonin | cytoplasm |
| recp04464 | 205 | PapA protein | outer membrane |
| recp04606 | 201 | Osmotically inducible protein Y precursor | periplasmic space |
| recp00609 | 749 | Ferrienterobactin receptor precursor | outer membrane |
| recp02100 | 350 | Fructose-bisphosphate aldolase class I | cytoplasm |
| recp02302 | 714 | Phosphate acetyltransferase | inner membrane |
| recp03559 | 660 | Hemin receptor | outer membrane |

**TABLE 2 - 7 preferred pathogenic *E.coli 536* strain sequences**

SEQ ID NO: 1 (RECP04801)

MTMKLIKTLVAVSALSMMSFGVFAQSVSATASTLDRAEAKIAAQAAEQGASYKITSARVENRVYMTAELLK

SEQ ID NO : 2 (RECP04667)

IRHILAVRQSTLLQIDTLIRQLAEISAMTVSIGGKTALDWAMKQDFRCGFWLMEKPEIAMKAIT
RSGMLSLMDAQARETWYRSLEYDNVPEISEANILNTFKQLHQNKDEVFERGVINVFRGLNWNYK
NNLVRWDRWGFHIVTGQQADRVADLERMLHLFSGWPIPDNRENIIIRLDDHIQSAQGQECYEYE
AHITFRKPELIDRLNDIIAKYYPEIIPYNI

SEQ ID NO : 3 (RECP03768)

MPTITTAQIKSTLQSAKQSAANKLHSAGQSTKDALKKAAEQTRNAGNRLILLIPKDYKGQGSSLNDLVRTADELGIE
VQYDEKNGTAITKQVFGTAEKLIGLTERGVTIFAPQLDKLLQKYQKAGNKLGGSAENIGDNLGKAGSVLSTFQNFLG
TALSSMKIDELIKKQKSGSNVSSSELAKASIELINQLVDTAASINNNVNSFSQQLNKLGSVLSNTKHLNGVGNKLQN
LPNLDNIGAGLDTVSGILSVISASFILSNADADTGTKAAAGVELTTKVLGNVGKGISQYIIAQRAAQGLSTSAAAAG
LIASAVTLAISPLSFLSIADKFKRANKIEEYSQRFKKLGYDGDSLLAAFHKETGAIDASLTTISTVLASVSSGISAA
ATTSLVGAPVSALVGAVTGIISGILEASKQAMFEHVASKMADVIAEWEKKHGKNYFENGYDARHAAFLEDNFKILSQ
YNKEYSVERSVLITQQHWDTLIGELAGVTRNGDKTLSGKSYIDYYEEGKRLEKKPDEFQKQVFDPLKGNIDLSDSKS
STLLKFVTPLLTPGEEIRERRQSGKYEYITELLVKGVDKWTVKGVQDKGSVYDYSNLIQHASVGNNQYREIRIESHL
GDGDDKVFLAAGSANIYAGKGHDVVYYDKTDTGYLTIDGTKATEAGNYTVTRVLGGDVKVLQEVVKEQEVSVGKRTE
KTQYRSYEFTHINGTDLTETDNLYSVEELIGTNRADKFFGSKFTDIFHGADGDDHIEGNDGNDRLYGDKGNDTLRGG
NGDDQLYGGDGNDKLTGGVGNNYLNGGDGDDELQVQGNSLAKNVLSGGKGNDKLYGSEGADLLDGGEGNDLLKGGYG
NDIYRYLSGYGHHIIDDDGGKDDKLSLADIDFRDVAFKREGNDLIMYKAEGNVLSIGHKNGITFRNWFEKESGDISN
HQIEQIFDKDGRVITPDSLKKAFEYQQSNNQANYVYGEYASTYADLDNLNPLINEISKIISAAGNFDVKEERSAASL
LQLSGNASDFSYGRNSITLTASA

SEQ ID NO : 4 (RECP03742)

MELIMPLSRRNFIQNAVLGISAAGLSAAPALAKNISSSTAHIISKTSGHADTSTSKSLHIISL
AYAYIAHGAGDEWTYHENRRAFSDYPLLPHRLSGVAAHSIDIRTDLLGHHLEHPLLIAPMGAH
AEKAGALYESSGASNRSLEDIAKASKGPKWFQLYFNADAGVTRSLLERAKAAGYSAIIITADA
PFPAGATFGNHDPRYGGKGDFFNQKVELTPADIEFVKKITGLPVIVKGILRGEDAVVAIDAGA
GVPSAISQLQEVAARVGHKVPVIFDSGIRRGIDVVRAISLGATAVAVGRPVLYGIAAGGVGGV
MLLSGARTLKDLSQGFIRNKETEH

SEQ ID NO : 2 (RECP01846)

(continued)

NKNQSALSSSIERLSSGLRINSAKDDAAGQAIANRFTSNIKGLTQAARNANDGISVAQT
TVQASTGTNSDSDLDSIQDEIKSRLDEIDRVSGQTQFNGVNVLAKDGSMKIQVGANDGQ
FNVNGKGAVANTAATKDDLVAASVSAAVGNEYTVSAGLSKSTAADVIASLTDGATVTAA
QANNTFTYNTTSTAAELQSYLTPKAGDTATFSVEIGSTKQDVVLASDGKITAKDGSKLY
TLNGLAFNHSGPAAAVQSTITTADGTSIVLAGSGDFGTTKTAGAINVTGAVISADALLS
VVKSGGNDVYNKADGTGLTTDNTTKYYLQDDGSVTNGSGKAVYVDATGKLTTDAETKAA
LDEAISSIDKFRSSLGAVQNRLDSAVTNLNNTTTNLSEAQSRIQDADYATEVSNMSKAQIIQQAGNSVL
QVLSLLQG

SEQ ID NO: 6 (RECP03559)

MSRPQFTSLRLSLLALAVSATLPTFAFATETMTVTATGNARSSFEAPMMVSVIDTSAPENQTATSATDLLRYVPGIT
LDGTGRTNGQDVNMRGYDHRGVLVLVDGVRQGTDTGHLNGTFLDPALIKRVEIVRGPSALLYGSGALGGVISYDTVD
AKDLLQEGQSSGFRVFGTGGTGDHSLGLGASAFGRTENLDGIVAWSSRDRGDLRQSNGETAPNDESINNMLAKGTWQ
IDSAQSLSGLVRYYNNDAREPKNPQTVEASDSSNPMVDRSTIQRDAQLSYKLAPQGNDWLNADAKIYWSEVRINAQN
TGSSGEYREQITKGARLENRSTLFADSFASHLLTYGGEYYRQEQHPGGATTGFPQAKIDFSSGWLQDEITLRDLPIT
LLGGTRYDSYRGSSDGYKDVDADKWSSRAGMTINPTNWLMLFGSYAQAFRAPTMGEMYNDSKHFSIGRFYTNYWVPN
PNLRPETNETQEYGFGLRFDDLMLSNDALEFKASYFDTKAKDYISTTVDFAAATTMSYNVPNAKIWGWDVMTKYTTD
LFSLDVAYNRTRGKDTDTGEYISSINPDTVTSTLNIPIAHSGFSVGWVGTFADRSTHISSSYSKQPGYGVNDFYVSY
QGQQALKGMTTTLVLGNAFDKEYWSPQGIPQDGRNGKIFVSYQW

SEQ ID NO: 6 (RECP04474)

(continued)

QIKSTLQSAKQSAANKLHSAGQSTKDALKKAAEQTRNAGNRLILLIPKDYKGQGSSLNDLVRTADELGIE
GTAITKQVFGTAEKLIGLTERGVTIFAPQLDKLLQKYQKAGNKLGGSAENIGDNLGKAGSVLSTFQNFLG
IDELIKKQKSGGNVSSSELAKASIELINQLVDTAASLNNNVNSFSQQLNKLGSVLSNTKHLNGVGNKLQN
GAGLDTVSGILSAISASFILSNADADTGTKAAAGVELTTKVLGNVGKGISQYIIAQRAAQGLSTSAAAAG
LAISPLSFLSIADKFKRANKIEEYSQRFKKLGYDGDSLLAAFHKETGAIDASLTTISTVLASVSSGISAA
APVSALVGAVTGIISGILEASKQAMFEHVASKMADVIAEWEKKHGKNYFENGYDARHAAFLEDNFEILSQ
ERSVLITQQHWDTLIGELAGVTRNGDKTLSGKSYIDYYEEGKRLEKEPDEFQKQVFDPLKGNIDLSVIKS
TPLLTPGKEIRERRQSGKYEYITELLVKGVDKWTVKGVQDKGSVYDYSNLIQHASVGNNQYREIRIESHL
TLSAGSANIYAGKGHDVVYYDKTDTGYLTIDGTKATEAGNYTVTRVLGGDVKVLQEVVKEQEVSVGKRTE
EFTHINGTDLTETDNLYSVEELIGTNRADKFFGSKFTDIFHGADGDDHIEGNDGNDRLYGDKGNDTLRGG
GGDGNDKLTGGVGNNYLNGGDGDDELQVQGNSLAKNVLSGGKGNDKLYGSEGADLLDGGEGNDLLKGGYG
SGYGHHIIDDDGGKDDKLSLADIDFRDVAFKREGNDLIMYKAEGNVLSIGHKNGITFRNWFEKESGDISN
DKDGRVITPDSLKKAFEYQQSNNQANYVYGEYASTYADLDNLNPLINEISKIISAAGNFDVKEERSAASL
SDFSYGRNSITLTASA

Table 3

| Antigens | Annotation | SEQ ID NO: | Animal model | | |
|---|---|---|---|---|---|
| | | | survival immun.(%) | Survival ctrl. (%) | Protection % |
| Recp02040 | Lipopolysaccharide N-acetylglucosaminyltransferase | 95 | 6/10 (60) | 3/10 (30) | 43 |
| Recp03760 | Hypothetical Protein | 80 | 4/10 (40) | 1/10 (10) | 33 |
| Recp03740 | F17 fimbrial protein precursor | 73 | 5/10 (50) | 3/10 (30) | 28,5 |
| Recp03761 | Hypothetical Protein | 81 | 5/10 (50) | 3/10 (30) | 28,5 |
| Recp02934 | PapH protein | 65 | 5/10 (50) | 3/10 (30) | 28,5 |
| Recp01398A | Putative surface-exposed virulence protein bigA | 168 | 3/8 (37,5) | 1/10(10) | 30 |
| Recp04460 | PapJ protein | 98 | 4/8 (50) | 1/10(10) | 44 |
| Recp04462 | PapC protein | 15 | 6/8 (75) | 1/10(10) | 72 |
| Recp04463 | PapH protein | 166 | 3/8 (37,5) | 1/10(10) | 30 |
| Recp04475 | Hemolysin C | 167 | 4/8 (50) | 1/10(10) | 44 |

TABLE 4

| SEQ ID NO | Name | | SEQ ID NO | Name | | SEQ ID NO | Name |
|---|---|---|---|---|---|---|---|
| 1 | RECP04801 | | 57 | RECP02717 | | 113 | RECP02221 |
| 2 | RECP04667 | | 58 | RECP02774 | | 114 | RECP03591 |
| 3 | RECP03768 | | 59 | RECP02778 | | 115 | RECP02237 |
| 4 | RECP03742 | | 60 | RECP02929 | | 116 | RECP00167 |
| 5 | RECP01846 | | 61 | RECP02930 | | 117 | RECP02453 |
| 6 | RECP03559 | | 62 | RECP02931 | | 118 | RECP00092 |
| 7 | RECP04474 | | 63 | RECP02932 | | 119 | RECP00183 |
| 8 | RECP02965 | | 64 | RECP02933 | | 120 | RECP01092 |
| 9 | RECP00299 | | 65 | RECP02934 | | 121 | RECP02481 |
| 10 | RECP02979 | | 66 | RECP02935 | | 122 | RECP03191 |
| 11 | RECP00065 | | 67 | RECP02956 | | 123 | RECP03194 |
| 12 | RECP00145 | | 68 | RECP02963 | | 124 | RECP03399 |
| 13 | RECP00286 | | 69 | RECP03142 | | 125 | RECP00778 |
| 14 | RECP02936 | | 70 | RECP03144 | | 126 | RECP01607 |
| 15 | RECP04462 | | 71 | RECP03145 | | 127 | RECP01205 |
| 16 | RECP04479 | | 72 | RECP03146 | | 128 | RECP01576 |
| 17 | RECP01388 | | 73 | RECP03740 | | 129 | RECP00323 |
| 18 | RECP04480 | | 74 | RECP03747 | | 130 | RECP00752 |
| 19 | RECP00323 | | 75 | RECP03755 | | 131 | RECP00933 |
| 20 | RECP01887 | | 76 | RECP03756 | | 132 | RECP01276 |
| 21 | RECP01896 | | 77 | RECP03757 | | 133 | RECP01416 |

(continued)

| SEQ ID NO | Name | | SEQ ID NO | Name | | SEQ ID NO | Name |
|---|---|---|---|---|---|---|---|
| 22 | RECP01897 | | 78 | RECP03758 | | 134 | RECP01834 |
| 23 | RECP01900 | | 79 | RECP03759 | | 135 | RECP02458 |
| 24 | RECP01903 | | 80 | RECP03760 | | 136 | RECP03533 |
| 25 | RECP01904 | | 81 | RECP03761 | | 137 | RECP03841 |
| 26 | RECP01907 | | 82 | RECP03764 | | 138 | RECP04121 |
| 27 | RECP02039 | | 83 | RECP03766 | | 139 | RECP04553 |
| 28 | RECP02042 | | 84 | RECP03776 | | 140 | RECP04625 |
| 29 | RECP02043 | | 85 | RECP04464 | | 141 | RECP01536 |
| 30 | RECP02338 | | 86 | RECP04575 | | 142 | RECP00086 |
| 31 | RECP04656 | | 87 | RECP04740 | | 143 | RECP00769 |
| 32 | RECP01901 | | 88 | RECP04742 | | 144 | RECP01006 |
| 33 | RECP02045 | | 89 | RECP00320 | | 145 | RECP01611 |
| 34 | RECP02046 | | 90 | RECP02779 | | 146 | RECP01750 |
| 35 | RECP03810 | | 91 | RECP02962 | | 147 | RECP02349 |
| 36 | RECP04478 | | 92 | RECP02993 | | 148 | RECP04188 |
| 37 | RECP00289 | | 93 | RECP03765 | | 149 | RECP00053 |
| 38 | RECP02782 | | 94 | RECP04686 | | 150 | RECP00054 |
| 39 | RECP03743 | | 95 | RECP02040 | | 151 | RECP00118 |
| 40 | RECP03745 | | 96 | RECP02195 | | 152 | RECP00535 |
| 41 | RECP04453 | | 97 | RECP02476 | | 153 | RECP00750 |
| 42 | RECP04456 | | 98 | RECP04460 | | 154 | RECP01673 |
| 43 | RECP04458 | | 99 | RECP03796 | | 155 | RECP02416 |
| 44 | RECP04496 | | 100 | RECP04046 | | 156 | RECP02876 |
| 45 | RECP04540 | | 101 | RECP00182 | | 157 | RECP03068 |
| 46 | RECP04705 | | 102 | RECP00955 | | 158 | RECP03340 |
| 47 | RECP03665 | | 103 | RECP03614 | | 159 | RECP03342 |
| 48 | RECP03771 | | 104 | RECP04758 | | 160 | RECP03556 |
| 49 | RECP04505 | | 105 | RECP00156 | | 161 | RECP04322 |
| 50 | RECP00271 | | 106 | RECP00821 | | 162 | RECP04606 |
| 51 | RECP00274 | | 107 | RECP01147 | | 163 | RECP00609 |
| 52 | RECP00318 | | 108 | RECP02564 | | 164 | RECP02100 |
| 53 | RECP01184 | | 109 | RECP03085 | | 165 | RECP02302 |
| 54 | RECP01306 | | 110 | RECP00198 | | 166 | RECP04463 |
| 55 | RECP01307 | | 111 | RECP00751 | | 167 | RECP04475 |
| 56 | RECP01398 | | 112 | RECP02152 | | 168 | RECP01398A |

**REFERENCES** (the contents of which are hereby incorporated by reference)

[1] Russo & Johnson (2000) J Infect Dis 181:1753-1754.

[2] Uehling et al. (1997) J Urol 157:2049-2052.
[3] Tammen (1990) Br J Urol 65:6-9.
[4] Langermann et al. (1997) Science 276:607-611.
[5] WO03/074553.
[6] WO01/66572.
[7] Janke et al. (2001) FEMS Microbiol Lett 199:61-66.
[8] W02004/005535.
[9] Dobrindt et al. (2002) Infect Immun 70:6365-6372.
[10] US2003/0165870.
[11] Welch et al. (2002) Proc Natl Acad Sci USA 99:17020-17024.
[12] American Type Culture Collection: ATCC 700928.
[13] European Journal of Biochemistry 2003; 1 Supplement 1 July: abstract P1.3-11.
[14] Geysen et al. (1984) PNAS USA 81:3998-4002.
[15] Carter (1994) Methods Mol Biol 36:207-223.
[16] Jameson, BA et al. 1988, CABIOS 4(1):181-186.
[17] Raddrizzani & Hammer (2000) Brief Bioinform 1(2):179-189.
[18] De Lalla et al. (1999) J. Immunol. 163:1725-1729.
[19] Brusic et al. (1998) Bioinformatics 14(2):121-130
[20] Meister et al. (1995) Vaccine 13(6):581-591.
[21] Roberts et al. (1996) AIDS Res Hum Retroviruses 12(7):593-610.
[22] Maksyutov & Zagrebelnaya (1993) Comput Appl Biosci 9(3):291-297.
[23] Feller & de la Cruz (1991) Nature 349(6311):720-721.
[24] Hopp (1993) Peptide Research 6:183-190.
[25] Welling et al. (1985) FEBS Lett. 188:215-218.
[26] Davenport et al. (1995) Immunogenetics 42:392-397.
[27] WO2006/091517.
[28] W02006/089264.
[29] Bodanszky (1993) Principles of Peptide Synthesis (ISBN: 0387564314).
[30] Fields et al. (1997) Meth Enzymol 289: Solid-Phase Peptide Synthesis. ISBN: 0121821900.
[31] Chan & White (2000) Fmoc Solid Phase Peptide Synthesis. ISBN: 0199637245.
[32] Kullmann (1987) Enzymatic Peptide Synthesis. ISBN: 0849368413.
[33] Ibba (1996) Biotechnol Genet Eng Rev 13:197-216.
[34] Breedveld (2000) Lancet 355(9205):735-740.
[35] Gorman & Clark (1990) Semin. Immunol. 2:457-466.
[36] US patent 5,707,829
*[37]* Current Protocols in Molecular Biology (F.M. Ausubel et al. eds., 1987) Supplement 30.
[38] EP-B-0509612.
[39] EP-B-0505012.
[40] Johnson & Stell (2001) J Clin Microbiol 39:3712-3717.
[41] Tang et al. (1997) Clin. Chem. 43:2021-2038.
[42] WO2006/046143
[43] Bernadac et al. (1998) J Bacteriol 180(18):4872-4878.
[44] EP-1441036.
[45] Sorensen & Mortensen (2005) Journal of Biotechnology 115:113-128.
[46] Meynial-Salles et al. (2005) Applied and Environmental Microbiology 71:2140-2144.
[47] US2004/0209370.
[48] WO00/68253.
[49] WO97/04110.
[50] Alper et al. (2005) Proc. Natl. Acad. Sci. USA 102:12678-12683.
[51] WO 01/09350.
[52] European patent 0624376.
*[53]* Vaccine Design... (1995) eds. Powell & Newman. ISBN: 030644867X. Plenum.
[54] WO00/23105.
[55] WO90/14837.
[56] Podda (2001) Vaccine 19:2673-2680.
[57] Frey et al. (2003) Vaccine 21:4234-4237.
[58] Vaccine Adjuvants: Preparation Methods and Research Protocols (Volume 42 of Methods in Molecular Medicine series). ISBN: 1-59259-083-7. Ed. O'Hagan.

[59] US Patent 6,299,884.
[60] US Patent 6,451,325.
[61] Allison & Byars (1992) Res Immunol 143:519-525.
[62] Hariharan et al. (1995) Cancer Res 55:3486-3489.
[63] US patent 5,057,540.
[64] WO96/33739.
[65] EP-A-0109942.
[66] WO96/11711.
[67] WO00/07621.
[68] Barr et al. (1998) Advanced Drug Delivery Reviews 32:247-271.
[69] Sjolanderet et al. (1998) Advanced Drug Delivery Reviews 32:321-338.
[70] Niikura et al. (2002) Virology 293:273-280.
[71] Lenz et al. (2001) J Immunol 166:5346-5355.
[72] Pinto et al. (2003) J Infect Dis 188:327-338.
[73] Gerber et al. (2001) Virol 75:4752-4760.
[74] WO03/024480
[75] WO03/024481
[76] Gluck et al. (2002) Vaccine 20:B10-B16.
[77] EP-A-0689454.
[78] Johnson et al. (1999) Bioorg Med Chem Lett 9:2273-2278.
[79] Evans et al. (2003) Expert Rev Vaccines 2:219-229.
[80] Meraldi et al. (2003) Vaccine 21:2485-2491.
[81] Pajak et al. (2003) Vaccine 21:836-842.
[82] Kandimalla et al. (2003) Nucleic Acids Research 31:2393-2400.
[83] WO02/26757.
[84] WO99/62923.
[85] Krieg (2003) Nature Medicine 9:831-835.
[86] McCluskie et al. (2002) FEMS Immunology and Medical Microbiology 32:179-185.
[87] WO98/40100.
[88] US patent 6,207,646.
[89] US patent 6,239,116.
[90] US patent 6,429,199.
[91] Kandimalla et al. (2003) Biochemical Society Transactions 31 (part 3):654-658.
[92] Blackwell et al. (2003) J Immunol 170:4061-4068.
[93] Krieg (2002) Trends Immunol 23:64-65.
[94] WO01/95935.
[95] Kandimalla et al. (2003) BBRC 306:948-953.
[96] Bhagat et al. (2003) BBRC 300:853-861.
[97] WO03/035836.
[98] WO95/17211.
[99] WO98/42375.
[100] Beignon et al. (2002) Infect Immun 70:3012-3019.
[101] Pizza et al. (2001) Vaccine 19:2534-2541.
[102] Pizza et al. (2000) Int J Med Microbiol 290:455-461.
[103] Scharton-Kersten et al. (2000) Infect Immun 68:5306-5313.
[104] Ryan et al. (1999) Infect Immun 67:6270-6280.
[105] Partidos et al. (1999) Immunol Lett 67:209-216.
[106] Peppoloni et al. (2003) Expert Rev Vaccines 2:285-293.
[107] Pine et al. (2002) J Control Release 85:263-270.
[108] Domenighini et al. (1995) Mol Microbiol 15:1165-1167.
[109] WO03/011223.
[110] WO99/40936.
[111] WO99/44636.
[112] Lillard JW et al., (2003) Blood 101(3):807-814. Epub 2002 Sep 12.
[113] Singh et al] (2001) J Cont Release 70:267-276.
[114] WO99/27960.
[115] US patent 6,090,406
[116] US patent 5,916,588

[117] EP-A-0626169.
[118] WO99/52549.
[119] WO01/21207.
[120] WO01/21152.
[121] Andrianov et al. (1998) Biomaterials 19:109-115.
[122] Payne et al. (1998) Adv Drug Delivery Review 31:185-196.
[123] US 4,680,338.
[124] US 4,988,815.
[125] WO92/15582.
[126] Stanley (2002) Clin Exp Dermatol 27:571-577.
[127] Wu et al. (2004) Antiviral Res. 64(2):79-83.
[128] Vasilakos et al. (2000) Cell Immunol. 204(1):64-74.
[129] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[130] Jones (2003) Curr Opin Investig Drugs 4:214-218.
[131] WO04/60308
[132] WO04/64759.
[133] US 6,924,271.
[134] US2005/0070556.
[135] US 5,658,731.
[136] Wong et al. (2003) J Clin Pharmacol 43(7):735-42.
[137] US2005/0215517.
[138] WO02/072012.
[139] Signorelli & Hadden (2003) Int Immunopharmacol 3(8):1177-1186.
[140] WO2004/064715.
[141] Cooper (1995) Pharm Biotechnol 6:559-580.
[142] WO2006/002422
[143] WO2004/87153.
[144] US 6,605,617.
[145] WO02/18383.
[146] WO2004/018455.
[147] WO03/082272.
[148] US patent 5,011,828.
[149] US-6586409.
[150] WO99/11241.
[151] WO94/00153.
[152] WO98/57659.
[153] European patent applications 0835318, 0735898 and 0761231.
[154] WO03/009869.
[155] Donnelly et al. (1997) Annu Rev Immunol 15:617-648.
[156] Strugnell et al. (1997) Immunol Cell Biol 75(4):364-369.
[157] Cui (2005) Adv Genet 54:257-89.
[158] Robinson & Torres (1997) Seminars in Immunol 9:271-283.
[159] Brunham et al. (2000) J Infect Dis 181 Suppl 3:S538-S543.
[160] Svanholin et al. (2000) Scand J Immunol 51(4):345-353.
[161] DNA Vaccination - Genetic Vaccination (1998) eds. Koprowski et al. (ISBN 3540633928).
[162] Gene Vaccination: Theory and Practice (1998) ed. Raz (ISBN 3540644288).
[163] Findeis et al., Trends Biotechnol. (1993) 11:202.
[164] Chiou et al. (1994) Gene Therapeutics: Methods And Applications Of Direct Gene Transfer. ed. Wolff.
[165] Wu et al., J. Biol. Chem. (1988) 263:621.
[166] Wu et al., J. Biol. Chem. (1994) 269:542.
[167] Zenke et al., Proc. Natl. Acad. Sci. (USA) (1990) 87:3655.
[168] Wu et al., J. Biol. Chem. (1991) 266:338.
[169] Jolly, Cancer Gene Therapy (1994) 1:51.
[170] Kimura, Human Gene Therapy (1994) 5:845.
[171] Connelly, Human Gene Therapy (1995) 1:185.

[172] Kaplitt, Nature Genetics (1994) 6:148.
[173] WO 90/07936.
[174] WO 94/03622.
[175] WO 93/25698.
[176] WO 93/25234.
[177] US 5,219,740.
[178] WO 93/11230.
[179] WO 93/10218.
[180] US 4,777,127.
[181] GB 2,200,651.
[182] EP-A-0 345 242.
[183] WO 91/02805.
[184] WO 94/12649.
[185] WO 93/03769.
[186] WO 93/19191.
[187] WO 94/28938.
[188] WO 95/11984.
[189] WO 95/00655.
[190] Curiel, Hum. Gene Ther. (1992) 3:147.
[191] Wu, J. Biol. Chem. (1989) 264:16985.
[192] US 5,814,482.
[193] WO 95/07994.
[194] WO 96/17072.
[195] WO 95/30763.
[196] WO 97/42338.
[197] WO 90/11092.
[198] US 5,580,859.
[199] US 5,422,120.
[200] WO 95/13796.
[201] WO 94/23697.
[202] WO 91/14445.
[203] EP 0524968.
[204] Philip, Mol. Cell Biol. (1994) 14:2411.
[205] Woffendin, Proc. Natl. Acad. Sci. (1994) 91:11581.
[206] US 5,206,152.
[207] WO 92/11033.
[208] US 5,149,655.
[209] Glezen & Alpers (1999) Clin. Infect. Dis. 28:219-224.
[210] Johnson et al. (2001) Infect Immun 69:1306-1314.
[211] Johnson et al. (2001) J Infect Dis 183:897-906 (see also 183:1546).
[212] Johnson, Hopkins (1998) Infection and Immunity 66:6063-6064.
[213] Bahrani-Mougeot et al.; Molecular Microbiology (2002) 45(4), 1079-1093
[214] Johnson et al., Infection and Immunity, (1998) 3059-3065
[215] Almeida & Alpar (1996) J. Drug Targeting 3:455-467.
[216] Agarwal & Mishra (1999) Indian J Exp Biol 37:6-16.
[217] Costantino et al. (1992) Vaccine 10:691-698.
[218] Costantino et al. (1999) Vaccine 17:1251-1263.
[219] International patent application WO03/007985.
[220] WO 99/24578.
[221] WO 99/36544.
[222] WO 99/57280.
[223] WO 00/66791.
[224] WO 01/64922.
[225] WO 01/64920.
[226] WO 03/020756.
[227] WO 2004/032958.
[228] WO 2004/048404.
[229] Watson (2000) Pediatr Infect Dis J 19:331-332.

[230] Rubin (2000) Pediatr Clin North Am 47:269-285.
[231] Jedrzejas (2001) Microbiol Mol Biol Rev 65:187-207.
[232] Bell (2000) Pediatr Infect Dis J 19:1187-1188.
[233] Iwarson (1995) APMIS 103:321-326.
[234] Gerlich et al. (1990) Vaccine 8 Suppl:S63-68 & 79-80.
[235] Hsu et al. (1999) Clin Liver Dis 3:901-915.
[236] Stratov et al. (2004) Curr Drug Tgts 5(1):71-88.
[237] Plotkin et al. (eds) (2003) Vaccines, 4th edition (W.B. Saunders Company)
[238] Del Guidice et al. (1998) Molecular Aspects of Medicine 19:1-70.
[239] Gustafsson et al. (1996) N. Engl. J. Med. 334:349-355.
[240] Rappuoli et al. (1991) TIBTECH9:232-238.
[241] Sutter et al. (2000) Pediatr Clin North Am 47:287-308.
[242] Zimmerman & Spann (1999) Am Fam Physician 59:113-118, 125-126.
[243] McMichael (2000) Vaccine 19 Suppl 1:S101-S107.
[244] Schuchat (1999) Lancet 353(9146):51-56.
[245] WO02/34771.
[246] WO 99/54457.
[247] WO 04/01846.
[248] WO 04/041157.
[249] WO 05/028618.
[250] WO 02/079243.
[251] WO 02/02606.
[252] Kalman et al. (1999) Nature Genetics 21:385-389.
[253] Read et al. (2000) Nucleic Acids Res 28:1397-1406.
[254] Shirai et al. (2000) J. Infect. Dis. 181(Suppl 3):S524-S527.
[255] WO 99/27105.
[256] WO 00/27994.
[257] WO 00/37494.
[258] WO2005/084306.
[259] WO2005/002619.
[260] Ross et al. (2001) Vaccine 19:4135-4142.
[261] Dreesen (1997) Vaccine 15 Suppl:S2-S6.
[262] MMWR Morb Mortal Wkly Rep 1998 Jan 16;47(1):12, 19.
[263] Anderson (2000) Vaccine 19 Suppl 1:S59-S65.
[264] Kahn (2000) Curr Opin Pediatr 12:257-262.
[265] Crowe (1995) Vaccine 13:415-421.
[266] Modlin et al. (2001) J Toxicol Clin Toxicol 39:85-100.
[267] Demicheli et al. (1998) Vaccine 16:880-884.
[268] Stepanov et al. (1996) J Biotechnol 44:155-160.
[269] Dale (1999) Infect Dis Clin North Am 13:227-43, viii.
[270] Ferretti et al. (2001) PNAS USA 98: 4658-4663.
[271] Kuroda et al. (2001) Lancet 357(9264):1225-1240; see also pages 1218-1219.
[272] WO 00/09699.
[273] WO 05/002619
[274] WO 05/084306
[275] EP-A-0372501
[276] EP-A-0378881
[277] EP-A-0427347
[278] WO93/17712
[279] WO94/03208
[280] WO98/58668
[281] EP-A-0471177
[282] EP-A-0594610.
[283] WO00/56360
[284] WO91/01146
[285] WO00/61761
[286] WO01/72337
[287] Falugi et al. (2001) Eur J Immunol 31:3816-3824.

[288] Baraldo et al, (2004) Infect Immun. 72:4884-4887
[289] WO02/091998.
[290] Kuo et al. (1995) Infect Immun 63:2706-2713.
[291] Research Disclosure, 453077 (Jan 2002)
[292] Needleman& Wunsch (1970) J. Mol. Biol. 48, 443-453.
[293] Rice et al. (2000) Trends Genet 16:276-277.
[294] Gennaro (2000) Remington: The Science and Practice of Pharmacy. 20th edition, ISBN: 0683306472.
*[295]* Methods In Enzymology (S. Colowick and N. Kaplan, eds., Academic Press, Inc.)
*[296]* Handbook of Experimental Immunology, Vols. I-IV (D.M. Weir and C.C. Blackwell, eds, 1986, Blackwell Scientific Publications)
[297] Sambrook et al. (2001) Molecular Cloning: A Laboratory Manual, 3rd edition (Cold Spring Harbor Laboratory Press).
*[298]* Handbook of Surface and Colloidal Chemistry (Birdi, K.S. ed., CRC Press, 1997)
[299] Ausubel et al. (eds) (2002) Short protocols in molecular biology, 5th edition (Current Protocols).
[300] Molecular Biology Techniques: An Intensive Laboratory Course, (Ream et al., eds., 1998, Academic Press)
[301] PCR (Introduction to Biotechniques Series), 2nd ed. (Newton & Graham eds., 1997, Springer Verlag)
[302] Murphy (1998) J. Bacteriol 180:2063-2071.

## Claims

1. A polypeptide comprising: (a) an amino acid sequence selected from the group consisting of SEQ ID NOs 80, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167 and 168; (b) an amino acid sequence having at least 80% sequence identity to an amino acid sequence of (a); (c) an amino acid sequence which is a fragment of at least 10 consecutive amino acids from an amino acid sequence of (a); or (d) an amino acid sequence having at least 80% sequence identity to an amino acid sequence of (a) and including a fragment of at least 10 consecutive amino acids from an amino acid sequence of (a).

2. The polypeptide of claim 1, wherein said fragment comprises at least one B-cell epitope of (a).

3. The polypeptide of claim 1 or claim 2, for use in medicine.

4. A pharmaceutical composition comprising the polypeptide of claim 1 or claim 2, in admixture with a pharmaceutically acceptable carrier.

5. A pharmaceutical composition comprising two or more polypeptides of claim 1 or claim 2, in admixture with a pharmaceutically acceptable carrier.

6. The composition of claim 4 or claim 5, further comprising a vaccine adjuvant.

7. An immunogenic composition comprising one or more outer membrane vesicles (OMVs) expressing one or more polypeptides comprising: (a) an amino acid sequence selected from the group consisting of SEQ ID NOs 80, 1, 2, 3, 4, 5, 6, and 7 (b) an amino acid sequence having at least 80% sequence identity to an amino acid sequence of (a); (c) an amino acid sequence which is a fragment of at least 10 consecutive amino acids from an amino acid sequence of (a); or (d) an amino acid sequence having at least 80% sequence identity to an amino acid sequence of (a) and including a fragment of at least 10 consecutive amino acids from an amino acid sequence of (a).

8. The immunogenic composition of claim 7, wherein said fragment comprises at least one B-cell epitope of (a).

9. Use of the polypeptide of claim 1 or claim 2, or the immunogenic composition of claim 7 or claim 8, in the manufacture of a medicament for raising an immune response in a patient.

10. A method for raising an immune response in a patient, comprising the step of administering to the patient the pharmaceutical composition of any one of claims 4 to 6 or the immunogenic composition of claim 7 or claim 8.

11. The use as claimed in claim 9, or the method of claim 10, wherein the immune response is protective against ExPEC infection.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 65463205 P
- US 71272005 P
- US 20030148262 A
- WO 02099035 A
- WO 03074553 A
- WO 0166572 A
- WO 2004005535 A
- US 20030165870 A
- WO 2006091517 A
- WO 2006089264 A
- US 5707829 A
- EP 0509612 B
- EP 0505012 B
- WO 2006046143 A
- EP 1441036 A
- US 20040209370 A
- WO 0068253 A
- WO 9704110 A
- WO 0109350 A
- EP 0624376 A
- WO 0023105 A
- WO 9014837 A
- US 6299884 B
- US 6451325 B
- US 5057540 A
- WO 9633739 A
- EP 0109942 A
- WO 9611711 A
- WO 0007621 A
- WO 03024480 A
- WO 03024481 A
- EP 0689454 A
- WO 0226757 A
- WO 9962923 A
- WO 9840100 A
- US 6207646 B
- US 6239116 B
- US 6429199 B
- WO 0195935 A
- WO 03035836 A
- WO 9517211 A
- WO 9842375 A
- WO 03011223 A
- WO 9940936 A
- WO 9944636 A
- WO 9927960 A
- US 6090406 A
- US 5916588 A
- EP 0626169 A
- WO 9952549 A
- WO 0121207 A
- WO 0121152 A
- US 4680338 A
- US 4988815 A
- WO 9215582 A
- US 4689338 A
- US 4929624 A
- US 5238944 A
- US 5266575 A
- US 5268376 A
- US 5346905 A
- US 5352784 A
- US 5389640 A
- US 5395937 A
- US 5482936 A
- US 5494916 A
- US 5525612 A
- US 6083505 A
- US 6440992 B
- US 6627640 B
- US 6656938 B
- US 6660735 B
- US 6660747 B
- US 6664260 B
- US 6664264 B
- US 6664265 B
- US 6667312 B
- US 6670372 B
- US 6677347 B
- US 6677348 B
- US 6677349 B
- US 6683088 B
- US 6703402 B
- US 6743920 B
- US 6800624 B
- US 6809203 B
- US 6888000 B
- US 6924293 B
- WO 0460308 A
- WO 0464759 A
- US 6924271 B
- US 20050070556 A
- US 5658731 A
- US 20050215517 A
- WO 02072012 A
- WO 2004064715 A
- WO 2006002422 A
- WO 200487153 A

- US 6605617 B
- WO 0218383 A
- WO 2004018455 A
- WO 03082272 A
- US 5011828 A
- US 6586409 B
- WO 9911241 A
- WO 9400153 A
- WO 9857659 A
- EP 0835318 A
- EP 0735898 A
- EP 0761231 A
- WO 03009869 A
- WO 9007936 A
- WO 9403622 A
- WO 9325698 A
- WO 9325234 A
- US 5219740 A
- WO 9311230 A
- WO 9310218 A
- US 4777127 A
- GB 2200651 A
- EP 0345242 A
- WO 9102805 A
- WO 9412649 A
- WO 9303769 A
- WO 9319191 A
- WO 9428938 A
- WO 9511984 A
- WO 9500655 A
- US 5814482 A
- WO 9507994 A
- WO 9617072 A
- WO 9530763 A
- WO 9742338 A
- WO 9011092 A
- US 5580859 A
- US 5422120 A
- WO 9513796 A
- WO 9423697 A
- WO 9114445 A
- EP 0524968 A
- US 5206152 A
- WO 9211033 A
- US 5149655 A
- WO 03007985 A
- WO 9924578 A
- WO 9936544 A
- WO 9957280 A
- WO 0066791 A
- WO 0164922 A
- WO 0164920 A
- WO 03020756 A
- WO 2004032958 A
- WO 2004048404 A
- WO 0234771 A
- WO 9954457 A
- WO 0401846 A
- WO 04041157 A
- WO 05028618 A
- WO 02079243 A
- WO 0202606 A
- WO 9927105 A
- WO 0027994 A
- WO 0037494 A
- WO 2005084306 A
- WO 2005002619 A
- WO 0009699 A
- WO 05002619 A
- WO 05084306 A
- EP 0372501 A
- EP 0378881 A
- EP 0427347 A
- WO 9317712 A
- WO 9403208 A
- WO 9858668 A
- EP 0471177 A
- EP 0594610 A
- WO 0056360 A
- WO 9101146 A
- WO 0061761 A
- WO 0172337 A
- WO 02091998 A

**Non-patent literature cited in the description**


- **RUSSO ; JOHNSON.** *J Infect Dis,* 2000, vol. 181, 1753-1754
- **UEHLING et al.** *J Urol,* 1997, vol. 157, 2049-2052
- **TAMMEN.** *Br J Urol,* 1990, vol. 65, 6-9
- **LANGERMANN et al.** *Science,* 1997, vol. 276, 607-611
- **JANKE et al.** *FEMS Microbiol Lett,* 2001, vol. 199, 61-66
- **DOBRINDT et al.** *Infect Immun,* 2002, vol. 70, 6365-6372
- **WELCH et al.** *Proc Natl Acad Sci USA,* 2002, vol. 99, 17020-17024
- *European Journal of Biochemistry,* 01 July 2003
- **GEYSEN et al.** *PNAS USA,* 1984, vol. 81, 3998-4002
- **CARTER.** *Methods Mol Biol,* 1994, vol. 36, 207-223
- **JAMESON, BA et al.** *CABIOS,* vol. 4 (1), 181-186
- **RADDRIZZANI ; HAMMER.** *Brief Bioinform,* 2000, vol. 1 (2), 179-189

- **DE LALLA et al.** *J. Immunol.,* 1999, vol. 163, 1725-1729
- **BRUSIC et al.** *Bioinformatics,* 1998, vol. 14 (2), 121-130
- **MEISTER et al.** *Vaccine,* 1995, vol. 13 (6), 581-591
- **ROBERTS et al.** *AIDS Res Hum Retroviruses,* 1996, vol. 12 (7), 593-610
- **MAKSYUTOV ; ZAGREBELNAYA.** *Comput Appl Biosci,* 1993, vol. 9 (3), 291-297
- **FELLER ; CRUZ.** *Nature,* 1991, vol. 349 (6311), 720-721
- **HOPP.** *Peptide Research,* 1993, vol. 6, 183-190
- **WELLING et al.** *FEBS Lett.,* 1985, vol. 188, 215-218
- **DAVENPORT et al.** *Immunogenetics,* 1995, vol. 42, 392-397
- **BODANSZKY.** *Principles of Peptide Synthesis,* 1993
- **FIELDS et al.** *Meth Enzymol 289: Solid-Phase Peptide Synthesis,* 1997, vol. 289, ISBN 0121821900
- **CHAN ; WHITE.** *Fmoc Solid Phase Peptide Synthesis,* 2000, ISBN 0199637245
- **KULLMANN.** *Enzymatic Peptide Synthesis,* 1987, ISBN 0849368413
- **IBBA.** *Biotechnol Genet Eng Rev,* 1996, vol. 13, 197-216
- **BREEDVELD.** *Lancet,* 2000, vol. 355 (9205), 735-740
- **GORMAN ; CLARK.** *Semin. Immunol.,* 1990, vol. 2, 457-466
- Current Protocols in Molecular Biology. 1987, vol. 30
- **JOHNSON ; STELL.** *J Clin Microbiol,* 2001, vol. 39, 3712-3717
- **TANG et al.** *Clin. Chem.,* 1997, vol. 43, 2021-2038
- **BERNADAC et al.** *J Bacteriol,* 1998, vol. 180 (18), 4872-4878
- **SORENSEN ; MORTENSEN.** *Journal of Biotechnology,* 2005, vol. 115, 113-128
- **MEYNIAL-SALLES et al.** *Applied and Environmental Microbiology,* 2005, vol. 71, 2140-2144
- **ALPER et al.** *Proc. Natl. Acad. Sci. USA,* 2005, vol. 102, 12678-12683
- Vaccine Design. Plenum, 1995
- **PODDA.** *Vaccine,* 2001, vol. 19, 2673-2680
- **FREY et al.** *Vaccine,* 2003, vol. 21, 4234-4237
- Methods in Molecular Medicine series. Vaccine Adjuvants: Preparation Methods and Research Protocols. vol. 42
- **ALLISON ; BYARS.** *Res Immunol,* 1992, vol. 143, 519-525
- **HARIHARAN et al.** *Cancer Res,* 1995, vol. 55, 3486-3489
- **BARR et al.** *Advanced Drug Delivery Reviews,* 1998, vol. 32, 247-271
- **SJOLANDERET et al.** *Advanced Drug Delivery Reviews,* 1998, vol. 32, 321-338
- **NIIKURA et al.** *Virology,* 2002, vol. 293, 273-280
- **LENZ et al.** *J Immunol,* 2001, vol. 166, 5346-5355
- **PINTO et al.** *J Infect Dis,* 2003, vol. 188, 327-338
- **GERBER et al.** *Virol,* 2001, vol. 75, 4752-4760
- **GLUCK et al.** *Vaccine,* 2002, vol. 20, B10-B16
- **JOHNSON et al.** *Bioorg Med Chem Lett,* 1999, vol. 9, 2273-2278
- **EVANS et al.** *Expert Rev Vaccines,* 2003, vol. 2, 219-229
- **MERALDI et al.** *Vaccine,* 2003, vol. 21, 2485-2491
- **PAJAK et al.** *Vaccine,* 2003, vol. 21, 836-842
- **KANDIMALLA et al.** *Nucleic Acids Research,* 2003, vol. 31, 2393-2400
- **KRIEG.** *Nature Medicine,* 2003, vol. 9, 831-835
- **MCCLUSKIE et al.** *FEMS Immunology and Medical Microbiology,* 2002, vol. 32, 179-185
- **KANDIMALLA et al.** *Biochemical Society Transactions,* 2003, vol. 31, 654-658
- **BLACKWELL et al.** *J Immunol,* 2003, vol. 170, 4061-4068
- **KRIEG.** *Trends Immunol,* 2002, vol. 23, 64-65
- **KANDIMALLA et al.** *BBRC,* 2003, vol. 306, 948-953
- **BHAGAT et al.** *BBRC,* 2003, vol. 300, 853-861
- **BEIGNON et al.** *Infect Immun,* 2002, vol. 70, 3012-3019
- **PIZZA et al.** *Vaccine,* 2001, vol. 19, 2534-2541
- **PIZZA et al.** *Int J Med Microbiol,* 2000, vol. 290, 455-461
- **SCHARTON-KERSTEN et al.** *Infect Immun,* 2000, vol. 68, 5306-5313
- **RYAN et al.** *Infect Immun,* 1999, vol. 67, 6270-6280
- **PARTIDOS et al.** *Immunol Lett,* 1999, vol. 67, 209-216
- **PEPPOLONI et al.** *Expert Rev Vaccines,* 2003, vol. 2, 285-293
- **PINE et al.** *J Control Release,* 2002, vol. 85, 263-270
- **DOMENIGHINI et al.** *Mol Microbiol,* 1995, vol. 15, 1165-1167
- **LILLARD JW et al.** *Blood,* 12 September 2002, vol. 101 (3), 807-814
- **SINGH et al.** *J Cont Release,* 2001, vol. 70, 267-276
- **ANDRIANOV et al.** *Biomaterials,* vol. 19, 109-115

- **PAYNE et al.** *Adv Drug Delivery Review,* vol. 31, 185-196
- **STANLEY.** *Clin Exp Dermatol,* 2002, vol. 27, 571-577
- **WU et al.** *Antiviral Res.,* 2004, vol. 64 (2), 79-83
- **VASILAKOS et al.** *Cell Immunol.,* 2000, vol. 204 (1), 64-74
- **JONES.** *Curr Opin Investig Drugs,* 2003, vol. 4, 214-218
- **WONG et al.** *J Clin Pharmacol,* 2003, vol. 43 (7), 735-42
- **SIGNORELLI ; HADDEN.** *Int Immunopharmacol,* 2003, vol. 3 (8), 1177-1186
- **COOPER.** *Pharm Biotechnol,* 1995, vol. 6, 559-580
- **DONNELLY et al.** *Annu Rev Immunol,* 1997, vol. 15, 617-648
- **STRUGNELL et al.** *Immunol Cell Biol,* 1997, vol. 75 (4), 364-369
- **CUI.** *Adv Genet,* 2005, vol. 54, 257-89
- **ROBINSON ; TORRES.** *Seminars in Immunol,* 1997, vol. 9, 271-283
- **BRUNHAM et al.** *J Infect Dis,* 2000, vol. 181 (3), S538-S543
- **SVANHOLIN et al.** *Scand J Immunol,* 2000, vol. 51 (4), 345-353
- DNA Vaccination - Genetic Vaccination. 1998
- Gene Vaccination: Theory and Practice. 1998
- **FINDEIS et al.** *Trends Biotechnol.,* vol. 11, 202
- Gene Therapeutics: Methods And Applications Of Direct Gene Transfer. 1994
- **WU et al.** *J. Biol. Chem.,* 1988, vol. 263, 621
- **WU et al.** *J. Biol. Chem.,* 1994, vol. 269, 542
- **ZENKE et al.** *Proc. Natl. Acad. Sci. (USA),* 1990, vol. 87, 3655
- **WU et al.** *J. Biol. Chem.,* 1991, vol. 266, 338
- **JOLLY.** *Cancer Gene Therapy,* 1994, vol. 1, 51
- **KIMURA.** *Human Gene Therapy,* 1994, vol. 5, 845
- **CONNELLY.** *Human Gene Therapy,* 1995, vol. 1, 185
- **KAPLITT.** *Nature Genetics,* 1994, vol. 6, 148
- **CURIEL.** *Hum. Gene Ther.,* 1992, vol. 3, 147
- **WU.** *J. Biol. Chem.,* 1989, vol. 264, 16985
- **PHILIP.** *Mol. Cell Biol.,* 1994, vol. 14, 2411
- **WOFFENDIN.** *Proc. Natl. Acad. Sci.,* 1994, vol. 91, 11581
- **GLEZEN ; ALPERS.** *Clin. Infect. Dis.,* 1999, vol. 28, 219-224
- **JOHNSON et al.** *Infect Immun,* 2001, vol. 69, 1306-1314
- **JOHNSON et al.** *J Infect Dis,* 2001, vol. 183, 897-906
- **JOHNSON ; HOPKINS.** *Infection and Immunity,* 1998, vol. 66, 6063-6064
- **BAHRANI-MOUGEOT et al.** *Molecular Microbiology,* 2002, vol. 45 (4), 1079-1093
- **JOHNSON et al.** *Infection and Immunity,* 1998, 3059-3065
- **ALMEIDA ; ALPAR.** *J. Drug Targeting,* 1996, vol. 3, 455-467
- **AGARWAL ; MISHRA.** *Indian J Exp Biol,* 1999, vol. 37, 6-16
- **COSTANTINO et al.** *Vaccine,* 1992, vol. 10, 691-698
- **COSTANTINO et al.** *Vaccine,* 1999, vol. 17, 1251-1263
- **WATSON.** *Pediatr Infect Dis J,* 2000, vol. 19, 331-332
- **RUBIN.** *Pediatr Clin North Am,* 2000, vol. 47, 269-285
- **JEDRZEJAS.** *Microbiol Mol Biol Rev,* 2001, vol. 65, 187-207
- **BELL.** *Pediatr Infect Dis J,* 2000, vol. 19, 1187-1188
- **IWARSON.** *APMIS,* 1995, vol. 103, 321-326
- **GERLICH et al.** *Vaccine,* 1990, vol. 8, 63-6879-80
- **HSU et al.** *Clin Liver Dis,* 1999, vol. 3, 901-915
- **STRATOV et al.** *Curr Drug Tgts,* 2004, vol. 5 (1), 71-88
- Vaccines. W.B. Saunders Company, 2003
- **DEL GUIDICE et al.** *Molecular Aspects of Medicine,* 1998, vol. 19, 1-70
- **GUSTAFSSON et al.** *N. Engl. J. Med.,* 1996, vol. 334, 349-355
- **RAPPUOLI et al.** *TIBTECH,* 1991, vol. 9, 232-238
- **SUTTER et al.** *Pediatr Clin North Am,* 2000, vol. 47, 287-308
- **ZIMMERMAN ; SPANN.** *Am Fam Physician,* 1999, vol. 59, 113-118125-126
- **MCMICHAEL.** *Vaccine,* 2000, vol. 19 (1), S101-S107
- **SCHUCHAT.** *Lancet,* 1999, vol. 353 (9146), 51-56
- **KALMAN et al.** *Nature Genetics,* 1999, vol. 21, 385-389
- **READ et al.** *Nucleic Acids Res,* 2000, vol. 28, 1397-1406
- **SHIRAI et al.** *J. Infect. Dis.,* 2000, vol. 181 (3), S524-S527
- **ROSS et al.** *Vaccine,* 2001, vol. 19, 4135-4142
- **DREESEN.** *Vaccine,* 1997, vol. 15, S2-S6
- *MMWR Morb Mortal Wkly Rep,* 16 January 1998, vol. 47 (1), 12, 19
- **ANDERSON.** *Vaccine,* 2000, vol. 19 (1), S59-S65
- **KAHN.** *Curr Opin Pediatr,* 2000, vol. 12, 257-262
- **CROWE.** *Vaccine,* 1995, vol. 13, 415-421

- **MODLIN et al.** *J Toxicol Clin Toxicol,* 2001, vol. 39, 85-100
- **DEMICHELI et al.** *Vaccine,* 1998, vol. 16, 880-884
- **STEPANOV et al.** *J Biotechnol,* 1996, vol. 44, 155-160
- **DALE.** *Infect Dis Clin North Am,* 1999, vol. 13, 227-43
- **FERRETTI et al.** *PNAS USA,* 2001, vol. 98, 4658-4663
- **KURODA et al.** *Lancet,* 2001, vol. 357 (9264), 1225-1240
- **FALUGI et al.** *Eur J Immunol,* 2001, vol. 31, 3816-3824
- **BARALDO et al.** *Infect Immun.,* 2004, vol. 72, 4884-4887
- **KUO et al.** *Infect Immun,* 1995, vol. 63, 2706-2713
- *Research Disclosure,* January 2002
- **NEEDLEMAN ; WUNSCH.** *J. Mol. Biol.,* 1970, vol. 48, 443-453
- **RICE et al.** *Trends Genet,* 2000, vol. 16, 276-277
- **GENNARO.** Remington: The Science and Practice of Pharmacy. 2000
- Methods In Enzymology. Academic Press, Inc,
- Handbook of Experimental Immunology. Blackwell Scientific Publications, 1986, vol. I-IV
- **SAMBROOK et al.** Molecular Cloning: A Laboratory Manual. Cold Spring Harbor Laboratory Press, 2001
- Handbook of Surface and Colloidal Chemistry. CRC Press, 1997
- Short protocols in molecular biology. Current Protocols, 2002
- Molecular Biology Techniques: An Intensive Laboratory Course. Academic Press, 1998
- Introduction to Biotechniques Series. PCR. Springer Verlag, 1997
- **MURPHY.** *J. Bacteriol,* 1998, vol. 180, 2063-2071